# EUROPEAN PATENT APPLICATION

(11) **EP 4 371 981 A1**
(43) Date of publication of application: **22.05.2024**
(21) Application number: 22841502.2
(22) Date of filing: 15.07.2022
(51) Int. Cl.: C07D 401/12, C07D 401/14, C07D 403/12, C07D 403/14, C07D 413/12, C07D 413/14, C07D 417/12, C07D 417/14, C07D 487/04

(54) **SULFUR/PHOSPHORUS-CONTAINING ARYL COMPOUND AND APPLICATION THEREOF**

(30) Priority: 15.07.2021 CN 202110801875; 30.07.2021 CN 202110875486; 30.09.2021 CN 202111162655; 29.10.2021 CN 202111273381; 13.01.2022 CN 202210039225; 16.03.2022 CN 202210260637; 24.06.2022 CN 202210731484
(71) Applicant: Medshine Discovery Inc., Nanjing, Jiangsu 210032 (CN)
(72) Inventor: WANG, Jianfei, Shanghai 200131 (CN); YANG, Guangwen, Shanghai 200131 (CN); AO, Zhihua, Shanghai 200131 (CN); SUN, Jikui, Shanghai 200131 (CN); ZHANG, Yang, Shanghai 200131 (CN); CHEN, Shuhui, Shanghai 200131 (CN)
(74) Representative: Calysta NV
(86) International application number: PCT/CN2022/106053
(87) International publication number: WO 2023/284869

(57) **Abstract**

Provided are a sulfur/phosphorus-containing aryl compound and an application thereof, and specifically disclosed are a compound represented by formula (V) and a pharmaceutically acceptable salt thereof.

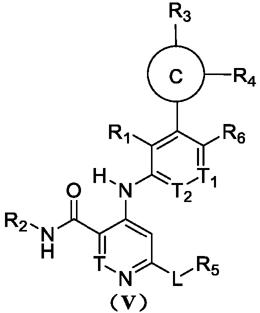

## Description

The present application claims the right of the following priorities:
CN202110801875.1, application date: July 15, 2021;
CN202110875486.3, application date: July 30, 2021;
CN202111162655.5, application date: September 30, 2021;
CN202111273381.7, application date: October 29, 2021;
CN202210039225.2, application date: January 13, 2022;
CN202210260637.9, application date: March 16, 2022;
CN202210731484.1, application date: June 24, 2022.

### TECHNICAL FIELD

The present disclosure relates to a new sulfur/phosphorus-containing aryl compound and a use thereof, and specifically relates to a compound of formula (V) and a pharmaceutically acceptable salt thereof.

### BACKGROUND

JAK is a category of non-receptor tyrosine kinases, comprising four subtypes: JAK1, JAK2, JAK3, and TYK2. The JAK-STAT signaling pathway mediated by them is related to cell proliferation, differentiation, apoptosis, and immune regulation. The JAK-STAT pathway is essential for immune responses, and during inflammation, the overactivation of JAK conversely promotes the progression of the disease. As research into the mechanism of the JAK-STAT pathway in various autoimmune diseases deepens, JAK inhibitors, as the latest type of targeted autoimmune drugs, have gradually received marketing approval for rheumatoid arthritis (RA), psoriatic arthritis (PsA), and atopic dermatitis, with more indications in late-stage clinical development, including ankylosing spondylitis (AS), ulcerative colitis (UC), Crohn's disease, and others. Research has revealed that TYK2 mediates the signaling of IL-6, IL-10, IL-12, IL-23, and type I interferon, encompassing the key cytokines IL-12 and IL-23, which are currently considered critical in the progression of psoriasis. Consequently, TYK2 inhibitors are considered to be important targets for treating a substantial population with another autoimmune disease - psoriasis. However, as of now, no TYK2 inhibitors have been approved.

BMS-986165, a selective allosteric TYK2 inhibitor, is the most advanced candidate compound in this field. Its phase III trials have demonstrated clinical efficacy comparable to first-line biologics, significantly superior to the oral standard therapy apremilast (a PDE4 inhibitor). Moreover, it has a low incidence of adverse reactions, with a discontinuation rate due to adverse events lower than that of both the apremilast group and the placebo group. The mechanism of action of BMS-986165 is unique. Unlike other JAK inhibitors, it targets the JH2 pseudokinase domain of TYK2. However, similar to orthosteric inhibitors, it suppresses the kinase activity of TYK2, thereby exerting its effect. Notably, it achieves high kinase selectivity (>1000-fold). This demonstrates the substantial clinical application potential of high-selectivity TYK2 inhibitors in the treatment of psoriasis and other targets, signifying considerable clinical application value.

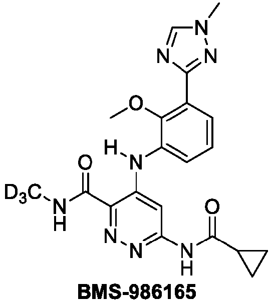

### CONTENT OF THE PRESENT INVENTION

The present disclosure provides a compound of formula (V) or a pharmaceutically acceptable salt thereof, 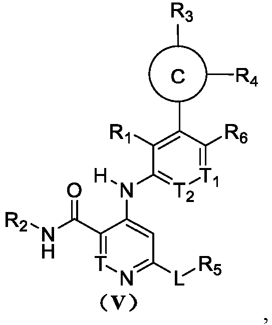 wherein
L is selected from -NH-, -NHC(=O)-, -NHC(=O)O-, and -NHC(=O)NH-;
T, T₁, and T₂ are each independently selected from N and CH, and the CH is optionally substituted by 1 halogen;
R₁ is selected from C₁₋₃ alkoxy, and the C₁₋₃ alkoxy is optionally substituted by 1, 2, or 3 Rₐ;
R₂ is selected from H and C₁₋₃ alkyl, and the C₁₋₃ alkyl is optionally substituted by 1, 2, or 3 R_{b};
ring C is selected from phenyl and 6-membered heteroaryl;
R₃ is selected from -P(=O)(C₁₋₃ alkyl)₂, -P(=O)(C₃₋₅ cycloalkyl)₂, -S(=O)ₙC₁₋₄ alkyl, - S(=O)ₙC₁₋₃ alkylamino, -S(=O)ₙ-4- to 5-membered heterocycloalkyl, -S(=O)ₙNH₂, - S(=O)(=NR)C₁₋₄ alkyl, -S(=O)(=NR)C₁₋₃ alkylamino, -S(=O)(=NR)C₃₋₅ cycloalkyl, 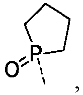 and 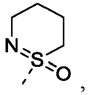 and the -P(=O)(C₁₋₃ alkyl)₂, -P(=O)(C₃₋₅ cycloalkyl)₂, -S(=O)ₙC₁₋₄ alkyl, - S(=O)ₙC₁₋₃ alkylamino, -S(=O)ₙ-4- to 5-membered heterocycloalkyl, -S(=O)ₙNH₂, - S(=O)(=NR)C₁₋₄ alkyl, -S(=O)(=NR)C₁₋₃ alkylamino, -S(=O)(=NR)C₃₋₅ cycloalkyl, 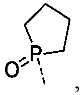 and 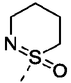 are each independently and optionally substituted by 1, 2, or 3 halogens;
R₅ is selected from C₁₋₃ alkyl, C₃₋₅ cycloalkyl, and 5- to 6-membered heteroaryl, and the C₁₋₃ alkyl, C₃₋₅ cycloalkyl, and 5- to 6-membered heteroaryl are each independently and optionally substituted by 1, 2, or 3 R_{c};
R₄ and R₆ are each independently selected from H, F, Cl, Br, and I;
alternatively, R₃, R₄ together with the carbon atom to which they are attached form 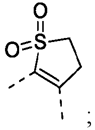
Rₐ and R_{b} are each independently selected from H, D, F, Cl, Br, and I;
R_{c} is selected from F, Cl, Br, I, and C₁₋₃ alkyl;
R is selected from H and C₁₋₃ alkyl;
n is 1 or 2;
provided that
when T is N, R₃ is not -S(=O)ₙC₁₋₄ alkyl;
"hetero" in the 4- to 5-membered heterocycloalkyl and 5- to 6-membered heteroaryl represents 1, 2, or 3 heteroatoms or heteroatom groups independently selected from -O-, -NH-, -S-, and -N-.

The present disclosure provides a compound of formula (V) or a pharmaceutically acceptable salt thereof, 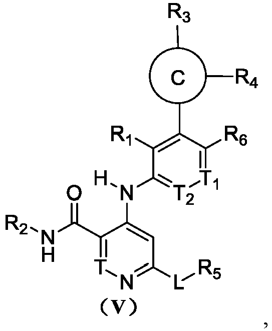 wherein
L is selected from -NH-, -NHC(=O)-, -NHC(=O)O-, and -NHC(=O)NH-;
T, T₁, and T₂ are each independently selected from N and CH, and the CH is optionally substituted by 1 halogen;
R₁ is selected from C₁₋₃ alkoxy, and the C₁₋₃ alkoxy is optionally substituted by 1, 2, or 3 Rₐ;
R₂ is selected from H and C₁₋₃ alkyl, and the C₁₋₃ alkyl is optionally substituted by 1, 2, or 3 R_{b};
ring C is selected from 5- to 6-membered heteroaryl;
R₃ is selected from -P(=O)(C₁₋₃ alkyl)₂, -P(=O)(C₃₋₅ cycloalkyl)₂, -S(=O)ₙC₁₋₄ alkyl, - S(=O)ₙC₁₋₃ alkylamino, -S(=O)ₙ-4- to 5-membered heterocycloalkyl, -S(=O)ₙNH₂, - S(=O)(=NR)C₁₋₄ alkyl, -S(=O)(=NR)C₁₋₃ alkylamino, -S(=O)(=NR)C₃₋₅ cycloalkyl, 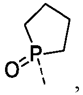 and 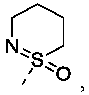 and the -P(=O)(C₁₋₃ alkyl)₂, -P(=O)(C₃₋₅ cycloalkyl)₂, -S(=O)ₙC₁₋₄ alkyl, - S(=O)ₙC₁₋₃ alkylamino, -S(=O)ₙ-4- to 5-membered heterocycloalkyl, -S(=O)ₙNH₂, - S(=O)(=NR)C₁₋₄ alkyl, -S(=O)(=NR)C₁₋₃ alkylamino, -S(=O)(=NR)C₃₋₅ cycloalkyl, 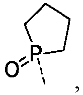 and 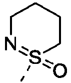 are each independently and optionally substituted by 1, 2, or 3 halogens;
R₅ is selected from C₁₋₃ alkyl, C₃₋₅ cycloalkyl, and 5- to 6-membered heteroaryl, and the C₁₋₃ alkyl, C₃₋₅ cycloalkyl, and 5- to 6-membered heteroaryl are each independently and optionally substituted by 1, 2, or 3 R_{c};
R₄ and R₆ are each independently selected from H, F, Cl, Br, and I;
alternatively, R₃, R₄ together with the carbon atom to which they are attached form 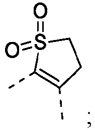
Rₐ and R_{b} are each independently selected from H, D, F, Cl, Br, and I;
R_{c} is selected from F, Cl, Br, I, and C₁₋₃ alkyl;
R is selected from H and C₁₋₃ alkyl;
n is 1 or 2;
provided that
when T is N, R₃ is not -S(=O)ₙC₁₋₄ alkyl;
"hetero" in the 4- to 5-membered heterocycloalkyl and 5- to 6-membered heteroaryl represents 1, 2, or 3 heteroatoms or heteroatom groups independently selected from -O-, -NH-, -S-, and -N-.

In some embodiments of the present disclosure, the L is selected from -NH- and - NHC(=O)-, and other variables are as defined in the present disclosure. In some embodiments of the present disclosure, the R₁ is selected from OCH₃, and the OCH₃ is optionally substituted by 1, 2, or 3 Rₐ, and other variables are as defined in the present disclosure.

In some embodiments of the present disclosure, the R₁ is selected from OCH₃ and OCF₃, and other variables are as defined in the present disclosure.

In some embodiments of the present disclosure, the R₁ is selected from OCH₃, and other variables are as defined in the present disclosure.

In some embodiments of the present disclosure, the R₂ is selected from H and CH₃, and the CH₃ is optionally substituted by 1, 2, or 3 R_{b}, and other variables are as defined in the present disclosure.

In some embodiments of the present disclosure, the R₂ is selected from H, CH₃, and CD₃, and other variables are as defined in the present disclosure.

In some embodiments of the present disclosure, the R₃ is selected from 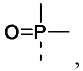 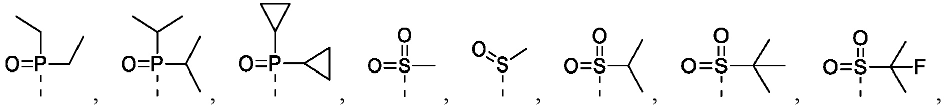 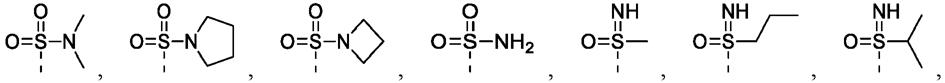 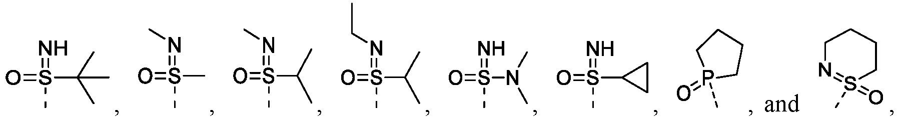 and other variables are as defined in the present disclosure.

In some embodiments of the present disclosure, the ring C is selected from phenyl, pyridyl, pyrimidinyl, pyridazinyl, and pyrazinyl, and other variables are as defined in the present disclosure.

In some embodiments of the present disclosure, the structural moiety 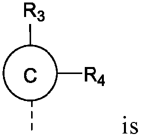 selected from 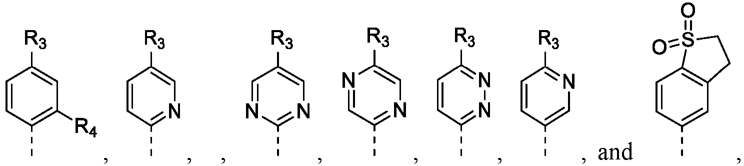 and other variables are as defined in the present disclosure.

In some embodiments of the present disclosure, the structural moiety 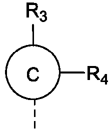 is selected from 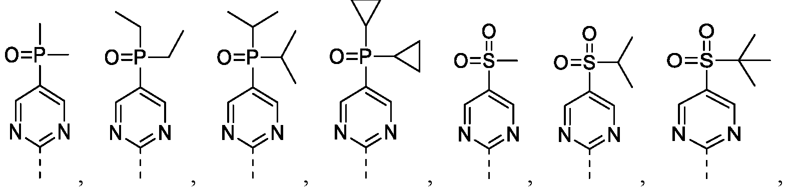 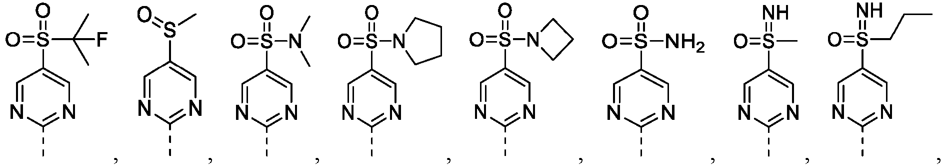 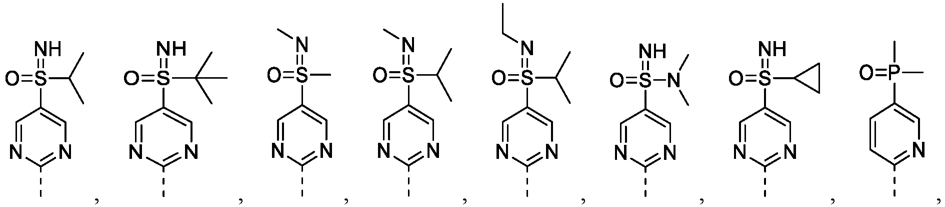 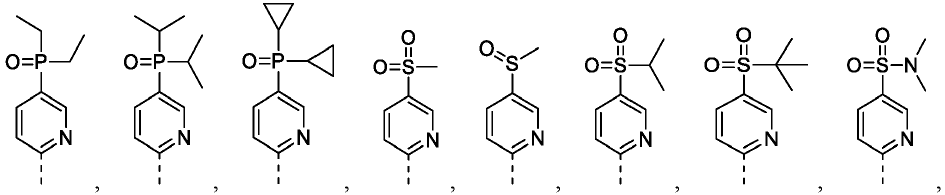 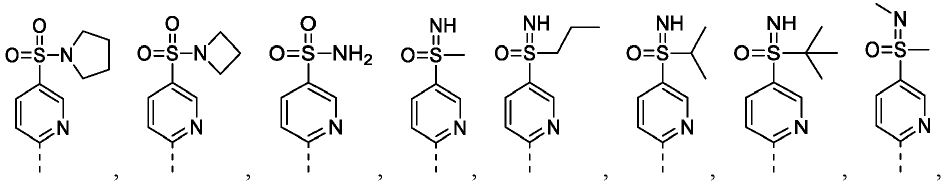 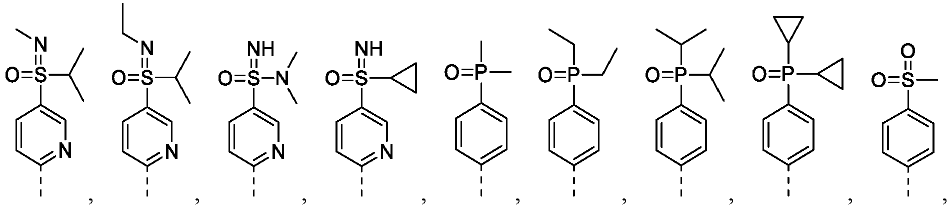 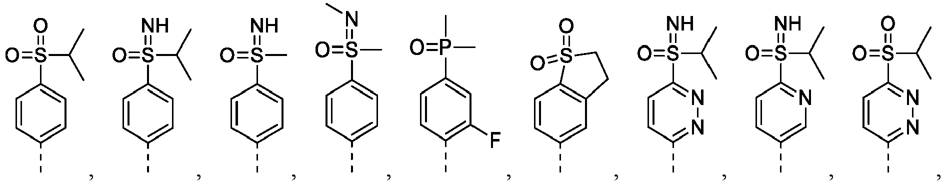 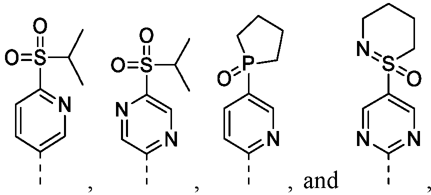 and other variables are as defined in the present disclosure.

In some embodiments of the present disclosure, the R_{c} is selected from F and CH₃, and other variables are as defined in the present disclosure.

In some embodiments of the present disclosure, the R₅ is selected from CH₃, cyclopropyl, imidazolyl, pyrazolyl, and pyridyl, and the CH₃, cyclopropyl, imidazolyl, pyrazolyl, and pyridyl are each independently and optionally substituted by 1, 2, or 3 R_{c}, and other variables are as defined in the present disclosure.

In some embodiments of the present disclosure, the R₅ is selected from CH₃, 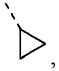 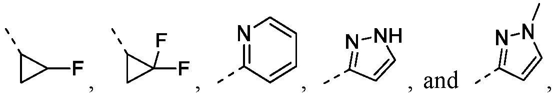 and other variables are as defined in the present disclosure.

In some embodiments of the present disclosure, the structural moiety 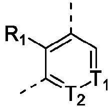 is selected from 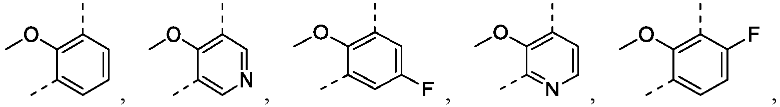 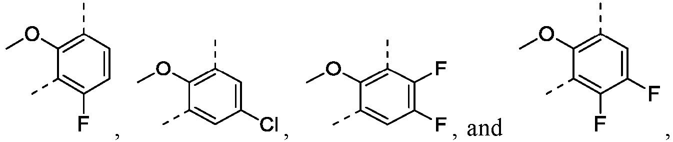 and other variables are as defined in the present disclosure.

In some embodiments of the present disclosure, the compound or the pharmaceutically acceptable salt thereof is provided, and the compound is selected from: 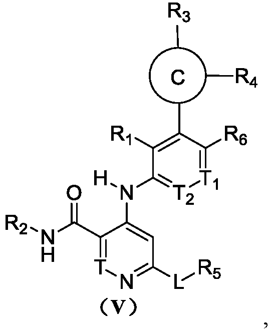 wherein
L is selected from -NH-, -NHC(=O)-, -NHC(=O)O-, and -NHC(=O)NH-;
T, T₁, and T₂ are each independently selected from N and CH, and the CH is optionally substituted by 1 halogen;
R₁ is selected from C₁₋₃ alkoxy, and the C₁₋₃ alkoxy is optionally substituted by 1, 2, or 3 Rₐ;
R₂ is selected from H and C₁₋₃ alkyl, and the C₁₋₃ alkyl is optionally substituted by 1, 2, or 3 R_{b};
ring C is selected from phenyl, pyridyl, pyrimidinyl, pyridazinyl, and pyrazinyl;
R₃ is selected from -P(=O)(C₁₋₃ alkyl)₂, -P(=O)(C₃₋₅ cycloalkyl)₂, -S(=O)ₙC₁₋₃ alkyl, - S(=O)ₙC₁₋₃ alkylamino, -S(=O)ₙ-4- to 5-membered heterocycloalkyl, -S(=O)ₙNH₂, - S(=O)(=NR)C₁₋₃ alkyl, -S(=O)(=NR)C₁₋₃ alkylamino, -S(=O)(=NR)C₃₋₅ cycloalkyl, 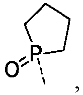 and 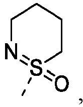 and the -P(=O)(C₁₋₃ alkyl)₂, -P(=O)(C₃₋₅ cycloalkyl)₂, -S(=O)ₙC₁₋₃ alkyl, - S(=O)ₙC₁₋₃ alkylamino, -S(=O)ₙ-4- to 5-membered heterocycloalkyl, -S(=O)ₙNH₂, - S(=O)(=NR)C₁₋₃ alkyl, -S(=O)(=NR)C₁₋₃ alkylamino, -S(=O)(=NR)C₃₋₅ cycloalkyl, 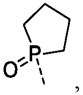 and 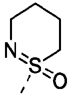 are each independently and optionally substituted by 1, 2, or 3 halogens;
R₅ is selected from C₁₋₃ alkyl, C₃₋₅ cycloalkyl, and 5- to 6-membered heteroaryl, and the C₁₋₃ alkyl, C₃₋₅ cycloalkyl, and 5- to 6-membered heteroaryl are each independently and optionally substituted by 1, 2, or 3 R_{c};
R₄ and R₆ are each independently selected from H, F, Cl, and Br;
alternatively, R₃, R₄ together with the carbon atom to which they are attached form 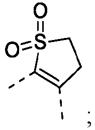
Rₐ and R_{b} are each independently selected from H, D, F, Cl, Br, and I;
R_{c} is selected from F, Cl, Br, I, and C₁₋₃ alkyl;
R is selected from H and C₁₋₃ alkyl;
n is 1 or 2;
"hetero" in the 4- to 5-membered heterocycloalkyl and 5- to 6-membered heteroaryl represents 1, 2, or 3 heteroatoms or heteroatom groups independently selected from -O-, -NH-, -S-, and -N-.

In some embodiments of the present disclosure, the R₁ is selected from OCH₃, and the OCH₃ is optionally substituted by 1, 2, or 3 Rₐ, and other variables are as defined in the present disclosure.

In some embodiments of the present disclosure, the R₁ is selected from OCH₃ and OCF₃, and other variables are as defined in the present disclosure.

In some embodiments of the present disclosure, the R₂ is selected from H and CH₃, and the CH₃ is optionally substituted by 1, 2, or 3 R_{b}, and other variables are as defined in the present disclosure.

In some embodiments of the present disclosure, the R₂ is selected from H, CH₃, and CD₃, and other variables are as defined in the present disclosure.

In some embodiments of the present disclosure, the R₃ is selected from 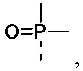 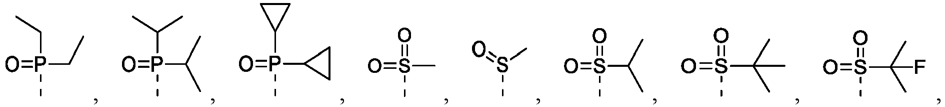 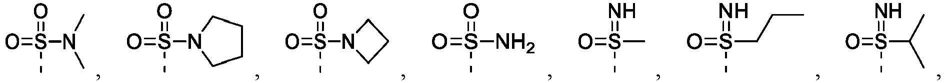 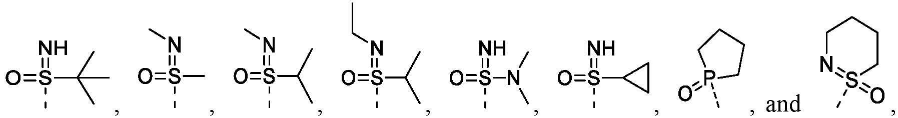 and other variables are as defined in the present disclosure.

In some embodiments of the present disclosure, the R₃ is selected from 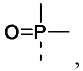 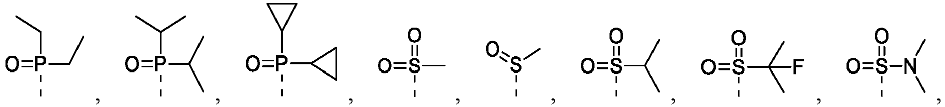 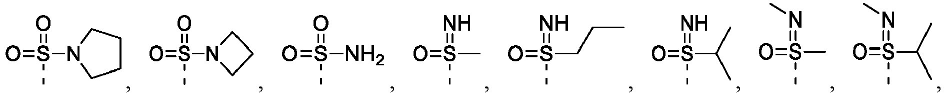 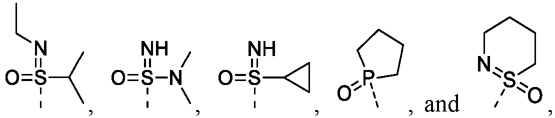 and other variables are as defined in the present disclosure.

In some embodiments of the present disclosure, the structural moiety 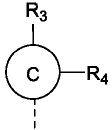 is selected from 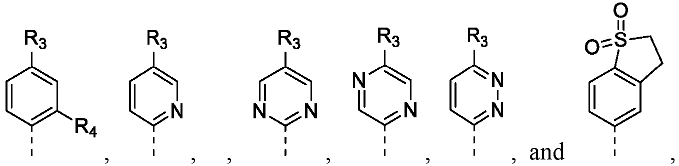 and other variables are as defined in the present disclosure.

In some embodiments of the present disclosure, the structural moiety 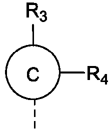 is selected from 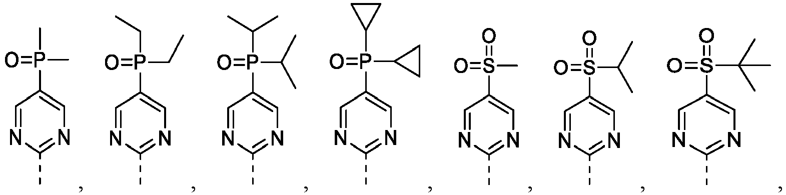 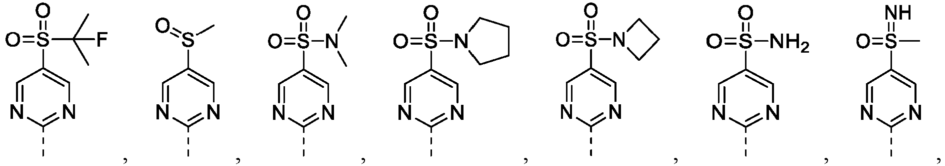 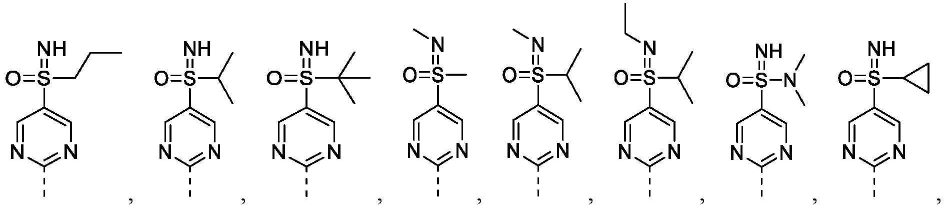 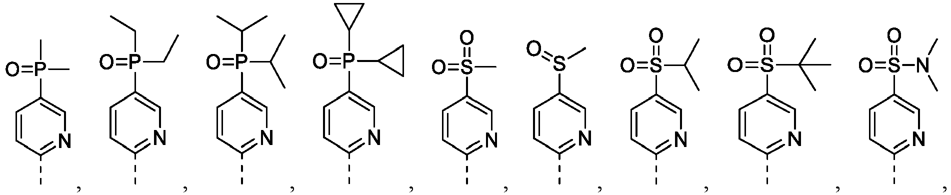 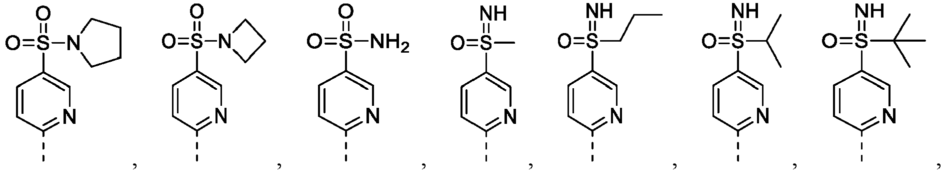 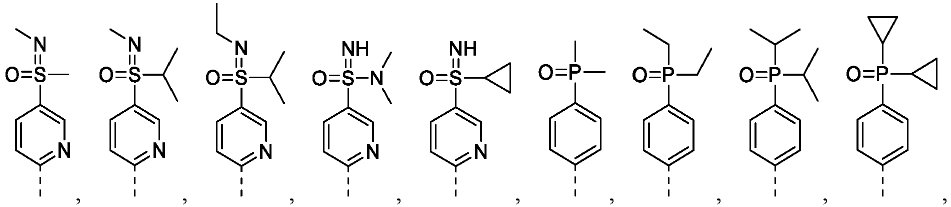 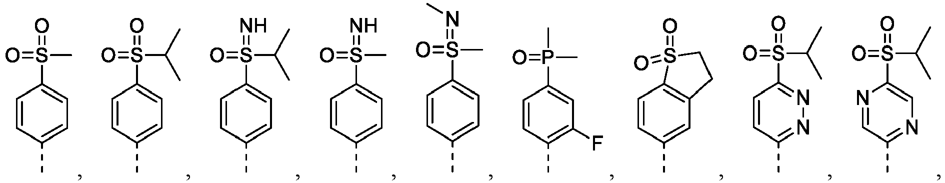 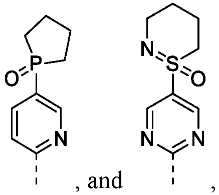 and other variables are as defined in the present disclosure.

In some embodiments of the present disclosure, the structural moiety 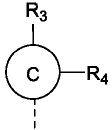 is selected from 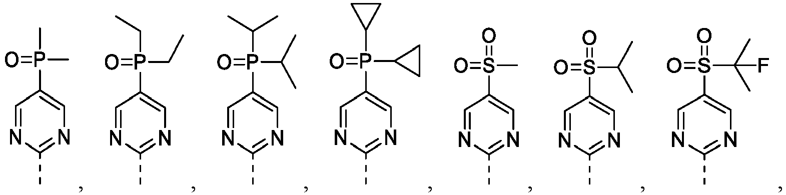 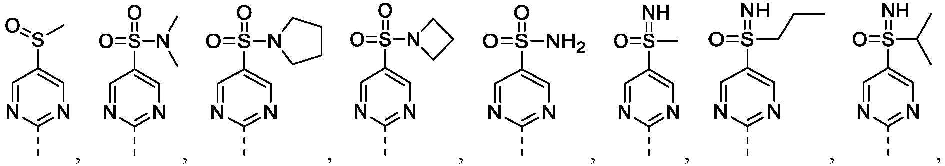 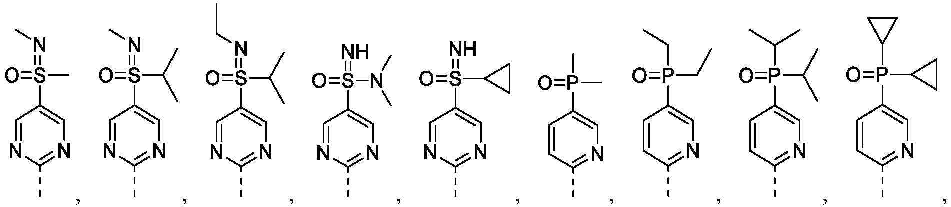 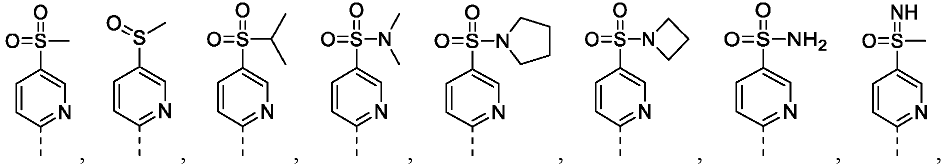 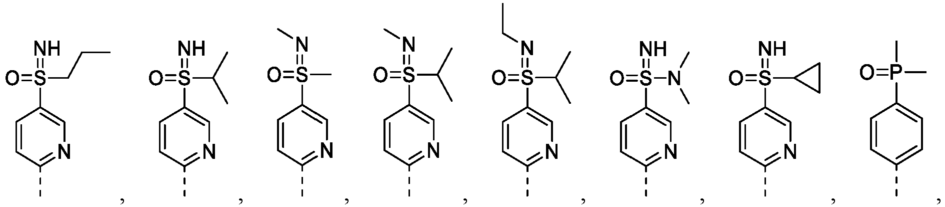 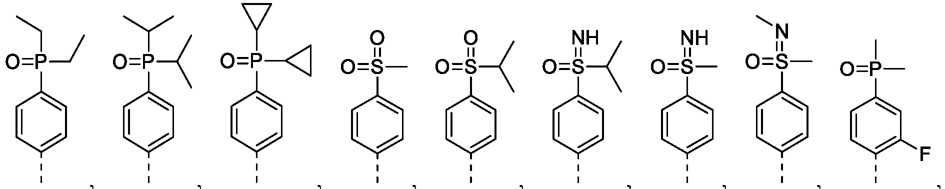 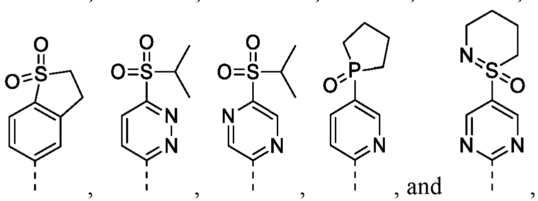 and other variables are as defined in the present disclosure.

In some embodiments of the present disclosure, the R₅ is selected from CH₃, cyclopropyl, imidazolyl, pyrazolyl, and pyridyl, and the CH₃, cyclopropyl, imidazolyl, pyrazolyl, and pyridyl are each independently and optionally substituted by 1, 2, or 3 R_{c}, and other variables are as defined in the present disclosure.

In some embodiments of the present disclosure, the R₅ is selected from CH₃, 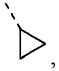 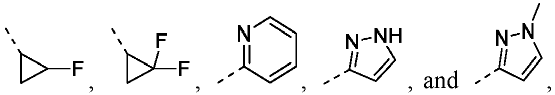 and other variables are as defined in the present disclosure.

In some embodiments of the present disclosure, the structural moiety 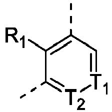 is selected from 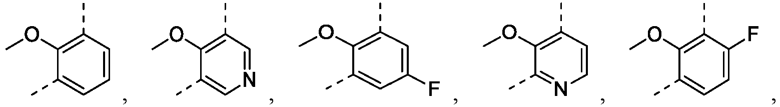 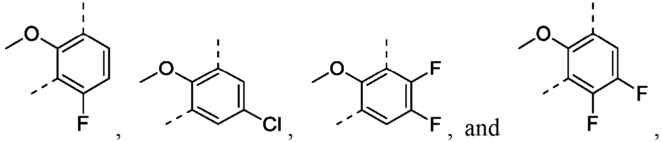 and other variables are as defined in the present disclosure.

In some embodiments of the present disclosure, the compound or the pharmaceutically acceptable salt thereof is provided, and the compound is selected from 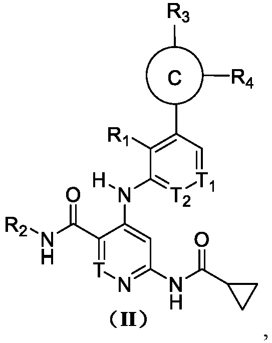 wherein
T, T₁, and T₂ are each independently selected from N and CH, and the CH is optionally substituted by 1 halogen;
R₁ is selected from C₁₋₃ alkoxy, and the C₁₋₃ alkoxy is optionally substituted by 1, 2, or 3 Rₐ;
R₂ is selected from H and C₁₋₃ alkyl, and the C₁₋₃ alkyl is optionally substituted by 1, 2, or 3 R_{b};
ring C is selected from phenyl, pyridyl, and pyrimidinyl;
R₃ is selected from -P(=O)(C₁₋₃ alkyl)₂, -P(=O)(C₃₋₅ cycloalkyl)₂, -S(=O)ₙC₁₋₃ alkyl, - S(=O)ₙC₁₋₃ alkylamino, -S(=O)ₙ-4- to 5-membered heterocyclyl, -S(=O)ₙNH₂, - S(=O)(=NR)C₁₋₃ alkyl, -S(=O)(=NR)C₁₋₃ alkylamino, -S(=O)(=NR)C₃₋₅ cycloalkyl, 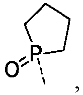 and 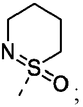
R₄ is selected from H, F, Cl, and Br;
alternatively, R₃, R₄ together with the carbon atom to which they are attached form 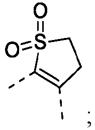
Rₐ and R_{b} are each independently selected from H, D, F, Cl, Br, and I;
R is selected from H and C₁₋₃ alkyl;
n is 1 or 2;
"hetero" in the 4- to 5-membered heterocyclyl represents 1, 2, or 3 heteroatoms or heteroatom groups independently selected from -O-, -NH-, -S-, and -N-.

In some embodiments of the present disclosure, the compound or the pharmaceutically acceptable salt thereof is provided, and the compound is selected from 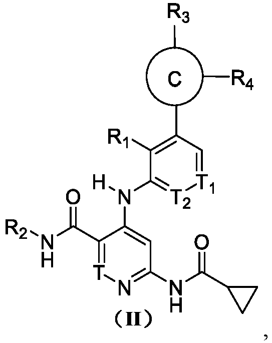 wherein
T, T₁, and T₂ are each independently selected from N and CH, and the CH is optionally substituted by 1 halogen;
R₁ is selected from C₁₋₃ alkoxy, and the C₁₋₃ alkoxy is optionally substituted by 1, 2, or 3 Rₐ;
R₂ is selected from C₁₋₃ alkyl, and the C₁₋₃ alkyl is optionally substituted by 1, 2, or 3 R_{b};
ring C is selected from phenyl, pyridyl, and pyrimidinyl;
R₃ is selected from -P(=O)(C₁₋₃ alkyl)₂, -P(=O)(C₃₋₅ cycloalkyl)₂, -S(=O)ₙC₁₋₃ alkyl, - S(=O)ₙC₁₋₃ alkylamino, -S(=O)ₙ-4- to 5-membered heterocyclyl, -S(=O)ₙNH₂, - S(=O)(=NR)C₁₋₃ alkyl, -S(=O)(=NR)C₁₋₃ alkylamino, and -S(=O)(=NR)C₃₋₅ cycloalkyl;
R₄ is selected from H, F, Cl, and Br;
alternatively, R₃, R₄ together with the carbon atom to which they are attached form 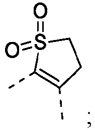
Rₐ and R_{b} are each independently selected from H, D, F, Cl, Br, and I;
R is selected from H and C₁₋₃ alkyl;
n is 1 or 2.

In some embodiments of the present disclosure, the R₁ is selected from OCH₃, and the OCH₃ is optionally substituted by 1, 2, or 3 Rₐ, and other variables are as defined in the present disclosure.

In some embodiments of the present disclosure, the R₁ is selected from OCH₃ and OCF₃, and other variables are as defined in the present disclosure.

In some embodiments of the present disclosure, the R₂ is selected from H and CH₃, and the CH₃ is optionally substituted by 1, 2, or 3 R_{b}, and other variables are as defined in the present disclosure.

In some embodiments of the present disclosure, the R₂ is selected from CH₃, and the CH₃ is optionally substituted by 1, 2, or 3 R_{b}, and other variables are as defined in the present disclosure.

In some embodiments of the present disclosure, the R₂ is selected from H, CH₃, and CD₃, and other variables are as defined in the present disclosure.

In some embodiments of the present disclosure, the R₂ is selected from CH₃ and CD₃, and other variables are as defined in the present disclosure.

In some embodiments of the present disclosure, the R₃ is independently selected from 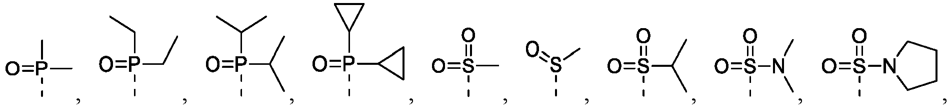 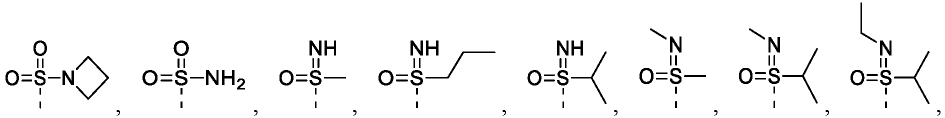 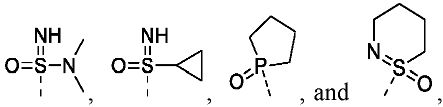 and other variables are as defined in the present disclosure.

In some embodiments of the present disclosure, the R₃ is independently selected from 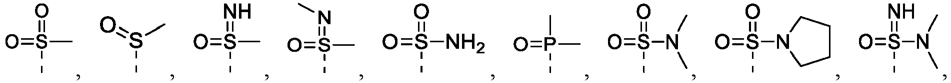 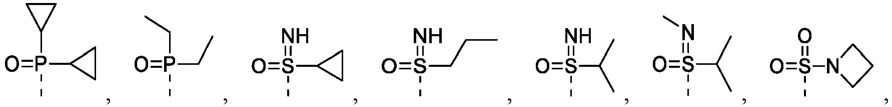 and 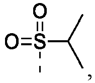 and other variables are as defined in the present disclosure.

In some embodiments of the present disclosure, the structural moiety 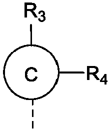 is selected from 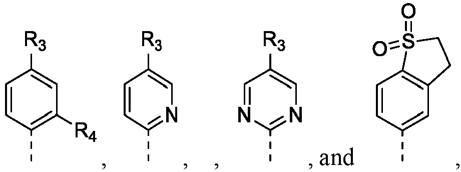 and other variables are as defined in the present disclosure.

In some embodiments of the present disclosure, the structural moiety 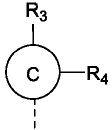 is selected from 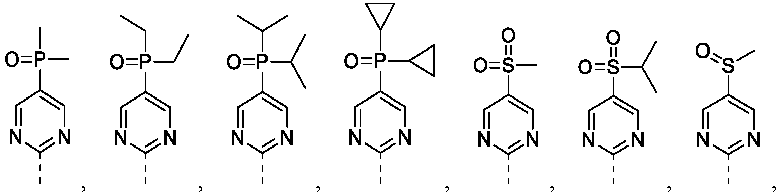 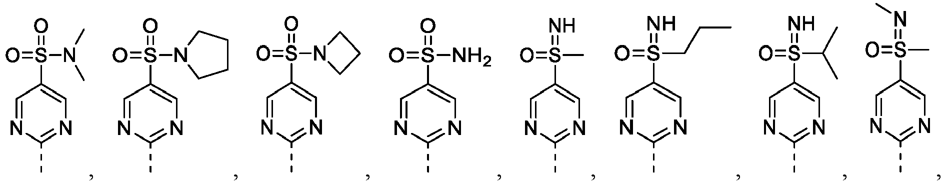 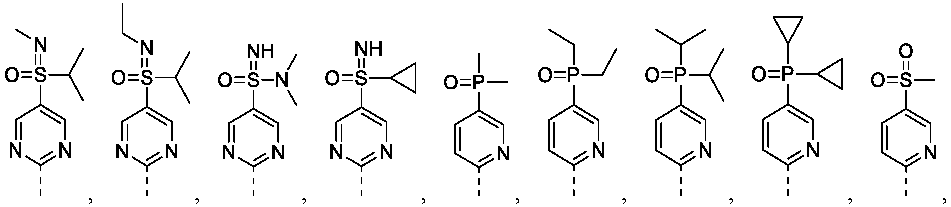 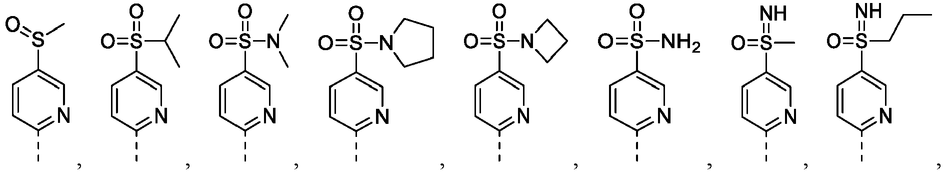 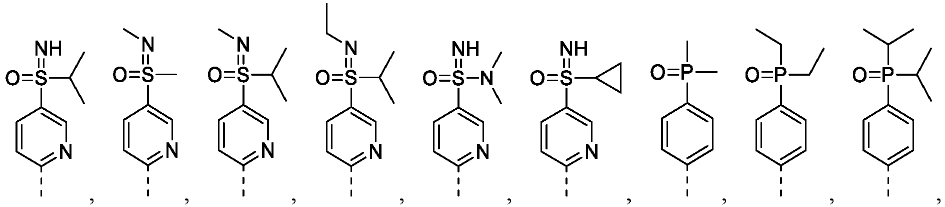 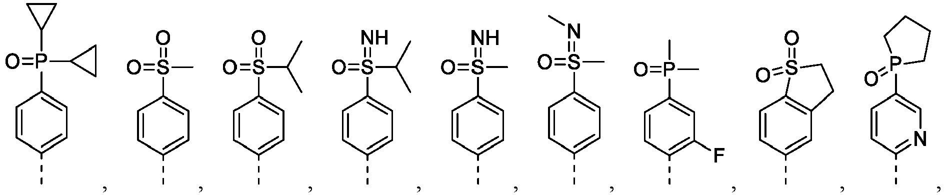 and 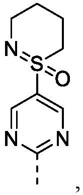 and other variables are as defined in the present disclosure.

In some embodiments of the present disclosure, the structural moiety 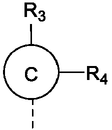 is selected from 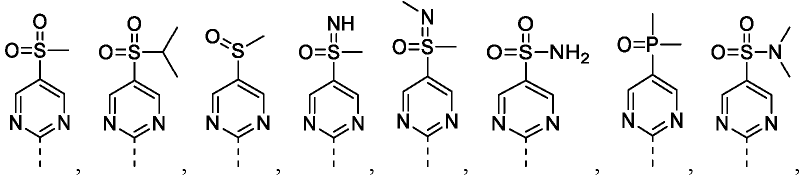 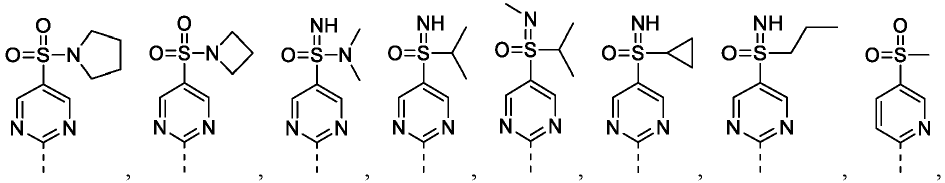 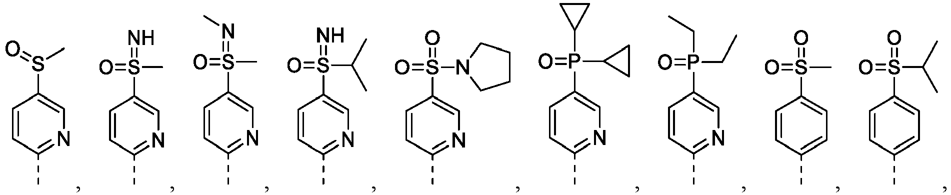 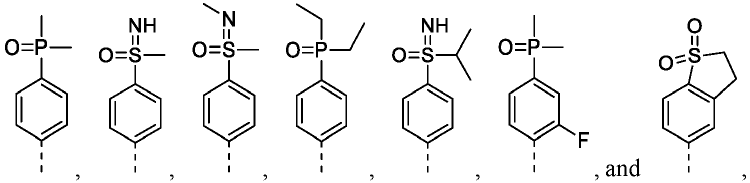 and other variables are as defined in the present disclosure.

In some embodiments of the present disclosure, the structural moiety 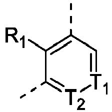 is selected from 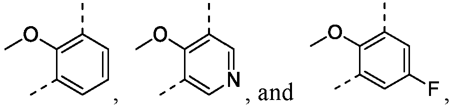 and other variables are as defined in the present disclosure.

The present disclosure provides a compound of formula (I) or a pharmaceutically acceptable salt thereof, 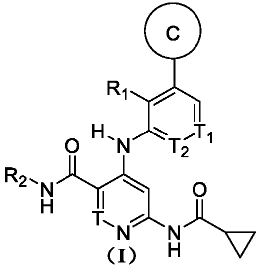 wherein
T, T₁, and T₂ are each independently selected from N and CH, and the CH is optionally substituted by 1 halogen;
R₁ is selected from C₁₋₃ alkoxy, and the C₁₋₃ alkoxy is optionally substituted by 1, 2, or 3 Rₐ;
R₂ is selected from C₁₋₃ alkyl, and the C₁₋₃ alkyl is optionally substituted by 1, 2, or 3 R_{b};
ring C is selected from phenyl, pyridine, pyrimidine, and 2,3-dihydrobenzo[b]thiophene 1,1-dioxide, and the phenyl, pyridine, pyrimidine, and 2,3-dihydrobenzo[b]thiophene 1,1-dioxide are each independently and optionally substituted by 1, 2, or 3 R_{c};
Rₐ and R_{b} are each independently selected from H, D, F, Cl, Br, and I;
each R_{c} is independently selected from -P(=O)(CH₃)₂, -P(=O)(C₃₋₅ cycloalkyl)₂, - S(=O)ₙC₁₋₃ alkyl, -S(=O)ₙC₁₋₃ alkylamino, -S(=O)ₙ-5-membered heterocyclyl, -S(=O)ₙNH₂, and -S(=O)(=NR)C₁₋₃ alkyl;
R is selected from H and C₁₋₃ alkyl;
n is 1 or 2.

In some embodiments of the present disclosure, the R₁ is selected from OCH₃, and the OCH₃ is optionally substituted by 1, 2, or 3 Rₐ, and other variables are as defined in the present disclosure.

In some embodiments of the present disclosure, the R₁ is selected from OCH₃ and OCF₃, and other variables are as defined in the present disclosure.

In some embodiments of the present disclosure, the R₂ is selected from CH₃, and the CH₃ is optionally substituted by 1, 2, or 3 R_{b}, and other variables are as defined in the present disclosure.

In some embodiments of the present disclosure, the R₂ is selected from CH₃ and CD₃, and other variables are as defined in the present disclosure.

In some embodiments of the present disclosure, the ring C is selected from 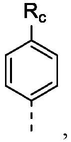 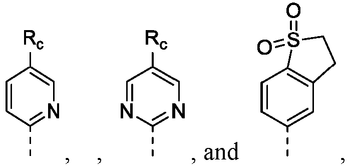 and other variables are as defined in the present disclosure.

In some embodiments of the present disclosure, the ring C is selected from 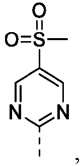 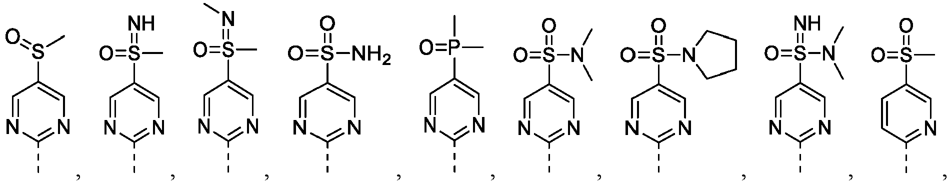 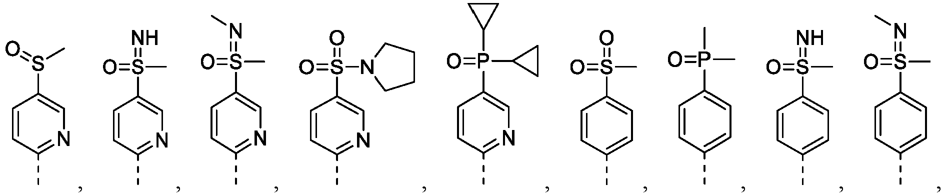 and 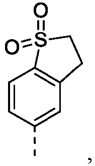 and other variables are as defined in the present disclosure.

In some embodiments of the present disclosure, the structural moiety 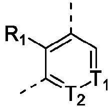 is selected from 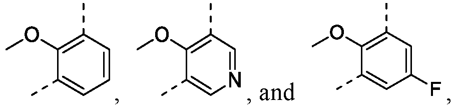 and other variables are as defined in the present disclosure.

There are still some embodiments of the present disclosure which are obtained by any combination of the above variables.

In some embodiments of the present disclosure, the compound or the pharmaceutically acceptable salt thereof is selected from: 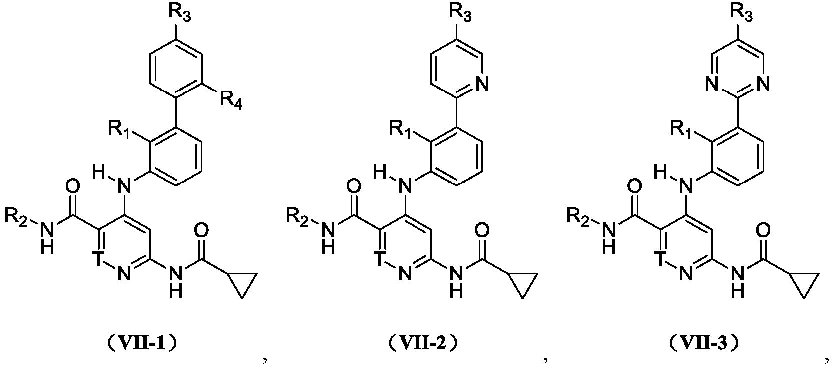 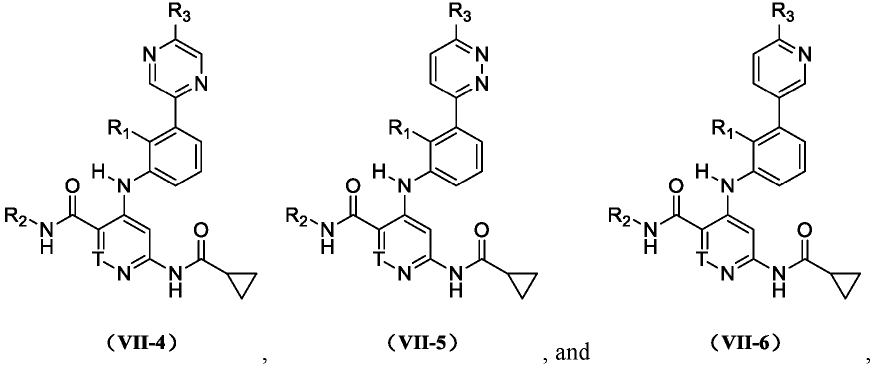 wherein
R₁, R₂, R₃, R₄ and T are as defined in the present disclosure.

The present disclosure provides a compound of the following formula or a pharmaceutically acceptable salt thereof,

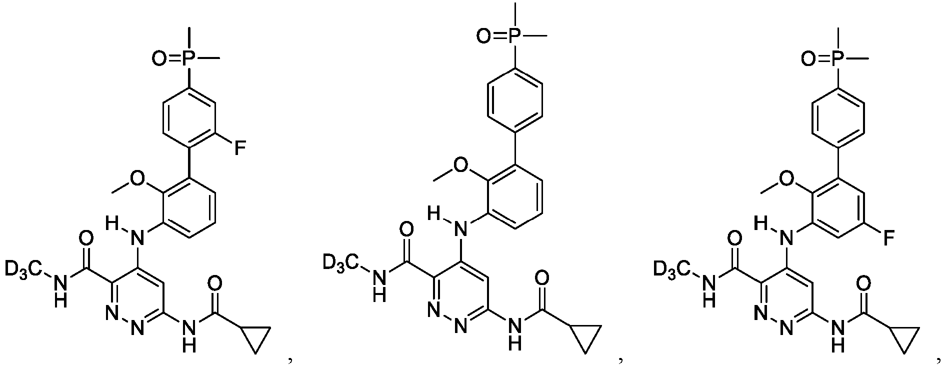

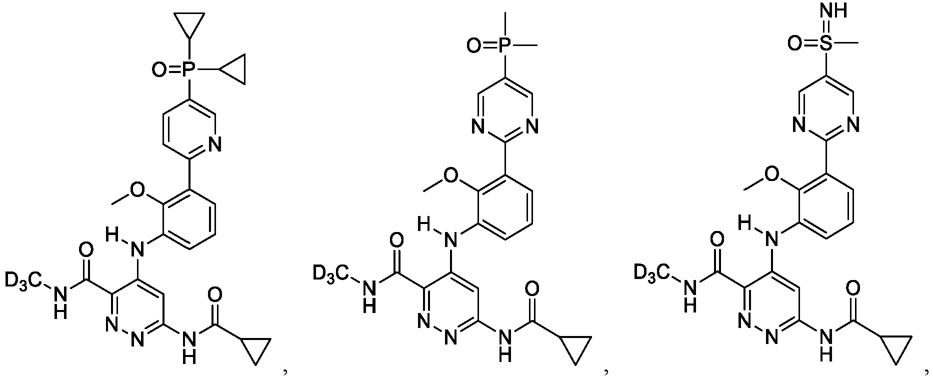

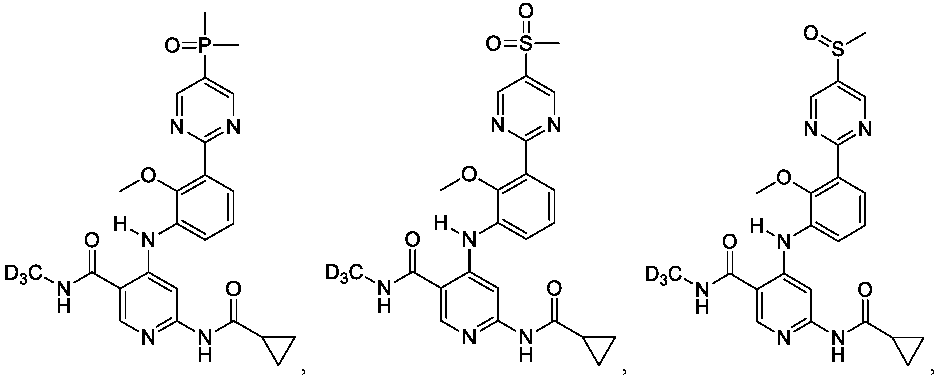

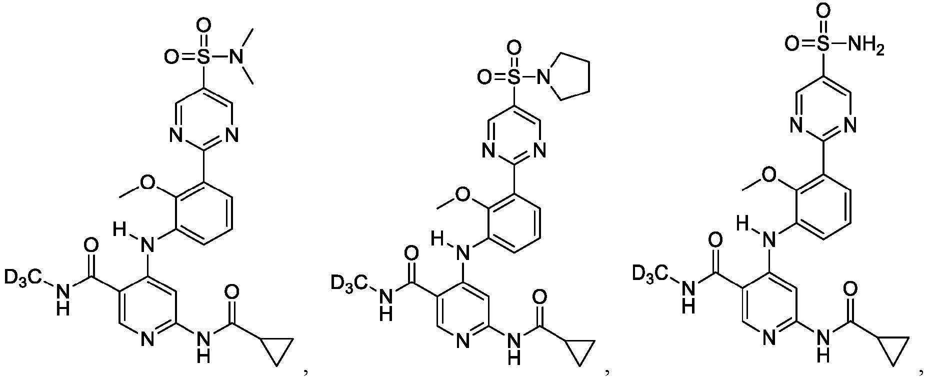

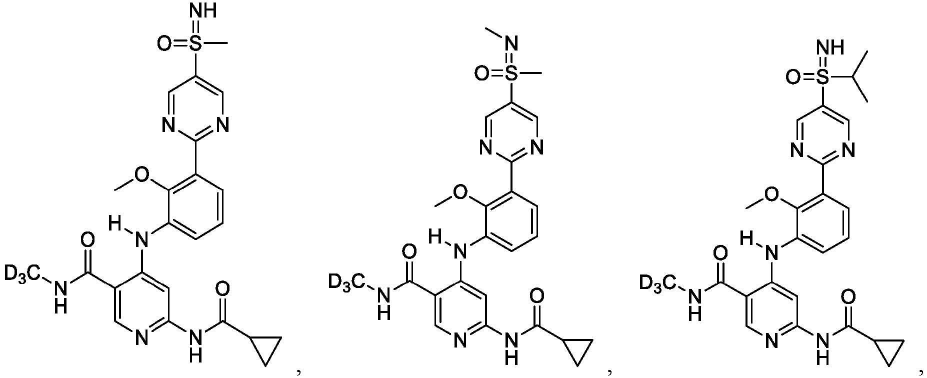

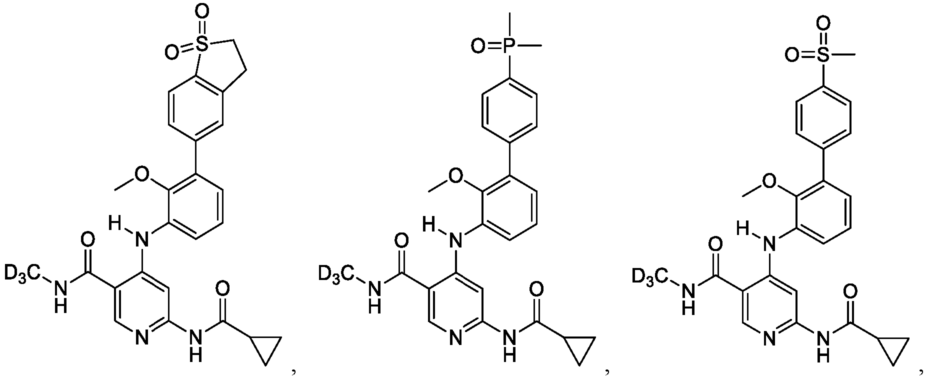

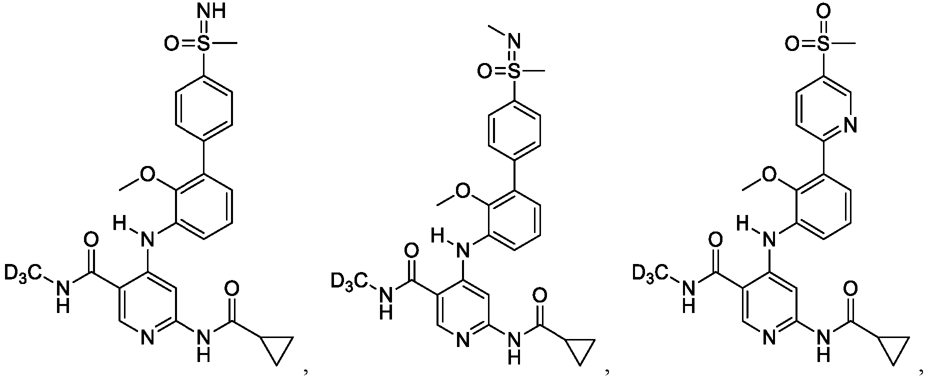

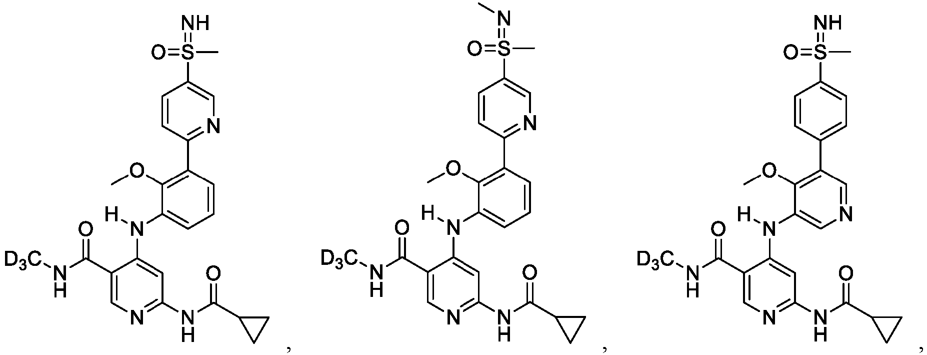

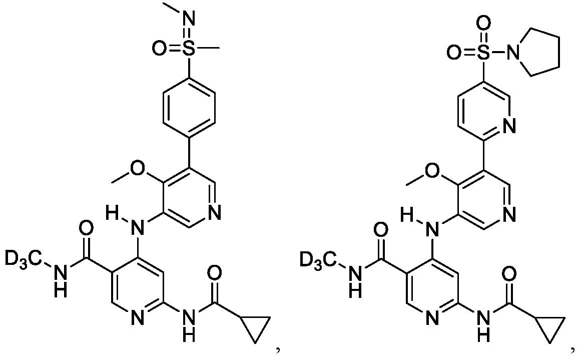

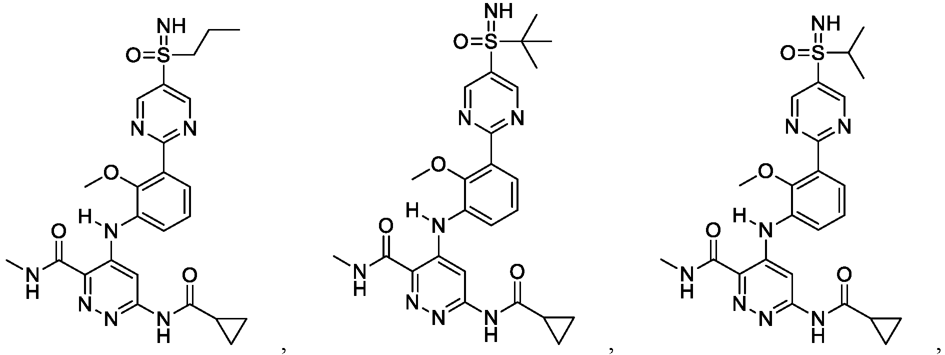

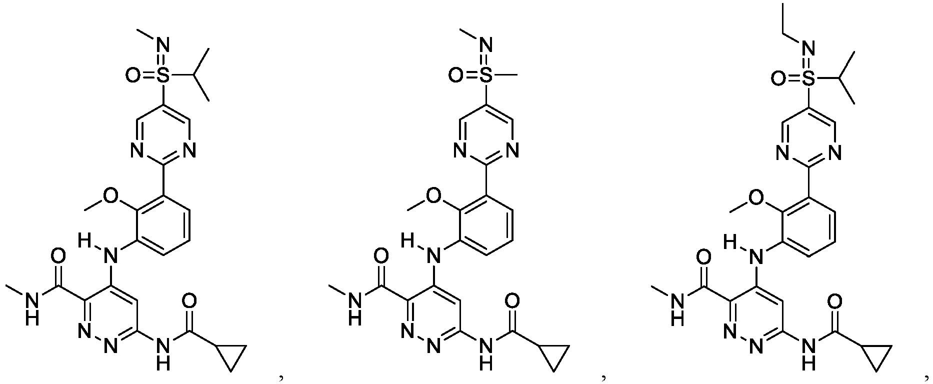

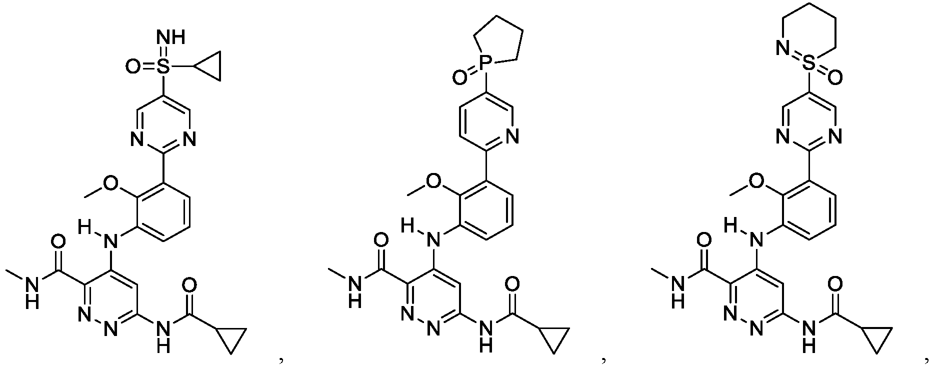

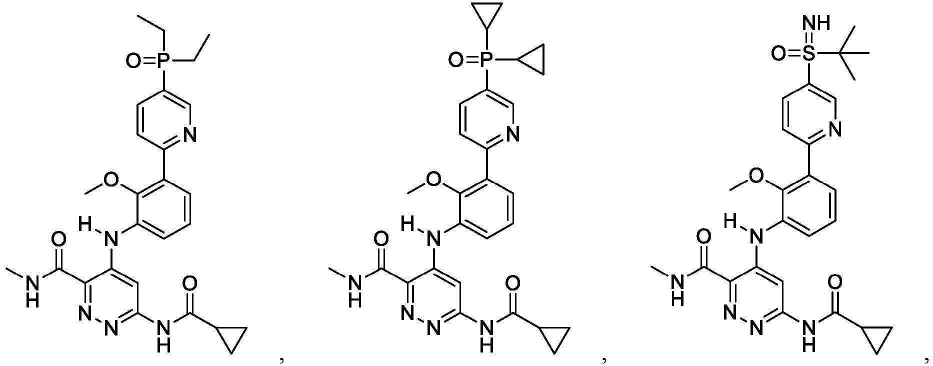

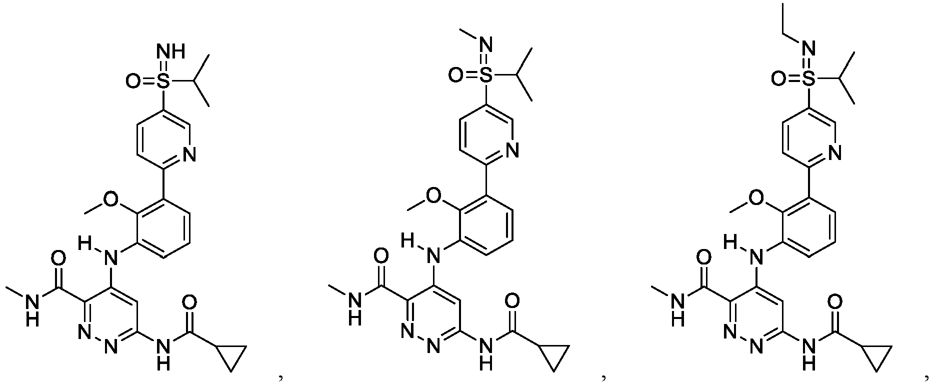

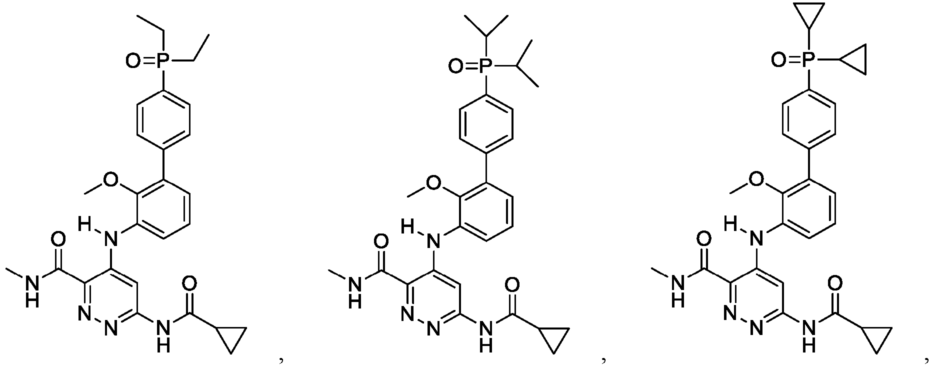

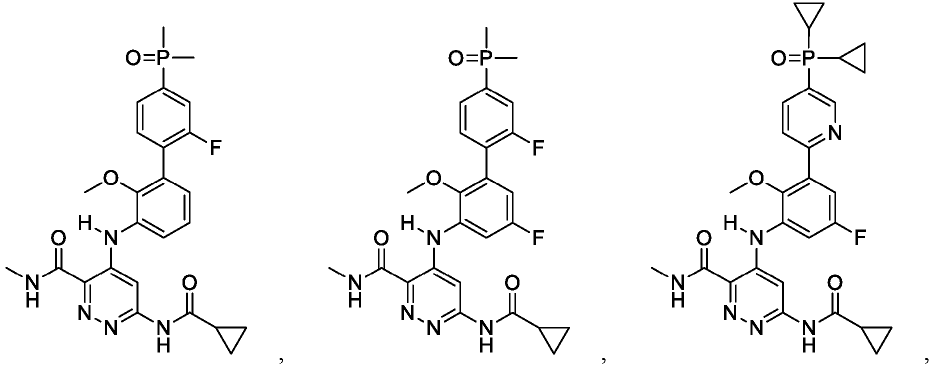

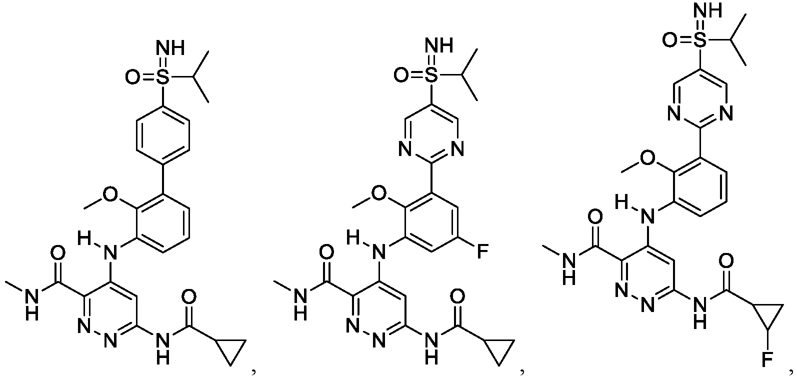

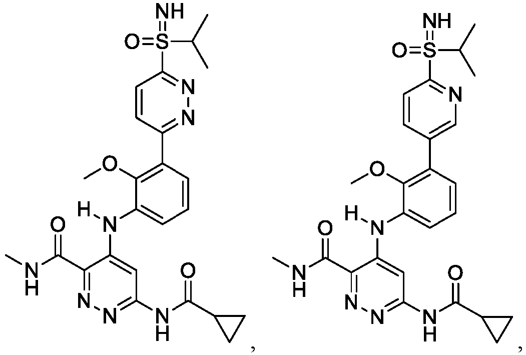

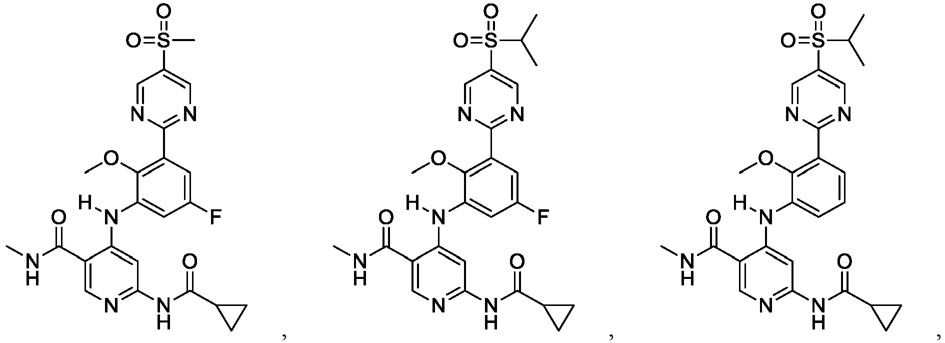

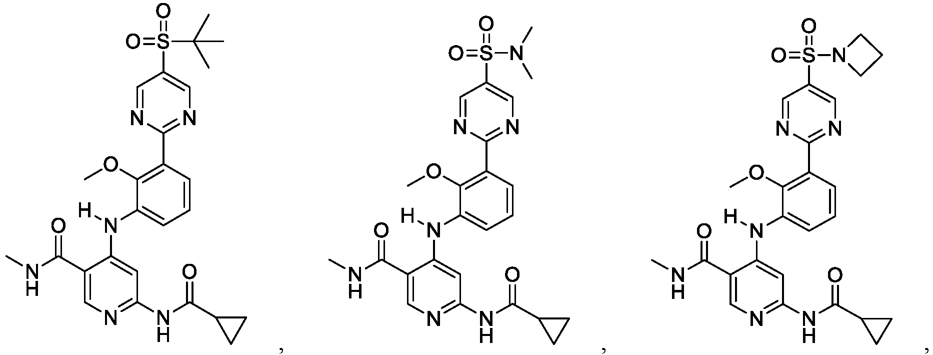

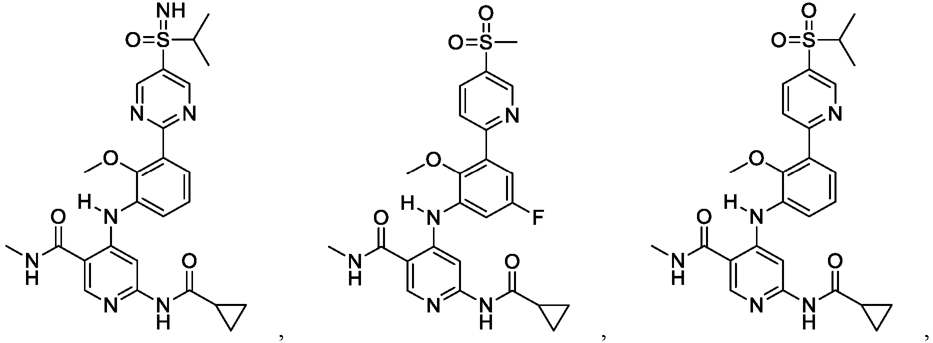

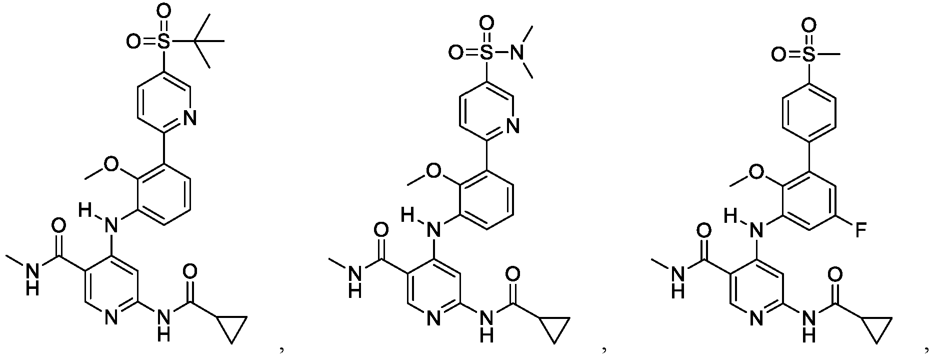

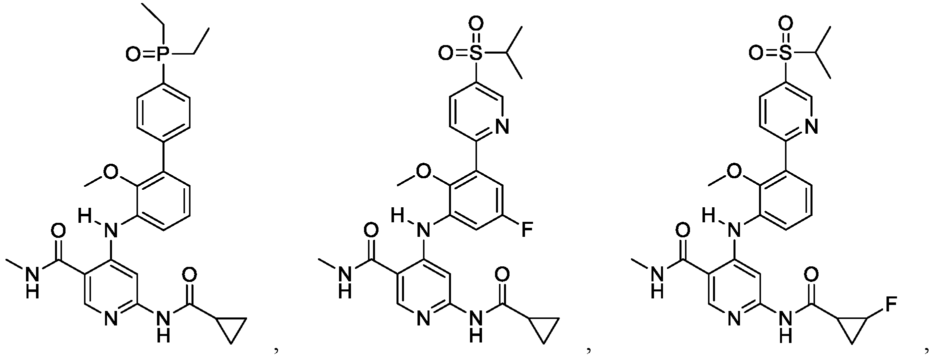

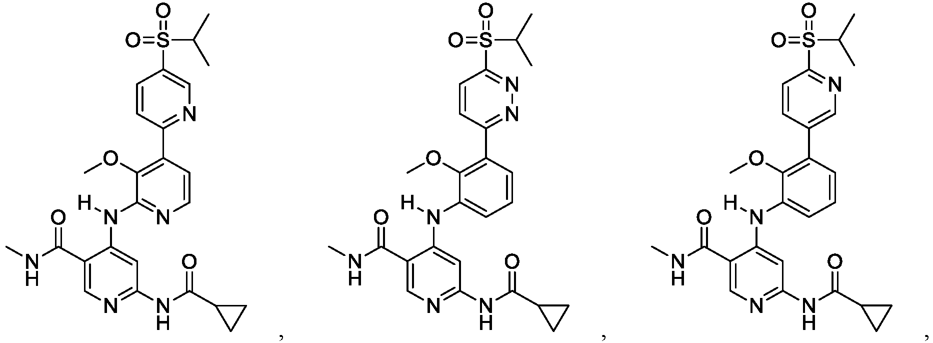

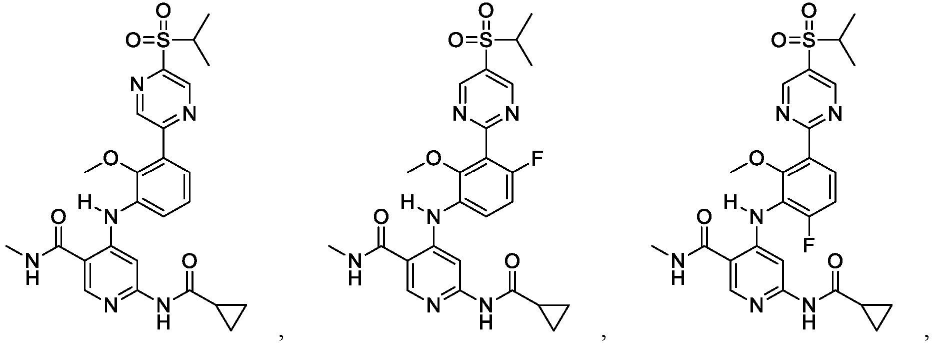

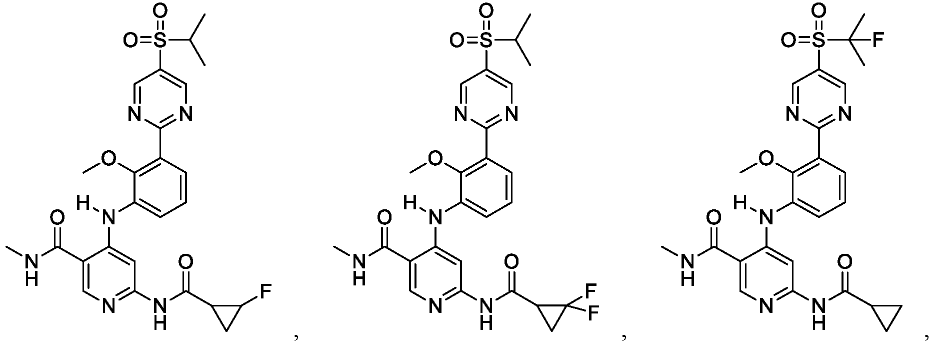

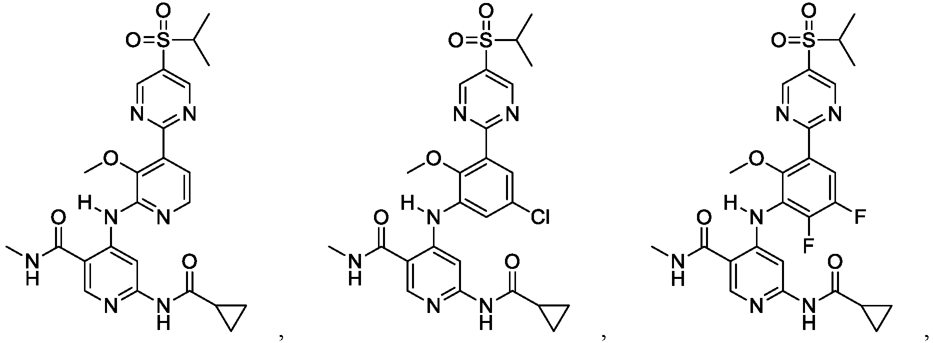

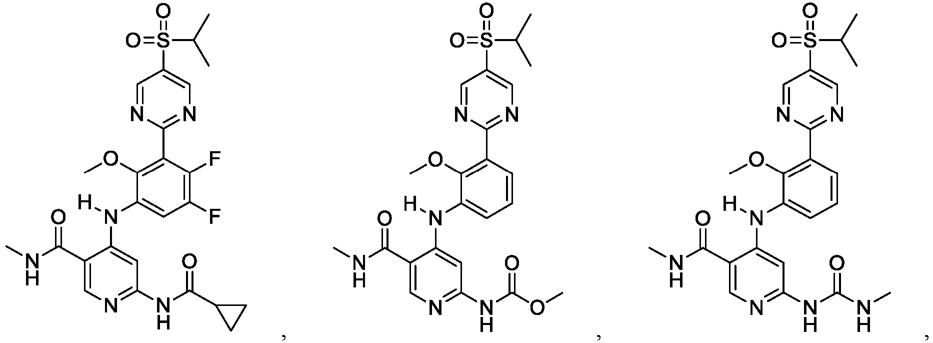

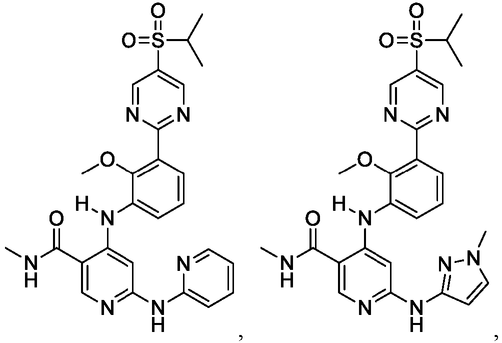

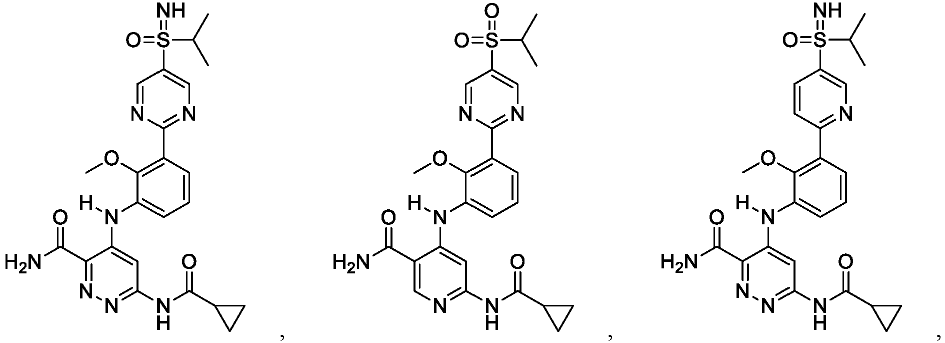

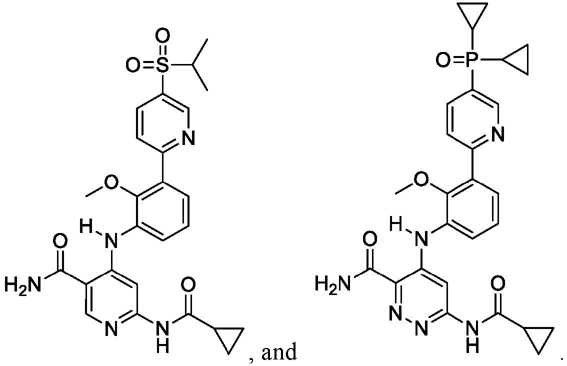

In some embodiments of the present disclosure, the compound or the pharmaceutically acceptable salt thereof is selected from:

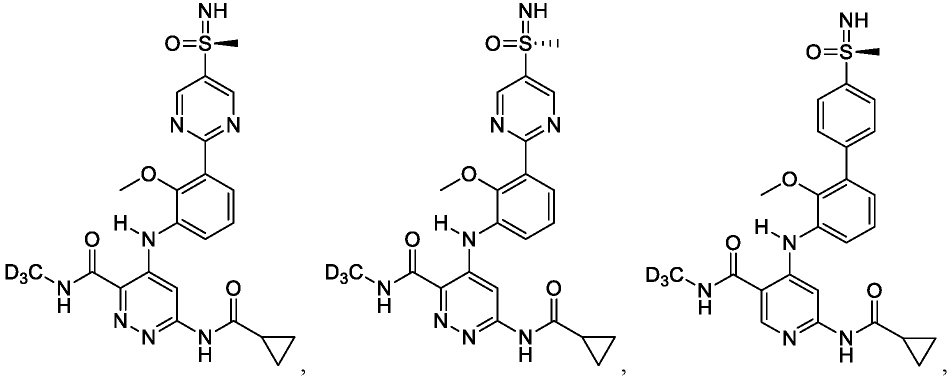

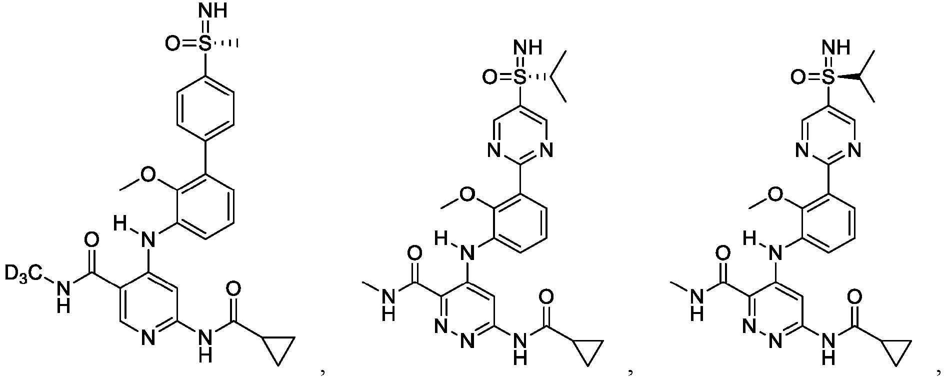

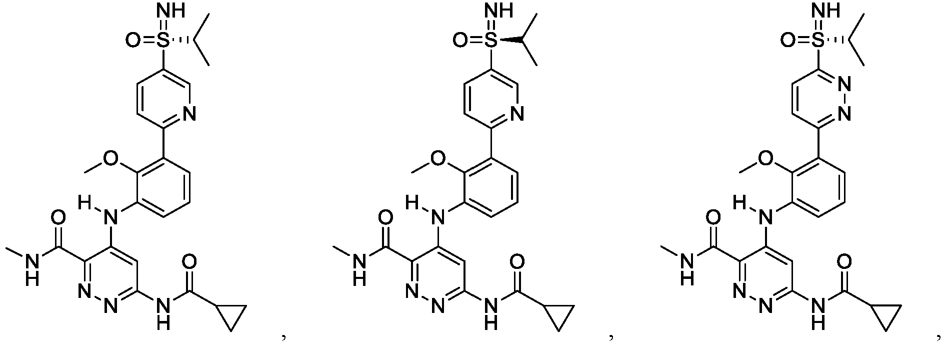

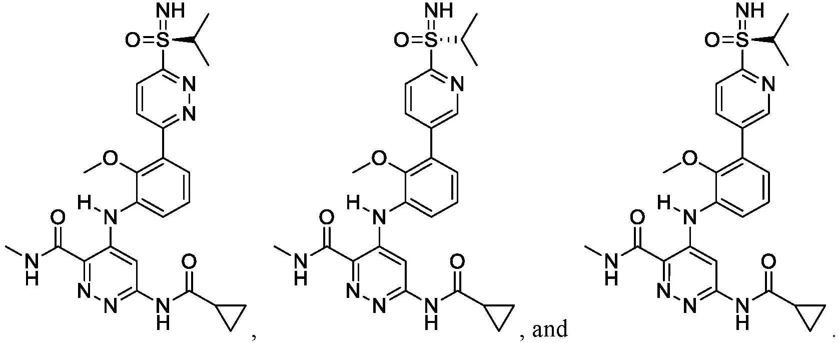

### Technical effect

The compound of the present disclosure acts on the JH2 pseudokinase domain of TYK2 and exhibits a good inhibitory effect on TYK2 kinase. The compound of the present disclosure shows high inhibitory activity against cell proliferation in the Ba/F3-FL-TYK2-E957D cell line, which has a point mutation in the TYK2 gene. In human PBMC cells, the compound of the present disclosure demonstrates strong inhibitory activity against the TYK2 signaling pathway activated by IFN-α stimulation, and also exhibits weak inhibitory activity against the JAK1/2 signaling pathway activated by IL-6 stimulation, the JAK2/2 signaling pathway activated by GM-CSF stimulation, and the JAK1/3 signaling pathway activated by IL-2 stimulation, thereby showing high selectivity. The compound of the present disclosure possesses excellent pharmacokinetic properties. The compound of the present disclosure has a significant dose-dependent inhibitory effect on the release of IFNγ in mice induced by IL-12/IL-18. Additionally, the compound of the present disclosure has a significant alleviating effect on colitis and psoriasis.

### Definition and description

Unless otherwise specified, the following terms and phrases when used herein have the following meanings. A specific term or phrase should not be considered indefinite or unclear in the absence of a particular definition, but should be understood in the ordinary sense. When a trading name appears herein, it is intended to refer to its corresponding commodity or active ingredient thereof.

The term "pharmaceutically acceptable" is used herein in terms of those compounds, materials, compositions, and/or dosage forms, which are suitable for use in contact with human and animal tissues within the scope of reliable medical judgment, with no excessive toxicity, irritation, an allergic reaction, or other problems or complications, commensurate with a reasonable benefit/risk ratio.

The term "pharmaceutically acceptable salt" refers to a salt of the compound of the present disclosure that is prepared by reacting the compound having a specific substituent of the present disclosure with a relatively non-toxic acid or base. When the compound of the present disclosure contains a relatively acidic functional group, a base addition salt can be obtained by contacting the compound with a sufficient amount of base in a pure solution or a suitable inert solvent. When the compound of the present disclosure contains a relatively basic functional group, an acid addition salt can be obtained by contacting the compound with a sufficient amount of acid in a pure solution or a suitable inert solvent. Certain specific compounds of the present disclosure contain both basic and acidic functional groups, thus can be converted to any base or acid addition salt.

The pharmaceutically acceptable salt of the present disclosure can be prepared from the parent compound that contains an acidic or basic moiety by conventional chemical method. Generally, such salt can be prepared by reacting the free acid or base form of the compound with a stoichiometric amount of an appropriate base or acid in water or an organic solvent or a mixture thereof.

Unless otherwise specified, the term "isomer" is intended to include a geometric isomer, a *cis-trans* isomer, a stereoisomer, an enantiomer, an optical isomer, a diastereoisomer, and a tautomeric isomer.

The compounds of the present disclosure may exist in specific geometric or stereoisomeric forms. The present disclosure contemplates all such compounds, including *cis* and *trans* isomers, (-)- and (+)-enantiomers, (*R*)- and (*S*)-enantiomers, diastereoisomers, (*D*)-isomers, (*L*)-isomers, racemic mixtures, and other mixtures thereof, such as enantiomers or diastereomer enriched mixtures, all of which are within the scope of the present disclosure. Additional asymmetric carbon atoms may be present in substituents such as alkyl. All these isomers and their mixtures are included within the scope of the present disclosure.

Unless otherwise specified, the term "enantiomer" or "optical isomer" refers to stereoisomers that are mirror images of each other.

Unless otherwise specified, the term *"cis-trans* isomer" or "geometric isomer" is caused by the inability to rotate freely of double bonds or single bonds of ring-forming carbon atoms.

Unless otherwise specified, the term "diastereomer" refers to a stereoisomer in which a molecule has two or more chiral centers and the relationship between the molecules is not mirror images.

Unless otherwise specified, "(+)" refers to dextrorotation, "(-)" refers to levorotation, and "(±)" refers to racemic.

Unless otherwise specified, the absolute configuration of a stereogenic center is represented by a wedged solid bond ( 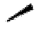 ) and a wedged dashed bond ( 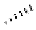 ), and the relative configuration of a stereogenic center is represented by a straight solid bond ( 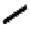 ) and a straight dashed bond ( 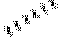 ), a wave line ( 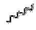 ) is used to represent a wedged solid bond ( 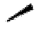 ) or a wedged dashed bond ( 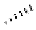 ), or the wave line ( 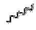 ) is used to represent a straight solid bond ( 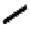 )or a straight dashed bond ( 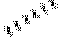 ).

Unless otherwise specified, the terms "enriched in one isomer", "enriched in isomers", "enriched in one enantiomer", or "enriched in enantiomers" refer to the content of one of the isomers or enantiomers is less than 100%, and the content of the isomer or enantiomer is greater than or equal to 60%, or greater than or equal to 70%, or greater than or equal to 80%, or greater than or equal to 90%, or greater than or equal to 95%, or greater than or equal to 96%, or greater than or equal to 97%, or greater than or equal to 98%, or greater than or equal to 99%, or greater than or equal to 99.5%, or greater than or equal to 99.6%, or greater than or equal to 99.7%, or greater than or equal to 99.8%, or greater than or equal to 99.9%.

Unless otherwise specified, the term "isomer excess" or "enantiomeric excess" refers to the difference between the relative percentages of two isomers or two enantiomers. For example, if the content of one isomer or enantiomer is 90%, and the content of the other isomer or enantiomer is 10%, the isomer or enantiomer excess (ee value) is 80%.

Optically active (*R*)- and (*S*)-isomers, or *D* and *L* isomers can be prepared using chiral synthesis, chiral reagents, or other conventional techniques. If one kind of enantiomer of certain compound of the present disclosure is to be obtained, it can be obtained by asymmetric synthesis or derivative action of chiral auxiliary, wherein the resulting diastereomeric mixture is separated and the auxiliary group is cleaved to provide the pure desired enantiomer. Alternatively, when the molecule contains a basic functional group (such as amino) or an acidic functional group (such as carboxyl), a salt of a diastereoisomer is formed with an appropriate optically active acid or base, and then diastereomeric resolution is performed by conventional methods known in the art, and then the pure enantiomer is recovered. In addition, the enantiomer and the diastereoisomer are generally separated through chromatography which uses a chiral stationary phase and optionally combines with a chemical derivative method (such as carbamate generated from amine).

The compound of the present disclosure may contain an unnatural proportion of atomic isotopes at one or more than one atom that constitutes the compound. For example, the compound can be radiolabeled with a radioactive isotope, such as tritium (³H), iodine-125 (¹²⁵I), or C-14 (¹⁴C). For another example, deuterated drugs can be formed by replacing hydrogen with deuterium, the bond formed by deuterium and carbon is stronger than that of ordinary hydrogen and carbon, compared with non-deuterated drugs, deuterated drugs have the advantages of reduced toxic and side effects, increased drug stability, enhanced efficacy, extended biological half-life of drugs, *etc.* All isotopic variations of the compound of the present disclosure, whether radioactive or not, are encompassed within the scope of the present disclosure.

The term "optional" or "optionally" means that the subsequent event or condition may occur but not requisite, that the term includes the instance in which the event or condition occurs and the instance in which the event or condition does not occur.

The term "substituted" means one or more than one hydrogen atom on a specific atom is substituted by the substituent, including deuterium and hydrogen variables, as long as the valence of the specific atom is normal and the substituted compound is stable. When the substituent is an oxygen (*i.e*., =O), it means two hydrogen atoms are substituted. Positions on an aromatic ring cannot be substituted with a ketone.

The term "optionally substituted" means an atom can be substituted by a substituent or not, unless otherwise specified, the type and number of the substituent may be arbitrary as long as being chemically achievable.

When any variable (such as R) occurs in the constitution or structure of the compound more than once, the definition of the variable at each occurrence is independent. Thus, for example, if a group is substituted by 0 to 2 R, the group can be optionally substituted by up to two R, wherein the definition of R at each occurrence is independent. Moreover, a combination of the substituent and/or the variant thereof is allowed only when the combination results in a stable compound.

When the number of a linking group is 0, such as -(CRR)₀-, it means that the linking group is a single bond.

When a substituent is 0 in number, it means that the substituent is absent. In the case of -A-(R)₀, the structure is actually -A.

When a substituent is vacant, it means that the substituent is absent, for example, when X is vacant in A-X, the structure of A-X is actually A.

When one of the variables is selected from a single bond, it means that the two groups linked by the single bond are connected directly. For example, when L in A-L-Z represents a single bond, the structure of A-L-Z is actually A-Z.

When the bond of a substituent can be cross-linked to two or more atoms on a ring, this substituent can be bonded to any atom on this ring. For example, the structural moiety 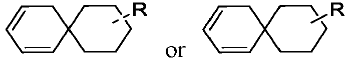 indicates that the substituent R can be substituted at any position on cyclohexyl or cyclohexadiene. When the enumerative substituent does not indicate by which atom it is linked to the group to be substituted, such substituent can be bonded by any atom thereof. For example, when pyridyl acts as a substituent, it can be linked to the group to be substituted by any carbon atom on the pyridine ring.

When the enumerative linking group does not indicate the direction for linking, the direction for linking is arbitrary, for example, the linking group L contained in 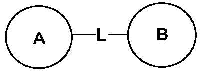 is -M-W-, then -M-W- can link ring A and ring B to form 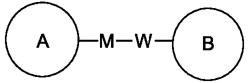 in the direction same as left-to-right reading order, and form 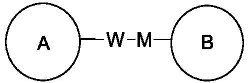 in the direction contrary to left-to-right reading order. A combination of the linking groups, substituents and/or variables thereof is allowed only when such combination can result in a stable compound.

Unless otherwise specified, when a group has one or more than one linkable site, any one or more than one site of the group can be linked to other groups through chemical bonds. When the linking site of the chemical bond is not positioned, and there is an H atom at the linkable site, then the number of H atoms at the site will decrease correspondingly with the number of the chemical bonds linking thereto so as to meet the corresponding valence. The chemical bond between the site and other groups can be represented by a straight solid bond 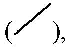 a straight dashed bond 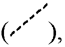 or a wavy line 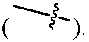 For example, the straight solid bond in -OCH₃ means that it is linked to other groups through the oxygen atom in the group; the straight dashed bond in 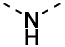 means that it is linked to other groups through the two ends of the nitrogen atom in the group; the wave line in 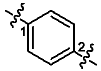 means that the phenyl group is linked to other groups through carbon atoms at position 1 and position 2; 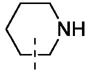 means that it can be linked to other groups through any linkable sites on the piperidinyl by one chemical bond, including at least four types of linkage, including 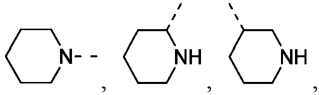 and 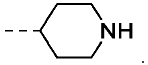 Even though the H atom is drawn on the -N-, 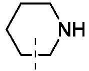 still includes the linkage of 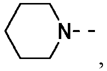 merely when one chemical bond was connected, the H of this site will be reduced by one to the corresponding monovalent piperidinyl.

Unless otherwise specified, the number of atoms in a ring is usually defined as the number of ring members, for example, "5- to 7-membered ring" refers to a "ring" in which 5 to 7 atoms are arranged around.

Unless otherwise specified, the term "C₁₋₄ alkyl" refers to a linear or branched saturated hydrocarbon group consisting of 1 to 4 carbon atoms. The C₁₋₄ alkyl includes C₁₋₂, C₁₋₃, C₂₋₃ alkyl, *etc.*; it can be monovalent (such as methyl), divalent (such as methylene), or multivalent (such as methine). Examples of C₁₋₄ alkyl include, but are not limited to, methyl (Me), ethyl (Et), propyl (including *n*-propyl and isopropyl), butyl (including *n*-butyl, isobutyl, *s*-butyl, and *t*-butyl), *etc.*

Unless otherwise specified, the term "C₁₋₃ alkyl" refers to a linear or branched saturated hydrocarbon group consisting of 1 to 3 carbon atoms. The C₁₋₃ alkyl includes C₁₋₂, C₂₋₃ alkyl, *etc.*; it can be monovalent (such as methyl), divalent (such as methylene), or multivalent (such as methine). Examples of C₁₋₃ alkyl include, but are not limited to, methyl (Me), ethyl (Et), propyl (including *n*-propyl and isopropyl), *etc.*

Unless otherwise specified, the term "C₁₋₃ alkoxy" refers to an alkyl group containing 1 to 3 carbon atoms that are connected to the rest of the molecule through an oxygen atom. The C₁₋₃ alkoxy includes C₁₋₂, C₂₋₃, C₃, C₂ alkoxy, *etc.* Examples of C₁₋₃ alkoxy include, but are not limited to, methoxy, ethoxy, propoxy (including n-propoxy and isopropoxy), *etc.*

Unless otherwise specified, the term "C₁₋₃ alkylamino" refers to an alkyl group containing 1 to 3 carbon atoms that are connected to the rest of the molecule through an amino group. The C₁₋₃ alkylamino includes C₁₋₂, C₃, C₂ alkylamino, *etc.* Examples of C₁₋₃ alkylamino include, but are not limited to, -NHCH₃, -N(CH₃)₂, -NHCH₂CH₃, -N(CH₃)CH₂CH₃, -NHCH₂CH₂CH₃, -NHCH₂(CH₃)₂, *etc.*

Unless otherwise specified, "C₃₋₅ cycloalkyl" refers to a saturated cyclic hydrocarbon group consisting of 3 to 5 carbon atoms, which is a monocyclic system, and the C₃₋₅ cycloalkyl includes C₃₋₄, C₄₋₅ cycloalkyl, *etc.*; it can be monovalent, divalent, or multivalent. Examples of C₃₋₅ cycloalkyl include, but are not limited to, cyclopropyl, cyclobutyl, cyclopentyl, *etc.*

Unless otherwise specified, the term "4- to 5-membered heterocycloalkyl" by itself or in combination with other terms refers to a saturated monocyclic group consisting of 4 to 5 ring atoms, wherein 1, 2, 3, or 4 ring atoms are heteroatoms independently selected from O, S, and N, and the rest are carbon atoms, wherein nitrogen atoms are optionally quaternized, and nitrogen and sulfur heteroatoms can be optionally oxidized (*i.e.,* NO and S(O)ₚ, p is 1 or 2). In addition, with regard to the "4- to 5-membered heterocycloalkyl", a heteroatom may occupy the connection position of the heterocycloalkyl with the rest of the molecule. The 4- to 5-membered heterocycloalkyl includes 4-membered and 5-membered heterocycloalkyl. Examples of 4- to 5-membered heterocycloalkyl include, but are not limited to, azetidinyl, oxetanyl, thietanyl, pyrrolidinyl, pyrazolidinyl, imidazolidinyl, tetrahydrothiophenyl (including tetrahydrothiophen-2-yl, tetrahydrothiophen-3-yl, etc.), tetrahydrofuranyl (including tetrahydrofuran-2-yl, *etc.*), *etc.*

Unless otherwise specified, the terms "5- to 6-membered heteroaryl ring" and "5- to 6- membered heteroaryl" in the present disclosure can be used interchangeably, and the term "5- to 6-membered heteroaryl" refers to a monocyclic group consisting of 5 to 6 ring atoms with a conjugated *π*-electron system, wherein 1, 2, 3, or 4 ring atoms are heteroatoms independently selected from O, S, and N, and the rest are carbon atoms, wherein nitrogen atoms are optionally quaternized, and nitrogen and sulfur heteroatoms can be optionally oxidized (*i.e.,* NO and S(O)ₚ, p is 1 or 2). The 5- to 6-membered heteroaryl can be linked to the rest of the molecule through a heteroatom or a carbon atom. The 5- to 6-membered heteroaryl includes 5-membered and 6-membered heteroaryl. Examples of the 5- to 6-membered heteroaryl include, but are not limited to, pyrrolyl (including *N*-pyrrolyl, 2-pyrrolyl, 3-pyrrolyl, *etc.),* pyrazolyl (including 2-pyrazolyl, 3-pyrazolyl, *etc*.), imidazolyl (including *N*-imidazolyl, 2-imidazolyl, 4-imidazolyl, 5-imidazolyl, *etc*.), oxazolyl (including 2-oxazolyl, 4-oxazolyl, 5-oxazolyl, *etc.),* triazolyl (including 1*H*-1,2,3-triazolyl, 2*H*-1,2,3-triazolyl, 1*H*-1,2,4-triazolyl, 4*H*-1,2,4-triazolyl, *etc.*)*,* tetrazolyl, isoxazolyl (including 3-isoxazolyl, 4-isoxazolyl, 5-isoxazolyl, *etc*.), thiazolyl (including 2-thiazolyl, 4-thiazolyl, 5-thiazolyl, *etc.*), furyl (including 2-furyl, 3-furyl, *etc.*), thienyl (including 2-thienyl, 3-thienyl, *etc.*), pyridyl (including 2-pyridyl, 3-pyridyl, 4-pyridyl, *etc.*), pyrazinyl, or pyrimidinyl (including 2-pyrimidinyl, 4-pyrimidinyl, *etc.).*

The structure of the compounds of the present disclosure can be confirmed by conventional methods known to those skilled in the art, and if the present disclosure involves an absolute configuration of a compound, then the absolute configuration can be confirmed by means of conventional techniques in the art. For example, in the case of single crystal X-ray diffraction (SXRD), diffraction intensity data are collected from the cultured single crystal using a Bruker D8 venture diffractometer with CuKα radiation as the light source and scanning mode: ϕ/ω scan, and after collecting the relevant data, the crystal structure is further analyzed by direct method (Shelxs97), so that the absolute configuration can be confirmed.

The compounds of the present disclosure can be prepared by a variety of synthetic methods known to those skilled in the art, including the specific embodiments listed below, the embodiments formed by their combination with other chemical synthesis methods, and equivalent alternatives known to those skilled in the art, with preferred embodiments including, but not limited to, the examples of the present disclosure.

The solvent used in the present disclosure is commercially available.

In the present disclosure, the following abbreviations are used: aq stands for water; eq stands for equivalent; DCM stands for dichloromethane; PE stands for petroleum ether; DMSO stands for dimethyl sulfoxide; EtOAc stands for ethyl acetate; EtOH stands for ethanol; MeOH stands for methanol; DMF stands for *N*,*N*-dimethylformamide; Cbz stands for benzyloxycarbonyl, which is an amine protecting group; Boc stands for *tert*-butoxycarbonyl, which is an amine protecting group; r.t. stands for room temperature; O/N stands for overnight; THF stands for tetrahydrofuran; Boc₂O stands for di-*tert*-butyl dicarbonate; TFA stands for trifluoroacetic acid; HCl stands for hydrochloric acid; mp stands for melting point; Pd(dppf)Cl₂ stands for [1,1'-bis(diphenylphosphino)ferrocene]dichloropalladium(II); Pd(dppf)Cl₂·CH₂Cl₂ stands for [1,1'-bis(diphenylphosphino)ferrocene]dichloropalladium(II) dichloromethane complex; TEA stands for triethylamine; Xantphos stands for 4,5-bis(diphenylphosphino)-9,9-dimethylxanthene; Pd₂(dba)₃ stands for tris(dibenzylideneacetone)dipalladium(0); EDCI stands for 1-(3-dimethylaminopropyl)-3-ethylcarbodiimide hydrochloride; HOBt stands for 1-hydroxybenzotriazole; NMP stands for *N*-methyl-2-pyrrolidone; DIPEA stands for *N*,*N*-diisopropylethylamine; LiHMDS stands for lithium bis(trimethylsilyl)amide; Pd₂(dba)₃.CHCl₃ stands for tris(dibenzylideneacetone)dipalladium(0)-chloroform adduct.

The compounds of the present disclosure are named according to the conventional naming principles in the art or by ChemDraw^{®} software, and the commercially available compounds use the supplier catalog names.

### DETAILED DESCRIPTION OF THE PREFERRED EMBODIMENT

The present disclosure is described in detail by the examples below, but it does not mean that there are any adverse restrictions on the present disclosure. The present disclosure has been described in detail herein, and its specific examples have also been disclosed; for one skilled in the art, it is obvious to make various modifications and improvements to the examples of the present disclosure without departing from the spirit and scope of the present disclosure.

### Reference example 1

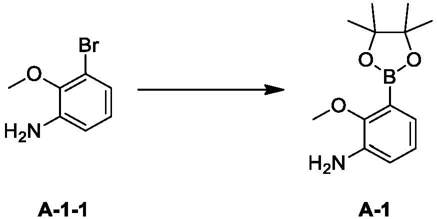

### Step 1: Synthesis of compound A-1

Compound **A-1-1** (10 g, 49.49 mmol, 1 *eq*), bis(pinacolato)diboron (18.85 g, 74.24 mmol, 1.5 *eq*), and potassium acetate (14.57 g, 148.48 mmol, 3 *eq*) were dissolved in 1,4-dioxane (200 mL). After replacing with nitrogen three times, Pd(dppf)Cl₂·CH₂Cl₂ (2.02 g, 2.47 mmol, 0.05 *eq*) was added, and the mixture was stirred at 100°C for 2 hours. Water (200 mL) was added to the reaction mixture, and the mixture was extracted with ethyl acetate (100 mL * 2). The organic phase was dried over anhydrous sodium sulfate, filtered, and the filtrate was concentrated under reduced pressure to obtain a crude product. The crude product was purified by silica gel column chromatography (V/V, ethyl acetate/petroleum ether = 0 to 25%) to obtain compound **A-1.** ¹HNMR (400 MHz, CDCl₃) δ: 7.13 - 7.11 (m, 1H), 6.93 (t, *J* = 15.2, 7.6 Hz, 1H), 6.88 - 6.85 (m, 1H), 3.81 (s, 3H), 1.36 (s, 12H); LCMS *m*/*z* = 250.1 [M+H]⁺.

### Reference example 2

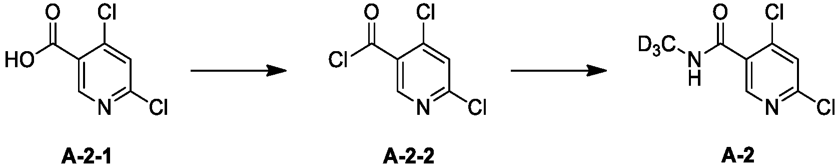

### Step 1: Synthesis of compound A-2-2

Compound **A-2-1** (18.5 g, 96.35 mmol, 1 *eq*) was dissolved in DCM (200 mL). Oxalyl chloride (15.90 g, 125.26 mmol, 10.96 mL, 1.3 *eq*) was then added to the mixture, and DMF (352.15 mg, 4.82 mmol, 370.68 µL, 0.05 *eq*) was added thereto. The reaction was carried out at 25°C for 16 hours. The reaction system was then concentrated, added with dichloromethane (50 mL), concentrated again, and directly used in the next step to obtain compound **A-2-2.**

### Step 2: Synthesis of compound A-2

Compound **A-2-2** (20 g, 95.04 mmol, 1 *eq*) was added to DCM (300 mL). Deuterated methylamine hydrochloride (5.36 g, 76.03 mmol, 0.8 *eq*) was then added. After cooling the mixture to 0°C, DIPEA (36.85 g, 285.11 mmol, 49.66 mL, 3 *eq*) was added, and the reaction was carried out at 20°C for 16 hours. After the reaction was completed, a saturated ammonium chloride aqueous solution (100 mL) was added to the system for extraction and phase separation. The aqueous phase was then extracted once with dichloromethane (60 mL). The organic phases were combined, washed once with saturated brine, dried over anhydrous sodium sulfate, filtered, and concentrated to obtain a crude product. *tert*-Butyl methyl ether (100 mL) was added to the crude product. The mixture was stirred for 2 hours, filtered, and the filter cake was concentrated under reduced pressure to obtain compound **A-2.** ¹H NMR (400 MHz, DMSO-*d*₆) δ: 8.59 (s, 1H), 8.48 (s, 1H), 7.91 (s, 1H); LCMS *m*/*z* = 208.1 [M+1]⁺.

### Reference example 3

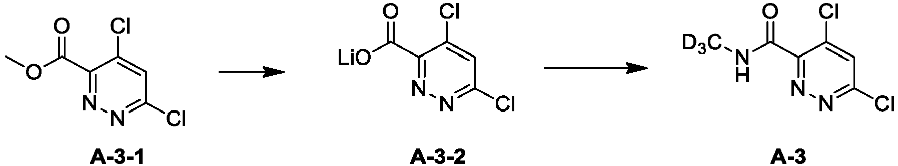

### Step 1: Synthesis of compound A-3-2

Compound **A-3-1** (20 g, 96.61 mmol, 1 *eq*) was dissolved in acetonitrile (100 mL) and water (15 mL). Lithium bromide (25.17 g, 289.84 mmol, 7.28 mL, 3 *eq*) and DIPEA (37.46 g, 289.84 mmol, 50.48 mL, 3 *eq*) were added thereto. The mixture was stirred and reacted at 20°C for 3 hours. The system was filtered, and the filter cake was rinsed with acetonitrile (50 mL). The filter cake was collected to obtain compound **A-3-2.** LCMS *m*/*z* = 192.9 [COOH+1]⁺.

### Step 2: Synthesis of compound A-3

Compound **A-3-2** (10 g, 50.27 mmol, 1 *eq*) was dissolved in DCM (150 mL). Oxalyl chloride (8.93 g, 70.38 mmol, 6.16 mL, 1.4 *eq*) was then added to the mixture, and DMF (183.72 mg, 2.51 mmol, 193.39 µL, 0.05 *eq*) was added thereto. The reaction was carried out at 20°C for 4 hours. The reaction system was concentrated, then dichloromethane (30 mL) was added, and the mixture was further concentrated. The concentrated mixture was added to dichloromethane (150 mL), and deuterated methylamine hydrochloride (3.37 g, 47.76 mmol, 0.95 *eq*) was added. After cooling to 0°C, DIPEA (19.49 g, 150.83 mmol, 26.27 mL, 3 *eq*) was added. The reaction was carried out at 20°C for 16 hours. After the reaction was completed, water (150 mL) was added to the system for extraction and phase separation. The aqueous phase was then extracted once with dichloromethane (150 mL). The organic phases were combined, washed once with saturated brine, dried over anhydrous sodium sulfate, filtered, and concentrated to obtain a crude product. The crude product was purified by silica gel column chromatography (ethyl acetate/petroleum ether = 0 to 20%) to obtain compound **A-3.** LCMS *m*/*z* = 209.0 [M+1]⁺.

### Reference example 4

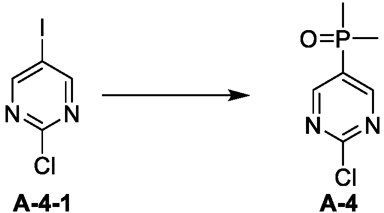

### Step 1: Synthesis of compound A-4

Compound **A-4-1** (3 g, 12.48 mmol, 1 *eq*), dimethylphosphine oxide (1.02 g, 13.10 mmol, 1.05 *eq*), potassium phosphate tribasic (3.97 g, 18.72 mmol, 1.5 *eq*), and Xantphos (721.98 mg, 1.25 mmol, 0.1 *eq*) were dissolved in 1 4-dioxane (30 mL). After replacing with nitrogen three times, palladium acetate (280.13 mg, 1.25 mmol, 0.1 *eq*) was added. The mixture was stirred at 120°C for 2 hours. The reaction mixture was filtered, and the filter cake was rinsed with ethyl acetate (30 mL * 2). The filtrate was extracted with water (30 mL), and the organic phase was dried over anhydrous sodium sulfate and then concentrated. The crude product was purified by silica gel column chromatography (methanol/dichloromethane = 0 to 20%) to obtain compound **A-4.** LCMS *m*/*z* = 191.0 [M+H]⁺.

### Reference example 5

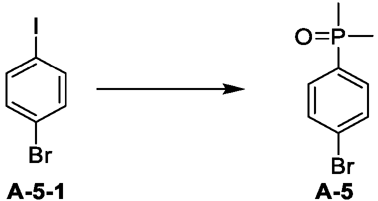

### Step 1: Synthesis of compound A-5

Compound **A-5-1** (10 g, 35.35 mmol, 1 *eq*), dimethylphosphine oxide (2.76 g, 35.35 mmol, 1 *eq*), triethylamine (4.18 g, 41.36 mmol, 5.76 mL, 1.17 *eq*), and Xantphos (204.53 mg, 353.48 µmol, 0.01 *eq*) were dissolved in 1 4-dioxane (50 mL) and THF (50 mL). After replacing with nitrogen three times, tris(dibenzylideneacetone)dipalladium(0) (161.84 mg, 176.74 µmol, 0.005 *eq*) was added. The mixture was stirred at 15°C for 2 hours. The reaction mixture was added with water (50 mL) and extracted with ethyl acetate (100 mL * 2). The organic phase was dried over anhydrous sodium sulfate, and filtered. The filtrate was concentrated under reduced pressure to obtain a crude product. The crude product was purified by silica gel column chromatography (methanol/dichloromethane = 0 to 9%) to obtain compound **A-5.** LCMS *m*/*z* = 232.8 [M+H]⁺.

To synthesize **reference examples 6 and 7** listed in Table 1, follow the synthetic steps of **reference example 5**, replacing dimethylphosphine oxide in step 1 with fragment 2, and replacing **A-5-1** with fragment 1.

**Table 1**

| Reference example | Compound | Fragment 1 | Fragment 2 | Structural formula | Spectrum |
|---|---|---|---|---|---|
| 6 | A-6 | 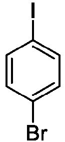 | 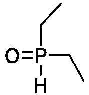 | 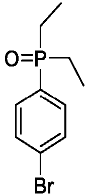 | LCMS *m*/*z* = 260.8 [M+1]⁺. |
| 7 | A-7 | 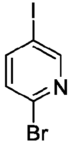 | 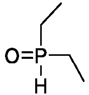 | 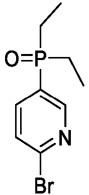 | LCMS *m*/*z* = 262.0 [M+1]⁺. |

### Reference example 8

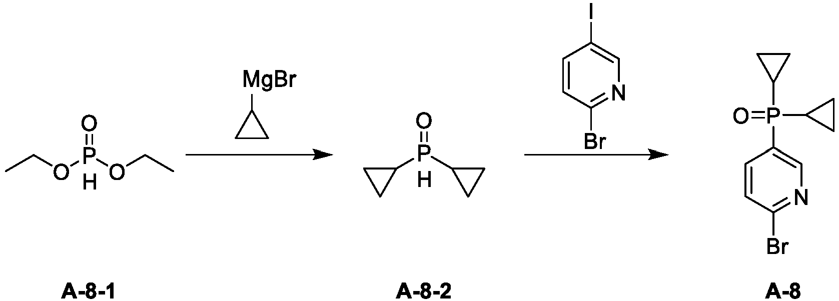

### Step 1: Synthesis of compound A-8-2

**A-8-1** (1.38 g, 10.00 mmol, 1.29 mL, 1 *eq*) was dissolved in THF (30 mL). The mixture was cooled to -70°C, and a solution of cyclopropylmagnesium bromide in tetrahydrofuran (0.5 M, 3.09 g, 30 mmol, 60 mL, 3 *eq*) was added dropwise thereto. The mixture was stirred for 2 hours, then slowly warmed to 25°C, and stirred for 3 hours. The mixture was then cooled to 0°C, added with 0.5 M hydrochloric acid aqueous solution (40 mL) to quench the reaction, and then extracted with ethyl acetate (30 mL * 3). The organic phases were combined, washed with saturated sodium chloride solution (20 mL), dried over anhydrous sodium sulfate, and filtered. The filtrate was concentrated under reduced pressure to obtain compound **A-8-2.** LCMS *m*/*z* = 131.2 [M+H]⁺.

### Step 2: Synthesis of compound A-8

2-Bromo-5-iodopyridine (800 mg, 2.82 mmol, 1 *eq*), **A-8-2** (366.69 mg, 2.82 mmol, 1 *eq*), and Xantphos (163.05 mg, 281.80 µmol, 0.1 *eq*) were dissolved in 1,4-dioxane (14 mL). Triethylamine (5.64 mmol, 5.64 mL, 2 *eq*) was added, followed by replacement with nitrogen. Finally, the catalyst Pd₂(dba)₃ (258.05 mg, 281.80 µmol, 0.1 *eq*) was added, followed by replacement with nitrogen. The reaction mixture was heated to 75°C and stirred for 12 hours. After the reaction was completed, the reaction mixture was cooled, added with 20 mL of water, and extracted with dichloromethane (30 mL * 2). The organic phases were combined, washed with saturated sodium chloride solution, dried over anhydrous sodium sulfate, and concentrated to obtain a crude product. The crude product was purified by silica gel column chromatography (dichloromethane: methanol = 100:0 to 50:1) to obtain compound **A-8.** LCMS *m*/*z* = 285.9 [M+1]⁺.

To synthesize **reference examples 9 and 10** listed in Table 2, follow the synthetic steps of **reference example 8**, replacing cyclopropylmagnesium bromide in step 1 with fragment 1, and replacing 2-bromo-5-iodopyridine in step 2 with fragment 2.

**Table 2**

| Reference example | Compound | Fragment 1 | Fragment 2 | Structural formula | Spectrum |
|---|---|---|---|---|---|
| **9** | **A-9** | 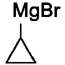 | 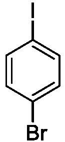 | 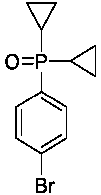 | LCMS *m*/*z* = 285.1 [M+H]⁺. |
| **10** | **A-10** | 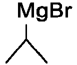 | 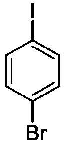 | 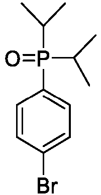 | LCMS *m*/*z* = 290.7 [M+H]⁺. |

### Reference example 11

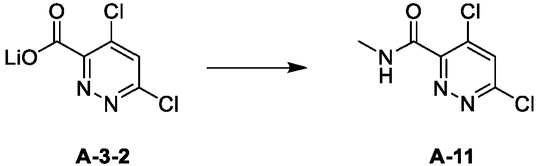

### Step 1: Synthesis of compound A-11

Compound **A-3-2** (7 g, 35.19 mmol, 1 *eq*) was dissolved in DCM (105 mL). Oxalyl chloride (6.25 g, 49.27 mmol, 4.31 mL, 1.4 *eq*) was then added to the mixture, and DMF (128.60 mg, 1.76 mmol, 135.37 µL, 0.05 *eq*) was added thereto. The reaction was carried out at 20°C for 3 hours. The reaction system was concentrated, then dichloromethane (80 mL) was added, and the mixture was further concentrated. The concentrated mixture was added to dichloromethane (150 mL), and methylamine hydrochloride (2.26 g, 33.43 mmol, 0.95 *eq*) was added. After cooling to -60°C, DIPEA (13.64 g, 105.57 mmol, 18.39 mL, 3 *eq*) was added. The reaction was carried out at 20°C for 10 hours. After the reaction was completed, water (80 mL) was added to the system for extraction and phase separation. The aqueous phase was then extracted with dichloromethane (100 mL). The organic phases were combined, washed with saturated brine, dried over anhydrous sodium sulfate, filtered, and concentrated to obtain a crude product. The crude product was purified by silica gel column chromatography (ethyl acetate/petroleum ether = 0 to 20%) to obtain compound **A-11.** LCMS *m*/*z* = 206.0 [M+1]⁺.

### Reference example 12

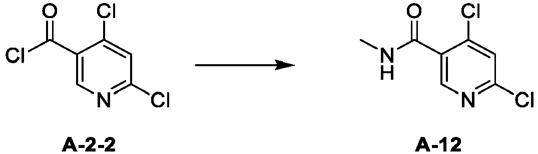

### Step 1: Synthesis of compound A-12

Compound **A-2-2** (8.77 g, 41.67 mmol, 1 *eq*) was dissolved in DCM (100 mL). Methylamine hydrochloride (2.81 g, 41.67 mmol, 1 *eq*) was then added to the mixture. After the mixture was cooled to 0°C, DIPEA (16.16 g, 125.01 mmol, 21.78 mL, 3 *eq*) was added thereto. The reaction was carried out at 20°C for 12 hours. After the reaction was completed, water (50 mL) was added to the system for extraction and phase separation. The aqueous phase was then extracted with dichloromethane (50 mL). The organic phases were combined, washed with saturated brine, dried over anhydrous sodium sulfate, filtered, and concentrated to obtain compound **A-12.** LCMS *m*/*z* = 205.0 [M+1]⁺.

### Reference example 13

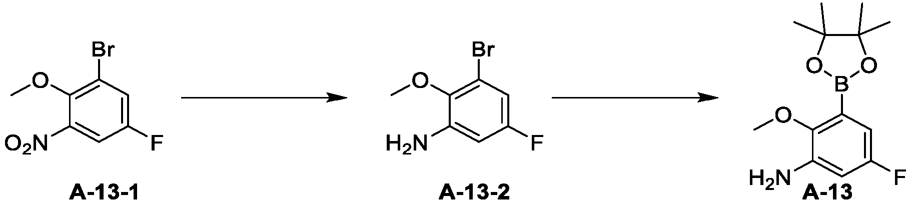

### Step 1: Synthesis of compound A-13-2

Compound **A-13-1** (6 g, 24.00 mmol, 1 *eq*) and stannous chloride dihydrate (21.66 g, 95.99 mmol, 4 *eq*) were dissolved in ethyl acetate (200 mL). The mixture was stirred at 70°C for 1 hour. After cooling to room temperature, the reaction mixture was added with water (200 mL), and extracted with ethyl acetate (100 mL * 2). The organic phase was dried over anhydrous sodium sulfate, filtered, and the filtrate was concentrated under reduced pressure to obtain a crude product. The crude product was purified by silica gel column chromatography (V/V, ethyl acetate/petroleum ether = 0 to 10%) to obtain compound **A-13-2.** LCMS *m*/*z* = 219.7 [M+H]⁺.

### Step 2: Synthesis of compound A-13

Compound **A-13-2** (2 g, 9.09 mmol, 1 *eq*), bis(pinacolato)diboron (3.46 g, 13.63 mmol, 1.5 *eq*)*,* and potassium acetate (2.68 g, 27.27 mmol, 3 *eq*) were dissolved in 1,4-dioxane (20 mL). After replacing with nitrogen three times, Pd(dppf)Cl₂·CH₂Cl₂ (371.13 mg, 0.45mmol, 0.05 *eq*) was added, and the mixture was stirred at 100°C for 18 hours. After cooling to room temperature, the reaction mixture was added with water (200 mL) and extracted with ethyl acetate (100 mL * 2). The organic phases were combined, dried over anhydrous sodium sulfate, and filtered. The filtrate was concentrated under reduced pressure to obtain a crude product. The crude product was purified by silica gel column chromatography (V/V, ethyl acetate/petroleum ether = 0 to 25%) to obtain compound **A-13.** LCMS *m*/*z* = 267.9 [M+H]⁺.

### Reference example 14

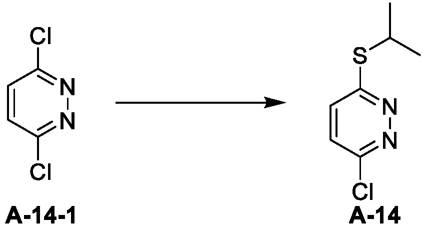

### Step 1: Synthesis of compound A-14

Compound **A-14-1** (1 g, 6.71 mmol, 1 *eq*) was dissolved in *N*,*N*-dimethylformamide (10 mL), and then sodium 2-propanethiolate (724.39 mg, 7.38 mmol, 1.1 *eq*) was added thereto. The mixture was stirred at 15°C for 1 hour. The reaction mixture was added with water (10 mL) and extracted with ethyl acetate (10 mL * 2). The organic phase was dried over anhydrous sodium sulfate, filtered, and the filtrate was concentrated under reduced pressure to obtain compound **A-14.** LCMS *m*/*z* = 189.2 [M+H]⁺.

### Reference example 15

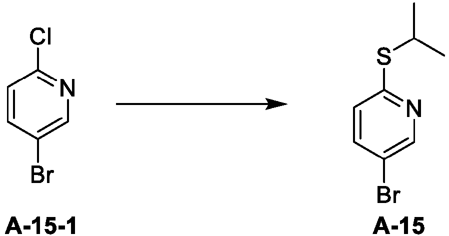

### Step 1: Synthesis of compound A-15

Compound **A-15-1** (1 g, 5.20 mmol, 1 *eq*) was dissolved in *N*,*N*-dimethylformamide (20 mL), and then sodium 2-propanethiolate (2.91 g, 29.62 mmol, 5.7 *eq*) was added thereto. The mixture was stirred at 15°C for 1 hour. The reaction mixture was added with water (20 mL) and extracted with ethyl acetate (20 mL * 2). The organic phase was dried over anhydrous sodium sulfate, filtered, and the filtrate was concentrated under reduced pressure to obtain compound **A-15.** LCMS *m*/*z* = 232.0 [M+H]⁺.

### Example 1 and example 2

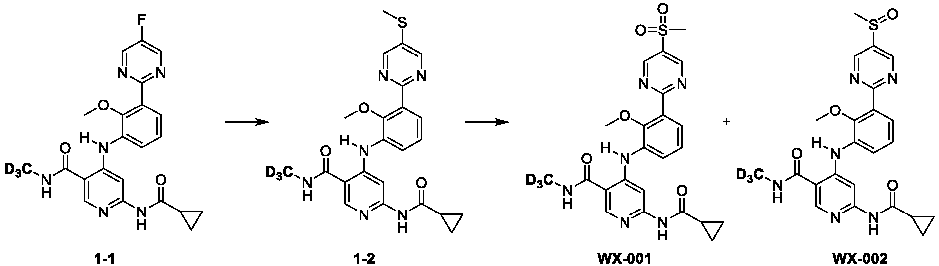

### Step 1: Synthesis of compound 1-2

Compound **1-1** (280 mg, 637.15 µmol, 1 *eq*) was dissolved in DMF (7 mL), and then sodium thiomethoxide (178.63 mg, 2.55 mmol, 162.39 µL, 4 *eq*) was added thereto. The mixture was stirred at 40°C for 2 hours. The reaction mixture was added with water (10 mL) and ethyl acetate (10 mL) for extraction and phase separation. The aqueous phase was then extracted with ethyl acetate (10 mL). The organic phases were combined, dried over anhydrous sodium sulfate, and filtered. The filtrate was concentrated under reduced pressure to obtain compound **1-2.** LCMS *m*/*z* = 468.1 [M+H]⁺.

### Step 2: Synthesis of hydrochloride of compound WX-001 and hydrochloride of compound WX-002

Compound **1-2** (40 mg, 85.55 µmol, 1 *eq*) was dissolved in DCM (2 mL). The mixture was cooled to 0°C and added with 3-chloroperoxybenzoic acid (26.05 mg, 128.33 µmol, purity of 85%, 1.5 *eq*)*.* The reaction was carried out at 20°C for 2 hours. 3-chloroperoxybenzoic acid was then added thereto (8.68 mg, 42.78 µmol, purity of 85%, 0.5 *eq*)*.* The reaction was carried out for another 2 hours. The system was added with water (10 mL) and ethyl acetate (10 mL) for extraction and phase separation. The aqueous phase was then extracted with ethyl acetate (10 mL). The organic phases were combined, washed with saturated sodium chloride aqueous solution, dried over anhydrous sodium sulfate, and concentrated to obtain a crude product. The crude product was separated by preparative high performance liquid chromatography (chromatographic column: Phenomenex Luna 80 * 30 mm * 3 µm; mobile phase: A (water containing 0.04% hydrochloric acid) and B (acetonitrile); gradient: B%: 5% to 35%, 8 minutes) to obtain **hydrochloride of** compound **WX-001** and **hydrochloride of** compound **WX-002.**

**Hydrochloride of WX-001:** ¹H NMR (400 MHz, CD₃OD) δ: 9.40 (s, 2H), 8.34 (s, 1H), 7.99 - 7.96 (m, 1H), 7.67 - 7.66 (m, 1H), 7.46 (t, *J* = 8.0 Hz, 1H), 6.54 (s, 1H), 3.82 (s, 3H), 3.35 (s, 3H), 1.81 - 1.70 (m, 1H), 1.11 - 1.01 (m,4H); LCMS *m*/*z* = 500.1 [M+H]⁺.

**Hydrochloride of WX-002:** ¹H NMR (400 MHz, CD₃OD) δ: 9.21 (s, 2H), 8.36 (s, 1H), 7.93 - 7.91 (m, 1H), 7.66 - 7.64 (m, 1H), 7.44 (t, *J* = 8.0 Hz, 1H), 6.56 (s, 1H), 3.78 (s, 3H), 3.06 (s, 3H), 1.81 - 1.72 (m, 1H), 1.11 - 1.01 (m, 4H); LCMS *m*/*z* = 484.1 [M+H]⁺.

### Example 3

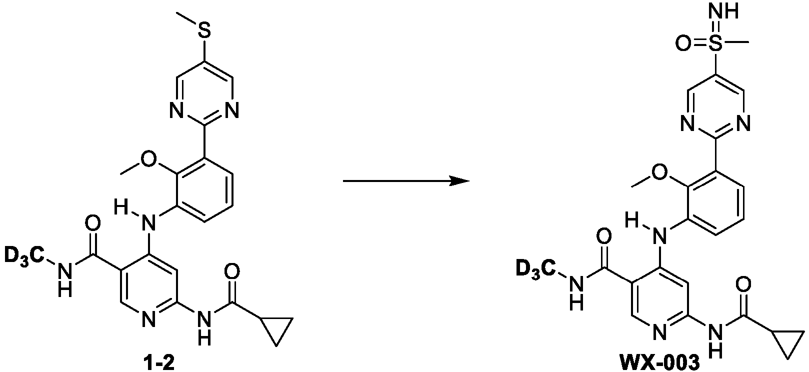

### Step 1: Synthesis of compound WX-003

Compound **1-2** (25 mg, 53.47 µmol, 1 *eq*) was dissolved in MeOH (0.2 mL), and then (diacetoxyiodo)benzene (51.67 mg, 160.41 µmol, 3 *eq*) and ammonium acetate (16.49 mg, 213.88 µmol, 4 *eq*) were added thereto. The reaction was carried out at 25°C for 2 hours. The system was added with water (5 mL) and ethyl acetate (5 mL) for extraction and phase separation. The aqueous phase was then extracted with ethyl acetate (5 mL). The organic phases were combined, washed with saturated sodium chloride aqueous solution, dried over anhydrous sodium sulfate, and concentrated to obtain a crude product. The crude product was purified by silica gel thin-layer chromatography (V:V:V, EtOAc: DCM: MeOH = 5:5:1) to obtain compound **WX-003.** ¹H NMR (400 MHz, CDCl₃) δ: 10.37 (s, 1H), 9.34 (s, 2H), 9.13 (s, 1H), 8.25 (s, 1H), 8.05 (s, 1H), 7.66 (d, *J* = 8.0 Hz, 2H), 7.25 (t, *J* = 8.0 Hz, 1H), 6.65 (s, 1H), 3.85 (s, 3H), 3.25 (s, 3H), 1.60 - 1.56 (m, 1H), 1.06 - 1.02 (m, 2H), 0.88 - 0.83 (m, 2H); LCMS *m*/*z* = 499.2 [M+H]⁺.

### Example 4

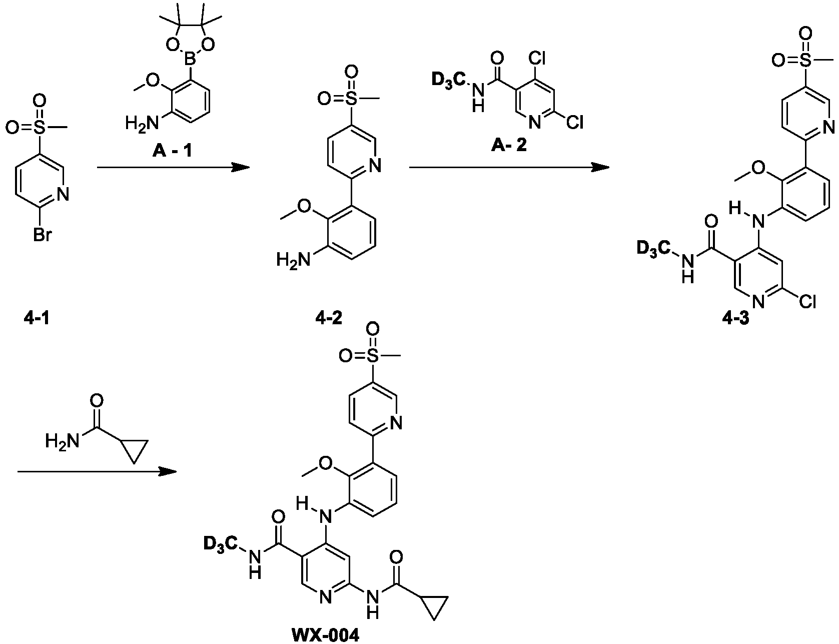

### Step 1: Synthesis of compound 4-2

Compound **4-1** (200 mg, 847.15 µmol, 1 *eq*), compound **A-1** (211.04 mg, 847.15 µmol, 1 *eq*), and sodium carbonate (269.37 mg, 2.54 mmol, 3 *eq*) were dissolved in DMF (4 mL) and water (0.8 mL). After replacing with nitrogen three times, Pd(dppf)Cl₂·CH₂Cl₂ (69.18 mg, 84.71 µmol, 0.1 *eq*) was added thereto. The reaction was carried out at 80°C for 12 hours. The reaction mixture was added with water (10 mL) and ethyl acetate (10 mL) for extraction and phase separation. The aqueous phase was then extracted with ethyl acetate (10 mL). The organic phases were combined, washed with saturated sodium chloride aqueous solution, dried over anhydrous sodium sulfate, and concentrated to obtain compound **4-2.** LCMS *m*/*z* = 279.0 [M+H]⁺.

### Step 2: Synthesis of compound 4-3

Compound **4-2** (180 mg, 646.72 µmol, 1 *eq*) and compound **A-2** (161.47 mg, 776.07 µmol, 1.2 *eq*) were added to THF (4.5 mL). After cooling to 0°C, the mixture was added with LiHMDS (1 M, 1.94 mL, 3.0 *eq*)*.* The reaction was carried out at 20°C for 2 hours. After the reaction was completed, the reaction mixture was added with water (20 mL) and ethyl acetate (20 mL) for extraction and phase separation. The aqueous phase was then extracted with ethyl acetate (20 mL). The organic phases were combined, washed with saturated sodium chloride aqueous solution, dried over anhydrous sodium sulfate, and concentrated to obtain compound **4-3.** LCMS *m*/*z* = 450.1 [M+H]⁺.

### Step 3: Synthesis of compound WX-004

Compound **4-3** (270 mg, 600.10 µmol, 1 *eq*) and cyclopropanecarboxamide (1.28 g, 15.00 mmol, 25 *eq*) were added to 1,4-dioxane (20 mL) and NMP (4 mL). Cesium carbonate (586.57 mg, 1.80 mmol, 3 *eq*) and Xantphos (52.08 mg, 90.01 µmol, 0.15 *eq*) were then added thereto. After replacing with nitrogen three times, Pd₂(dba)₃ (82.43 mg, 90.01 µmol, 0.15 *eq*) was added. The reaction was carried out at 120°C for 18 hours. After the reaction was completed, the reaction mixture was added with water (30 mL) and ethyl acetate (30 mL) for extraction and phase separation. The aqueous phase was then extracted with ethyl acetate (30 mL). The organic phases were combined, washed with saturated sodium chloride aqueous solution, dried over anhydrous sodium sulfate, and concentrated to obtain a crude product. The crude product was purified by silica gel column chromatography (ethyl acetate/petroleum ether = 0 to 100%) to obtain compound **WX-004.** ¹H NMR (400 MHz, CDCl₃) δ: 10.55 (s, 1H), 9.22 (s, 1H), 8.74 (s, 1H), 8.30 (s, 1H), 8.23 - 8.18 (m, 2H), 8.10 (s, 1H), 7.60 (d, *J* = 8.0 Hz, 2H), 7.30 (t, *J* = 8.0 Hz, 1H), 6.55 (s, 1H), 3.57 (s, 3H), 3.17 (s, 3H), 1.59 - 1.55 (m, 1H), 1.09 - 1.05 (m, 2H), 0.91 - 0.86 (m, 2H); LCMS *m*/*z* = 499.1 [M+H]⁺.

To synthesize **example 7** listed in Table 3, follow the synthesis steps of **example 4**, replacing **4-1** in step 1 with fragment **7-1** in the table below.

**Table 3**

| Example | Compound | Fragment | Structural formula | Spectrum |
|---|---|---|---|---|
| **7** | **Hydrochloride of WX-007** | 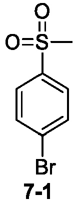 | 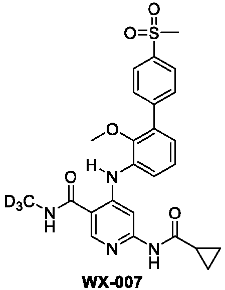 | ¹H NMR (400 MHz, MeOD) δ: 8.34 (s, 1H), 8.06 (d, *J* = 8.0 Hz, 2H), 7.88 (d, *J* = 8.0 Hz, 2H), 7.56 - 7.53 (m, 1H), 7.49 - 7.46 (m, 1H), 7.41 (t, *J* = 8.0 Hz, 1H), 6.68 (s, 1H), 3.47 (s, 3H), 3.19 (s, 3H), 1.80 - 1.76 (m, 1H), 1.12 |
| | | | | - 1.09 (m, 2H), 1.06 - 1.02 (m, 2H); LCMS *m*/*z* = 498.1 [M+H]⁺. |
| | | | | Purification method: preparative high performance liquid chromatography (chromatographic column: |
| | | | | Phenomenex Luna C18 80 * 40 mm * 3 µm; mobile phase: A (water containing 0.04% hydrochloric acid) and B (acetonitrile); gradient: B%: 23% to 42%, 6 minutes). |

To synthesize **example 26** listed in Table 4, follow the synthesis steps of **example 4**, replacing **4-1** in step 1 with fragment **7-1** in the table below, replacing **A-1** with **A-13**, and replacing **A-2** in step 2 with **A-12.**

**Table 4**

| Example | Compound | Fragment | Structural formula | Spectrum |
|---|---|---|---|---|
| **26** | **Trifluoroacetate of WX-026** | 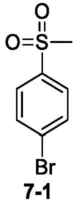 | 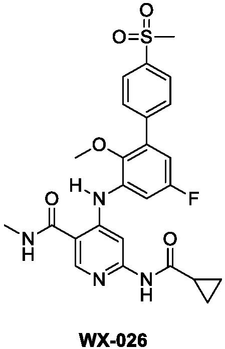 | ¹H NMR (400 MHz, CDCl₃) δ: 12.45 (s, 1H), 11.32 (s, 1H), 8.27 (s, 1H), 8.19 (s, 1H), 7.97 (d, *J* = 8.4 Hz, 2H), 7.75 (d, *J* = 8.4 Hz, 2H), 7.17 (dd, *J* = 3.0, 8.6 Hz, 1H), 6.94 (dd, *J =* 3.0, 8.4 Hz, 2H), 3.31 (s, 3H), 3.06 (s, 3H), 3.06 (d, *J* = 4.8 Hz, 3H), 1.91 - 1.87 (m, 1H), 1.06 - 0.99 (m, 2H), 0.96 - 0.89 (m, 2H); LCMS *m*/*z* = 513.1 [M+1]⁺. |
| | | | | Purification method: preparative high performance liquid chromatography (chromatographic column: Welch Xtimate C18 100 * 40 mm * 3 µm; mobile phase: A (water containing 0.075% trifluoroacetic acid) and B (acetonitrile); gradient: B%: 26% to 56%, 8 minutes). |

### Example 5

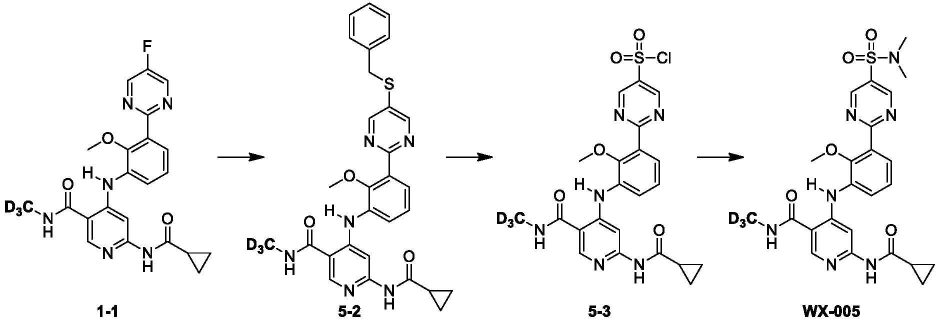

### Step 1: Synthesis of compound 5-2

Compound **1-1** (0.45 g, 1.02 mmol, 1 *eq*) was dissolved in DMF (5 mL). Cesium carbonate (500.45 mg, 1.54 mmol, 1.5 *eq*) and benzyl mercaptan (254.37 mg, 2.05 mmol, 239.97 µL, 2 *eq*) were added thereto, and the mixture was stirred at 40°C for 1 hour. The reaction mixture was added with water (10 mL) and ethyl acetate (10 mL) for extraction and phase separation. The aqueous phase was then extracted with ethyl acetate (10 mL). The organic phases were combined, dried over anhydrous sodium sulfate, and filtered. The filtrate was concentrated under reduced pressure to obtain compound **5-2.** LCMS *m*/*z* = 544.3 [M+H]⁺.

### Step 2: Synthesis of compound 5-3

Compound **5-2** (200 mg, 367.88 µmol, 1 *eq*) was dissolved in acetic acid (2 mL) and water (0.6 mL). The mixture was then cooled to 0°C, and N-chlorosuccinimide (221.06 mg, 1.66 mmol, 4.5 *eq*) was added thereto. The mixture was stirred at 20°C for 2 hours. Sodium sulfate was added to the system for drying, and the system was directly used in the next step. Compound **5-3** was obtained. LCMS *m*/*z* = 520.0 [M+H]⁺.

### Step 3: Synthesis of compound WX-005

Compound **5-3** (50 mg, 96.16 µmol, 1 *eq*) was dissolved in THF (2 mL). The mixture was then cooled to -20°C, and dimethylamine hydrochloride (392.06 mg, 4.81 mmol, 440.52 µL, 50 *eq*) and triethylamine (1.46 g, 14.42 mmol, 2.01 mL, 150 *eq*) were added thereto. The reaction was carried out at 20°C for 2 hours. After the reaction was completed, the reaction mixture was added with water (10 mL) and ethyl acetate (10 mL) for extraction and phase separation. The aqueous phase was then extracted with ethyl acetate (10 mL). The organic phases were combined, washed with saturated sodium chloride aqueous solution, dried over anhydrous sodium sulfate, and concentrated to obtain a crude product. The crude product was separated by preparative high performance liquid chromatography (chromatographic column: Waters Xbridge BEH C18 100 * 30 mm * 10 µm; mobile phase: A (water containing 10 mM ammonium bicarbonate) and B (acetonitrile); gradient: B%: 30% to 65%, 10 minutes) to obtain compound **WX-005.** ¹HNMR (400 MHz, CDCl₃) δ: 10.44 (s, 1H), 9.18 (s, 2H), 8.30 (s, 1H), 8.26 (s, 1H), 8.12 (s, 1H), 7.75 - 7.69 (m, 2H), 7.33 (t, *J* = 8.0 Hz, 1H), 6.20 (s, 1H), 3.87 (s, 3H), 2.87 (s, 6H), 1.54 - 1.51 (m, 1H), 1.11 - 1.08 (m, 2H), 0.92 - 0.87 (m, 2H); LCMS *m*/*z* = 529.2 [M+H]⁺.

### Example 6

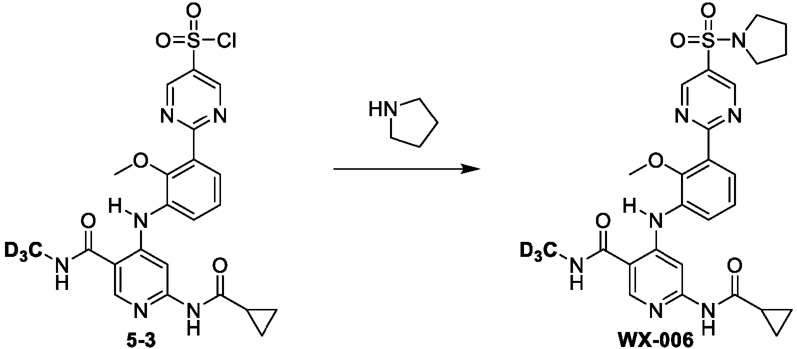

### Step 1: Synthesis of hydrochloride of compound WX-006

Compound **5-3** (95.6 mg, 183.85 µmol, 1 *eq*) was dissolved in THF (2 mL). The mixture was then cooled to -20°C, and tetrahydropyrrole (13.08 mg, 183.85 µmol, 15.35 µL, 1 *eq*) and triethylamine (2.79 g, 27.58 mmol, 3.84 mL, 150 *eq*) were added thereto. The reaction was carried out at 20°C for 2 hours. After the reaction was completed, the reaction mixture was added with water (10 mL) and ethyl acetate (10 mL) for extraction and phase separation. The aqueous phase was then extracted with ethyl acetate (10 mL). The organic phases were combined, washed with saturated sodium chloride aqueous solution, dried over anhydrous sodium sulfate, and concentrated to obtain a crude product. The crude product was separated by preparative high performance liquid chromatography (chromatographic column: Phenomenex Luna C18 80 * 30 mm * 3 µm; mobile phase: A (water containing 0.04% hydrochloric acid) and B (acetonitrile); gradient: B%: 15% to 45%, 8 minutes) to obtain **hydrochloride of** compound **WX-006.** ¹H NMR (400 MHz, CD₃OD) δ: 9.30 (s, 2H), 8.35 (s, 1H), 7.98 - 7.95 (m, 1H), 7.68 - 7.65 (m, 1H), 7.47 - 7.43 (m, 1H), 6.58 (s, 1H), 3.81 (s, 3H), 3.39 - 3.32 (m, 4H), 1.88 - 1.86 (m, 4H), 1.85 - 1.70 (m, 1H), 1.12 - 1.08 (m, 2H), 1.04 - 1.02 (m, 2H); LCMS *m*/*z* = 555.2 [M+H]⁺.

### Example 8

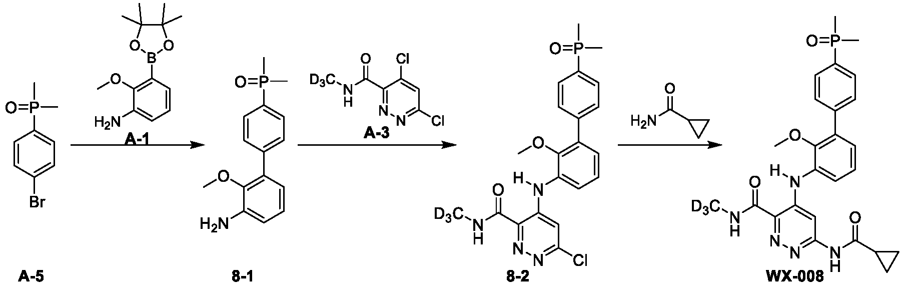

### Step 1: Synthesis of compound 8-1

Compound **A-5** (1 g, 4.29 mmol, 1 *eq*), compound **A-1** (1.07 g, 4.29 mmol, 1 *eq*), and potassium phosphate tribasic (1.82 g, 8.58 mmol, 2 *eq*) were dissolved in water (4 mL) and 1,4-dioxane (20 mL). After replacing with nitrogen three times, Pd(dppf)Cl₂·CH₂Cl₂ (350.43 mg, 429.11 µmol, 0.1 *eq*) was added. The mixture was stirred at 80°C for 2 hours. The reaction mixture was added with water (10 mL) and extracted with ethyl acetate (20 mL * 2). The organic phase was dried over anhydrous sodium sulfate, filtered, and the filtrate was concentrated under reduced pressure to obtain a crude product. The crude product was purified by silica gel column chromatography (methanol/dichloromethane = 0 to 10%) to obtain compound **8-1.** ¹HNMR (400 MHz, CDCl₃) δ: 7.81 - 7.71 (m, 4H), 7.02 - 6.98 (m, 1H), 6.81 - 6.72 (m, 2H), 3.95 (s, 2H), 3.40 (s, 3H), 1.81 (s, 3H), 1.78 (s, 3H); LCMS *m*/*z* = 276.1 [M+H]⁺.

### Step 2: Synthesis of compound 8-2

Compound **8-1** (200 mg, 726.53 µmol, 1 *eq*) and compound **A-3** (151.88 mg, 726.53 µmol, 1 *eq*) were dissolved in THF (10 mL). LiHMDS (1 M, 2.91 mL, 4 *eq*) was added. The mixture was stirred at 15°C for 1 hour. The reaction mixture was added with water (10 mL) and extracted with ethyl acetate (20 mL * 2). The organic phase was dried over anhydrous sodium sulfate, filtered, and the filtrate was concentrated under reduced pressure to obtain a crude product. The crude product was purified by silica gel column chromatography (methanol/dichloromethane = 0 to 10%) to obtain compound **8-2.** LCMS *m*/*z* = 448.1 [M+H]⁺.

### Step 3: Synthesis of hydrochloride of compound WX-008.

Compound **8-2** (0.1 g, 223.28 µmol, 1 *eq*), cyclopropanecarboxamide (475.05 mg, 5.58 mmol, 25 *eq*), cesium carbonate (218.25 mg, 669.84 µmol, 3 *eq*), and Xantphos (12.92 mg, 22.33 µmol, 0.1 *eq*) were dissolved in dioxane (5 mL). After replacing with nitrogen three times, Pd₂(dba)₃ (20.45 mg, 22.33 µmol, 0.1 *eq*) was added. The mixture was stirred at 120°C for 4 hours. The reaction mixture was added with water (5 mL) and extracted with ethyl acetate (10 mL * 3). The organic phase was dried over anhydrous sodium sulfate, filtered, and the filtrate was concentrated under reduced pressure to obtain a crude product. The crude product was separated by preparative high performance liquid chromatography (chromatographic column: Phenomenex Luna C18 80 * 40 mm * 3 µm; mobile phase: A (water containing 0.04% hydrochloric acid) and B (acetonitrile); gradient: B%: 24% to 46%, 7 minutes) to obtain **hydrochloride of** compound **WX-008.** ¹HNMR (400 MHz, CD₃OD) δ: 7.94 - 7.88 (m, 2H), 7.84 - 7.81 (m, 2H), 7.58 - 7.55 (m, 1H), 7.51 - 7.48 (m, 1H), 7.43 - 7.39 (m, 1H), 6.95 (s, 1H), 3.47 (s, 3H), 1.86 (s, 3H), 1.83 (s, 3H), 1.68 - 1.61 (m, 1H), 1.18 - 1.06 (m, 4H); LCMS *m*/*z* = 497.1 [M+H]⁺.

To synthesize **example 27** listed in Table 5, follow the synthesis steps of **example 8**, replacing **A-1** in step 1 with **A-13.**

**Table 5**

| Example | Compound | Fragment | Structural formula | Spectrum |
|---|---|---|---|---|
| 27 | **Hydrochloride of WX-027** | 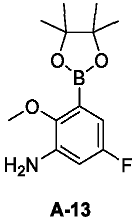 | 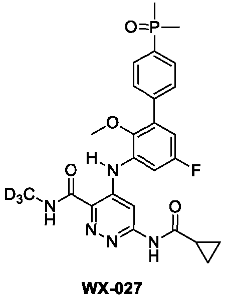 | ¹H NMR (400 MHz, CDCl₃) δ: 11.20 (s, 1H), 8.91 (br s, 1H), 8.28 (s, 1H), 8.07 (s, 1H), 7.77 - 7.65 (m, 4H), 7.16 (dd, *J* = 2.9, 9.2 Hz, 1H), 6.80 (dd, *J* = 2.9, 8.7 Hz, 1H), 3.33 (s, 3H), 1.73 (d, *J* = 12.8 Hz, 6H), 1.66- 1.60 (m, 1H), 1.11 - 1.02 (m, 2H), 0.93 - 0.87 (m, 2H); LCMS *m*/*z* = 537.1 [M+Na]⁺. |
| | | | | Purification method: preparative high performance liquid chromatography (chromatographic column: Phenomenex Luna C18 80 * 40 mm * 3 µm; mobile phase: A (water containing 0.04% hydrochloric acid) and B (acetonitrile); gradient: B%: 24% to 46%, 7 minutes). |

### Example 9

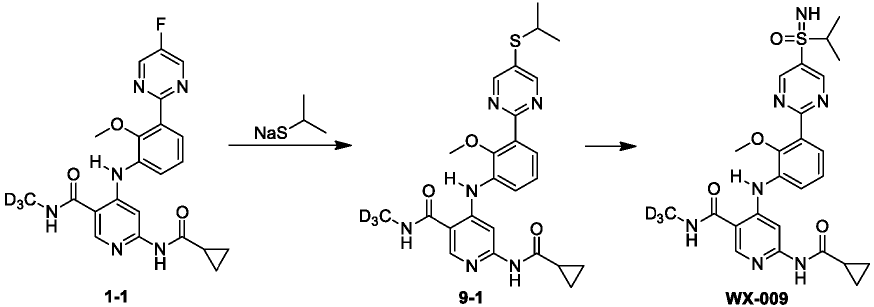

### Step 1: Synthesis of compound 9-1

Compound **1-1** (50 mg, 113.78 µmol, 1 *eq)* was dissolved in DMF (1 mL), and then sodium 2-propanethiolate (44.67 mg, 455.11 µmol, 4 *eq)* was added thereto. The reaction was carried out at 40°C for 2 hours. The reaction mixture was added with water (10 mL) and ethyl acetate (10 mL) for extraction and phase separation. The aqueous phase was then extracted with ethyl acetate (10 mL). The organic phases were combined, dried over anhydrous sodium sulfate, and filtered. The filtrate was concentrated under reduced pressure to obtain a crude product. The crude product was purified by silica gel column chromatography (ethyl acetate: petroleum ether = 10% to 30%) to obtain compound **9-1.** LCMS m/z = 496.2 [M+H]⁺.

### Step 2: Synthesis of trifluoroacetate of compound WX-009

Compound **9-1** (56 mg, 112.99 µmol, 1 *eq)* was added to MeOH (1 mL), and (diacetoxyiodo)benzene (109.18 mg, 338.98 µmol, 3 *eq)* and ammonium acetate (34.84 mg, 451.97 µmol, 4 *eq)* were added thereto. The reaction was carried out at 25°C for 2 hours. The system was added with water (10 mL) and ethyl acetate (10 mL) for extraction and phase separation. The aqueous phase was then extracted with ethyl acetate (10 mL). The organic phases were combined, washed with saturated sodium chloride aqueous solution, dried over anhydrous sodium sulfate, and concentrated to obtain a crude product. The crude product was purified by preparative high performance liquid chromatography (column: Welch Xtimate C18 100 * 40 mm * 3 µm; mobile phase: A (water containing 0.075% trifluoroacetic acid) and B (acetonitrile); gradient: B%: 23 to 63%, 8 minutes) to obtain **trifluoroacetate of** compound **WX-009.** ¹H NMR (400 MHz, CDCl₃) δ: 11.97 - 11.77 (m, 1H), 11.60 - 11.42 (m, 1H), 9.33 (s, 2H), 8.73 - 8.65 (m, 1H), 8.73 -8.65 (m, 1H), 8.01 - 7.93 (m, 1H), 7.67 - 7.62 (m, 1H), 7.46 - 7.40 (m, 1H), 3.90 - 3.83 (m, 3H), 3.52 - 3.41 (m, 3H), 1.93 - 1.83 (m, 1H), 1.52 - 1.38 (m, 6H), 1.13 - 1.07 (m, 2H), 1.05 - 0.95 (m, 2H); LCMS *m*/*z* = 527.2 [M+H]⁺.

### Example 10

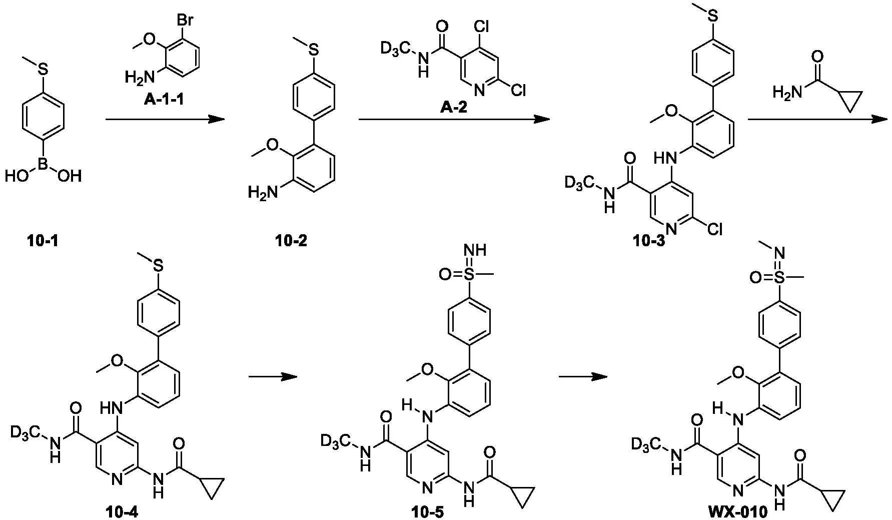

### Step 1: Synthesis of compound 10-2

Compound **10-1** (997.91 mg, 5.94 mmol, 2 *eq),* compound **A-1-1** (600 mg, 2.97 mmol, 1 *eq),* and potassium phosphate tribasic (1.89 g, 8.91 mmol, 3 *eq)* were added to 1,4-dioxane (24 mL). After replacing with nitrogen, Pd(dppf)Cl₂·CH₂Cl₂ (242.51 mg, 296.96 µmol, 0.1 *eq)* was added. The reaction was carried out at 100°C for 3 hours. The reaction mixture was added with water (40 mL) and ethyl acetate (40 mL) for extraction and phase separation. The aqueous phase was then extracted with ethyl acetate (40 mL). The organic phases were combined, washed twice with saturated sodium chloride aqueous solution, dried over anhydrous sodium sulfate, and concentrated to obtain a crude product. The crude product was purified by silica gel column chromatography (ethyl acetate/petroleum ether = 0 to 20%) to obtain compound **10-2.** LCMS *m*/*z* = 246.1 [M+H]⁺.

### Step 2: Synthesis of compound 10-3

Compound **10-2** (300 mg, 1.22 mmol, 1 *eq)* and compound **A-2** (279.86 mg, 1.35 mmol, 1.1 *eq)* were added to THF (6 mL). After cooling to 0°C, the mixture was added with LiHMDS (1 M, 4.89 mL, 4 *eq).* The reaction was carried out at 20°C for 2 hours. After the reaction was completed, the reaction was quenched by methanol. The reaction mixture was added with water (20 mL) and ethyl acetate (20 mL) for extraction and phase separation. The aqueous phase was then extracted with ethyl acetate (20 mL). The organic phases were combined, washed with saturated sodium chloride aqueous solution, dried over anhydrous sodium sulfate, and concentrated to obtain compound **10-3.** LCMS *m*/*z* = 417.1 [M+H]⁺.

### Step 3: Synthesis of compound 10-4

Compound **10-3** (300 mg, 719.53 µmol, 1 *eq)* and cyclopropanecarboxamide (1.53 g, 17.99 mmol, 25 *eq)* were added to 1,4-dioxane (2 mL), NMP (0.4 mL), and water (0.4 mL). Cesium carbonate (703.31 mg, 2.16 mmol, 3 *eq)* and Xantphos (62.45 mg, 107.93 µmol, 0.15 *eq)* were then added thereto. After replacing with nitrogen, Pd₂(dba)₃ (98.83 mg, 107.93 µmol, 0.15 *eq)* was added. After replacing with nitrogen, the reaction was carried out at 120°C for 3 hours. After the reaction was completed, the reaction mixture was added with water (10 mL) and ethyl acetate (10 mL) for extraction and phase separation. The aqueous phase was then extracted with ethyl acetate (10 mL). The organic phases were combined, washed twice with saturated sodium chloride aqueous solution, dried over anhydrous sodium sulfate, and concentrated to obtain a crude product. The crude product was purified by silica gel column chromatography (ethyl acetate/petroleum ether = 0 to 100%) to obtain compound **10-4.** LCMS *m*/*z* = 466.1 [M+H]⁺.

### Step 4: Synthesis of compound 10-5

Compound **10-4** (200 mg, 429.57 µmol, 1 *eq)* was added to methanol (4 mL), and then (diacetoxyiodo)benzene (415.09 mg, 1.29 mmol, 3 *eq)* and ammonium acetate (132.45 mg, 1.72 mmol, 4 *eq)* were added thereto. The reaction was carried out at 25°C for 3 hours. The system was added with water (10 mL) and ethyl acetate (10 mL) for extraction and phase separation. The aqueous phase was then extracted with ethyl acetate (10 mL). The organic phases were combined, washed with saturated sodium chloride aqueous solution, dried over anhydrous sodium sulfate, and concentrated to obtain a crude product. The crude product was purified by preparative thin-layer chromatography on a silica gel plate (ethyl acetate: dichloromethane: methanol = 5:5:1) to obtain compound **10-5.** ¹H NMR (400 MHz, CDCl₃) δ: 10.47 (s, 1H), 8.27 (s, 1H), 8.18 (s, 2H), 8.06 (d, *J=* 8.0 Hz, 2H), 7.81 (d, *J=* 8.0 Hz, 2H), 7.56 (d, *J* = 8.0 Hz, 1H), 7.26 (t, *J* = 8.0 Hz, 1H), 7.11 - 7.08 (m, 1H), 6.20 (s, 1H), 3.45 (s, 3H), 3.18 (s, 3H), 2.76 (s, 1H), 1.57 - 1.52 (m, 1H), 1.11 - 1.08 (m, 2H), 0.92 - 0.87 (m, 2H); LCMS *m*/*z* = 497.2 [M+H]⁺.

### Step 5: Synthesis of hydrochloride of compound WX-010

Compound **10-5** (40 mg, 80.55 µmol, 1 *eq)* was dissolved in 1,4-dioxane (3 mL), and then Cu(OAc)₂ (21.95 mg, 120.82 µmol, 1.5 *eq)* and pyridine (15.29 mg, 193.32 µmol, 15.60 µL, 2.4 *eq)* were added thereto. The mixture was stirred in an open container for 10 minutes, and then added with methylboronic acid (9.64 mg, 161.10 µmol, 2 *eq).* The reaction was carried out at 100°C for 12 hours. After the reaction was completed, the reaction mixture was added with water (10 mL) and ethyl acetate (10 mL) for extraction and phase separation. The aqueous phase was then extracted with ethyl acetate (10 mL). The organic phases were combined, washed with saturated sodium chloride aqueous solution, dried over anhydrous sodium sulfate, and concentrated to obtain a crude product. The crude product was separated by preparative high performance liquid chromatography (chromatographic column: Phenomenex Luna C18 80 * 30 mm * 3 µm; mobile phase: A (water containing 0.04% hydrochloric acid) and B (acetonitrile); gradient: B%: 10% to 30%, 8 minutes) to obtain **hydrochloride of** compound **WX-010.** ¹H NMR (400 MHz, CDCl₃) δ: 10.47 (s, 1H), 8.27 - 8.24 (m, 2H), 8.19 (s, 1H), 7.94 (d, *J* = 8.0 Hz, 2H), 7.82 (d, *J=* 8.0 Hz, 2H), 7.57 - 7.55 (m, 1H), 7.28 - 7.24 (m, 1H), 7.12 - 7.10 (m, 1H), 6.19 (s, 1H), 3.47 (s, 3H), 3.14 (s, 3H), 2.72 (s, 3H), 1.56 - 1.53 (m, 1H), 1.12 - 1.08 (m, 2H), 0.92 - 0.87 (m, 2H); LCMS *m*/*z* = 511.2 [M+H]⁺.

### Example 11

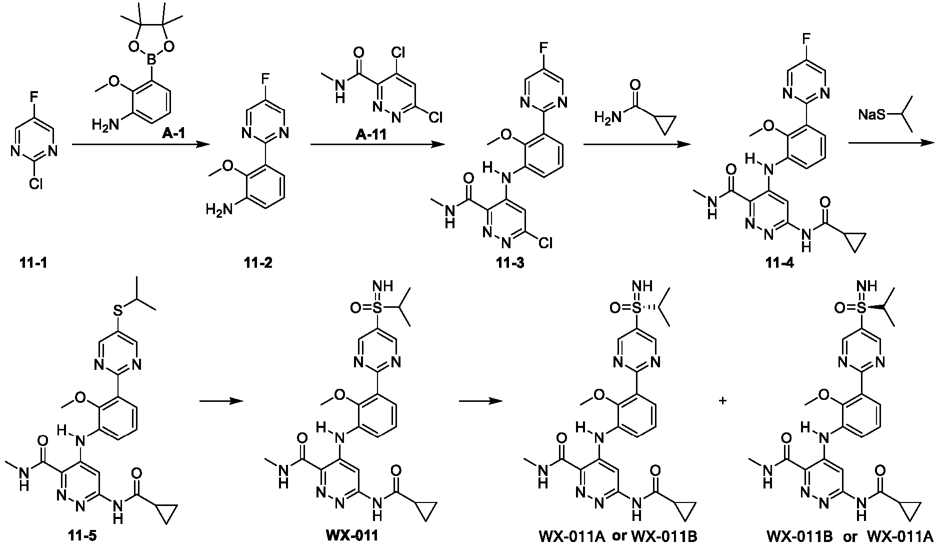

### Step 1: Synthesis of compound 11-2

Compound **11-1** (3.83 g, 28.90 mmol, 3.58 mL, 0.9 *eq),* compound **A-1** (8 g, 32.11 mmol, 1 *eq),* and potassium phosphate tribasic (20.45 g, 96.34 mmol, 3 *eq)* were added to 1,4-dioxane (120 mL) and water (24 mL). After replacing with nitrogen, Pd(dppf)Cl₂·CH₂Cl₂ (1.57 g, 1.93 mmol, 0.06 *eq)* was added. The reaction was carried out at 100°C for 6 hours. The reaction mixture was added with water (100 mL) and ethyl acetate (100 mL) for extraction and phase separation. The aqueous phase was then extracted with ethyl acetate (100 mL). The organic phases were combined, washed with saturated sodium chloride aqueous solution, dried over anhydrous sodium sulfate, and concentrated to obtain a crude product. The crude product was purified by silica gel column chromatography (ethyl acetate/petroleum ether = 0 to 20%) to obtain compound **11-2.** LCMS *m*/*z* = 220.0 [M+H]⁺.

### Step 2: Synthesis of compound 11-3

Compound **11-2** (3.0 g, 13.69 mmol, 1 *eq)* and compound **A-11** (3.67 g, 17.79 mmol, 1.3 *eq)* were added to THF (30 mL). After cooling to -60°C, the mixture was added with LiHMDS (1 M, 41.06 mL, 3 *eq).* The reaction was carried out at 20°C for 2 hours. After the reaction was completed, the reaction was quenched by methanol (10 mL). The reaction mixture was added with water (50 mL) and ethyl acetate (50 mL) for extraction and phase separation. The aqueous phase was then extracted with ethyl acetate (50 mL). The organic phases were combined, washed with saturated sodium chloride aqueous solution, dried over anhydrous sodium sulfate, and concentrated to obtain compound **11-3.** LCMS *m*/*z* = 389.0 [M+H]⁺.

### Step 3: Synthesis of compound 11-4

Compound **11-3** (4.2 g, 10.80 mmol, 1 *eq)* and cyclopropanecarboxamide (9.19 g, 108.03 mmol, 10 *eq)* were added to NMP (80 mL). Cesium carbonate (10.56 g, 32.41 mmol, *3 eq)* and Xantphos (937.62 mg, 1.62 mmol, 0.15 *eq)* were then added thereto. After replacing with nitrogen, Pd₂(dba)₃ (1.48 g, 1.62 mmol, 0.15 *eq)* was added. After replacing with nitrogen, the reaction was carried out at 140°C for 2 hours. After the reaction was completed, the reaction mixture was added with water (100 mL) and ethyl acetate (100 mL) for extraction and phase separation. The aqueous phase was then extracted with ethyl acetate (100 mL). The organic phases were combined, washed twice with saturated sodium chloride aqueous solution, dried over anhydrous sodium sulfate, and concentrated to obtain a crude product. The crude product was purified by silica gel column chromatography (methanol/dichloromethane = 0 to 10%) to obtain compound **11-4.** LCMS *m*/*z* = 438.1 [M+H]⁺.

### Step 4: Synthesis of compound 11-5

Compound **11-4** (300 mg, 685.83 µmol, 1 *eq)* was dissolved in DMF (5 mL), and then sodium 2-propanethiolate (100.96 mg, 1.03 mmol, 1.5 *eq)* was added thereto. The reaction was carried out at 60°C for 2 hours. The reaction mixture was added with water (10 mL) and ethyl acetate (10 mL) for extraction and phase separation. The aqueous phase was then extracted with ethyl acetate (10 mL). The organic phases were combined, dried over anhydrous sodium sulfate, and filtered. The filtrate was concentrated under reduced pressure to obtain compound **11-5.** LCMS *m*/*z* = 494.1 [M+H]⁺.

### Step 5: Synthesis of compound WX-011

Compound **11-5** (400 mg, 810.41 µmol, 1 *eq)* was added to MeOH (30 mL), and then (diacetoxyiodo)benzene (783.08 mg, 2.43 mmol, 3 *eq)* and ammonium acetate (249.86 mg, 3.24 mmol, 4 *eq)* were added thereto. The reaction was carried out at 25°C for 2 hours. The system was added with water (10 mL) and ethyl acetate (10 mL) for extraction and phase separation. The aqueous phase was then extracted with ethyl acetate (10 mL). The organic phases were combined, washed with saturated sodium chloride aqueous solution, dried over anhydrous sodium sulfate, and concentrated to obtain a crude product. The crude product was separated by preparative high performance liquid chromatography (chromatographic column: Phenomenex Luna C18 80 * 30 mm * 3 µm; mobile phase: A (water containing 0.04% hydrochloric acid) and B (acetonitrile); gradient: B%: 45% to 45%, 8 minutes). The obtained solution was concentrated under vacuum at 40°C to remove acetonitrile, then adjusted to alkalinity (pH = 8) with saturated sodium bicarbonate, and extracted with dichloromethane (30 mL * 3). The organic phases were combined, washed with saturated brine (50 mL), dried over anhydrous sodium sulfate, and filtered. The filtrate was concentrated under vacuum and dried to obtain compound **WX-011.** LCMS *m*/*z* = 525.2 [M+H]⁺.

### Step 6: Synthesis of compounds WX-011A and WX-011B

**WX-011** was subjected to resolution by SFC (column: DAICEL CHIRALCEL OD-H (250 mm * 30 mm, 5 µm); mobile phase: A (CO₂) and B (ethanol containing 0.1% ammonia water); gradient: B% = 65% to 65%, 15 minutes) to obtain **WX-011A** and **WX-011B.**

**WX-011A:** ¹H NMR (400 MHz, CDCl₃) δ: 11.07 (s, 1H), 10.05 (s, 1H), 9.28 (s, 2H), 8.25 (s, 1H), 8.13 - 8.12 (m, 1H), 7.80 (d, *J* = 8.0 Hz, 1H), 7.64 (d, *J* = 8.0 Hz, 1H), 7.33 (t, *J* = 8.0 Hz, 1H), 3.83 (s, 3H), 3.38 - 3.33 (m, 1H), 3.03 (s, 3H), 1.91 - 1.88 (m, 1H), 1.41 (t, *J* = 8.0 Hz, 6H), 1.10 - 1.08 (m, 2H), 0.92 - 0.89 (m, 2H); LCMS *m*/*z* = 525.2 [M+H]⁺. SFC detection method: (column: Chiralpak AD-3, 3 µm, 0.46 cm id × 5 cm L; mobile phase: A(CO₂) and B (EtOH containing 0.1% isopropylamine); gradient: B% = 50 to 50%, 5 minutes; flow rate: 4.0 mL/min; wavelength: 220 nm; pressure: 100 bar), with Rt of 2.538 min, and a chiral isomer excess of 100%.

**WX-011B:** ¹H NMR (400 MHz, CDCl₃) δ: 11.07 (s, 1H), 9.88 (s, 1H), 9.28 (s, 2H), 8.25 (s, 1H), 8.10 - 8.09 (m, 1H), 7.80 (d, *J* = 8.0 Hz, 1H), 7.64 (d, *J* = 8.0 Hz, 1H), 7.33 (t, *J* = 8.0 Hz, 1H), 3.84 (s, 3H), 3.36 - 3.33 (m, 1H), 3.03 (s, 3H), 1.85 - 1.82 (m, 1H), 1.41 (t, *J* = 8.0 Hz, 6H), 1.11 - 1.09 (m, 2H), 0.93 - 0.91 (m, 2H); LCMS *m*/*z* = 525.2 [M+H]⁺. SFC detection method: (column: Chiralpak AD-3, 3 µm, 0.46 cm id × 5 cm L; mobile phase: A(CO₂) and B (EtOH containing 0.1% isopropylamine); gradient: B% = 50 to 50%, 5 minutes; flow rate: 4.0 mL/min; wavelength: 220 nm; pressure: 100 bar), with Rt of 3.011 min, and a chiral isomer excess of 98.60%.

### Step 7: Synthesis of hydrochloride of compound WX-011A

Compound **WX-011A** (790 mg, 1.51 mmol, 1 *eq)* was dissolved in MeCN (6 mL), and HCl/EtOAc (4 M, 1.13 mL, 3 *eq)* was added thereto. The mixture was stirred until clear. Water (50 mL) was then added to the system to obtain **hydrochloride of** compound **WX-011A.** ¹H NMR (400 MHz, CD₃OD) δ: 9.42 (s, 2H), 8.08 (dd, *J* = 1.6, 8.0 Hz, 1H), 7.78 (dd, *J* = 8.0 Hz, 1H), 7.51 (t, *J* = 8.0 Hz, 1H), 6.95 (s, 1H), 3.88 (s, 3H), 3.86 - 3.77 (m, 1H), 3.02 (s, 3H), 1.91 - 1.78 (m, 1H), 1.47 (dd, *J* = 6.8, 12.4 Hz, 6H), 1.19 - 1.04 (m, 4H); LCMS *m*/*z* = 525.0 [M+H]⁺.

### Example 12

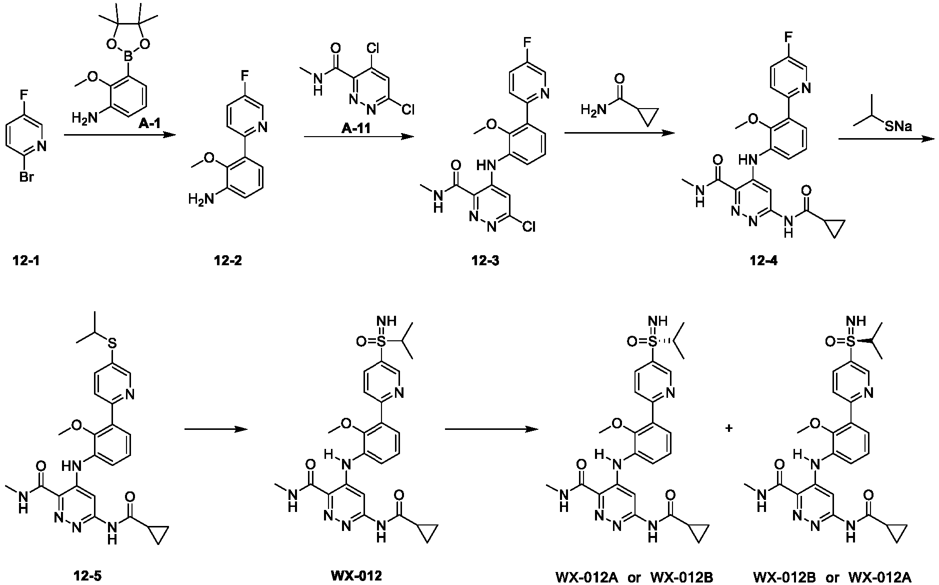

### Step 1: Synthesis of compound 12-2

Compound **12-1** (3 g, 17.05 mmol, 4.97 mL, 1 *eq),* **A-1** (5.10 g, 20.46 mmol, 1.2 *eq),* and potassium carbonate (4.71 g, 34.09 mmol, 2 *eq)* were dissolved in 1,4-dioxane (50 mL) and water (10 mL). After replacing with nitrogen three times, Pd(dppf)Cl₂ (1.25 g, 1.70 mmol, 0.1 *eq)* was added thereto. After replacing with nitrogen once more, the mixture was heated to 80°C and stirred for 4 hours. The reaction mixture was added with water (50 mL) and extracted with ethyl acetate (50 mL * 2). The organic phases were combined and washed with saturated brine (30 mL * 2). The organic phases were dried over anhydrous sodium sulfate, filtered, and the filtrate was concentrated under vacuum to obtain a crude product. The crude product was purified by silica gel column chromatography (ethyl acetate/petroleum ether = 0 to 30%) to obtain compound **12-2.** LCMS *m*/*z* = 219.1 [M+1]⁺.

### Step 2: Synthesis of compound 12-3

Under a nitrogen atmosphere, compound **12-2** (2 g, 9.16 mmol, 1 *eq)* was added to THF (5 mL). **A-11** (1.89 g, 9.16 mmol, 1 *eq)* was added thereto, and the mixture was stirred at 25°C (room temperature) until dissolved. LiHMDS (1 M, 22.91 mL, 2.5 *eq)* was slowly added dropwise thereto, and the mixture was stirred at 25°C (room temperature) for another 1 hour. The reaction mixture was added with saturated ammonium chloride aqueous solution (20 mL) and water (50 mL), and extracted with ethyl acetate (50 mL * 2). The organic phases were combined and washed with saturated brine (50 mL * 2). The organic phases were dried over anhydrous sodium sulfate, filtered, and the filtrate was concentrated under vacuum to obtain a crude product. The crude product was purified by silica gel column chromatography (ethyl acetate/petroleum ether = 0 to 50%) to obtain compound **12-3.** LCMS *m*/*z* = 387.9 [M+1]⁺.

### Step 3: Synthesis of compound 12-4

Compound **12-3** (800 mg, 2.06 mmol, 1 *eq)* and cyclopropanecarboxamide (1.76 g, 20.63 mmol, 10 *eq)* were added to 1,4-dioxane (3 mL) and NMP (0.3 mL). Cesium carbonate (2.69 g, 8.25 mmol, 4 *eq)* and Xantphos (358.10 mg, 618.88 µmol, 0.15 *eq)* were then added thereto. After replacing with nitrogen three times, Pd₂(dba)₃ (320.30 mg, 309.44 µmol, 0.15 *eq)* was added. The reaction was carried out at 130°C for 18 hours. After cooling to room temperature, the reaction mixture was added with water (30 mL) and ethyl acetate (30 mL) for extraction and phase separation. The aqueous phase was then extracted with ethyl acetate (30 mL). The organic phases were combined, washed with saturated sodium chloride aqueous solution, dried over anhydrous sodium sulfate, and concentrated to obtain a crude product. The crude product was purified by silica gel column chromatography (ethyl acetate/petroleum ether = 0 to 50%) to obtain compound **12-4.** LCMS *m*/*z* = 437.3 [M+1]⁺.

### Step 4: Synthesis of compound 12-5

Compound **12-4** (600 mg, 1.37 mmol, 1 *eq)* was dissolved in DMF (1 mL), and then sodium 2-propanethiolate (539.69 mg, 5.50 mmol, 4 *eq)* was added thereto. The reaction was carried out at 40°C for 2 hours. The reaction mixture was added with water (10 mL) and ethyl acetate (10 mL) for extraction and phase separation. The aqueous phase was then extracted with ethyl acetate (10 mL). The organic phases were combined, dried over anhydrous sodium sulfate, and filtered. The filtrate was concentrated under reduced pressure. The crude product was purified by silica gel column chromatography (ethyl acetate/petroleum ether = 0 to 50%) to obtain compound **12-5.** The crude product was directly used in the next step without purification. LCMS *m*/*z* = 493.2 [M+1⁺.

### Step 5: Synthesis of compound WX-012

**12-5** (350 mg, 710.53 µmol, 1 *eq)* was added to MeOH (20 mL), and (diacetoxyiodo)benzene (686.57 mg, 2.13 mmol, 3 *eq)* and ammonium acetate (219.08 mg, 2.84 mmol, 4 *eq)* were added thereto. The reaction was carried out at 25°C for 2 hours. The reaction mixture was added with water (20 mL) and ethyl acetate (20 mL) for extraction and phase separation. The aqueous phase was then extracted with ethyl acetate (20 mL). The organic phases were combined, washed with saturated sodium chloride aqueous solution (20 mL), dried over anhydrous sodium sulfate, and concentrated to obtain a crude product. The crude product was purified by silica gel column chromatography (methanol/dichloromethane = 0 to 3%) to obtain compound **WX-012.** ¹H NMR (400 MHz, CDCl₃) δ: 11.16 (s, 1H), 9.21 (d, *J* = 2.4 Hz, 1H), 8.53 (br dd, *J* = 2.5, 6.5 Hz, 1H), 8.29 - 8.14 (m, 4H), 7.69 (dd, *J* = 1.5, 7.8 Hz, 1H), 7.57 (dd, *J* = 1.4, 7.9 Hz, 1H), 7.38 - 7.32 (m, 1H), 3.59 - 3.52 (m, 1H), 3.56 (s, 3H), 3.39 - 3.29 (m, 1H), 3.07 (d, *J* = 5.0 Hz, 3H), 2.82 (br s, 1H), 1.69 - 1.61 (m, 1H), 1.39 (dd, *J* = 6.8, 13.3 Hz, 6H), 1.18 - 1.07 (m, 2H), 1.00 - 0.93 (m, 2H); LCMS *m*/*z* = 524.1 [M+1]⁺.

### Step 6: Synthesis of compounds WX-012A and WX-012B

WX-012 was subjected to resolution by SFC (column: DAICEL CHIRALPAK AD (250 mm * 30 mm, 10 µm); mobile phase: A (CO₂) and B (isopropanol containing 0.1% ammonia water); gradient: B% = 50% to 50%, 15 minutes) to obtain **WX-012A** and **WX-012B.**

**WX-012A:** ¹H NMR (400 MHz, CDCl₃) δ: 11.16 (s, 1H), 9.22 (d, 2.4 Hz, 1H), 8.54 (br s, 1H), 8.30 - 8.15 (m, 4H), 7.70 (dd, *J* = 1.5, 7.8 Hz, 1H), 7.58 (dd, *J* = 1.5, 8.0 Hz, 1H), 7.42 - 7.32 (m, 1H), 3.57 (s, 3H), 3.40 - 3.30 (m, 1H), 3.08 (d, *J* = 5.2 Hz, 3H), 2.84 (br s, 1H), 1.70 - 1.62 (m, 1H), 1.40 (dd, *J* = 6.8, 13.3 Hz, 6H), 1.17 - 1.12 (m, 2H), 1.02 - 0.95 (m, 2H); LCMS *m*/*z* = 524.0 [M+H]⁺. SFC detection method: (column: Chiralpak AD-3, 3 µm, 0.46 cm id × 15 cm L; mobile phase: A (CO₂) and B (isopropanol containing 0.05% diethylamine); gradient: B% = 40%, 8 minutes; flow rate: 2.5 mL/min; wavelength: 220 nm; pressure: 100 bar), with Rt of 4.941 min, and a chiral isomer excess of 99.66%.

**WX-012B:** ¹H NMR (400 MHz, CDCl₃) δ: 11.15 (s, 1H), 9.22 (d, *J* = 2.4 Hz, 1H), 8.61 (s, 1H), 8.28 - 8.19 (m, 2H), 8.21 - 8.13 (m, 2H), 7.70 (dd, *J* = 1.4, 7.7 Hz, 1H), 7.58 (dd, *J* = 1.6, 7.9 Hz, 1H), 7.36 (t, *J* = 7.9 Hz, 1H), 3.57 (s, 3H), 3.39 - 3.29 (m, 1H), 3.08 (d, *J* = 5.0 Hz, 3H), 2.84 (br s, 1H), 1.66 (br d, *J* = 4.0 Hz, 1H), 1.40 (dd, *J* = 6.8, 13.3 Hz, 6H), 1.17 - 1.11 (m, 2H), 1.01 - 0.94 (m, 2H); LCMS *m*/*z* = 524.0 [M+H]⁺. SFC detection method: (column: Chiralpak AD-3, 3 µm, 0.46 cm id × 15 cm L; mobile phase: A (CO₂) and B (Isopropanol containing 0.05% diethylamine); gradient: B% = 40%, 8 minutes; flow rate: 2.5 mL/min; wavelength: 220 nm; pressure: 100 bar), with Rt of 6.054 min, and a chiral isomer excess of 98.66%.

### Example 13

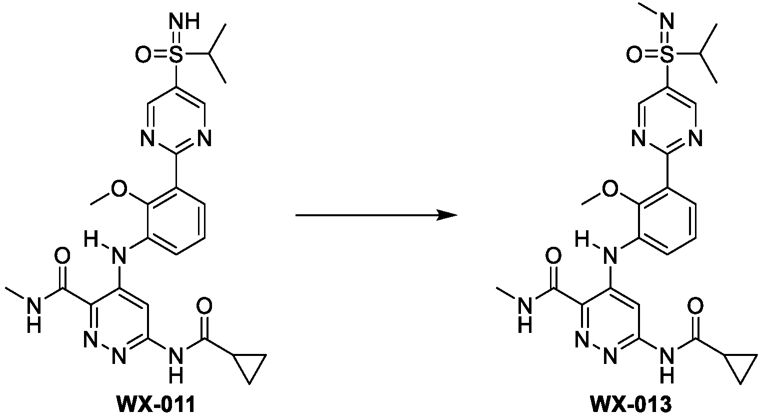

### Step 1: Synthesis of compound WX-013

Compound **WX-011** (20 mg, 38.12 µmol, 1 *eq)* was added to 1,4-dioxane (2 mL), and then Cu(OAc)₂ (20.77 mg, 114.37 µmol, 3 *eq)* and pyridine (7.24 mg, 91.50 µmol, 7.39 µL, 2.4 *eq)* were added thereto. The mixture was stirred in an open container for 10 minutes, and then added with methylboronic acid (6.85 mg, 114.37 µmol, 3 *eq).* The reaction was carried out at 100°C for 3 hours. The system was added with water (10 mL) and ethyl acetate (10 mL) for extraction and phase separation. The aqueous phase was then extracted with ethyl acetate (10 mL). The organic phases were combined, washed with saturated sodium chloride aqueous solution, dried over anhydrous sodium sulfate, and concentrated to obtain a crude product. The crude product was separated by preparative high performance liquid chromatography (chromatographic column: Phenomenex Luna C18 80 * 30 mm * 3 µm; mobile phase: A (water containing 0.04% hydrochloric acid) and B (acetonitrile); gradient: B%: 15% to 30%, 8 minutes) to obtain a crude product. The crude product was further purified by preparative thin-layer chromatography on a silica gel plate (ethyl acetate: dichloromethane: methanol = 5:5:1) to obtain compound **WX-013.** ¹H NMR (400 MHz, CD₃OD) δ: 9.31 (s, 2H), 8.10 - 8.06 (m, 1H), 7.79 - 7.73 (m,1H), 7.50 (t, *J* = 8.0 Hz, 2H), 3.83 (s, 3H), 3.03 (s, 3H), 2.92 - 2.90 (m, 1H), 2.81 (s, 3H), 1.83 - 1.82 (m, 1H), 1.50 (d, *J* = 8.0 Hz, 3H), 1.40 (d, *J* = 8.0 Hz, 3H), 1.14 - 1.08 (m, 4H); LCMS *m*/*z* = 539.2 [M+H]⁺.

To synthesize **example 14** listed in Table 6, follow the synthesis steps of **example 13**, replacing **WX-011** in step 1 with **WX-012.**

**Table 6**

| Example | Compound | Structural formula | Spectrum |
|---|---|---|---|
| **14** | **Trifluoroacetate of WX-014** | 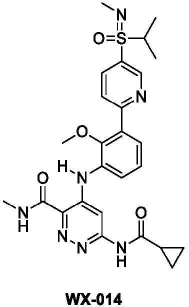 | ¹H NMR (400 MHz, CDCl₃) *δ*: 13.31 (br s, 1H), 11.90 (s, 1H), 9.21 (s, 1H), 8.58 - 8.39 (m, 2H), 8.31 (d, *J* = 8.5 Hz, 1H), 7.92 (dd, *J* = 1.4, 7.9 Hz, 1H), 7.88 - 7.81 (m, 1H), 7.58 (dd, *J* = 1.3, 7.8 Hz, 1H), 7.49 - 7.42 (m, 1H), 4.31 - 4.19 (m, 1H), 3.60 (s, 3H), 3.08 (d, *J* = 5.3 Hz, 3H), 2.82 (s, 3H), 2.11 - 2.01 (m, 1H), 1.62 (d, *J* = 6.4 Hz, 3H), 1.33 (d, *J* = 6.4 Hz, 3H), 1.15 - 1.09 (m, 2H), 1.08 - 1.02 (m, 2H); LCMS *m*/*z* = 538.1 [M+1]⁺. |
| | | | Purification method: preparative high performance liquid chromatography (chromatographic column: Welch Xtimate C18 100 * 40 mm * 3 µm; mobile phase: A (water containing 0.04% trifluoroacetic acid) and B (acetonitrile); gradient: B%: 13% to 43%, 8 minutes). |

### Example 15

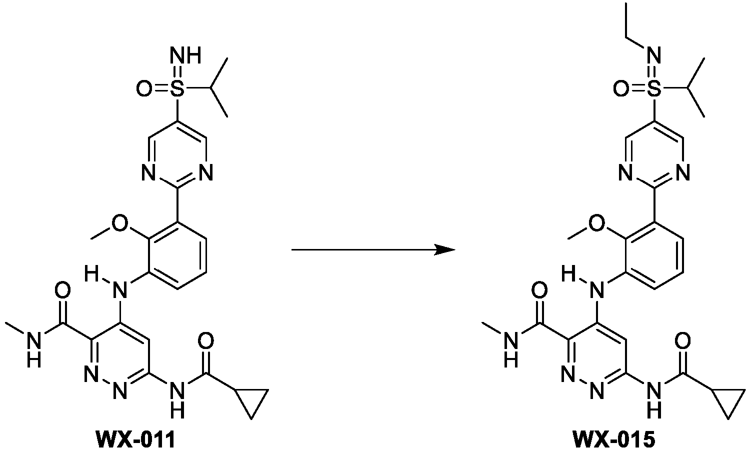

### Step 1: Synthesis of hydrochloride of compound WX-015

Compound **WX-011** (100 mg, 190.62 µmol, 1 *eq)* was added to 1,4-dioxane (2 mL), and then Cu(OAc)₂ (103.87 mg, 571.87 µmol, 3 *eq)* and pyridine (36.19 mg, 457.50 µmol, 36.93 µL, 2.4 *eq*) were added thereto. The mixture was stirred in an open container for 10 minutes, and then added with ethylboronic acid (21.13 mg, 285.94 µmol, 1.5 *eq).* The reaction was carried out at 100°C for 4 hours. The system was added with water (10 mL) and ethyl acetate (10 mL) for extraction and phase separation. The aqueous phase was then extracted with ethyl acetate (10 mL). The organic phases were combined, washed with saturated sodium chloride aqueous solution, dried over anhydrous sodium sulfate, and concentrated to obtain a crude product. The crude product was separated by preparative high performance liquid chromatography (chromatographic column: Phenomenex Luna C18 80 * 30 mm * 3 µm; mobile phase: A (water containing 0.04% hydrochloric acid) and B (acetonitrile); gradient: B%: 20% to 40%, 8 minutes) to obtain **hydrochloride of** compound **WX-015.** ¹H NMR (400 MHz, CDCl₃) δ: 11.07 (s, 1H), 9.16 (s, 2H), 9.04 (s, 1H), 8.21 (s, 1H), 8.14 - 8.12 (m, 1H), 7.84 (d, *J* = 8.0 Hz, 1H), 7.64 (d, *J* = 8.0 Hz, 1H), 7.36 (t, *J* = 8.0 Hz, 1H), 3.86 (s, 3H), 3.48 - 3.14 (m, 2H), 3.03 (s, 3H), 1.57 - 1.54 (m, 1H), 1.44 (d, *J* = 8.0 Hz, 3H), 1.34 (d, *J* = 8.0 Hz, 3H), 1.26 - 1.24 (m, 3H), 1.10 - 0.93 (m, 4H); LCMS *m*/*z* = 553.2 [M+H]⁺.

To synthesize **example 16** listed in Table 7, follow the synthesis steps of **example 15,** replacing **WX-011** in step 1 with **WX-012.**

**Table 7**

| Example | Compound | Structural formula | Spectrum |
|---|---|---|---|
| **16** | **Hydrochloride of WX-016** | 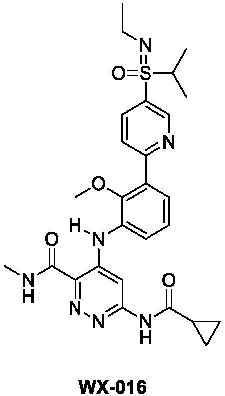 | LCMS *m*/*z* = 552.2 [M+H]⁺. |
| | | | Purification method: preparative high performance liquid chromatography (chromatographic column: Xtimate C18 150 * 40 mm * 5 µm; mobile phase: A (water containing 0.04% hydrochloric acid) and B (acetonitrile); gradient: B%: 20% to 40%, 10 minutes). |

### Example 17

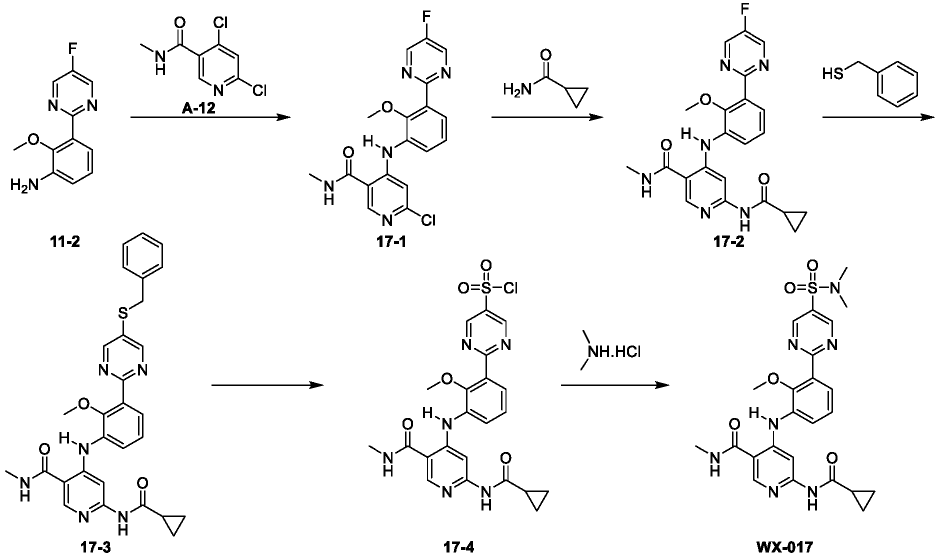

### Step 1: Synthesis of compound 17-1

Compound **11-2** (2.0 g, 9.12 mmol, 1 *eq)* and compound **A-12** (2.06 g, 10.04 mmol, 1.1 *eq)* were added to THF (20 mL). After cooling to -60°C, the mixture was added with LiHMDS (1 M, 27.37 mL, 3 *eq).* The reaction was carried out at 20°C for 2 hours. After the reaction was completed, the reaction was quenched by methanol (10 mL). The reaction mixture was added with water (50 mL) and ethyl acetate (50 mL) for extraction and phase separation. The aqueous phase was then extracted with ethyl acetate (50 mL). The organic phases were combined, washed with saturated sodium chloride aqueous solution, dried over anhydrous sodium sulfate, and concentrated to obtain compound **17-1.** LCMS *m*/*z* = 388.0 [M+H]⁺.

### Step 2: Synthesis of compound 17-2

Compound **17-1** (0.52 g, 1.34 mmol, 1 *eq)* and cyclopropanecarboxamide (1.14 g, 13.41 mmol, 10 *eq)* were added to NMP (7 mL). Cesium carbonate (1.31 g, 4.02 mmol, 3 *eq)* and Xantphos (116.38 mg, 201.14 µmol, 0.15 *eq)* were then added thereto. After replacing with nitrogen, Pd₂(dba)₃ (184.19 mg, 201.14 µmol, 0.15 *eq)* was added. After replacing with nitrogen, the reaction was carried out at 140°C for 2 hours. After the reaction was completed, the reaction mixture was added with water (30 mL) and ethyl acetate (30 mL) for extraction and phase separation. The aqueous phase was then extracted with ethyl acetate (30 mL). The organic phases were combined, washed twice with saturated sodium chloride aqueous solution, dried over anhydrous sodium sulfate, and concentrated to obtain a crude product. The crude product was purified by silica gel column chromatography (methanol/dichloromethane = 0 to 10%) to obtain compound **17-2.** LCMS *m*/*z* = 437.1 [M+H]⁺.

### Step 3: Synthesis of compound 17-3

Compound **17-2** (500 mg, 1.15 mmol, 1 *eq)* was dissolved in DMF (5 mL). Cesium carbonate (559.91 mg, 1.72 mmol, 1.5 *eq)* and benzyl mercaptan (284.58 mg, 2.29 mmol, 268.48 µL, 2 *eq)* were added. The mixture was stirred at 40°C for 2 hours. The reaction mixture was added with water (10 mL) and ethyl acetate (10 mL) for extraction and phase separation. The aqueous phase was then extracted with ethyl acetate (10 mL). The organic phases were combined, dried over anhydrous sodium sulfate, and filtered. The filtrate was concentrated under reduced pressure to obtain compound **17-3.** LCMS *m*/*z* = 541.2 [M+H]⁺.

### Step 4: Synthesis of compound 17-4

Compound **17-3** (400 mg, 739.87 µmol, 1 *eq)* was dissolved in acetic acid (4 mL) and water (0.5 mL). The mixture was then cooled to 0°C, and *N*-chlorosuccinimide (395.18 mg, 2.96 mmol, 4 *eq)* was added thereto. The mixture was stirred at 25°C for 4 hours. Sodium sulfate was added to the system for drying, and the system was directly used in the next step. Compound **17-4** was obtained. LCMS *m*/*z* = 517.0 [M+H]⁺.

### Step 5: Synthesis of hydrochloride of compound WX-017

Compound **17-4** (190 mg, 367.54 µmol, 1 *eq)* was added to THF (2 mL). The mixture was then cooled to -60°C, and dimethylamine hydrochloride (1.50 g, 18.38 mmol, 50 *eq)* and triethylamine (5.58 g, 55.13 mmol, 7.67 mL, 150 *eq)* were added. The reaction was carried out at 20°C for 12 hours. The system was added with water (10 mL) and ethyl acetate (10 mL) for extraction and phase separation. The aqueous phase was then extracted with ethyl acetate (10 mL). The organic phases were combined, washed with saturated sodium chloride aqueous solution, dried over anhydrous sodium sulfate, and concentrated to obtain a crude product. The crude product was separated by preparative high performance liquid chromatography (chromatographic column: Phenomenex Luna C18 80 * 30 mm * 3 µm; mobile phase: A (water containing 0.04% hydrochloric acid) and B (acetonitrile); gradient: B%: 17% to 37%, 7 minutes) to obtain **hydrochloride of** compound **WX-017.** ¹H NMR (400 MHz, CDCl₃) δ: 10.35 (s, 1H), 9.17 (s, 2H), 8.27 (s, 2H), 8.12 (s, 1H), 7.72 - 7.68 (m, *J* = 8.0 Hz, 1H), 7.33 (t, *J* = 8.0 Hz, 1H), 6.20 (s, 1H), 3.87 (s, 3H), 3.03 (s, 3H), 2.86 (s, 6H), 1.68 - 1.53 (m, 1H), 1.20 - 1.18 (m, 2H), 0.90 - 0.86 (m, 2H); LCMS *m*/*z* = 526.1 [M+H]⁺.

### Example 18

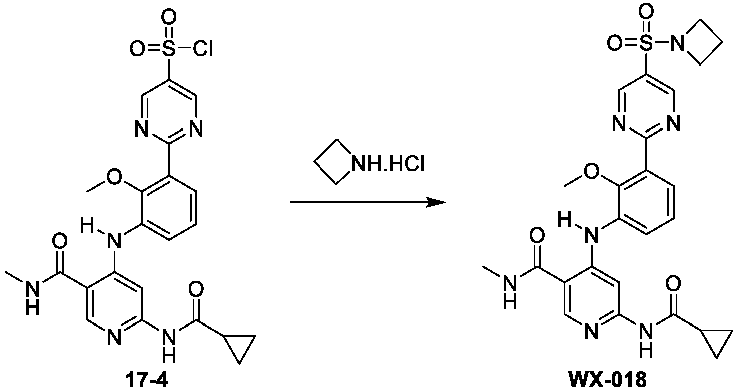

### Step 1: Synthesis of hydrochloride of compound WX-018

Compound **17-4** (190 mg, 367.54 µmol, 1 *eq)* was added to THF (2 mL). The mixture was then cooled to -60°C, and azetidine hydrochloride (1.72 g, 18.38 mmol, 50 *eq)* and triethylamine (5.58 g, 55.13 mmol, 7.67 mL, 150 *eq)* were added. The reaction was carried out at 20°C for 12 hours. The system was added with water (10 mL) and ethyl acetate (10 mL) for extraction and phase separation. The aqueous phase was then extracted with ethyl acetate (10 mL). The organic phases were combined, washed with saturated sodium chloride aqueous solution, dried over anhydrous sodium sulfate, and concentrated to obtain a crude product. The crude product was separated by preparative high performance liquid chromatography (chromatographic column: Phenomenex Luna C18 80 * 30 mm * 3 µm; mobile phase: A (water containing 0.04% hydrochloric acid) and B (acetonitrile); gradient: B%: 17% to 37%, 7 minutes) to obtain **hydrochloride of** compound **WX-018.** ¹H NMR (400 MHz, CDCl₃) δ 10.35 (s, 1H), 9.19 (s, 2H), 8.27 (s, 2H), 8.09 (s, 1H), 7.71 - 7.67 (m, 2H), 7.33 (t, *J* = 8.0 Hz, 1H), 6.22 (s, 1H), 3.93 - 3.91 (m, 4H), 3.85 (s, 3H), 2.98 (s, 3H), 2.22 - 2.19 (s, 2H), 1.51 - 1.49 (m, 1H), 1.05 - 1.03 (m, 2H), 0.90 - 0.86 (m, 2H); LCMS *m*/*z* = 538.2 [M+H]⁺.

### Example 19

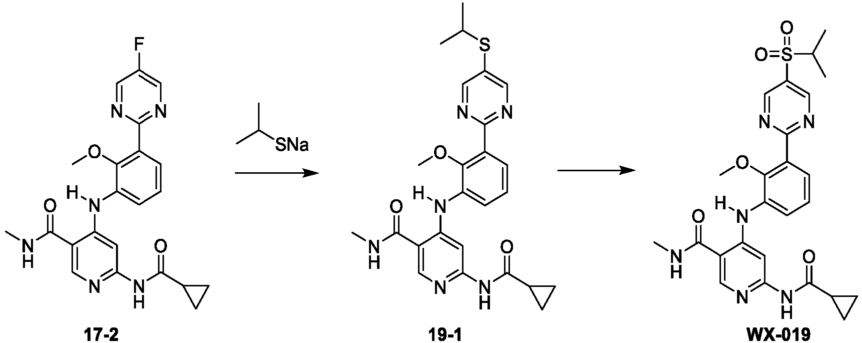

### Step 1: Synthesis of compound 19-1

Compound **17-2** (200 mg, 458.26 µmol, 1 *eq)* was dissolved in DMF (7 mL), and then sodium 2-propanethiolate (179.90 mg, 1.83 mmol, 4 *eq)* was added thereto. The reaction was carried out at 40°C for 16 hours. After cooling to room temperature, the reaction mixture was added with water (10 mL) and ethyl acetate (10 mL) for extraction and phase separation. The aqueous phase was then extracted with ethyl acetate (10 mL). The organic phases were combined, dried over anhydrous sodium sulfate, and filtered. The filtrate was concentrated under reduced pressure to obtain a crude product of compound **19-1.** The crude product was directly used in the next step without purification. ¹H NMR (400 MHz, CDCl₃) δ: 10.30 (s, 1H), 8.85 (s, 2H), 8.36 - 8.25 (m, 2H), 8.12 (s, 1H), 7.63 (dd, *J* = 7.8, 15.6 Hz, 2H), 6.24 (br s, 1H), 3.80 (s, 3H), 3.44 (quin, *J* = 6.7 Hz, 1H), 3.01 (d, *J* = 4.4 Hz, 3H), 1.58 - 1.50 (m, 1H), 1.38 (d, *J* = 6.5 Hz, 6H), 1.13 - 1.05 (m, 2H), 0.92 - 0.82 (m, 2H); LCMS *m*/*z* = 493.1 [M+H]⁺.

### Step 2: Synthesis of compound WX-019

Compound **19-1** (100 mg, 203.01 µmol, 1 *eq)* was dissolved in DCM (2 mL). The mixture was then cooled to 0°C and added with 3-chloroperoxybenzoic acid (52.55 mg, 304.51 µmol, purity of 85%, 1.5 *eq).* The reaction was carried out at 20°C for 16 hours. The system was added with water (10 mL) and ethyl acetate (10 mL) for extraction and phase separation. The aqueous phase was then extracted with ethyl acetate (10 mL). The organic phases were combined, washed with saturated sodium chloride aqueous solution, dried over anhydrous sodium sulfate, and concentrated to obtain a crude product. The crude product was separated by preparative high performance liquid chromatography (chromatographic column: Phenomenex Luna 80 * 40 mm * 3 µm; mobile phase: A (water containing 0.04% hydrochloric acid) and B (acetonitrile); gradient: B%: 5% to 35%, 8 minutes). The obtained solution was concentrated under vacuum at 40°C to remove acetonitrile, then adjusted to alkalinity (pH = 8) with saturated sodium bicarbonate, and extracted with dichloromethane (30 mL * 3). The organic phases were combined, washed with saturated brine (50 mL), dried over anhydrous sodium sulfate, and filtered. The filtrate was concentrated under vacuum and dried to obtain compound **WX-019.** ¹H NMR (400 MHz, CDCl₃) δ: 10.39 (s, 1H), 9.26 (s, 2H), 8.31 - 8.24 (m, 1H), 8.17 - 8.11 (m, 1H), 7.80 - 7.70 (m, 2H), 7.39 - 7.31 (m, 1H), 6.14 - 6.07 (m, 1H), 3.89 (s, 3H), 3.37 - 3.25 (m, 1H), 3.04 (d, *J* = 4.8 Hz, 2H), 1.59 - 1.52 (m, 1H), 1.43 (d, 6H), 1.32 - 1.27 (m, 2H), 1.12 - 1.07 (m, 2H). LCMS *m*/*z* = 525.1 [M+H]⁺.

### Example 20

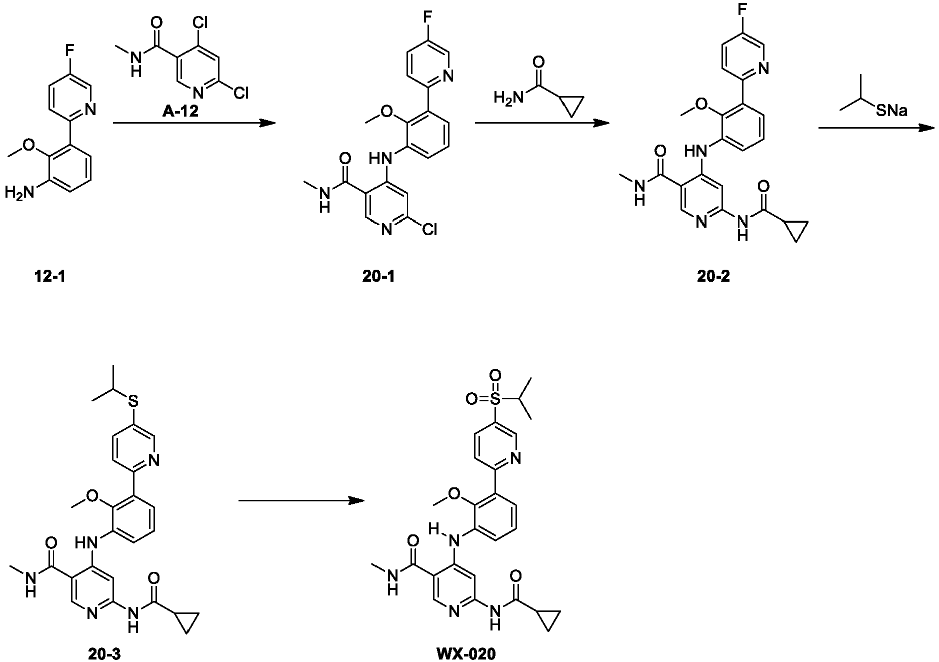

### Step 1: Synthesis of compound 20-1

Under a nitrogen atmosphere, compound **12-1** (1 g, 4.58 mmol, 1.35 *eq)* and compound **A-12** (695.62 mg, 3.39 mmol, 1 *eq)* were dissolved in THF (5 mL). The mixture was stirred at 25°C (room temperature). LiHMDS (1 M, 8.48 mL, 2.5 *eq)* was slowly added dropwise thereto. The mixture was stirred at 25°C (room temperature) for another 1 hour. The reaction mixture was added with saturated ammonium chloride aqueous solution (20 mL) and water (50 mL), and extracted with ethyl acetate (50 mL * 2). The organic phases were combined and washed with saturated brine (50 mL * 2). The organic phases were dried over anhydrous sodium sulfate, filtered, and the filtrate was concentrated under vacuum to obtain a crude product. The crude product was purified by silica gel column chromatography (ethyl acetate/petroleum ether = 0 to 50%) to obtain compound **20-1.** LCMS *m*/*z* = 387.0 [M+1]⁺.

### Step 2: Synthesis of compound 20-2

Compound **20-1** (1.27 g, 3.28 mmol, 1 *eq)* and cyclopropanecarboxamide (2.79 g, 32.83 mmol, 10 *eq)* were added to a mixed solvent of 1,4-dioxane (2 mL) and NMP (0.5 mL). Cesium carbonate (3.21 g, 9.85 mmol, 3 *eq)* and Xantphos (284.97 mg, 492.49 µmol, 0.15 *eq)* were then added thereto. After replacing with nitrogen three times, Pd₂(dba)₃ (450.99 mg, 492.49 µmol, 0.15 *eq)* was added. The reaction was carried out at 130°C for 18 hours. After cooling to room temperature, the reaction mixture was added with water (30 mL) and ethyl acetate (30 mL) for extraction and phase separation. The aqueous phase was then extracted with ethyl acetate (30 mL). The organic phases were combined, washed with saturated sodium chloride aqueous solution, dried over anhydrous sodium sulfate, and concentrated to obtain a crude product. The crude product was purified by silica gel column chromatography (ethyl acetate/petroleum ether = 0 to 50%) to obtain compound **20-2.** LCMS *m*/*z* = 436.1 [M+1]⁺.

### Step 3: Synthesis of compound 20-3

Compound **20-2** (500 mg, 1.15 mmol, 1 *eq)* was dissolved in DMF (5 mL), and then sodium 2-propanethiolate (450.76 mg, 4.59 mmol, 4 *eq)* was added thereto. The reaction was carried out at 40°C for 2 hours. The reaction mixture was added with water (10 mL) and extracted with ethyl acetate (10 mL * 2). The organic phases were combined, dried over anhydrous sodium sulfate, filtered, and the filtrate was concentrated under vacuum. The crude product was purified by silica gel column chromatography (ethyl acetate/petroleum ether = 0 to 50%) to obtain compound **20-3.** LCMS *m*/*z* = 492.1 [M+1]⁺.

### Step 4: Synthesis of hydrochloride of compound WX-020

Compound **20-3** (100.20 mg, 203.83 µmol, 1 *eq)* was dissolved in DCM (10 mL). The mixture was then cooled to 0°C and added with 3-chloroperoxybenzoic acid (82.76 mg, 407.65 µmol, purity of 85%, 2 *eq).* The reaction was carried out at 25°C for 2 hours. After the reaction was completed, the reaction mixture was added with water (30 mL) and ethyl acetate (30 mL) for extraction and phase separation. The aqueous phase was then extracted with ethyl acetate (30 mL). The organic phases were combined, washed with saturated sodium chloride aqueous solution, dried over anhydrous sodium sulfate, and concentrated under vacuum to obtain a crude product. The crude product was separated by preparative high performance liquid chromatography (chromatographic column: Phenomenex C18 150 * 40 mm * 5 µm; mobile phase: A (water containing 0.04% hydrochloric acid) and B (acetonitrile); gradient: B%: 15% to 45%, 10 minutes) to obtain **hydrochloride of** compound **WX-020.** ¹H NMR (400 MHz, DMSO-*d*₆) δ: 11.85 (br s, 1H), 11.03 (s, 1H), 9.12 (d, *J* = 1.6 Hz, 1H), 9.06 (br s, 1H), 8.51 (s, 1H), 8.36 (dd, *J* = 2.3, 8.4 Hz, 1H), 8.19 (d, *J* = 8.4 Hz, 1H), 7.73 (br d, *J* = 7.8 Hz, 1H), 7.62 (br d, *J* = 7.5 Hz, 1H), 7.41 (t, *J* = 7.9 Hz, 1H), 7.31 (brs, 1H), 3.53 (s, 3H), 3.46 - 3.30 (m, 1H), 2.82 (d, *J* = 4.4 Hz, 3H), 1.99 - 1.89 (m, 1H), 1.24 (d, *J* = 6.8 Hz, 6H), 0.99 - 0.85 (m, 4H); LCMS *m*/*z* = 524.1 [M+1]⁺.

### Step 5: Synthesis of compound WX-020

Compound **20-3** (1.8 g, 3.66 mmol, 1 *eq)* was dissolved in ethanol (18 mL). At 0°C, a solution of potassium hydrogenperoxomonosulphate (3.38 g, 5.49 mmol, 1.5 *eq)* in water (18 mL) was added thereto. The mixture was then gradually returned to 25°C, and stirred and reacted for 16 hours. After the reaction was completed, the reaction mixture was added with saturated sodium bicarbonate (15 mL) and sodium sulfite solution (15 mL) to quench, and extracted with ethyl acetate (30 mL * 2). The organic phases were combined, washed with saturated sodium chloride solution, filtered, and the filtrate was dried over anhydrous sodium sulfate. The crude product was purified by silica gel column chromatography (dichloromethane: methanol = 100:0 to 100:2) to obtain a crude product. The crude product was stirred in methanol (5 mL) at 60°C for 1 hour, and filtered. The filter cake was stirred in acetone (5 mL) at 60°C for another 1 hour, and filtered. The filter cake was dried under vacuum to obtain compound **WX-020.** ¹H NMR (400 MHz, DMSO-*d*₆) δ: 10.79 (brs, 1H), 10.73 (brs, 1H), 9.09 (d, *J* = 1.8 Hz, 1H), 8.64 (br d, *J* = 4.5 Hz, 1H), 8.53 (s, 1H), 8.33 (dd, *J* = 2.4, 8.4 Hz, 1H), 8.17 (d, *J* = 8.4 Hz, 1H), 8.07 (s, 1H), 7.60 - 7.51 (m, 2H), 7.38 - 7.21 (m, 1H), 3.69 - 3.57 (m, 1H), 3.51 (s, 3H), 2.80 (d, *J* = 4.4 Hz, 3H), 2.03 - 1.93 (m, 1H), 1.23 (d, *J* = 6.8 Hz, 6H), 0.83 - 0.74 (m, 4H); LCMS m/z = 524.0 [M+1]⁺.

### Step 6: Synthesis of sulfate of compound WX-020

Compound **WX-020** (0.4 g, 763.94 µmol, 1 *eq)* was added to acetone (4 mL), and the reaction mixture was stirred. Dilute sulfuric acid aqueous solution (0.25 M, 7.64 mL, 2.5 *eq)* was then added thereto. The reaction mixture was heated to 55°C, stirred for 30 minutes, and then naturally cooled to room temperature. The reaction mixture was filtered. The filter cake was washed with a small amount of ethyl acetate (10 mL), and dried under vacuum to obtain **sulfate of WX-020.** ¹H NMR (400 MHz, DMSO-*d*₆) δ: 11.33 (brs, 1H), 10.93 (brs, 1H), 9.11 (d, *J* = 1.8 Hz, 1H), 8.87 (brs, 1H), 8.48 (s, 1H), 8.36 (dd, *J* = 2.4, 8.4 Hz, 1H), 8.17 (d, *J* = 8.4 Hz, 1H), 7.70 (br d, *J* = 7.8 Hz, 1H), 7.61 (dd, *J* = 1.2, 8.0 Hz, 1H), 7.39 (t, *J* = 7.9 Hz, 1H), 3.61 (quin, *J* = 6.8 Hz, 1H), 3.54 (s, 3H), 2.82 (d, *J* = 4.4 Hz, 3H), 1.93 - 1.87 (m, 1H), 1.24 (d, *J* = 6.8 Hz, 6H), 0.92 - 0.86 (m, 4H); LCMS m/z = 523.9 [M+1]⁺.

### Example 21

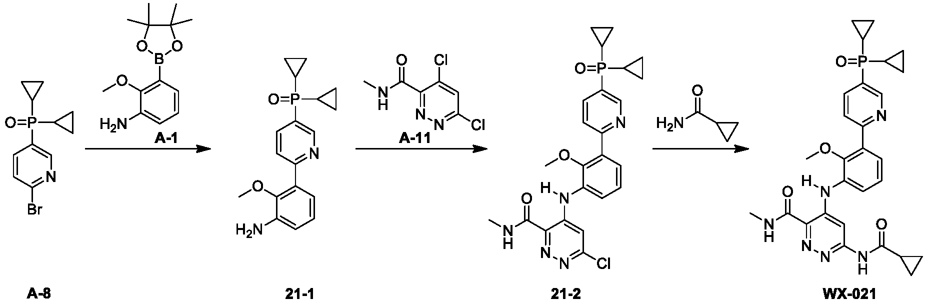

### Step 1: Synthesis of compound 21-1

Compound **A-8** (300 mg, 1.05 mmol, 1 *eq)* and **A-1** (261.21 mg, 1.05 mmol, 1 *eq)* were added to a mixed solvent of 1,4-dioxane (16 mL) and water (1 mL). Cesium carbonate (434.76 mg, 3.15 mmol, 3 *eq)* was then added thereto. After replacing with nitrogen, Pd(dppf)Cl₂ (76.72 mg, 104.86 µmol, 0.1 *eq)* was finally added thereto. Under a nitrogen atmosphere, the reaction mixture was stirred at 90°C for 6 hours. After cooling to room temperature, the reaction mixture was added with water (25 mL) and extracted with ethyl acetate (35 mL * 2). The organic phases were combined, dried over anhydrous sodium sulfate, and concentrated under vacuum to obtain a crude product. The crude product was purified by silica gel column chromatography (methanol: dichloromethane = 0 to 30%) to obtain compound **21-1.** LCMS *m*/*z* = 329.1 [M+1]⁺.

### Step 2: Synthesis of compound 21-2

Compound **21-1** (90 mg, 274.10 µmol, 1 *eq)* and compound **A-11** (56.47 mg, 274.10 µmol, 1 *eq)* were dissolved in THF (10 mL). Under a nitrogen atmosphere, LiHMDS (1 M, 1.10 mL, 4 *eq)* was slowly added thereto at 0°C. The mixture was then warmed to 25°C and stirred for 2 hours. After the reaction was completed, the reaction mixture was added with methanol (8 mL) and then concentrated under reduced pressure. The reaction mixture was then added with water (20 mL) and extracted with dichloromethane (30 mL * 2). The organic phases were combined, dried over anhydrous sodium sulfate, and concentrated under vacuum to obtain a crude product. The crude product was purified by silica gel column chromatography (methanol: dichloromethane = 0 to 40%) to obtain compound **21-2.** LCMS *m*/*z* = 498.1 [M+1]⁺.

### Step 4: Synthesis of compound WX-021

Compound **21-2** (50 mg, 100.42 µmol, 1 *eq),* cyclopropanecarboxamide (213.65 mg, 2.51 mmol, 25 *eq),* and Xantphos (8.72 mg, 15.06 µmol, 0.15 *eq)* were dissolved in a mixed solvent of 1.4-dioxane (6 mL) and NMP (1 mL). Cesium carbonate (98.16 mg, 301.26 µmol, *3 eq)* was added thereto. The system was replaced with nitrogen. Pd₂(dba)₃ (13.79 mg, 15.06 µmol, 0.15 *eq)* was finally added thereto. Under a nitrogen atmosphere, the mixture was stirred at 130°C for 12 hours. After cooling to room temperature, the reaction mixture was added with water (10 mL) and extracted with dichloromethane (20 mL * 2). The organic phases were combined, dried over anhydrous sodium sulfate, and concentrated under vacuum to obtain a crude product. The crude product was purified by silica gel column chromatography (dichloromethane: methanol = 100:0 to 30:1) to obtain compound **WX-021.** ¹H NMR (400 MHz, CDCl₃) δ: 11.15 (s, 1H), 9.13 (dd, *J* = 1.4, 5.4 Hz, 1H), 8.89 (br s, 1H), 8.28 (s, 1H), 8.23 - 8.16 (m, 2H), 8.10 (br d, *J* **=** 7.3 Hz, 1H), 7.66 (dd, *J* = 1.4, 8.0 Hz, 1H), 7.56 (d, *J* = 8.0 Hz, 1H), 7.37 - 7.32 (m, 1H), 3.58 (s, 3H), 3.08 (d, *J*=5.0 Hz, 3H), 1.74 - 1.65 (m, 1H), 1.20 - 0.95 (m, 14H); LCMS m/z = 547.1 [M+1]⁺.

To synthesize **examples 22**, **23**, and **24**, follow the synthesis steps of **example 21**, replacing **A-8** in step 1 with fragments in the table below.

**Table 8**

| Example | Compound | Fragment | Structural formula | Spectrum |
|---|---|---|---|---|
| **22** | **WX-022** | 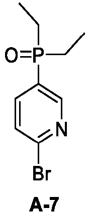 | 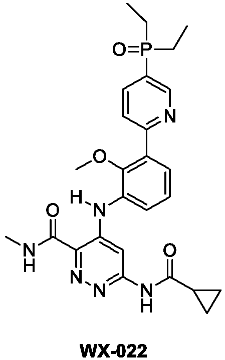 | ¹H NMR (400 MHz, CDCl₃) δ: 11.06 (s, 1H), 8.84 (dd, *J* = 1.1, 4.4 Hz, 1H), 8.73 - 8.63 (m, 1H), 8.17 (s, 1H), 8.11 - 8.02 (m, 3H), 7.60 - 7.57 (m, 1H), 7.48 - 7.45 (m, 1H), 7.25 (t, *J* = 7.9 Hz, 1H), 3.48 (s, 3H), 2.99 (d, *J* = 5.0 Hz, 3H), 2.07 - 1.86 (m, 4H), 1.63 - 1.59 (m, 1H), 1.18 - 1.09 (m, 6H), 1.07 - 1.03 (m, 2H), 0.89 - 0.84 (m, 2H); |
| | | | | LCMS *m*/*z* = 523.2 [M+H]⁺. |
| **23** | **WX-023** | 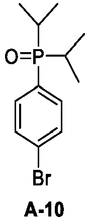 | 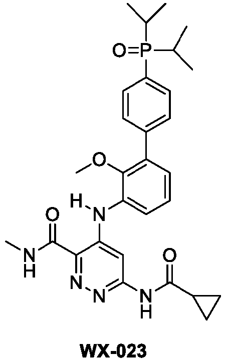 | ¹H NMR (400 MHz, CDCl₃) δ: 11.14 (br s, 1H), 9.23 (br s, 1H), 8.34 (s, 1H), 8.21 - 8.11 (m, 1H), 7.79 - 7.73 (m, 4H), 7.54 - 7.48 (m, 1H), 7.31 - 7.23 (m, 1H), 7.22 - 7.18 (m, 1H), 3.43 (s, 3H), 3.07 (d, *J* = 5.0 Hz, 3H), 2.44 - 2.31 (m, 2H), 1.81 - 1.64 (m, 1H), 1.30 - 1.24 (m, 6H), 1.18 - 1.08 (m, 8H), 0.98 - 0.92 (m, 2H); LCMS *m*/*z* = 550.1 [M+H]⁺. |
| **24** | **WX-024** | 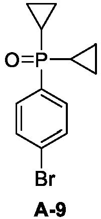 | 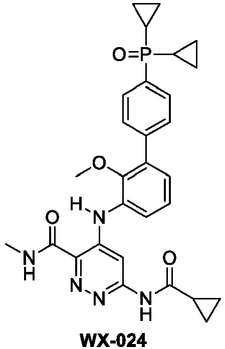 | ¹H NMR (400 MHz, CDCl₃) δ: 11.14 (s, 1H), 9.02 (br s, 1H), 8.32 (s, 1H), 8.16 (br d, *J* = 4.8 Hz, 1H), 7.93 - 7.87 (m, 2H), 7.74 (dd, *J* = 2.5, 8.3 Hz, 2H), 7.51 (dd, *J* = 1.4, 7.9 Hz, 1H), 7.31-7.25 (m, 1H), 7.19 (dd, *J* = 1.5, 7.8 Hz, 1H), 3.45 (s, 3H), 3.07 (d, *J* = 5.0 Hz, 3H), 1.75 - 1.65 (m, 1H), 1.17 - 0.87 (m, 14H); LCMS *m*/*z* = 546.2 [M+H]⁺. |

To synthesize **example 25** listed in Table 9, follow the synthesis steps of **example 21**, replacing **A-8** in step 1 with **A-6**, and **A-11** in step 2 with **A-12.**

**Table 9**

| | | | | |
|---|---|---|---|---|
| **2 5** | **WX-025** | 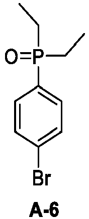 | 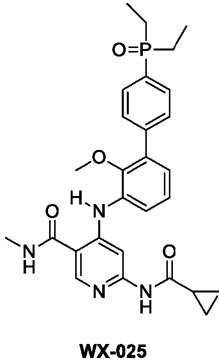 | ¹H NMR (400 MHz, CDCl₃) δ: 10.47 (s, 1H), 8.30 - 8.17 (m, 3H), 7.76 (d, *J* = 6.0 Hz, 4H), 7.59 - 7.53 (m, 1H), 7.26 (s, 1H), 7.13 (s, 1H), 6.34 - 6.21 (m, 1H), 3.45 (s, 3H), 3.04 (d, *J* = 4.8 Hz, 3H), 1.96 (br d, *J* = 9.8 Hz, 4H), 1.59 - 1.55 (m, 1H), 1.23 - 1.14 (m, 6H), 1.13 - 1.06 (m, 2H), 0.95 - 0.86 (m, 2H); LCMS *m*/*z* = 521.1 [M+H]⁺. |

### Example 28

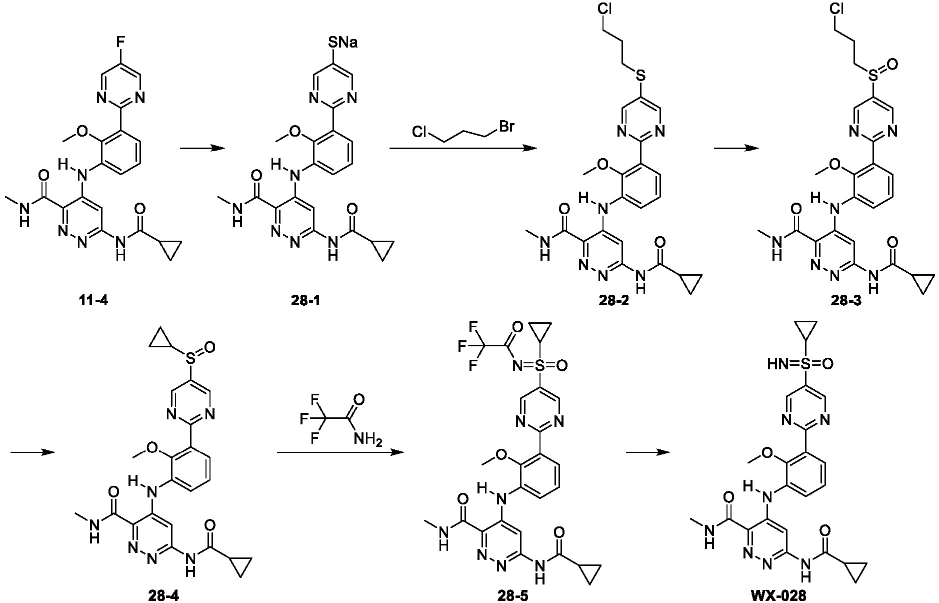

### Step 1: Synthesis of compound 28-1

Compound **11-4** (100 mg, 228.61 µmol, 1 *eq)* was added to DMSO (1 mL), and then sodium sulfide (35.68 mg, 457.22 µmol, 19.18 µL, 2 *eq)* was added thereto. The reaction was carried out at 70°C for 2 hours, and the mixture was cooled to room temperature to obtain a crude solution of compound **28-1.** The crude solution was directly used in the next step without purification. LCMS *m*/*z* = 452.1 [M+H]⁺.

### Step 2: Synthesis of compound 28-2

The crude DMSO solution of compound **28-1** (theoretical content of 108 mg, 228.10 µmol, 1 *eq)* obtained in the previous step was dissolved in DMF (1 mL). Potassium carbonate (45.67 mg, 330.44 µmol, 1.45 *eq)* and 1-bromo-4-chloropropane (52.31 mg, 305.08 µmol, 35.11 µL, 1.34 *eq)* were added thereto, and the mixture was stirred at 25°C for 10 minutes. The reaction mixture was added with water (3 mL) and extracted twice with ethyl acetate (6 mL). The organic phase was washed once with saturated brine (5 mL), dried over anhydrous sodium sulfate, and filtered. The filtrate was concentrated under reduced pressure to obtain compound **28-2.** LCMS *m*/*z* = 528.2 [M+H]⁺.

### Step 3: Synthesis of compound 28-3

Compound **28-2** (0.86 g, 1.63 mmol, 1 *eq)* was dissolved in dichloromethane (20 mL). 3-Chloroperoxybenzoic acid (330.66 mg, 1.63 mmol, purity of 85%, 1 *eq)* was added thereto at 0°C. The reaction was carried out at 25°C for 0.5 hours. The reaction mixture was washed once with 10% sodium thiosulfate aqueous solution (20 mL). The organic phase was dried over anhydrous sodium sulfate, filtered, and the filtrate was concentrated under reduced pressure to obtain a crude product. The crude product was purified by silica gel column chromatography (methanol/dichloromethane = 0 to 10%) to obtain compound **28-3.** LCMS *m*/*z* = 544.2 [M+H]⁺.

### Step 4: Synthesis of compound 28-4

Compound **28-3** (0.27 g, 496.30 µmol, 1 *eq)* was dissolved in DMF (9 mL), and potassium *tert*-butoxide (111.38 mg, 992.60 µmol, 2 *eq)* was added thereto at 25°C. The mixture was stirred for 1 hour. The reaction mixture was added with saturated ammonium chloride aqueous solution (9 mL) and extracted with dichloromethane (15 mL * 2). The organic phase was dried over anhydrous sodium sulfate, filtered, and the filtrate was concentrated under reduced pressure to obtain a crude product. The crude product was purified by silica gel column chromatography (methanol/dichloromethane = 0 to 10%) to obtain compound **28-4.** LCMS *m*/*z* = 508.1 [M+H]⁺.

### Step 5: Synthesis of compound 28-5

Compound **28-4** (0.15 g, 295.53 µmol, 1 *eq)* and trifluoroacetamide (33.41 mg, 295.53 µmol, 1 *eq)* were dissolved in dichloromethane (7.5 mL). (Diacetoxyiodo)benzene (142.78 mg, 443.29 µmol, 1.5 *eq),* magnesium oxide (47.64 mg, 1.18 mmol, 13.31 µL, 4 *eq),* and rhodium acetate (26.12 mg, 59.11 µmol, 0.2 *eq)* were added thereto. The mixture was stirred at 40°C for 1 hour. The reaction mixture was added with water (5 mL), and settled for phase separation. The organic phase was washed with 10% sodium thiosulfate aqueous solution (10 mL), dried over anhydrous sodium sulfate, filtered, and the filtrate was concentrated under reduced pressure to obtain a crude product. The crude product was purified by preparative thin-layer chromatography on a silica gel plate (dichloromethane: methanol = 10:1) to obtain compound **28-5.** LCMS *m*/*z* = 619.1 [M+H]⁺.

### Step 6: Synthesis of compound WX-028

Compound **28-5** (10 mg, 16.17 µmol, 1 *eq)* was dissolved in methanol (1 mL), and potassium carbonate (2.23 mg, 16.17 µmol, 1 *eq)* was added thereto. The mixture was stirred at 25°C for 10 minutes. The reaction mixture was added with water (1 mL) and extracted with dichloromethane (2 mL). The organic phase was dried over anhydrous sodium sulfate, filtered, and the filtrate was concentrated under reduced pressure to obtain a crude product. The crude product was purified by preparative thin-layer chromatography on a silica gel plate (dichloromethane: methanol = 10:1) to obtain compound **WX-028.** ¹H NMR (400 MHz, CDCl₃) δ: 11.19 (s, 1H), 9.34 (s, 2H), 8.54 - 8.24 (m, 2H), 7.84 - 7.82 (m, 1H), 7.72 (d, *J* = 7.2 Hz, 1H), 7.41 - 7.37 (m, 1H), 3.90 (s, 3H), 3.06 (d, *J* = 4.8 Hz, 3H), 2.71 - 2.65 (m, 1H), 2.41 - 2.24 (m, 1H), 1.14 - 1.06 (m, 8H); LCMS *m*/*z* = 523.2 [M+H]⁺.

### Example 29

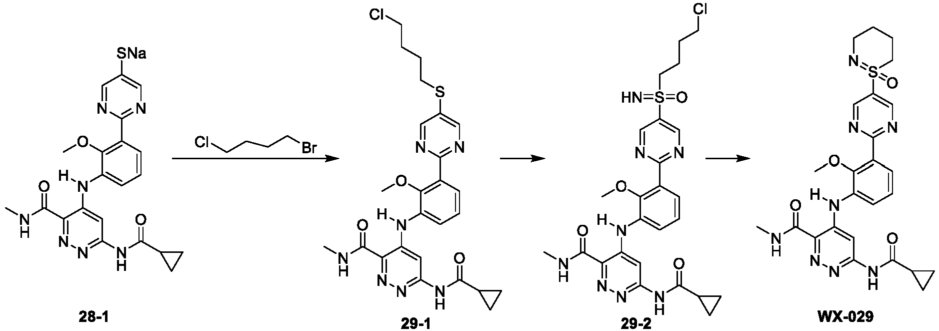

### Step 1: Synthesis of compound 29-1

The crude DMSO solution of compound **28-1** (theoretical content of 108 mg, 228.10 µmol, 1 *eq)* was dissolved in DMF (1 mL). Potassium carbonate (45.67 mg, 330.44 µmol, 1.45 *eq)* and 1-bromo-4-chlorobutane (52.31 mg, 305.08 µmol, 35.11 µL, 1.34 *eq)* were added thereto, and the mixture was stirred at 25°C for 10 minutes. The reaction mixture was added with water (3 mL) and extracted with ethyl acetate (6 mL * 2). The organic phase was washed with saturated brine (5 mL), dried over anhydrous sodium sulfate, filtered, and the filtrate was concentrated under reduced pressure to obtain compound **29-1.** LCMS *m*/*z* = 542.2 [M+H]⁺.

### Step 2: Synthesis of compound 29-2

Compound **29-1** (120 mg, 221.38 µmol, 1 *eq)* was dissolved in methanol (5 mL), and (diacetoxyiodo)benzene (213.92 mg, 664.14 µmol, 3 *eq)* and ammonium acetate (51.19 mg, 664.14 µmol, 3 *eq)* were added thereto. The mixture was stirred at 25°C for 10 minutes. The reaction mixture was extracted with dichloromethane (10 mL) and water (5 mL). The organic phase was dried over anhydrous sodium sulfate, filtered, and the filtrate was concentrated under reduced pressure to obtain a crude product. The crude product was purified by silica gel column chromatography (methanol/dichloromethane = 0 to 10%) to obtain compound **29-2.** LCMS *m*/*z* = 573.2 [M+H]⁺.

### Step 5: Synthesis of hydrochloride of compound WX-29

Compound **29-2** (35 mg, 61.08 µmol, 1 *eq)* was dissolved in DMF (1 mL), and sodium hydride (9.77 mg, 244.30 µmol, purity of 60%, 4 *eq)* was added thereto at 0°C. The mixture was stirred at 25°C for 0.5 hours. The reaction mixture was added with saturated ammonium chloride aqueous solution (2 mL), then added with water (1 mL), and extracted with dichloromethane (3 mL * 2). The organic phase was dried over anhydrous sodium sulfate, filtered, and the filtrate was concentrated under reduced pressure to obtain a crude product. The crude product was separated by preparative high performance liquid chromatography (chromatographic column: Phenomenex Luna 80 * 30 mm * 3 µm; mobile phase: A (water containing 0.04% hydrochloric acid) and B (acetonitrile); gradient: B%: 10% to 40%, 8 minutes) to obtain **hydrochloride of** compound **WX-29.** ¹HNMR (400 MHz, CD₃OD) δ: 9.64 (s, 2H), 8.12 - 8.09 (m, 1H), 7.83 - 7.81 (m, 1H), 7.51 (t, *J* = 8.0 Hz, 1H), 7.10 (s, 1H), 4.31 - 4.21 (m, 1H), 4.12 - 4.08 (m, 1H), 3.89 (s, 3H), 3.85 - 3.73 (m, 2H), 3.00 (s, 3H), 2.66 - 2.55 (m, 2H), 2.26 - 2.15 (m, 1H), 2.09 - 2.02 (m, 1H), 1.92 - 1.84 (m, 1H), 1.16 - 1.05 (m, 4H); LCMS *m*/*z* = 537.3 [M+H]⁺.

### Example 30

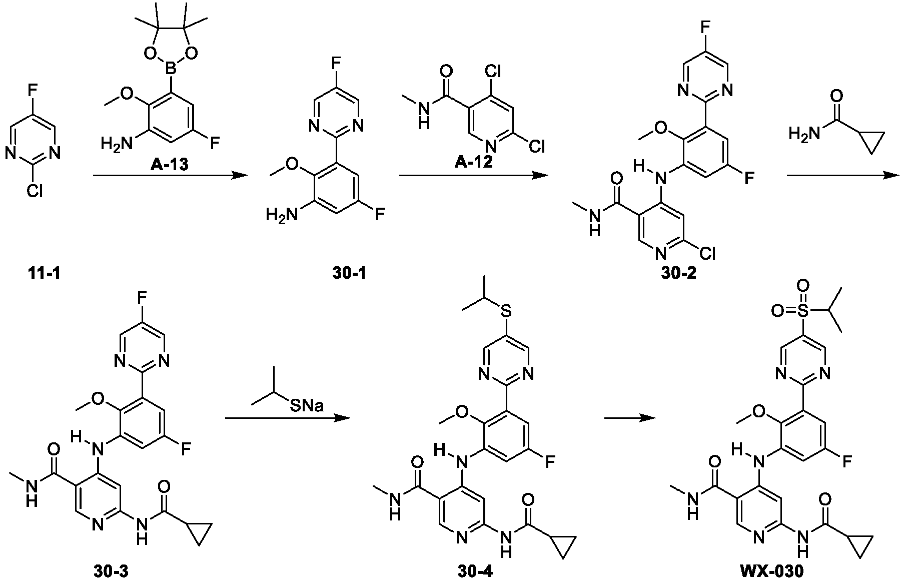

### Step 1: Synthesis of compound 30-1

Compound **11-1** (1.24 g, 9.36 mmol, 1.16 mL, 1 *eq),* **A-13** (2.5 g, 9.36 mmol, 1 *eq),* and potassium carbonate (2.59 g, 18.72 mmol, 2 *eq)* were dissolved in a mixed solvent of 1,4-dioxane (80 mL) and water (15 mL). After replacing with nitrogen three times, Pd(dppf)Cl₂ (684.86 mg, 936.00 µmol, 0.1 *eq)* was added thereto. Under a nitrogen atmosphere, the mixture was heated to 80°C and stirred for 4 hours. After cooling to room temperature, the reaction mixture was added with saturated ammonium chloride aqueous solution (100 mL) and water (100 mL), and then extracted with ethyl acetate (100 mL * 2). The organic phase was washed with saturated brine (100 mL * 2), dried over anhydrous sodium sulfate, filtered, and the filtrate was concentrated under reduced pressure to obtain a crude product. The crude product was purified by silica gel column chromatography (ethyl acetate/petroleum ether = 10 to 30%) to obtain compound **30-1.** LCMS *m*/*z* = 238.0 [M+1]⁺.

### Step 2: Synthesis of compound 30-2

Under a nitrogen atmosphere, compound **30-1** (1.8 g, 3.79 mmol, purity of 50%, 1 *eq)* and **A-12** (777.96 mg, 3.79 mmol, 1 *eq)* were added to THF (10 mL), and the mixture was stirred until dissolved. The mixture was cooled to 0°C, and LiHMDS (1 M, 9.49 mL, 2.5 *eq)* was added dropwise thereto. The mixture was then returned to 25°C and stirred for another 2 hours. After the reaction was completed, the reaction mixture was added with water (100 mL) and extracted with ethyl acetate (50 mL * 2). The organic phases were combined, washed with saturated brine (50 mL * 2), dried over anhydrous sodium sulfate, filtered, and the filtrate was concentrated under reduced pressure to obtain a crude product. The crude product was purified by silica gel column chromatography (methanol/dichloromethane = 3%) to obtain compound **30-2.** LCMS *m*/*z* = 406.0 [M+1]⁺.

### Step 3: Synthesis of compound 30-3

Compound **30-2** (1.18 g, 2.91 mmol, 1 *eq)* and cyclopropanecarboxamide (6.19 g, 72.70 mmol, 25 *eq)* were added to 1,4-dioxane (80 mL) and NMP (2 mL). Cesium carbonate (2.84 g, 8.72 mmol, 3 *eq)* and Xantphos (252.39 mg, 436.19 µmol, 0.15 *eq)* were then added thereto. After replacing with nitrogen three times, Pd₂(dba)₃ (399.43 mg, 436.19 µmol, 0.15 *eq)* was added. The mixture was refluxed and reacted at 130°C for 18 hours. After the reaction was completed, the reaction mixture was added with water (100) and extracted with ethyl acetate (50 mL * 2). The organic phases were combined, washed with saturated sodium chloride aqueous solution (50 mL * 2), dried over anhydrous sodium sulfate, and concentrated to obtain a crude product. The crude product was purified by silica gel column chromatography (methanol/dichloromethane = 3%) to obtain compound **30-3.** LCMS *m*/*z* = 455.1 [M+1]⁺.

### Step 4: Synthesis of compound 30-4

**30-3** (200 mg, 440.11 µmol, 1 *eq)* was dissolved in DMF (10 mL), and sodium 2-propanethiolate (86.39 mg, 880.23 µmol, 2 *eq)* was added thereto. The reaction was carried out at 40°C for 16 hours. After the reaction was completed, the reaction mixture was added with water (10 mL) and ethyl acetate (10 mL) for extraction and phase separation. The aqueous phase was then extracted once with ethyl acetate (10 mL). The organic phases were combined, washed once with saturated sodium chloride aqueous solution, dried over anhydrous sodium sulfate, and concentrated under vacuum to obtain a crude product of compound **30-4.** The crude product was directly used in the next step without purification. LCMS m/z = 511.2 [M+1]⁺.

### Step 5: Synthesis of compound WX-030

Compound **30-4** (200 mg, 391.71 µmol, 1 *eq)* was added to a mixed solution of ethanol (10 mL) and water (5 mL), and then potassium hydrogenperoxomonosulphate (361.21 mg, 587.56 µmol, 1.5 *eq)* was added thereto. The reaction was carried out at 25°C for 2 hours. After the reaction was completed, the reaction mixture was added with saturated sodium bisulfite aqueous solution (10 mL), and extracted with ethyl acetate (20 mL * 2). The organic phase was washed with saturated brine (10 mL * 2), dried over anhydrous sodium sulfate, filtered, and the filtrate was concentrated under reduced pressure to obtain a crude product. The crude product was separated by preparative high performance liquid chromatography (chromatographic column: Welch Xtimate C18 100 * 40 mm * 3 µm; mobile phase: A (water containing 0.075% trifluoroacetic acid) and B (acetonitrile); gradient: B%: 13% to 43%, 8 minutes). The obtained solution was concentrated under vacuum at 40°C to remove acetonitrile, then adjusted to alkalinity (pH = 8) with saturated sodium bicarbonate, and extracted with dichloromethane (30 mL * 3). The organic phases were combined, washed with saturated brine (50 mL), dried over anhydrous sodium sulfate, filtered, and the filtrate was concentrated under vacuum to obtain compound **WX-030.** ¹H NMR (400 MHz, CDCl₃) δ: 10.47 (s, 1H), 9.17 (s, 2H), 8.53 (br s, 1H), 8.22 (s, 1H), 8.12 (s, 1H), 7.38 (d, *J* = 9.0 Hz, 2H), 6.31 - 6.23 (m, 1H), 3.80 (s, 3H), 3.28 - 3.18 (m, 1H), 2.94 (d, *J* = 4.8 Hz, 3H), 1.51 - 1.44 (m, 1H), 1.34 (d, *J* = 6.8 Hz, 6H), 1.12 - 0.96 (m, 2H), 0.87 - 0.80 (m, 2H); LCMS *m*/*z* = 543.2 [M+1]⁺.

### Example 31

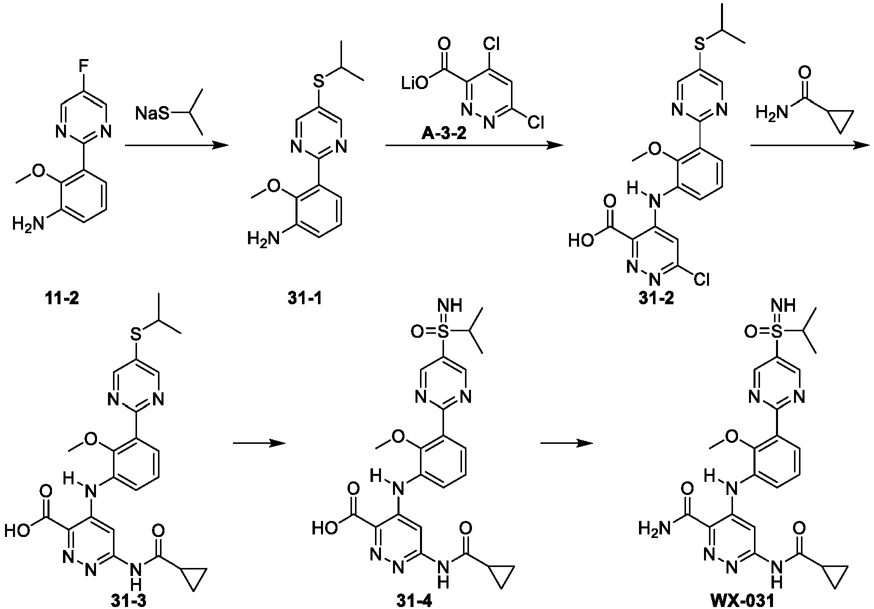

### Step 1: Synthesis of compound 31-1

Compound **11-2** (2 g, 9.12 mmol, 1 *eq)* and sodium 2-propanethiolate (906.59 mg, 9.24 mmol, 1.5 *eq)* were dissolved in DMF (20 mL), and the mixture was reacted at 40°C for 1 hour. The reaction mixture was added with ethyl acetate (20 mL) and water (20 mL). The aqueous phase was then extracted once with ethyl acetate (20 mL), and the organic phase was washed three times with saturated sodium chloride aqueous solution (20 mL), dried over anhydrous sodium chloride, and concentrated under reduced pressure to obtain compound **31-1.** LCMS *m*/*z* = 276.1 [M+H]⁺.

### Step 2: Synthesis of compound 31-2

Compound **31-1** (0.8 g, 2.91 mmol, 1 *eq)* and compound **A-3-2** (577.90 mg, 2.91 mmol, 1 *eq)* were dissolved in THF (16 mL), and LiHMDS (1 mol/L, 8.72 mL, 3 *eq)* was added thereto at -60°C. The mixture was warmed to 25°C and reacted for 3 hours. The reaction mixture was added with methanol (5 mL) to quench, then added with 2 mol/L hydrochloric acid solution to adjust the pH to 7 to 8, and added with ethyl acetate (20 mL) and water (20 mL). The aqueous phase was then extracted once with ethyl acetate (20 mL). The organic phase was washed once with saturated sodium chloride aqueous solution (20 mL), dried over anhydrous sodium sulfate, and concentrated. The obtained crude product was subjected to silica gel column chromatography (methanol/dichloromethane = 0 to 10%) to obtain compound **31-2.** LCMS *m*/*z* = 432.1 [M+H]⁺.

### Step 3: Synthesis of compound 31-3

Compound **31-2** (0.46 g, 1.07 mmol, 1 *eq)* and cyclopropanecarboxamide (906.42 mg, 10.65 mmol, 10 *eq*) were dissolved in 1,4-dioxane (16 mL). Cesium carbonate (867.55 mg, 2.66 mmol, 2.5 *eq*) was then added thereto. Under a nitrogen atmosphere, Pd₂(dba)₃ (30.53 mg, 33.34 µmol, 0.1 *eq*) and Xantphos (38.58 mg, 66.68 µmol, 0.2 *eq*) were added thereto. The reaction was carried out at 120°C for 4 hours. The reaction system was cooled to 20 to 30°C. The reaction mixture was added with 2 mol/L hydrochloric acid solution to adjust the pH to 3, and added with dichloromethane (20 mL) and water (20 mL). The aqueous phase was then extracted once with dichloromethane (20 mL). The organic phase was washed once with saturated sodium chloride aqueous solution (20 mL), dried over anhydrous sodium sulfate, and then concentrated under reduced pressure. The obtained crude product was subjected to silica gel column chromatography (methanol/dichloromethane = 0 to 10%) to obtain compound **31-3.** LCMS *m*/*z* = 481.0 [M+H]⁺.

### Step 4: Synthesis of compound 31-4

Compound **30-3** (0.42 g, 524.41 µmol, purity of 60%, 1 *eq*) was dissolved in MeOH (10 mL). Ammonium acetate (160.41 mg, 2.08 mmol, 4 *eq*) and (diacetoxyiodo)benzene (502.71 mg, 1.56 mmol, 3 *eq*) were then added thereto, and the reaction was carried out at 25°C for 4 hours. The reaction mixture was added with ethyl acetate (10 mL) and water (10 mL). The aqueous phase was then extracted once with ethyl acetate (10 mL). The organic phase was washed with saturated sodium chloride aqueous solution (10 mL * 3), dried over anhydrous sodium sulfate, and then concentrated. Compound **31-4** was obtained. LCMS *m*/*z* = 512.2 [M+H]⁺. The crude product was directly used in the next step without purification.

### Step 5: Synthesis of hydrochloride of compound WX-031

Compound **31-4** (450 mg, 175.94 µmol, purity of 20%, 1 *eq*) and ammonium chloride (470.55 mg, 8.80 mmol, 50 *eq*) were dissolved in DMF (10 mL). HATU (200.69 mg, 527.81 µmol, 3 *eq*) and DIPEA (27.29 mg, 211.12 µmol, 36.77 µL, 1.2 *eq*) were then added thereto. The reaction was carried out at 25°C for 3 hours. The reaction mixture was added with ethyl acetate (10 mL) and water (10 mL). The aqueous phase was then extracted once with ethyl acetate (10 mL). The organic phase was washed with saturated sodium chloride aqueous solution (10 mL * 3), dried over anhydrous sodium sulfate, and then concentrated. The obtained crude product was subjected to silica gel column chromatography (methanol/dichloromethane = 0 to 10%), and then separated by preparative high performance liquid chromatography (chromatographic column: Phenomenex Luna 80 * 30 mm * 3 µm; mobile phase: A (water containing 0.04% hydrochloric acid) and B (acetonitrile); gradient: B%: 10% to 35%) to obtain **hydrochloride of** compound **WX-031.** ¹H NMR (400 MHz, CD₃OD) δ: 9.47 (s, 2H), 8.10 (dd, *J* = 0.8, 8.0 Hz, 1H), 7.78 (dd, *J* = 0.9, 7.9 Hz, 1H), 7.50 (t, *J* = 8.0 Hz, 1H), 6.91 (s, 1H), 4.12 - 4.04 (m, 1H), 3.88 (s, 3H), 1.86 - 1.80 (m, 1H), 1.52 (dd, *J* = 8.0, 18.8 Hz, 6H), 1.18 - 1.08 (m, 4H); LCMS *m*/*z* = 511.1 [M+H]⁺.

### Example 32

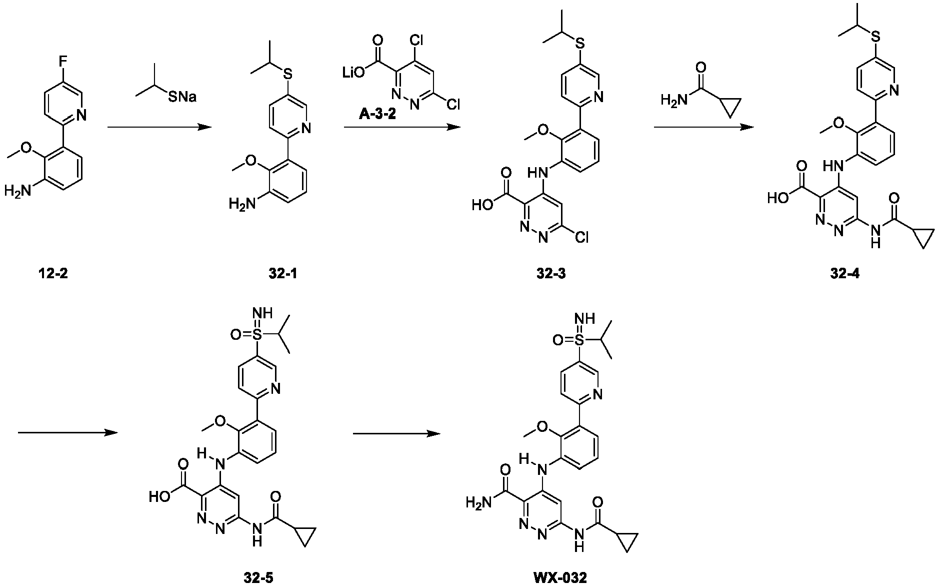

### Step 1: Synthesis of compound 32-1

Compound **12-2** (1.12 g, 5.13 mmol, 1 *eq*) was dissolved in DMF (10 mL), and then sodium 2-propanethiolate (2.01 g, 20.53 mmol, 4 *eq*) was added thereto. The reaction was carried out at 40°C for 2 hours. The reaction mixture was added with water (10 mL) and ethyl acetate (10 mL) for extraction and phase separation. The aqueous phase was then extracted once with ethyl acetate (10 mL). The organic phases were combined, dried over anhydrous sodium sulfate, filtered, and the filtrate was concentrated under reduced pressure to obtain a crude product. The crude product was purified by silica gel column chromatography (ethyl acetate/petroleum ether = 10%) to obtain compound **32-1.** LCMS *m*/*z* = 275.1 [M+1]⁺.

### Step 2: Synthesis of compound 32-2

Under a nitrogen atmosphere, compound **32-1** (250 mg, 911.14 µmol, 1 *eq)* and **A-3-2** (181.25 mg, 911.14 µmol, 1 eq) were added to THF (10 mL). The mixture was stirred at 25°C, and LiHMDS(1 M, 2.28 mL, 2.5 *eq*) was slowly added dropwise thereto. The mixture was stirred at 25°C for another 2 hours. The reaction mixture was added with saturated ammonium chloride aqueous solution (20 mL) and water (50 mL), and extracted with ethyl acetate (50 mL * 2). The organic phases were combined, washed with saturated brine (50 mL * 2), dried over anhydrous sodium sulfate, filtered, and the filtrate was concentrated under reduced pressure to obtain a crude product. The crude product was purified by silica gel column chromatography (methanol/dichloromethane = 10%) to obtain compound **32-2.** LCMS *m*/*z* = 431.0 [M+1]⁺.

### Step 3: Synthesis of compound 32-3

Compound **32-2** (240 mg, 556.96 µmol, 1 *eq*) and cyclopropanecarboxamide (1.19 g, 13.92 mmol, 25 *eq*) were added to a mixed solvent of 1,4-dioxane (10 mL) and NMP (1 mL). Cesium carbonate (544.41 mg, 1.67 mmol, 3 *eq*) and Xantphos (48.34 mg, 83.54 µmol, 0.15 *eq*) were then added thereto. After replacing with nitrogen three times, Pd₂(dba)₃ (76.50 mg, 83.54 µmol, 0.15 *eq*) was added. The mixture was stirred and reacted at 130°C for 18 hours. After cooling to room temperature, the reaction mixture was added with water (30 mL) and ethyl acetate (30 mL) for extraction and phase separation. The aqueous phase was then extracted once with ethyl acetate (30 mL). The organic phases were combined, washed once with saturated sodium chloride aqueous solution, dried over anhydrous sodium sulfate, filtered, and the filtrate was concentrated under vacuum to obtain a crude product. The crude product was purified by silica gel column chromatography (methanol/dichloromethane = 3%) to obtain compound **32-3.** LCMS *m*/*z* = 480.1 [M+1]⁺.

### Step 4: Synthesis of compound 32-4

Compound **32-3** (187 mg, 389.95 µmol, 1 *eq)* was added to methanol (1 mL), and then (diacetoxyiodo)benzene (376.80 mg, 1.17 mmol, 3 *eq)* and ammonium acetate (120.23 mg, 1.56 mmol, 4 *eq)* were added thereto. The reaction was carried out at 25°C for 2 hours. The reaction mixture was added with water (10 mL) and ethyl acetate (10 mL) for extraction and phase separation. The aqueous phase was then extracted once with ethyl acetate (10 mL). The organic phases were combined, washed once with saturated sodium chloride aqueous solution (10 mL), dried over anhydrous sodium sulfate, filtered, and the filtrate was concentrated under vacuum to obtain a crude product of **32-4.** LCMS *m*/*z* = 511.1 [M+1]⁺. The crude product was directly used in the next step without purification.

### Step 5: Synthesis of hydrochloride of compound WX-032

Compound **32-4** (199 mg, 389.76 µmol, 1 *eq*) was dissolved in DMF (10 mL). Ammonium chloride (1.04 g, 19.49 mmol, 50 *eq*), HATU (222.30 mg, 584.65 µmol, 1.5 *eq*), and DIPEA (151.12 mg, 1.17 mmol, 203.67 µL, 3 *eq*) were then sequentially added thereto. The reaction was carried out at 25°C for 1 hour. The reaction system was added with water (10 mL) and ethyl acetate (10 mL) for extraction and phase separation. The aqueous phase was then extracted once with ethyl acetate (10 mL). The organic phases were combined, washed once with saturated sodium chloride aqueous solution (10 mL), dried over anhydrous sodium sulfate, filtered, and the filtrate was concentrated under vacuum to obtain a crude product. The crude product was separated by preparative high performance liquid chromatography (chromatographic column: Xtimate C18, 150 * 40 mm * 3 µm; mobile phase: A (water containing 0.04% hydrochloric acid) and B (acetonitrile); gradient: B%: 15% to 45%, 10 minutes) to obtain **hydrochloride of** compound **WX-032.** ¹H NMR (400 MHz, DMSO-*d*₆) δ: 11.49 (brs, 1H), 11.12 (brs, 1H), 9.27 - 9.08 (m, 1H), 8.67 - 8.52 (m, 1H), 8.48 - 8.36 (m, 1H), 8.28 - 8.17 (m, 1H), 8.12 - 8.08 (m, 1H), 7.98 - 7.92 (m, 1H), 7.73 - 7.59 (m, 2H), 7.44 - 7.36 (m, 1H),), 3.97 - 3.84 (m, 1H), 3.54 - 3.52 (m, 3H), 2.16 - 2.00 (m, 1H), 1.41 - 1.25 (m, 6H), 0.93 - 0.78 (m, 4H); LCMS *m*/*z* = 510.1[M+1]⁺.

### Example 33

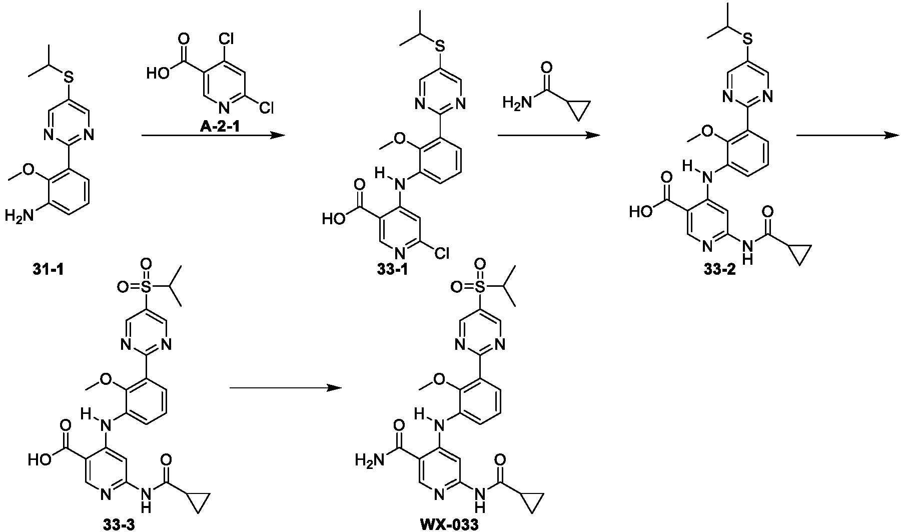

### Step 1: Synthesis of compound 33-1

Under a nitrogen atmosphere, compound **A-2-1** (627.52 mg, 3.27 mmol, 1 *eq*) and **31-1** (900 mg, 3.27 mmol, 1 *eq*) were added to tetrahydrofuran (10 mL), and the mixture was stirred until dissolved. LiHMDS (1 M, 8.17 mL, 2.5 *eq*) was slowly added dropwise at 25°C, and then the mixture was stirred at 25°C for another 1 hour. After the reaction was completed, the reaction mixture was added with water (30 mL) and ethyl acetate (30 mL) for extraction and phase separation. The aqueous phase was then extracted once with ethyl acetate (30 mL). The organic phases were combined, washed once with saturated sodium chloride aqueous solution, dried over anhydrous sodium sulfate, filtered, and the filtrate was concentrated under vacuum to obtain a crude product. The crude product was purified by silica gel column chromatography (methanol/dichloromethane = 0 to 10%) to obtain compound **33-1.** ¹H NMR (400 MHz, DMSO-*d*₆) δ: 11.39 (br s, 1H), 8.94 (s, 2H), 8.65 (s, 1H), 7.563 - 7.55 (m, 2H), 7.31 (t, *J* = 8.0 Hz, 1H), 6.85 (s, 1H), 3.77 - 3.69 (m, 1H), 3.66 (s, 3H), 1.31 (d, *J* = 6.8 Hz, 6H); LCMS *m*/*z* = 431.1 [M+1]⁺.

### Step 2: Synthesis of compound 33-2

Compound **33-1** (500 mg, 1.16 mmol, 1 *eq*), cyclopropanecarboxamide (2.47 g, 29.01 mmol, 25 *eq*), and Xantphos (201.42 mg, 348.10 µmol, 0.3 *eq*) were sequentially added to a mixed solvent of NMP (1 mL) and 1,4-dioxane (10 mL). The reaction mixture was replaced with nitrogen three times, and cesium carbonate (1.51 g, 4.64 mmol, 4 *eq*) and Pd₂(dba)₃·CHCl₃ (180.16 mg, 174.05 µmol, 0.15 *eq*) were added thereto. After replacing with nitrogen three times, the reaction mixture was heated to reflux (external temperature of 130°C) and reacted for 16 hours. After cooling to room temperature, the reaction mixture was added with water (20 mL) and ethyl acetate (20 mL) for extraction and phase separation. The aqueous phase was then extracted once with ethyl acetate (10 mL). The organic phases were combined, dried over anhydrous sodium sulfate, filtered, and the filtrate was concentrated under vacuum to obtain a crude product. The crude product was purified by silica gel column chromatography (methanol/dichloromethane = 0 to 10%) to obtain compound **33-2.** LCMS *m*/*z* = 480.1 [M+1]⁺.

### Step 3: Synthesis of compound 33-3

Compound **33-2** (150 mg, 312.79 µmol, 1 *eq*) was dissolved in DCM (2 mL). 3-Chloroperoxybenzoic acid (80.97 mg, 469.19 µmol, 1.5 *eq*) was added thereto. The reaction was carried out at 20°C for 16 hours. After the reaction was completed, the reaction mixture was added with water (20 mL) and ethyl acetate (20 mL) for extraction and phase separation. The aqueous phase was then extracted once with ethyl acetate (10 mL). The organic phases were combined, dried over anhydrous sodium sulfate, filtered, and the filtrate was concentrated under reduced pressure to obtain a crude product of **33-3.** LCMS *m*/*z* = 512.1 [M+1]⁺. The crude product was directly used in the next step without purification.

### Step 4: Synthesis of compound WX-033

Compound **33-3** (140 mg, 273.68 µmol, 1 *eq*) and ammonium chloride (731.97 mg, 13.68 mmol, 50 *eq*) were added to DMF (10 mL). HATU (312.18 mg, 821.04 µmol, 3 *eq*) and DIPEA (212.23 mg, 1.64 mmol, 286.02 µL, 6 *eq*) were sequentially added thereto. The mixture was stirred and reacted at 25°C for 1 hour. The reaction system was added with water (10 mL) and ethyl acetate (10 mL) for extraction and phase separation. The aqueous phase was then extracted once with ethyl acetate (10 mL). The organic phases were combined, washed once with saturated sodium chloride aqueous solution, dried over anhydrous sodium sulfate, filtered, and the filtrate was concentrated under vacuum to obtain a crude product. The crude product was separated by preparative high performance liquid chromatography (chromatographic column: Welch Xtimate C18 100 * 40 mm * 3 µm; mobile phase: A (water containing 0.075% trifluoroacetic acid) and B (acetonitrile); gradient: B%: 10% to 40%, 8 minutes). The obtained solution was concentrated under vacuum at 40°C to remove acetonitrile, then adjusted to alkalinity (pH = 8) with saturated sodium bicarbonate, and extracted with dichloromethane (30 mL * 3). The organic phases were combined, washed with saturated brine (50 mL), dried over anhydrous sodium sulfate, filtered, and the filtrate was concentrated under vacuum to obtain compound **WX-033.** ¹H NMR (400 MHz, CDCl₃) δ: 10.67 (br s, 1H), 9.27 (s, 2H), 8.38 (s, 1H), 8.16 - 8.04 (m, 1H), 7.82 (d, *J* = 8.4 Hz, 1H), 7.73 (d, *J* = 6.8 Hz, 1H), 7.40 - 7.34 (m 1H), 5.83 (brs, 2H), 3.85 (s, 2H), 3.38 - 3.24 (m, 1H), 1.74 - 1.65 (m, 1H), 1.44 (d, *J* = 6.8 Hz, 6H), 1.13 - 1.06 (m, 2H), 0.95 - 0.89 (m, 2H); LCMS *m*/*z* = 511.2 [M+1]⁺.

### Example 34

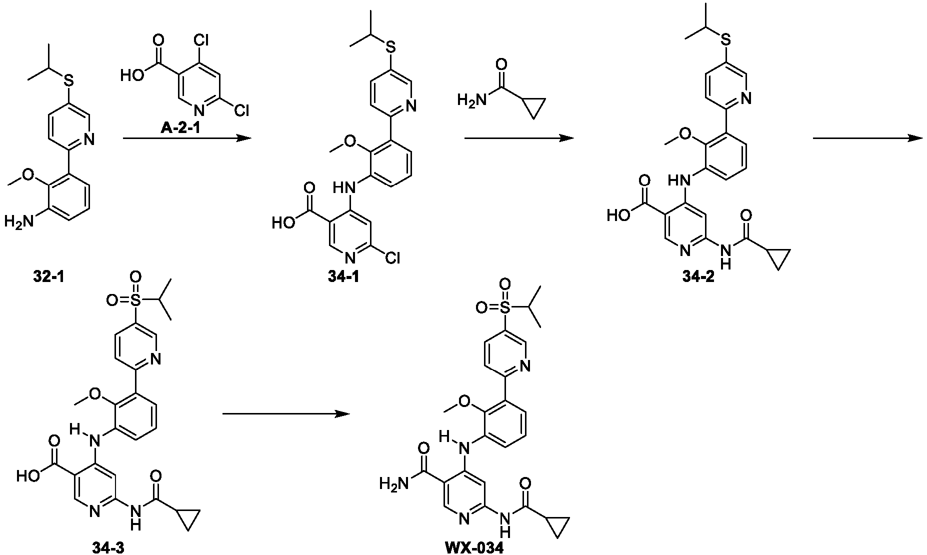

### Step 1: Synthesis of compound 34-1

Under a nitrogen atmosphere, **32-1** (250 mg, 911.14 µmol, 1 *eq)* was added to THF (10 mL), then **A-2-1** (174.94 mg, 911.14 µmol, 1 *eq)* was added thereto, and the mixture was stirred until dissolved. LiHMDS (1 M, 2.28 mL, 2.5 *eq*) was added dropwise thereto at 25°C, and the mixture was stirred for another 2 hours. After the reaction was completed, the reaction mixture was added with saturated ammonium chloride aqueous solution (20 mL) and water (50 mL), and extracted with ethyl acetate (50 mL * 2). The organic phases were combined, washed with saturated brine (50 mL * 2), dried over anhydrous sodium sulfate, filtered, and the filtrate was concentrated under vacuum to obtain a crude product. The crude product was purified by silica gel column chromatography (methanol/dichloromethane = 10%) to obtain compound **34-1.** LCMS *m*/*z* = 430.0 [M+1]⁺.

### Step 2: Synthesis of compound 34-2

Compound **34-1** (550 mg, 1.28 mmol, 1 *eq*) and cyclopropanecarboxamide (2.72 g, 31.98 mmol, 25 *eq*) were added to a mixed solvent of 1,4-dioxane (20 mL) and NMP (2 mL). Cesium carbonate (1.25 g, 3.84 mmol, 3 *eq*) and Xantphos (111.04 mg, 191.90 µmol, 0.15 *eq*) were then added thereto. After replacing with nitrogen three times, Pd₂(dba)₃ (175.72 mg, 191.90 µmol, 0.15 *eq*) was added. The mixture was reacted at 130°C for 18 hours. After the reaction was cooled to room temperature, the reaction mixture was added with water (30 mL) and ethyl acetate (30 mL) for extraction and phase separation. The aqueous phase was then extracted once with ethyl acetate (30 mL). The organic phases were combined, washed once with saturated sodium chloride aqueous solution, dried over anhydrous sodium sulfate, filtered, and the filtrate was concentrated under vacuum to obtain a crude product. The crude product was purified by silica gel column chromatography (methanol/dichloromethane = 3%) to obtain compound **34-2.** LCMS *m*/*z* = 479.2 [M+1]⁺.

### Step 3: Synthesis of compound 34-3

Compound **34-2** (430 mg, 898.52 µmol, 1 *eq*) was dissolved in dichloromethane (10 mL). The mixture was then cooled to 0°C and added with 3-chloroperoxybenzoic acid (364.84 mg, 1.80 mmol, purity of 85%, 2 *eq*). The reaction was then carried out at 25°C for 2 hours. After the reaction was completed, the reaction mixture was added with water (30 mL) and ethyl acetate (30 mL) for extraction and phase separation. The aqueous phase was then extracted once with ethyl acetate (30 mL). The organic phases were combined, washed once with saturated sodium chloride aqueous solution, dried over anhydrous sodium sulfate, filtered, and the filtrate was concentrated under vacuum to obtain a crude product of **34-3.** LCMS *m*/*z* = 511.1 [M+1]⁺. The crude product was directly used in the next step without further purification.

### Step 4: Synthesis of trifluoroacetate of compound WX-034

Compound **34-3** (458 mg, 897.05 µmol, 1 *eq*) was added to DMF (10 mL). Ammonium chloride (2.40 g, 44.85 mmol, 50 *eq*), HATU (511.63 mg, 1.35 mmol, 1.5 *eq*), and DIPEA (347.81 mg, 2.69 mmol, 468.74 µL, 3 *eq*) were then added thereto. The reaction was carried out at 25°C for 1 hour. After the reaction was completed, the reaction mixture was added with water (10 mL) and ethyl acetate (10 mL) for extraction and phase separation. The aqueous phase was then extracted once with ethyl acetate (10 mL). The organic phases were combined, washed once with saturated sodium chloride aqueous solution, dried over anhydrous sodium sulfate, filtered, and the filtrate was concentrated under vacuum to obtain a crude product. The crude product was separated by preparative high performance liquid chromatography (chromatographic column: Welch Xtimate C18 100 * 40 mm * 3 µm; mobile phase: A (water containing 0.075% trifluoroacetic acid) and B (acetonitrile); gradient: B%: 15 to 45%, 8 minutes) to obtain **trifluoroacetate of** compound **WX-034.** ¹H NMR (400 MHz, DMSO-*d*₆) δ: 11.34 - 10.93 (m, 2H), 9.22 - 9.00 (m, 1H), 8.59 (s, 1H), 8.50 - 8.23 (m, 2H), 8.17 (d, *J* = 8.4 Hz, 1H), 7.77 - 7.56 (m, 4H), 7.42 - 7.30 (m, 1H), 3.65 - 3.58 (m, 1H), 3.52 (s, 3H), 2.01 - 1.87 (m, 1H), 1.24 (d, *J* = 6.8 Hz, 6H), 0.93 - 0.79 (m, 4H); LCMS *m*/*z* = 510.1 [M+1]⁺.

### Example 35

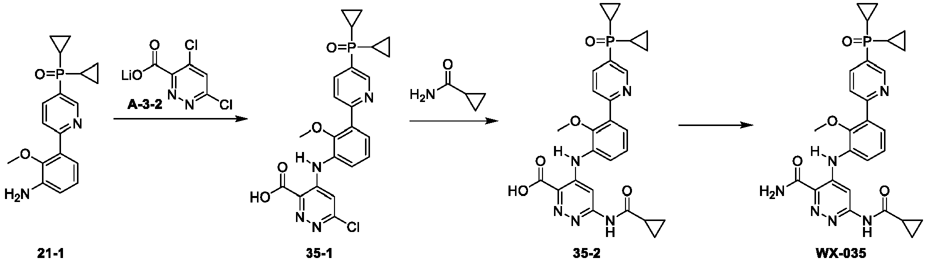

### Step 1: Synthesis of compound 35-1

Compound **21-1** (100 mg, 304.56 µmol, 1 *eq*) and **A-3-2** (60.58 mg, 304.56 µmol, 1 *eq*) were dissolved in THF (10 mL). LiHMDS (1 M, 1.22 mL, 4 *eq*) was added dropwise thereto at 0°C, and then the mixture was stirred and reacted at 25°C for 2 hours. The reaction mixture was added with 8 mL of methanol, concentrated under reduced pressure, then added with water (20 mL), and extracted with dichloromethane (30 mL * 2). The organic phase was washed with saturated sodium chloride solution (25 mL), dried over anhydrous sodium sulfate, filtered, and the filtrate was concentrated under vacuum to obtain a crude product. The crude product was purified by silica gel column chromatography (dichloromethane: methanol = 100:0 to 100:5) to obtain compound **35-1.** LCMS m/z = 485.0 [M+1]⁺.

### Step 4: Synthesis of compound 35-2

Compound **35-1** (50 mg, 103.12 µmol, 1 *eq*), cyclopropanecarboxamide (219.40 mg, 2.58 mmol, 25 *eq*), and Xantphos (8.95 mg, 15.47 µmol, 0.15 *eq*) were dissolved in a mixed solvent of 1,4-dioxane (1 mL) and NMP (0.5 mL). Cesium carbonate (100.80 mg, 309.36 µmol, 3 *eq*) was then added thereto. After replacing with nitrogen, Pd₂(dba)₃ (14.16 mg, 15.47 µmol, 0.15 *eq)* was added thereto. After replacing with nitrogen, the reaction mixture was heated to reflux and reacted for 12 hours. After the reaction was completed, the reaction mixture was cooled to room temperature, and concentrated under reduced pressure to remove the organic solvent. The crude product was purified by silica gel column chromatography (dichloromethane: methanol = 100:0 to 1:1) to obtain compound **35-2.** LCMS *m*/*z* = 534.1 [M+1]⁺.

### Step 5: Synthesis of trifluoroacetate of compound WX-035

Compound **35-2** (30 mg, 56.23 µmol, 1 *eq*), ammonium chloride (150.39 mg, 2.81 mmol, 50 *eq*), and HATU (32.07 mg, 84.35 µmol, 1.5 *eq*) were dissolved in DMF (2 mL). DIPEA (21.80 mg, 168.69 µmol, 29.38 µL, 3 *eq*) was then added thereto. The mixture was stirred and reacted at 25°C for 12 hours. After the reaction was completed, the reaction mixture was added with water (6 mL) and extracted with ethyl acetate (10 mL * 2). The organic phase was washed with saturated sodium chloride solution, dried over anhydrous sodium sulfate, filtered, and the filtrate was concentrated under vacuum to obtain a crude product. The crude product was purified by preparative high performance liquid chromatography (chromatographic column: Welch Xtimate C18 100 * 40 mm * 3 µm; mobile phase: A (water containing 0.075% trifluoroacetic acid) and B (acetonitrile); gradient: B%: 8 to 38%, 8 minutes) to obtain **trifluoroacetate of** compound **WX-035.** ¹H NMR (400 MHz, CDCl₃) δ: 13.05 (br s, 1H), 11.53 (s, 1H), 9.06 (d, *J* = 4.4 Hz, 1H), 8.40 (s, 1H), 8.19 - 8.13 (m, 1H), 7.96 (br d, *J* = 8.0 Hz, 1H), 7.71 (d, *J* = 7.8 Hz, 1H), 7.61 (br s, 1H), 7.45 (d, *J* = 6.8 Hz, 1H), 7.35 - 7.30 (m, 1H), 5.81 (br s, 1H), 3.48 (s, 3H), 2.00 - 1.93 (m, 1H), 1.06 - 0.87 (m, 14H); LCMS m/z = 533.2 [M+1]⁺.

### Example 36

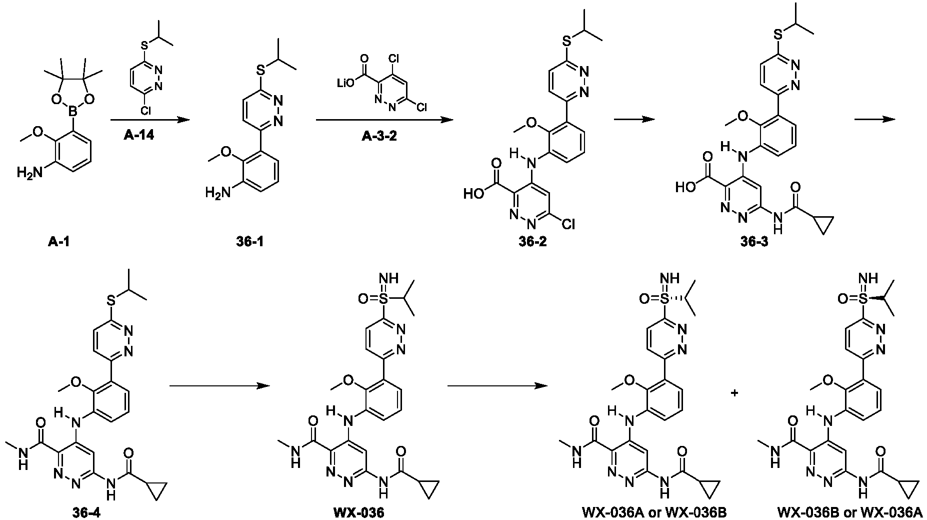

### Step 1: Synthesis of compound 36-1

Compound **A-1** (2 g, 8.03 mmol, 1 *eq*), compound **A-14** (1.36 g, 7.23 mmol, 0.9 *eq*), and potassium phosphate tribasic (5.11 g, 24.09 mmol, 3 *eq*) were dissolved in 1,4-dioxane (20 mL) and water (10 mL). After replacing with nitrogen three times, Pd(dppf)Cl₂·CH₂Cl₂ (393.38 mg, 481.71 µmol, 0.06 *eq*) was added. The mixture was stirred at 100°C for 2 hours. The reaction mixture was added with water (10 mL) and extracted with ethyl acetate (20 mL * 2). The organic phase was dried over anhydrous sodium sulfate, filtered, and the filtrate was concentrated under reduced pressure to obtain a crude product. The crude product was purified by silica gel column chromatography (ethyl acetate/petroleum ether = 0 to 50%) to obtain compound **36-1.** ¹HNMR (400 MHz, CDCl₃) δ: 7.83 - 7.80 (m, 1H), 7.30 - 7.29 (m, 1H), 7.28 - 7.27 (m, 1H), 7.08 - 7.04 (m, 1H), 6.87 (dd, *J* = 8.0, 1.6 Hz, 1H), 4.39 - 4.33 (m, 1H), 3.53 (s, 3H), 1.51 (d, *J* = 6.8 Hz, 6H); LCMS *m*/*z* = 276.1 [M+H]⁺.

### Step 2: Synthesis of compound 36-2

Compound **36-1** (1 g, 3.63 mmol, 1 *eq*) and compound **A-3-2** (830.74 mg, 4.18 mmol, 1.15 *eq*) were dissolved in isopropanol (10 mL) and water (3 mL). Zinc acetate (799.56 mg, 4.36 mmol, 1.2 *eq*) was added thereto. The mixture was stirred at 80°C for 16 hours. The reaction mixture was added with water (30 mL), filtered, and the filter cake was collected to obtain compound **36-2.** LCMS *m*/*z* = 432.2 [M+H]⁺.

### Step 3: Synthesis of compound 36-3

Compound **36-2** (1 g, 2.32 mmol, 1 *eq*), cyclopropanecarboxamide (788.19 mg, 9.26 mmol, 4 *eq*), cesium carbonate (1.51 g, 4.63 mmol, 2 *eq*), and Xantphos (133.97 mg, 231.54 µmol, 0.1 *eq*) were dissolved in dioxane (10 mL). After replacing with nitrogen three times, Pd₂(dba)₃ (212.02 mg, 231.54 µmol, 0.1 *eq*) was added. The mixture was stirred at 120°C for 3 hours. The reaction mixture was added with water (10 mL) and extracted with dichloromethane (10 mL * 2). The organic phase was dried over anhydrous sodium sulfate, filtered, and the filtrate was concentrated under reduced pressure to obtain a crude product. The crude product was added with *tert*-butyl methyl ether (20 mL), stirred at 20°C for 1 hour, and then filtered. The filter cake was collected to obtain compound **36-3.** LCMS *m*/*z* = 481.3 [M+H]⁺.

### Step 4: Synthesis of compound 36-4

Compound **36-3** (1.42 g, 2.96 mmol, 1 *eq*) was dissolved in NMP (10 mL) and acetonitrile (5 mL). 1-Ethyl-3-(3-dimethylaminopropyl)carbodiimide hydrochloride (793.08 mg, 4.14 mmol, 1.4 *eq*), 1-hydroxybenzotriazole (199.65 mg, 1.48 mmol, 0.5 *eq*), methylamine hydrochloride (199.52 mg, 2.96 mmol, 1 *eq*), and methylimidazole (727.85 mg, 8.87 mmol, 706.65 µL, 3 *eq*) were then added thereto. The mixture was stirred at 65°C for 1 hour. The reaction mixture was added with water (10 mL) and extracted twice with dichloromethane (10 mL). The organic phase was dried over anhydrous sodium sulfate, filtered, and the filtrate was concentrated under reduced pressure to obtain a crude product. The crude product was purified by silica gel column chromatography (ethyl acetate/petroleum ether = 0 to 50%) to obtain compound **36-4.** LCMS *m*/*z* = 494.2 [M+H]⁺.

### Step 5: Synthesis of compounds WX-036A and WX-036B

Compound **36-4** (0.7 g, 1.42 mmol, 1 *eq*) was dissolved in methanol (14 mL). (Diacetoxyiodo)benzene (1.14 g, 3.55 mmol, 2.5 *eq*) and ammonium acetate (273.30 mg, 3.55 mmol, 2.5 *eq*) were added thereto, and the mixture was stirred at 15°C for 2 hours. The reaction mixture was added with water (15 mL) and extracted with dichloromethane (15 mL * 2). The organic phase was washed with 5% sodium thiosulfate aqueous solution (20 mL), dried over anhydrous sodium sulfate, filtered, and the filtrate was concentrated under reduced pressure to obtain a crude product. The crude product was purified by silica gel column chromatography (methanol/ethyl acetate = 0 to 10%) to obtain compound **WX-036.** Compound **WX-036** was subjected to resolution by SFC (column: Chiralpak IH (250 mm * 30 mm, 10 µm); mobile phase: A (CO₂) and B (ethanol containing 0.1% ammonia water); gradient: B% = 50% to 50%, 15 minutes) to obtain compound **WX-036A** and compound **WX-036B.**

**WX-036A:** ¹HNMR (400 MHz, CDCl₃) δ: 11.14 (s, 1H), 8.94 (s, 1H), 8.40 - 8.38 (m, 1H), 8.27 - 8.23 (m, 2H), 8.19 - 8.14 (m, 1H), 7.85 - 7.83 (m, 1H), 7.62 (dd, *J* = 8.0, 1.6 Hz, 1H), 7.42 - 7.38 (m, 1H), 4.08 - 4.01 (m, 2H), 3.56 (s, 3H), 3.07 (d, *J* = 5.2 Hz, 3H), 1.75 - 1.69 (m, 1H), 1.48 (d, *J* = 7.0 Hz, 3H), 1.42 (d, *J* = 6.8 Hz, 3H), 1.15 - 1.11 (m, 2H), 0.98 - 0.93 (m, 2H); LCMS *m*/*z* = 525.2 [M+H]⁺. SFC (column: Chiralpak IH-3, 3 µm, 0.46 cm id × 5 cm L; mobile phase: A (CO₂) and B (EtOH containing 0.1% isopropylamine); gradient: B% = 5 to 50%, 3 minutes; flow rate: 3.4 mL/min; wavelength: 220 nm; pressure: 100 bar, Rt = 1.615 min) with a chiral isomer excess of 100%.

**WX-036B:** ¹HNMR (400 MHz, CDCl₃) δ: 11.14 (s, 1H), 8.92 (s, 1H), 8.40 - 8.38 (m, 1H), 8.27 - 8.22 (m, 2H), 8.19 - 8.17 (m, 1H), 7.84 (dd, *J* = 8.0, 1.6 Hz, 1H), 7.62 (dd, *J* = 8.0, 1.6 Hz, 1H), 7.42 - 7.38 (m, 1H), 4.08 - 4.01 (m, 1H), 3.56 (s, 3H), 3.07 (d, *J* = 5.2 Hz, 3H), 1.74 - 1.69 (m, 2H), 1.48 (d, *J* = 6.8 Hz, 3H), 1.42 (d, *J* = 7.2 Hz, 3H), 1.15 - 1.11 (m, 2H), 0.98 - 0.94 (m, 2H); LCMS *m*/*z* = 525.2 [M+H]⁺. SFC (column: Chiralpak IH-3, 3 µm, 0.46 cm id × 5 cm L; mobile phase: A (CO₂) and B (EtOH containing 0.1% isopropylamine); gradient: B% = 5 to 50%, 3 minutes; flow rate: 3.4 mL/min; wavelength: 220 nm; pressure: 100 bar, Rt = 1.771 min) with a chiral isomer excess of 99.38%.

### Example 37

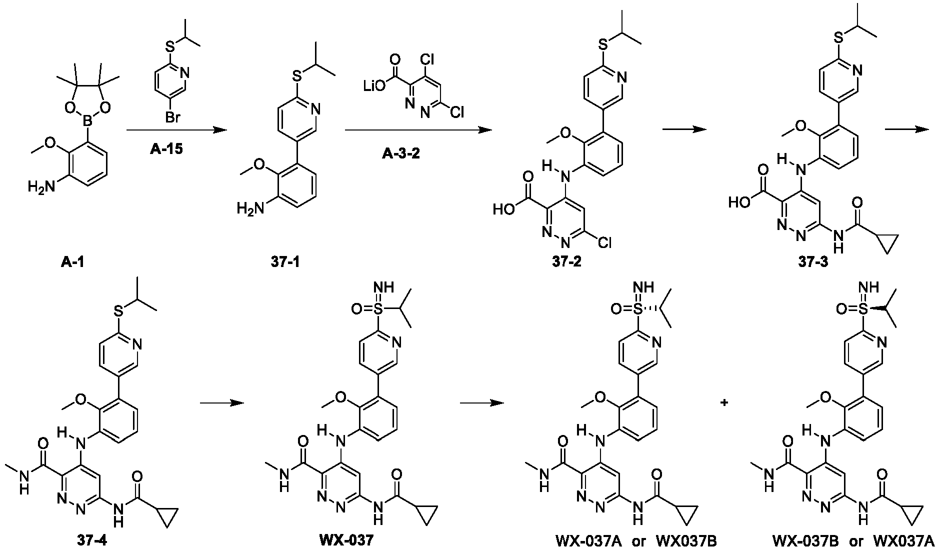

### Step 1: Synthesis of compound 37-1

Compound **A-1** (2 g, 8.03 mmol, 1 *eq*), compound **A-15** (1.12 g, 4.82 mmol, 0.6 *eq*), and potassium phosphate tribasic (5.11 g, 24.09 mmol, 3 *eq*) were dissolved in dioxane (20 mL) and water (10 mL). After replacing with nitrogen three times, Pd(dppf)Cl₂·CH₂Cl₂ (393.38 mg, 481.71 µmol, 0.06 *eq*) was added. The mixture was stirred at 100°C for 1.5 hours. The reaction mixture was added with water (10 mL) and extracted twice with ethyl acetate (20 mL). The organic phase was dried over anhydrous sodium sulfate, filtered, and the filtrate was concentrated under reduced pressure to obtain a crude product. The crude product was purified by silica gel column chromatography (ethyl acetate/petroleum ether = 0 to 50%) to obtain compound **37-1.** ¹HNMR (400 MHz, CDCl₃) δ: 8.67 - 8.65 (m, 1H), 7.76 (dd, *J* = 8.4, 2.4 Hz, 1H), 7.23 - 7.21 (m, 1H), 7.02 - 6.98 (m, 1H), 6.79 - 6.71 (m, 2H), 4.08 - 3.98 (m, 3H), 3.44 (s, 3H), 1.45 (d, *J* = 6.8 Hz, 6H); LCMS *m*/*z* = 275.0 [M+H]⁺.

### Step 2: Synthesis of compound 37-2

Compound **37-1** (950 mg, 3.46 mmol, 1 *eq*) and compound **A-3-2** (757.60 mg, 3.81 mmol, 1.1 *eq)* were dissolved in isopropanol (9.5 mL) and water (3.2 mL). Zinc acetate (762.32 mg, 4.15 mmol, 1.2 *eq)* was then added thereto. The mixture was stirred at 80°C for 16 hours. The reaction mixture was added with water (30 mL), filtered, and the filter cake was collected to obtain compound **37-2.** LCMS *m*/*z* = 431.0 [M+H]⁺.

### Step 3: Synthesis of compound 37-3

Compound **37-2** (1 g, 2.32 mmol, 1 *eq*), cyclopropanecarboxamide (790.00 mg, 9.28 mmol, 4 *eq*), and cesium carbonate (1.51 g, 4.64 mmol, 2 *eq)* were dissolved in dioxane (10 mL). After replacing with nitrogen three times, Xantphos (134.28 mg, 232.07 µmol, 0.1 *eq*) and Pd₂(dba)₃ (212.51 mg, 232.07 µmol, 0.1 *eq*) were added. The mixture was stirred at 120°C for 3 hours. The reaction mixture was added with water (10 mL) and extracted with dichloromethane (10 mL * 2). The organic phase was dried over anhydrous sodium sulfate, filtered, and the filtrate was concentrated under reduced pressure to obtain a crude product. The crude product was added with tert-butyl methyl ether (20 mL), stirred at 20°C for 1 hour, and then filtered. The filter cake was collected to obtain compound **37-3.** LCMS *m*/*z* = 480.3 [M+H]⁺.

### Step 4: Synthesis of compound 37-4

Compound **37-3** (1.06 g, 2.21 mmol, 1 *eq*) was dissolved in NMP (10 mL) and acetonitrile (5 mL). 1-Ethyl-3-(3-dimethylaminopropyl)carbodiimide hydrochloride (593.23 mg, 3.09 mmol, 1.4 *eq*), 1-hydroxybenzotriazole (149.33 mg, 1.11 mmol, 0.5 *eq*), methylamine hydrochloride (149.24 mg, 2.21 mmol, 1 *eq*), and N-methylimidazole (544.42 mg, 6.63 mmol, 528.57 µL, 3 *eq*) were then added thereto. The mixture was stirred at 65°C for 1 hour. The reaction mixture was added with water (10 mL) and extracted with dichloromethane (10 mL * 2). The organic phase was dried over anhydrous sodium sulfate, filtered, and the filtrate was concentrated under reduced pressure to obtain a crude product. The crude product was purified by silica gel column chromatography (ethyl acetate/petroleum ether = 0 to 50%) to obtain compound **37-4.** LCMS *m*/*z* = 493.2 [M+H]⁺.

### Step 5: Synthesis of compounds WX-037A and WX-037B

Compound **37-4** (0.9 g, 1.83 mmol, 1 *eq*) was dissolved in methanol (18 mL). (Diacetoxyiodo)benzene (1.47 g, 4.57 mmol, 2.5 *eq*) and ammonium acetate (352.08 mg, 4.57 mmol, 2.5 *eq*) were added thereto, and the mixture was stirred at 15°C for 2 hours. The reaction mixture was added with water (15 mL) and extracted with dichloromethane (15 mL * 2). The organic phase was washed with 5% sodium thiosulfate aqueous solution (20 mL), dried over anhydrous sodium sulfate, filtered, and the filtrate was concentrated under reduced pressure to obtain a crude product. The crude product was purified by silica gel column chromatography (methanol/ethyl acetate = 0 to 10%) to obtain compound **WX-037.** Compound **WX-037** was subjected to resolution by SFC (column: Chiralpak IH (250 mm * 30 mm, 10 µm); mobile phase: A (CO₂) and B (methanol containing 0.1% ammonia water); gradient: B% = 45% to 45%, 10 minutes) to obtain **WX-037A and WX-037B.**

**WX-037A:** ¹HNMR (400 MHz, CDCl₃) δ: 11.14 (s, 1H), 8.97 - 8.96 (m, 1H), 8.84 - 8.75 (m, 1H), 8.26 (s, 1H), 8.22 - 8.16 (m, 3H), 7.55 (dd, *J* = 8.0, 1.6 Hz, 1H), 7.35 - 7.31 (m, 1H), 7.23 - 7.21 (m, 1H), 3.83 - 3.76 (m, 1H), 3.46 (s, 3H), 3.07 (d, *J* = 5.2 Hz, 3H), 1.73 - 1.69 (m, 1H), 1.42 (d, *J* = 7.2 Hz, 3H), 1.36 (d, *J* = 6.8 Hz, 3H), 1.15 - 1.12 (m, 2H), 0.99 - 0.94 (m, 2H); LCMS *m*/*z* = 524.2 [M+H]⁺. SFC (column: Chiralpak IH-3, 3 µm, 0.46 cm id × 10 cm L; mobile phase: A (CO₂) and B (MeOH containing 0.1% isopropylamine); gradient: B% = 10% to 50%, 4 minutes; flow rate: 3.4 mL/min; wavelength: 220 nm; pressure: 100 bar), with Rt of 2.795 min, and a chiral isomer excess of 100%.

**WX-037B:** ¹HNMR (400 MHz, CDCl₃) δ: 11.15 (s, 1H), 9.03 - 8.78 (m, 2H), 8.38 - 8.08 (m, 4H), 7.56 - 7.53 (m, 1H), 7.35 - 7.31 (m, 1H), 7.22 - 7.21 (m, 1H), 3.85 - 3.76 (m, 1H), 3.46 (s, 3H), 3.07 (d, *J* = 4.4 Hz, 3H), 1.74 - 1.69 (m, 1H), 1.42 (d, *J* = 6.0 Hz, 3H), 1.42 (d, *J* = 6.4 Hz, 3H), 1.14 - 1.13 (m, 2H), 0.99 - 0.94 (m, 2H); LCMS *m*/*z* = 524.2 [M+H]⁺. SFC (column: Chiralpak IH-3, 3 µm, 0.46 cm id × 10 cm L; mobile phase: A (CO₂) and B (MeOH containing 0.1% isopropylamine); gradient: B% = 10% to 50%, 4 minutes; flow rate: 3.4 mL/min; wavelength: 220 nm; pressure: 100 bar), with Rt of 2.983 min, and a chiral isomer excess of 98.50%.

### Example 38

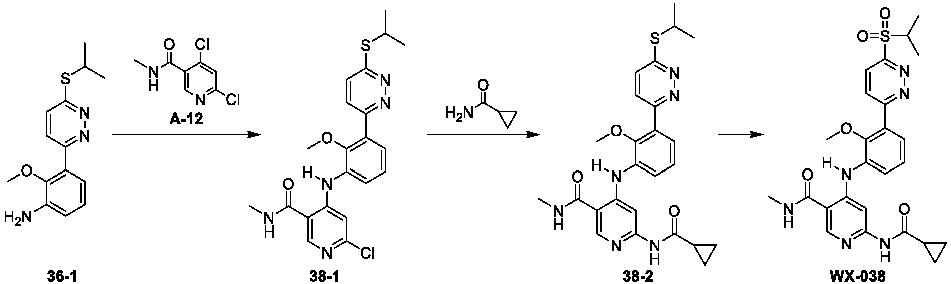

### Step 1: Synthesis of compound 38-1

Under a nitrogen atmosphere, compound **36-1** (1.82 g, 6.61 mmol, 1 *eq*) and **A-12** (1.36 g, 6.61 mmol, 1 *eq*) were sequentially added to tetrahydrofuran (50 mL), and the mixture was stirred until dissolved. LiHMDS (1 M, 16.52 mL, 2.5 *eq*) was slowly added dropwise thereto at 0°C, and the mixture was stirred at 25°C for another 2 hours. After the reaction was completed, the reaction mixture was added with water (100 mL) and extracted with ethyl acetate (100 mL * 2). The organic phases were combined, washed with saturated brine (100 mL * 2), dried over anhydrous sodium sulfate, filtered, and the filtrate was concentrated under reduced pressure to obtain a crude product. The crude product was purified by silica gel column chromatography (dichloromethane: methanol = 100:3) to obtain compound **38-1.** LCMS *m*/*z* = 444.0 [M+1]⁺.

### Step 2: Synthesis of compound 38-2

Compound **38-1** (2.1 g, 4.73 mmol, 1 *eq)* and cyclopropanecarboxamide (4.03 g, 47.30 mmol, 10 *eq)* were dissolved in dioxane (40 mL) and NMP (2 mL). Cesium carbonate (4.62 g, 14.19 mmol, 3 *eq)* and Xantphos (410.55 mg, 709.54 µmol, 0.15 *eq)* were then added thereto. After replacing with nitrogen three times, Pd₂(dba)₃ (649.74 mg, 709.54 µmol, 0.15 *eq)* was added. The mixture was stirred at 130°C for 16 hours. After the reaction was completed, the reaction mixture was cooled to room temperature, then added with water (40 mL), and extracted with ethyl acetate (40 mL * 2). The organic phase was washed with saturated brine (40 mL * 2), dried over anhydrous sodium sulfate, filtered, and the filtrate was concentrated under reduced pressure to obtain a crude product. The crude product was purified by silica gel column chromatography (dichloromethane: methanol = 10:1) to obtain compound **38-2.** LCMS *m*/*z* = 493.1 [M+1]⁺.

### Step 3: Synthesis of compound WX-038

Compound **38-2** (200 mg, 406.02 µmol, 1 *eq)* was added to a mixed solution of ethanol (10 mL) and water (5 mL), and potassium hydrogenperoxomonosulphate (374.41 mg, 609.02 µmol, 1.5 *eq)* was added thereto. The reaction was carried out at 25°C for 2 hours. The reaction mixture was directly filtered to obtain compound **WX-038.** ¹H NMR (400 MHz, CDCl₃) δ: 11.97 (br s, 1H), 9.39 (br s, 1H), 9.27 (s, 1H), 8.39 (d, *J* = 8.8 Hz, 1H), 8.27 - 8.16 (m, 2H), 7.97 (dd, *J* = 1.4, 7.9 Hz, 1H), 7.62 (d, *J* = 6.8 Hz, 1H), 7.44 (t, *J* = 7.9 Hz, 1H), 4.19 - 4.09 (m, 1H), 3.55 (s, 3H), 3.06 (d, *J*= 4.3 Hz, 3H), 2.09 - 1.97 (m, 1H), 1.48 (d, *J* = 7.0 Hz, 6H), 1.05 - 0.96 (m, 2H), 0.94 - 0.85 (m, 2H); LCMS *m*/*z* = 525.1 [M+1]⁺.

### Example 39

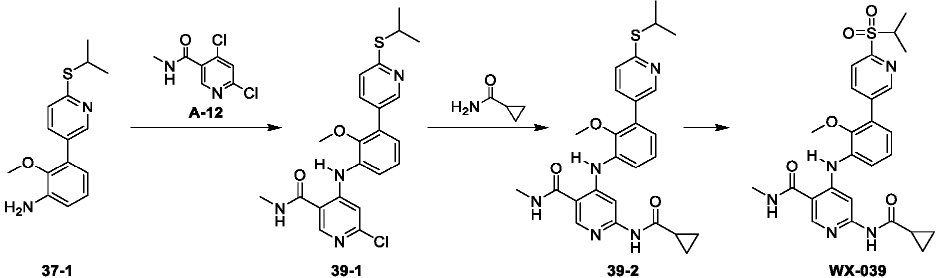

### Step 1: Synthesis of compound 39-1

Under a nitrogen atmosphere, compound **37-1** (532 mg, 1.94 mmol, 1 *eq*) and **A-12** (397.56 mg, 1.94 mmol, 1 *eq*) were sequentially added to tetrahydrofuran (20 mL), and the mixture was stirred until dissolved. LiHMDS (1 M, 4.85 mL, 2.5 *eq*) was slowly added dropwise thereto at 0°C, and the mixture was stirred at 25°C for another 2 hours. After the reaction was completed, the reaction mixture was added with water (50 mL) and extracted with ethyl acetate (50 mL * 2). The organic phases were combined, washed with saturated brine (100 mL * 2), dried over anhydrous sodium sulfate, filtered, and the filtrate was concentrated under reduced pressure to obtain a crude product. The crude product was purified by silica gel column chromatography (dichloromethane: methanol = 100:3) to obtain compound **39-1.** LCMS *m*/*z* = 443.0 [M+1]⁺.

### Step 2: Synthesis of compound 39-2

Compound **39-1** (786 mg, 1.77 mmol, 1 *eq*) and cyclopropanecarboxamide (1.51 g, 17.74 mmol, 10 *eq*) were dissolved in dioxane (20 mL) and NMP (0.5 mL). Cesium carbonate (1.73 g, 5.32 mmol, 3 *eq*) and Xantphos (154.01 mg, 266.16 µmol, 0.15 *eq*) were then added thereto. After replacing with nitrogen three times, Pd₂(dba)₃ (243.73 mg, 266.16 µmol, 0.15 *eq*) was added. The mixture was stirred at 130°C for 16 hours. After the reaction was completed, the reaction mixture was cooled to room temperature, then added with water (20 mL), and extracted with ethyl acetate (20 mL * 2). The organic phase was washed with saturated brine (20 mL * 2), dried over anhydrous sodium sulfate, filtered, and the filtrate was concentrated under reduced pressure to obtain a crude product. The crude product was purified by silica gel column chromatography (dichloromethane: methanol = 10:1) to obtain compound **38-2.** LCMS *m*/*z* = 492.2 [M+1]⁺.

### Step 3: Synthesis of WX-039

Compound **39-2** (200 mg, 406.83 µmol, 1 *eq*) was added to a mixed solution of ethanol (10 mL) and water (5 mL), and potassium hydrogenperoxomonosulphate (375.16 mg, 610.25 µmol, 1.5 *eq*) was added thereto. The reaction was carried out at 25°C for 2 hours. After the reaction was completed, the reaction mixture was added with saturated sodium bisulfite aqueous solution (10 mL), and extracted with ethyl acetate (20 mL * 2). The organic phase was washed with saturated brine (10 mL * 2), dried over anhydrous sodium sulfate, filtered, and the filtrate was concentrated under reduced pressure to obtain a crude product. The crude product was purified by silica gel column chromatography (DCM: MeOH = 10:1) to obtain compound **WX-039.** ¹H NMR (400 MHz, CDCl₃) δ: 10.58 (s, 1H), 9.10 - 8.90 (m, 1H), 8.35 (s, 2H), 8.28 - 8.21 (m, 1H), 8.20 - 8.14 (m, 2H), 7.62 (dd, *J* = 1.3, 8.0 Hz, 1H), 7.33 (t, *J* = 7.9 Hz, 1H), 7.16 (dd, *J* = 1.5, 7.8 Hz, 1H), 6.35 (br s, 1H), 3.89 - 3.79 (m, 1H), 3.49 (s, 3H), 3.05 (d, *J* = 4.8 Hz, 3H), 1.61 - 1.53 (m, 1H), 1.40 (d, *J* = 7.0 Hz, 6H), 1.15 - 1.07 (m, 2H), 0.96 - 0.88 (m, 2H); LCMS *m*/*z* = 524.1 [M+1]⁺.

### Biological test data

### Experimental example 1: Inhibitory activity of compounds on TYK2 JH2 pseudokinase

In this experiment, the inhibitory effect of compounds on TYK2 JH2 pseudokinase was tested using the method of time-resolved fluorescence resonance energy transfer (TR-FRET). In the experiment, TYK2 JH2 or JAK1 JH2 pseudokinase can simultaneously bind to fluorescently labeled Tracer and Tb antibody to form a "sandwich structure". Tb antibody, functioning as a fluorescent donor, produces fluorescence at a wavelength of 495 nm under the excitation of light at a specific wavelength. Tracer, functioning as a fluorescent acceptor, can receive fluorescence at a wavelength of 495 nm and thus produce fluorescence at a wavelength of 520 nm, *i.e.,* a time-resolved fluorescence resonance energy transfer (TR-FRET) signal, only when it is within the "sandwich structure", *i.e.,* when it is sufficiently close to Tb antibody. When the compound is added to compete with Tracer for binding to the pseudokinase, the binding of Tracer decreases, leading to a weakened TR-FRET signal. The inhibitory activity of the compound binding to the pseudokinase can be reflected by the ratio of signal values at 520 nm/495 nm.

### 1 Experimental reagents: see Table 10

**Table 10: Information on experimental reagents**

| Reagent name | Supplier | Storage conditions |
|---|---|---|
| TYK2/JAK1 | Bioduro | -80°C |
| Tracer | Bioduro | -80°C |
| Tb antibody | Cisbio | -80°C |
| HEPES | Invitrogen | 4°C |
| MgCl₂ 1 M | Sigma | Room temperature |
| Brij L23 solution (Brij-35) | Sigma | Room temperature |
| DTT | Sigma | -20°C |
| BSA | Sigma | 4°C |

### 2 Experimental methods

### 1) Preparation of 1X experimental working solution

The working solution consists of the following components: HEPES (pH 7.5) at a final concentration of 20 mM; MgCh at a final concentration of 10 mM; Brij-35 at a final concentration of 0.015%; DTT at a final concentration of 2 mM; and BSA at a final concentration of 50 µg/mL.

### 2) Experimental steps:

a) The compound was dissolved in DMSO to achieve a storage concentration of 10 mM.
b) Various concentrations of the compound, 200 times the final concentration, were prepared in a compound dilution plate and transferred to an Echo plate.
c) The Echo instrument was used to transfer 150 nL of the compound from the Echo plate to a 384-well experimental plate.
d) 5 µL of TYK2 JH2 kinase, at three times the final concentration, was added to the 384-well experimental plate.
e) 5 µL of Tb, at three times the final concentration, was added to the 384-well experimental plate.
f) 5 µL of Tracer, at three times the final concentration, was added to the 384-well experimental plate.
g) The plate was centrifuged for 30 seconds and then incubated at room temperature for 60 minutes.
h) The fluorescence signal values at 520 nm/495 nm were measured using an Envision plate reader (PerkinElmer).

### 3) Data analysis

Data analysis was performed using the XL-Fit software to determine the IC₅₀ of the compounds. The results are shown in Table 11:

**Table 11: Kinase half maximal inhibitory concentration IC₅₀ (nM)**

| **Test samples** | **TYK2 JH2** |
|---|---|
| **Hydrochloride of WX-001** | 0.27 |
| **Hydrochloride of WX-002** | 0.19 |
| **WX-003** | 0.34 |
| **WX-004** | 0.20 |
| **WX-011A** | 0.10 |
| **WX-011B** | 0.08 |
| **WX-012A** | 0.08 |
| **WX-012B** | 0.08 |
| **Hydrochloride of WX-018** | 0.13 |
| **WX-019** | 0.09 |
| **Hydrochloride of WX-020** | 0.08 |
| **WX-021** | 0.08 |

**Conclusion:** The compounds of the present disclosure exhibit good inhibitory effect on TYK2 JH2 pseudokinase.

### Experimental example 2: Inhibitory activity of compounds on the proliferation of Ba/F3-FL-TYK2-E957D and Ba/F3-TEL-TYK2 cells

Adenosine triphosphate (ATP) serves as a universal energy carrier in various life processes, representing the smallest unit of energy storage and transfer. The CellTiter-Glo^{™} Luminescent Cell Viability Assay Kit utilizes luciferase as a detection agent. During the luminescent process, luciferase requires the participation of ATP. By adding CellTiter-Glo^{™} reagent to the cell culture medium and measuring the luminescence, the light signal was directly proportional to the amount of ATP in the system, and ATP was positively correlated with the number of viable cells. Therefore, cell proliferation can be detected by measuring ATP content using the CellTiter-Glo kit. In this test, the cell lines used were Ba/F3-FL-TYK2-E957D and Ba/F3-TEL-TYK2. Ba/F3-FL-TYK2-E957D cells can stably express the exogenously introduced human TYK2-E957D gene, and the TYK2-E957D gene sequence contains both JH1 and JH2 domains. On the other hand, Ba/F3-TEL-TYK2 cells can stably express the exogenously introduced human TEL-TYK2 gene, and the TEL-TYK2 gene sequence contains only the JH1 domain of TYK2.

### IC₅₀ measurement process:

### 1) Cell culture

The cell lines were cultured in an incubator at 37°C with 5% CO₂. Regular passaging was performed, and cells in the logarithmic growth phase were selected for plating.

### 2) Preparation of compound storage plate

a) The test compounds were prepared into a 10 mM solution with DMSO, and then diluted to 0.3 or 1 mM with DMSO.
b) Preparation of 1000× compound storage plate (tube): The compounds were 3-fold serially diluted from the highest concentration to the lowest concentration, creating 9 different concentrations.
c) Preparation of 20× compound working solution: 49 µL of cell culture medium was added to a flat-bottomed 96-well transparent drug plate. From the 1000× compound storage plate, 1 µL of the compound was pipetted into the cell culture medium in the 96-well transparent drug plate. In the solvent control, 1 µL of DMSO was added. After the compound or DMSO was added, the mixture was homogenized using a multi-channel pipette.

### 3) Cell plating and drug administration

a) Cells were stained with trypan blue and viable cells were counted to ensure a cell viability of 90% or more.
b) 95 µL of cell suspension (2000 cells/well) was added to each well of the compound assay cell plate, and cell-free culture medium containing 0.1% DMSO was added to the Min control wells.
c) Administration in the compound assay cell plate: 5 µL of 20× compound working solution was added to the cell culture plate. 5 µL of DMSO-cell culture medium mixture was added to the Max control wells. The final concentration of DMSO was 0.1%.
d) The culture plate was then incubated in an incubator at 37°C with 5% CO₂ for 72 hours.

### 4) Cell viability assay using CellTiter-Glo luminescence method

The following steps were conducted in accordance with the instructions of the Promega CellTiter-Glo Luminescent Cell Viability Assay Kit (Promega-G7573).
a) CellTiter-Glo buffer was thawed and brought to room temperature.
b) CellTiter-Glo substrate was also brought to room temperature.
c) CellTiter-Glo buffer was added to a bottle of CellTiter-Glo substrate to dissolve the substrate, thereby preparing CellTiter-Glo working solution.
d) The mixture was slowly vortexed and shaken to be fully dissolved.
e) The cell culture plate was removed and allowed to equilibrate to room temperature for 10 minutes.
f) 50 µL (equal to half the volume of cell culture medium in each well) of CellTiter-Glo working solution was added to each well.
g) The culture plate was shaken on an orbital shaker for 2 minutes to induce cell lysis.
h) The plate was then left at room temperature for 10 minutes to stabilize the luminescent signal.
i) The luminescent signal was detected using a SpectraMax Paradigm plate reader.

### 5) Data processing

Data analysis was conducted using GraphPad Prism 5.0 software. Nonlinear S-curve regression was employed to fit the data and generate dose-response curves. From these curves, the IC₅₀ values were calculated. The data are shown in Table 12.

**Table 12: Half maximal inhibitory concentration IC₅₀ (nM) for cells**

| **Compound** | **Ba/F3-FL-TYK2-E957D** | **Ba/F3-TEL-TYK2** |
|---|---|---|
| **Hydrochloride of WX-001** | 13.7 | - |
| **Hydrochloride of WX-002** | 19.2 | - |
| **WX-004** | 5.5 | - |
| **WX-005** | 2.2 | - |
| **Hydrochloride of WX-006** | 10.6 | - |
| **Hydrochloride of WX-007** | 9.5 | - |
| **Hydrochloride of WX-008** | 16.0 | - |
| **Trifluoroacetate of WX-009** | 12.1 | - |
| **Hydrochloride of WX-010** | 12.6 | - |
| **WX-011A** | 5.3 | >10000 |
| **WX-011B** | 3.3 | >10000 |
| **WX-012A** | 3.3 | >10000 |
| **WX-012B** | 2.4 | >10000 |
| **WX-013** | 12.1 | - |
| **Trifluoroacetate of WX-014** | 10.9 | - |
| **Hydrochloride of WX-015** | 13.0 | - |
| **Hydrochloride of WX-016** | 11.7 | - |
| **Hydrochloride of WX-017** | 4.0 | - |
| **Hydrochloride of WX-018** | 4.2 | - |
| **WX-019** | 2.0 | >10000 |
| **Hydrochloride of WX-020** | 1.5 | >10000 |
| **WX-021** | 2.5 | >10000 |
| **WX-022** | 13.7 | - |
| **WX-023** | 13.8 | - |
| **WX-024** | 10.0 | - |
| **WX-025** | 12.7 | - |
| **Trifluoroacetate of WX-026** | 18.8 | - |
| **Hydrochloride of WX-027** | 18.3 | - |
| **WX-028** | 4.0 | - |
| **Hydrochloride of WX-029** | 3.8 | - |
| **WX-030** | 2.0 | - |
| **Hydrochloride of WX-031** | 2.6 | - |
| **Hydrochloride of WX-032** | 2.9 | - |
| **WX-033** | 0.1 | >10000 |
| **Trifluoroacetate of WX-034** | 0.1 | >10000 |
| **Trifluoroacetate of WX-035** | 6.7 | - |
| **WX-036A** | 8.3 | - |
| **WX-036B** | 17.3 | |
| **WX-037A** | 15.0 | - |
| **WX-037B** | 17.0 | - |
| **WX-038** | 3.0 | - |
| **WX-039** | 4.7 | - |

| | | |
|---|---|---|
| "-" indicates that the measurement was not conducted. | | |

**Conclusion:** The compounds of the present disclosure exhibit strong inhibitory activity against the proliferation of Ba/F3 cells transfected with the human TYK2-E957D gene (containing both JH1 and JH2 domains of TYK2). However, they have no inhibitory activity against the proliferation of Ba/F3 cells transfected with the human TEL-TYK2 gene (which contains only the JH1 domain of TYK2). This suggests that the compounds of the present disclosure are highly selective allosteric inhibitors of TYK2 JH2.

### Experimental example 3: Inhibitory activity of compounds on TYK2, JAK1/2, JAK2/2, and JAK1/3 signaling pathways in human PBMC cells

The purpose of this experiment was to detect the inhibitory effect of the compounds on cytokine-activated JAK-STAT signaling pathways in human peripheral blood mononuclear cells (PBMC).

### 1 Main reagents and instruments:

### 1) Cells: Human peripheral blood mononuclear cells (PBMC) (Supplier: Sailybio)

### 2) Reagents: see Table 13

**Table 13: Information on reagents**

| **Name** | **Supplier** |
|---|---|
| 1640 culture medium | Gibco |
| Non-essential amino acids | Gibco |
| Fetal bovine serum | ExCell Bio |
| Double antibiotic (Penicillin, Streptomycin) | Millipore |
| Human interleukin 6 (IL-6) | Absin |
| Human interferon α (IFN-α) | PBL Assay Science |
| Human interleukin 2 (IL-2) | Absin |
| Human granulocyte-macrophage colony-stimulating factor (GM-CSF) | Peprotech |
| Human CD4 antibody | Biolegend |
| Human CD33 antibody | Biolegend |
| Human pSTAT1 antibody | BD |
| Human pSTAT5 antibody | BD |
| Staining solution | Biolegend |
| Fixation buffer | BD |
| Permeabilization buffer | BD |
| Dulbecco's phosphate buffered saline (DPBS) | Corning |

### 3) Instruments

### Flow cytometer: Brand: BD; model: Fortessa

### 2 Reagent preparation

Culture medium: 1640 culture medium + 10% fetal bovine serum + 1% double antibiotic + 1% non-essential amino acids (all percentages are volume ratios)

### 3 Experimental steps

a) PBMCs frozen in liquid nitrogen were thawed in a 37°C water bath, and culture medium was added. The mixture was then centrifuged at 320 g for 3 minutes.
b) Cells were resuspended in culture medium, counted, and the cell concentration was adjusted to 5×10⁵ cells/mL with culture medium. Subsequently, the cell suspension was inoculated into two 96-well round-bottom plates, with 200 µL per well. The plates were incubated at 37°C with 5% CO₂ for 90 minutes.
c) Different concentrations of the test compound (starting at 2 µM, 5-fold serial dilution, a total of 8 concentrations) were added, followed by incubation at 37°C with 5% CO₂ for 30 minutes.
d) IL-6 (final concentration at 20 ng/mL) was added to the first round-bottom plate and incubated at 37°C with 5% CO₂ for 15 minutes.
e) IFNα (final concentration at 1000 U/mL) was added to the second round-bottom plate and incubated at 37°C with 5% CO₂ for 15 minutes.
f) IL-2 (final concentration at 4 ng/mL) was added to the third round-bottom plate and incubated at 37°C with 5% CO₂ for 15 minutes.
g) GM-CSF (final concentration at 20 pg/mL) was added to the fourth round-bottom plate and incubated at 37°C with 5% CO₂ for 15 minutes.
h) Cells were centrifuged at 320 g for 3 minutes, and each well was washed once with 200 µL of staining solution.
i) 50 µL of staining solution containing human CD33 antibody was added to each well of the GM-CSF-stimulated culture plate, and 50 µL of staining solution containing CD4 antibody was added to the remaining three culture plates. The staining was performed at 4°C for 30 minutes. The cells were washed twice with the staining solution.
j) 100 µL of fixation buffer was added to each well, and the cells were fixed at 4°C for 15 minutes. The cells were washed once with the staining solution.
k) 100 µL of permeabilization buffer was added to each well, and the cells were permeabilized at 4°C for 20 minutes. The cells were washed twice with the staining solution.
l) 50 µL of staining solution containing human pSTAT1 antibody was added to each well of the IL-6 and IFN-α-stimulated culture plates, and 50 µL of staining solution containing human pSTAT5 antibody was added to each well of the IL-2 and GM-CSF-stimulated culture plates. The staining was performed at room temperature for 15 minutes. The cells were washed twice with the staining solution.
m) The cells were resuspended in 150 µL of staining solution.
n) The fluorescence intensity of pSTAT1 (stimulated by IL-6 and IFN-α) or pSTAT5 (stimulated by IL-2) in CD4-positive cells was detected using flow cytometer. The fluorescence intensity of pSTAT5 (stimulated by GM-CSF) in CD33-positive cells was also detected.

### 4 Data analysis

Data analysis was performed using Flowjo software to determine the IC₅₀ of the compounds. The results are shown in Table 14:

**Table 14: Half maximal inhibitory concentration IC₅₀ (nM) for cells**

| **Compound** | **IFN-α/pSTAT1 (TYK2)** | **IL-6/pSTAT1 (JAK1/2)** | **GM-CSF/pSTAT5 (JAK2/2)** | **IL-2/pSTAT5 (JAK1/3)** |
|---|---|---|---|---|
| **WX-011A** | 2.6 | 87.0 | 1656 | 1311 |
| **WX-011B** | 2.4 | 72.5 | 1348 | 773 |
| **WX-012A** | 2.0 | 52.4 | - | - |
| **WX-012B** | 1.5 | 32.5 | - | - |
| **WX-019** | 1.5 | 99.7 | 1438 | 1576 |
| **Hydrochloride of WX-020** | 1.3 | 163.9 | 1072 | 1396 |
| **WX-021** | 2.1 | 63.4 | 580 | 540 |

| | | | | |
|---|---|---|---|---|
| "-" indicates that the measurement was not conducted. | | | | |

**Conclusion:** The compound of the present disclosure exhibits high inhibitory activity against the TYK2 signaling pathway activated by IFN-α stimulation in human PBMC cells. Meanwhile, it exhibits weak inhibitory activity against the JAK1/2 signaling pathway activated by IL-6 stimulation, the JAK2/2 signaling pathway activated by GM-CSF stimulation, and the JAK1/3 signaling pathway activated by IL-2 stimulation, thereby showing high selectivity.

### Experimental example 4: Pharmacokinetic testing of compounds in mice

### Experimental purpose:

The objective was to study the pharmacokinetic behavior of the compound of the present disclosure in mice and evaluate the pharmacokinetic characteristics, using male CD-1 mice aged 7 to 9 weeks as test animals. The drug concentrations in plasma at different time points after a single intravenous injection (IV) and oral administration (PO) of the compound were determined using LC/MS/MS.

### Experimental operation:

The pharmacokinetic characteristics of the compound in rodents following intravenous injection and oral administration were tested using a standard protocol. Test animals were fasted for 10 to 14 hours before administration and allowed to feed 4 hours post-administration. The compound was prepared into a clear solution with the appropriate solvent for both IV (intravenous injection) and PO (oral gavage) administration. The solvent was 10% DMSO + 10% solutol + 80% (10% HP-β-CD aqueous solution). Whole blood samples were collected within 24 hours, centrifuged at 6000 g for 3 minutes, and the supernatant was separated to obtain plasma samples. Protein precipitation was performed by adding four times the volume of acetonitrile solution containing internal standard to the plasma samples. After centrifugation, the supernatant was added with an equal volume of water and then centrifuged before being subjected to LC-MS/MS analysis for quantifying plasma drug concentration. Pharmacokinetic parameters such as peak concentration, time to peak concentration, clearance, half-life, area under the drug-time curve, and bioavailability were calculated.

The results of the pharmacokinetic parameters are shown in Table 15.

**Table 15: Results of pharmacokinetic testing in mice**

| | | **WX-011** | **WX-011A** | **WX-012** | **WX-019** | **WX-020 (Hydrochloride)** | **WX-021** |
|---|---|---|---|---|---|---|---|
| **IV** | **Dose (mg/kg)** | 1 | 3 | 1 | 3 | 3 | 3 |
| | **Starting concentration C₀ (nM)** | 5422 | 9915 | 2747 | 16325 | 8045 | 9889 |
| | **Half-life T_{1/2} (h)** | 3.2 | 4.2 | 4.4 | 1.1 | 2.3 | 2.9 |
| | **Apparent volume of distribution Vd (L/kg)** | 0.7 | 0.8 | 2.9 | 0.4 | 1.1 | 0.9 |
| | **Apparent clearance Cl (mL/Kg/min)** | 6.6 | 14.1 | 12.8 | 4.7 | 7.0 | 8.5 |
| | **Area under the curve AUC₀₋ₗₐₛₜ (nM.hr)** | 4791 | 6908 | 2462 | 21042 | 14130 | 11869 |
| **PO** | **Dose (mg/kg)** | 10 | 10 | 10 | 10 | 10 | 10 |
| | **Peak concentration Cₘₐₓ (nM)** | 10169 | 16814 | 12436 | 17335 | 15539 | 11337 |
| | **Time to peak concentration Tₘₐₓ (h)** | 0.5 | 0.3 | 0.3 | 0.8 | 0.5 | 0.3 |
| | **Area under the curve AUC₀₋ₗₐₛₜ (nM.hr)** | 24983 | 26715 | 16661 | 61215 | 56784 | 20720 |
| | **Bioavailability F%** | 52% | 51% | 68% | 87% | 121% | 52% |

**Conclusion:** The compounds of the present disclosure exhibit excellent pharmacokinetic properties in mice.

### Experimental example 5: Pharmacokinetic testing of compounds in rats

### Experimental purpose:

The objective was to study the pharmacokinetic behavior of the compound of the present disclosure in rats and evaluate the pharmacokinetic characteristics, using male SD rats aged 7 to 9 weeks as test animals. The drug concentrations in plasma at different time points after a single intravenous injection (IV) and oral administration (PO) of the compound were determined using LC/MS/MS.

### Experimental operation:

The pharmacokinetic characteristics of the compound in rodents following intravenous injection and oral administration were tested using a standard protocol. Test animals were fasted for 10 to 14 hours before administration and allowed to feed 4 hours post-administration. The compound was prepared into a clear solution with the appropriate solvent for both IV (intravenous injection) and PO (oral gavage) administration. The solvent was 10% DMSO + 10% solutol + 80% (10% HP-β-CD aqueous solution). Whole blood samples were collected within 24 hours, centrifuged at 6000 g for 3 minutes, and the supernatant was separated to obtain plasma samples. Protein precipitation was performed by adding four times the volume of acetonitrile solution containing internal standard to the plasma samples. After centrifugation, the supernatant was added with an equal volume of water and then centrifuged before being subjected to LC-MS/MS analysis for quantifying plasma drug concentration. Pharmacokinetic parameters such as peak concentration, time to peak concentration, clearance, half-life, area under the drug-time curve, and bioavailability were calculated.

The results of the pharmacokinetic parameters are shown in Table 16.

**Table 16: Results of pharmacokinetic testing in rats**

| | | **Hydrochloride of WX-011A** | **WX-020** |
|---|---|---|---|
| **IV** | **Dose (mg/kg)** | 1 | 3 |
| | **Starting concentration C₀ (nM)** | 4545 | 3026 |
| | **Half-life T_{1/2} (h)** | 0.6 | 0.9 |
| | **Apparent volume of distribution Vd (L/kg)** | 0.5 | 0.9 |
| | **Apparent clearance Cl (mL/Kg/min)** | 16.8 | 13.1 |
| | **Area under the curve AUC₀₋ₗₐₛₜ (nM.hr)** | 2075 | 2606 |
| **PO** | **Dose (mg/kg)** | 10 | 10 |
| | **Peak concentration Cₘₐₓ (nM)** | 4394 | 3377 |
| | **Time to peak concentration Tₘₐₓ (h)** | 0.5 | 0.8 |
| | **Area under the curve AUC₀₋ₗₐₛₜ (nM.hr)** | 8202 | 9762 |
| | **Bioavailability F%** | 41% | 39% |

**Conclusion:** The compounds of the present disclosure exhibit excellent pharmacokinetic properties in rats.

### Experimental example 6: Inhibition experiment of compounds on IFN-α-induced STAT1 phosphorylation in mouse whole blood

The purpose of this experiment was to detect the inhibitory effect of the compound on the JAK-STAT signaling pathway activated by IFN-α in mouse whole blood. Fresh mouse whole blood was collected and placed in a 96-well plate. The test compound was added and incubated for 1 hour, followed by stimulation with IFN-α. The corresponding STAT1 phosphorylation level in the CD3⁺ cell population was analyzed using flow cytometer by means of surface antibody staining and intracellular phosphorylated antibody staining. The IFN-α-induced STAT1 phosphorylation in mouse whole blood is dependent on TYK2 activity. By detecting the inhibitory activity of the compound on downstream STATS phosphorylation, the half maximal inhibitory concentration (IC₅₀) of the compound on TYK2 signaling pathway activity can be determined.

### 1 Main reagents and instruments

### 1) Main reagents: see Table 17

**Table 17: Information on reagents**

| **Name** | **Supplier** |
|---|---|
| Dimethyl sulfoxide | Sigma |
| Perm buffer III (permeabilization buffer) | BD Biosciences |
| Lyse/Fix buffer (lysis/fixation buffer) | BD Biosciences |
| EDTA (ethylenediaminetetraacetic acid) | Invitrogen |
| PBS (phosphate buffered saline) | BI |
| Brilliant Violet 421 anti-mouse CD3 antibody | Biolegend |
| Alexa Fluor647 anti-STAT1 phospho (Ser727) antibody | Biolegend |
| Recombinant mouse IFNalpha | Miltenyi |

### 2) Experimental consumables

### 96-well V-bottom microplate, Greiner; 96 square-well deep well plate, Thermo; 96-well flat-bottom microplate, Corning

### 3) Instruments

CO₂ incubator: MCO-15AC (Thermo);
Single-channel pipettes: 0.2 to 10 µL, 20 to 200 µL, 200 to 1000 µL (Thermo);
Multi-channel pipettes: 0.2 to 10 µL, 5 to 50 µL, 20 to 300 µL (Raining);
Centrifuges: Thermo Centrifuge ST 40R; Thermo LEGEND Micro 21R;
Water purification system: Millipore Milli-Q Reference system;
Vortex mixer: EARTH REQUIRED;
Shaker: QI LIN BEI ER; MH-2;
Flow cytometer: Beckman CytoFlex.

### 2 Experimental steps

### 1) Compound dilution

a) The compound was prepared into a 10 mM solution with dimethyl sulfoxide (DMSO), and diluted in DMSO to a solution with various concentrations, 500 times the final concentration.
b) 5 µL of the diluted compound was transferred to 120 µL of phosphate buffered saline (PBS) containing 0.1% bovine serum albumin (BSA).
c) Positive and negative control groups were set up, with both finally containing 0.2% DMSO.

### 2) Experimental process

a) 67.5 µL of mouse whole blood was added to each well of a 96-well cell culture plate.
b) 3.5 µL of the diluted compound was added thereto and mixed thoroughly.
c) The plate was incubated in a 37°C incubator for 60 minutes.
d) Mouse interferon recombinant protein was diluted 125-fold in PBS containing 0.1% BSA, and anti-mouse CD3 antibody was diluted 5-fold in PBS containing 0.1% BSA. 5 µL of diluted anti-mouse CD3 antibody and 4 µL of diluted mouse interferon recombinant protein were added to each well.
e) The plate was incubated in a 37°C incubator for 30 minutes.
f) All cells were transferred to a 96-well deep well plate, and 1 mL of 37°C pre-warmed 1x lysis/fixation buffer was added thereto.
g) The cells were incubated in the dark at 37°C for 10 minutes.
h) After centrifugation at 600 g for 5 minutes, the supernatant was discarded, and the cells were washed twice with 1 mL of PBS by centrifugation.
i) 0.4 mL per well of permeabilization buffer was added to the cell pellet and mixed thoroughly. The cells were incubated in the dark at 4°C for 30 minutes.
j) After centrifugation at 600 g for 5 minutes, the supernatant was discarded, and the cells were washed twice with 1 mL of flow cytometry staining buffer (PBS + 0.2% BSA + 1 mM EDTA) by centrifugation.
k) Anti-STAT1 antibody was diluted 65-fold in flow cytometry staining buffer, and added to cell-containing wells at 100 µL per well and mixed thoroughly.
l) The cells were incubated at room temperature for 40 minutes.
m) 1 mL per well of flow cytometry staining buffer was added, and the cells were washed twice by centrifugation at 600 g for 5 minutes.
n) The supernatant was discarded, and the cell pellet was resuspended in 300 µL of flow cytometry staining buffer.
o) The samples were loaded for analysis using the Beckman CytoFlex flow cytometer.

### 3 Data analysis

Data were analyzed using FlowJo software. Curve fitting was performed and the IC₅₀ was calculated using GraphPad Prism 8 software. The results are shown in Table 18.

**Table 18: Half maximal inhibitory concentration IC₅₀ (nM)**

| **Compound** | **IFN-α/pSTAT1 (TYK2)** |
|---|---|
| **WX-011A** | **148.2** |
| **WX-011B** | **375.0** |
| **WX-012B** | **418.6** |
| **WX-019** | **427.2** |
| **WX-020** | **369.3** |
| **WX-021** | **452.0** |
| **WX-033** | **315.9** |
| **Trifluoroacetate of WX-034** | **93.2** |

**Conclusion:** In mouse whole blood, the compound of the present disclosure exhibits high inhibitory activity against the TYK2 signaling pathway activated by IFNα stimulation.

### Experimental example 7: Inhibition experiment of compounds on cytokine-induced STAT phosphorylation in human whole blood/platelets

The purpose of this experiment was to detect the inhibitory effect of the compound on the JAK-STAT signaling pathway activated by cytokines in human whole blood or platelet-rich plasma. Fresh human whole blood/platelets were placed in a 96-well plate. The test compound was added and incubated for 1 hour, followed by stimulation with various cytokines. The corresponding STAT phosphorylation levels in different cell populations were analyzed using flow cytometer by means of surface antibody staining and intracellular phosphorylated antibody staining. The experiments using the cytokines IFN-α, IL-6, and IL-2 as stimulants were conducted in human whole blood, whereas the experiment using the cytokine TPO as a stimulant was conducted in platelet-rich plasma.

### 1 Main reagents: see Table 19

**Table 19: Information on reagents**

| **Name** | **Supplier** |
|---|---|
| 1640 culture medium | BI |
| Alexa Fluor 647 Mouse Anti-Stat5 (pY694) (AF647-labeled mouse anti-STATS (pY694) antibody) | BD |
| FITC Mouse Anti-Human CD3 (FITC-labeled mouse anti-human CD3 antibody) | BD |
| PE anti-human CD61 Antibody (PE-labeled mouse anti-human CD61 antibody) | Biolegend |
| Alexa Fluor 647 mouse IgG1, k Isotype Ctrl antibody (AF647-labeled mouse IgG1 isotype control antibody) | Biolegend |
| Recombinant human IL-2 | PEPROTECH |
| Universal Type I IFN (1MU) (IFN-α) | R&D |
| Recombinant Human TPO (thrombopoietin) | Stem cell |
| 96 Well Microplate | Beaver |
| 2 mL 96 Well Standard Certified RNase/DNase Free Non-Sterile (2 mL 96-well deep well plate) | Costar |
| Phosflow Lyse/Fix Buffer 5X (5X concentrated fixation buffer for lysing red blood cells) | BD |
| Perm Buffer III (permeabilization buffer III) | BD |

### 2 Experimental steps

### 1) Cytokine-induced phosphorylation experiment in human whole blood

a) Fresh blood from volunteers was collected using EDTA anticoagulant tubes.
b) 90 µL per well of whole blood was rapidly seeded into a 96-well plate and incubated in a cell culture incubator for 15 minutes. 10 µL of the test compound was added thereto, with each assay performed in single wells. Both the negative control group and the positive control group were added with an equal volume of 1640 culture medium, equivalent to the DMSO content in the experimental group, with each assay performed in triplicate. The plate was then incubated in the incubator for 60 minutes.
c) After incubation, 25 µL of IL-2 at a final concentration of 20 ng/mL, IL-6 at a final concentration of 50 ng/mL, or IFN-α at a final concentration of 1000 U/mL was added to each well to induce stimulation. The negative control group was added with an equal volume of 1640 culture medium. The plate was then incubated in the incubator for 15 minutes.
d) 100 µL of 1× Lyse/Fix buffer was added to each well, and the plate was placed on ice to stop the reaction. The samples were then transferred to a 96-well deep well plate containing 10 times the volume of the blood sample of 37°C pre-warmed 1× Lyse/Fix buffer, mixed thoroughly, and left to stand at 37°C for 15 minutes.
e) 500 µL of ice-cold PBS was added to each well, followed by centrifugation at 500×g for 8 minutes. The supernatant was then discarded.
f) Cells were resuspended in 250 µL of pre-cooled PBS and transferred to a 96-well shallow well plate. The plate was centrifuged at 500×g for 8 minutes, and the supernatant was discarded.
g) The cell pellet was washed once with pre-cooled PBS.
h) After discarding the supernatant, anti-human CD3 antibody was added thereto, followed by incubation at room temperature for 40 minutes.
i) The plate was centrifuged at 500×g for 8 minutes, then the supernatant was discarded, and the cells were washed twice with 200 µL of pre-cooled PBS.
j) The cells were resuspended in 200 µL of pre-cooled Perm Buffer III per well and permeabilized on ice for 60 minutes.
k) The plate was centrifuged at 600×g for 8 minutes, then the supernatant was discarded, and the cells were washed twice with pre-cooled PBS.
l) Intracellular staining with anti-pSTAT5 antibody was carried out, followed by incubation at room temperature for 60 minutes.
m) The cells were centrifuged at 600×g for 8 minutes, washed twice with PBS, resuspended in 200 µL of PBS, and detected using the CytoFlex S flow cytometer.
n) Data analysis was conducted using FlowJo and GraphPad Prism 8 software.

### 2) Cytokine-induced phosphorylation experiment in human platelet

a) Fresh blood from volunteers was collected using EDTA anticoagulant tubes and centrifuged at 200 g for 20 minutes to prepare platelet-rich plasma (PRP).
b) 90 µL per well of PRP was rapidly seeded into a 96-well plate and incubated in a cell culture incubator for 15 minutes. 10 µL of the test compound was added thereto, with each assay performed in single wells. Both the negative control group and the positive control group were added with an equal volume of 1640 culture medium, equivalent to the DMSO content in the experimental group, with each assay performed in triplicate. The plate was then incubated in the incubator for 60 minutes.
c) 25 µL of TPO at a final concentration of 200 ng/mL was added to induce stimulation. The negative control group was added with an equal volume of 1640 culture medium. The plate was then incubated in the incubator for 15 minutes.
d) After incubation, the plate was centrifuged at 1000×g for 10 minutes, then the supernatant was discarded, and the cells were washed twice with 200 µL of PBS.
e) PE-anti-human CD61 antibody was added to each well, followed by incubation at room temperature for 40 minutes.
f) After incubation, the plate was centrifuged at 1000×g for 10 minutes, then the supernatant was discarded, and the cells were washed twice with 200 µL of PBS.
g) The cells were resuspended in 200 µL of pre-cooled Perm Buffer III and permeabilized on ice for 60 minutes.
h) The plate was centrifuged at 1000×g for 10 minutes, then the supernatant was discarded, and the cells were washed twice with pre-cooled PBS.
i) Intracellular anti-pSTAT5 phosphorylated antibody was added thereto, followed by incubation at room temperature for 60 minutes.
j) The cells were washed twice with PBS and resuspended in 200 µL of PBS, and then detected using the CytoFlex S flow cytometer.

### 3 Data analysis

Data were analyzed using FlowJo software. Curve fitting was performed and the IC₅₀ was calculated using GraphPad Prism 8 software. Data were presented as mean and standard deviation (SD). The inhibition rate of the compound was defined as follows: Inhibition% = (1 - (A - B) / (C - B)) * 100. Where: Ais the MFI (mean fluorescence intensity) for experimental wells containing both the compound and the cytokine; B is the MFI (minimum mean fluorescence intensity) for control wells without the cytokine; C is the MFI (maximum mean fluorescence intensity) for control wells containing only the cytokine. The results are shown in Table 20.

**Table 20: Half maximal inhibitory concentration IC₅₀ (nM)**

| **Compound** | **IFN-α/pSTAT5 (TYK2)** | **IL-6/pSTAT3 (JAK1/2)** | **TPO/pSTAT5 (JAK2/2)** | **IL-2/pSTAT5 (JAK1/3)** |
|---|---|---|---|---|
| **WX-011A** | 23.1 | 10754 | >100000 | 11188 |
| **WX-019** | 29.7 | 33851 | >100000 | 33754 |
| **WX-020** | 40.3 | >100000 | >100000 | >100000 |

**Conclusion:** In human whole blood or platelets, the compound of the present disclosure exhibits high inhibitory activity against the TYK2 signaling pathway activated by IFNα stimulation. Meanwhile, it exhibits weak inhibitory activity against the JAK1/2 signaling pathway activated by IL-6 stimulation, the JAK2/2 signaling pathway activated by TPO stimulation, and the JAK1/3 signaling pathway activated by IL-2 stimulation, thereby showing high selectivity.

### Experimental example 8: Pharmacodynamic study of compounds on IL-12/IL-18-induced IFN-γ secretion by mouse immune cells in an in vivo model

The purpose of this experiment was to evaluate the inhibitory effect of the compound on IL-12/IL-18-induced IFN-γ secretion by immune cells in mice. The IFN-γ protein level in serum samples of mice treated with the compound was detected using the enzyme-linked immunosorbent assay (ELISA) method.

### 1 Experimental materials

### a) Experimental animals

C57BL/6J mice, aged 9 to 10 weeks, weighing 17.82 to 21.42 g, female, supplied by Shanghai Lingchang Biotechnology Co., Ltd.

### b) Information on recombinant proteins: see Table 21

**Table 21: Information on recombinant proteins**

| **Product name** | **Supplier** |
|---|---|
| Recombinant Mouse IL-12 Protein | R&D |
| Recombinant Mouse IL-18/IL-1F4 Protein | R&D |
| Mouse IFN-gamma Quantikine ELISA Kit | R&D |

### 2 Experimental methods and procedures

### 1) Experimental design and drug preparation

This experiment was conducted twice. The specific grouping of experimental animals and administration regimen are shown in Tables 22 and 23, respectively.

The compounds were all prepared into a clear solution with 10% DMSO + 10% solutol + 80% (10% HP-β-CD) as a solvent for PO (oral gavage) administration. The frequency of administration was once daily (QD).

Preparation of recombinant mouse IL-12 protein: An appropriate amount of antibody was transferred to a centrifuge tube, and a certain volume of PBS buffer was added to prepare a solution with a concentration of 0.1 µg/mL. The solution was gently shaken to mix and used immediately.

Preparation of recombinant mouse IL-18/IL-1F4 protein: An appropriate amount of antibody was transferred to a centrifuge tube, and a certain volume of PBS buffer was added to prepare a solution with a concentration of 10 µg/mL. The solution was gently shaken to mix and used immediately.

For the Normal group, the administration consisted of PO (oral gavage) with blank solvent and intraperitoneal injection of blank PBS solution. For the blank control group, the administration consisted of PO (oral gavage) with blank solvent and intraperitoneal injection of inducer IL-12/IL-18.

**Table 22: Grouping of experimental animals and administration regimen (first experiment)**

| **Group** | **Drug** | **Dose (mg/kg)** | **Administration volume (mL/kg)** | **Frequency of administration** | **N** |
|---|---|---|---|---|---|
| 1 | Normal group | / | 10 | QD | 3 |
| 2 | Blank control group | / | 10 | QD | 6 |
| 3 | WX-011A | 3 | 10 | QD | 6 |
| 4 | WX-011A | 10 | 10 | QD | 6 |

**Table 23: Grouping of experimental animals and administration regimen (second experiment)**

| **Group** | **Drug** | **Dose (mg/kg)** | **Administration volume (mL/kg)** | **Frequency of administration** | **N** |
|---|---|---|---|---|---|
| 1 | Normal group | / | 10 | QD | 3 |
| 2 | Blank control group | / | 10 | QD | 6 |
| 3 | WX-020 | 3 | 10 | QD | 6 |
| 4 | WX-020 | 10 | 10 | QD | 6 |

### 2) Administration and sample collection

a) According to the grouping, the mice were administered by gavage (PO) with either the compound formulation or solvent control.
b) One hour later, each mouse received an intraperitoneal injection of IL-12 (0.01 µg per mouse) or PBS buffer.
c) One hour after IL-12 administration, each mouse received an intraperitoneal injection of IL-18 (1 µg per mouse) or PBS buffer.
d) Blood was collected three hours after IL-18 administration, and serum was separated.

### 3) ELISA testing of serum samples

a) Prior to use, all reagents were mixed thoroughly to avoid foaming.
b) The number of strips required was determined based on the number of experimental wells (blanks and standards). Duplicate tests were performed for samples (including standards) and blanks.
c) Sample addition: 100 µL/well of the diluted Cytokine standard was added to the standard wells, 100 µL/well of the sample was added to the sample wells, and 100 µL/well of Dilution buffer R (1×) was added to the blank control wells.
d) Detection antibody addition: 50 µL/well of Biotinylated antibody working solution was added. After mixing, the plate was covered with a sealing film and incubated at 37°C for 90 minutes.
e) Plate washing: The liquid was discarded from the wells, then 300 µL/well of 1× Washing buffer working solution was added, and allowed to stand for 1 minute before the liquid was discarded from the wells. This process was repeated 4 times, with drying on filter paper each time.
f) Enzyme addition: 100 µL/well of Streptavidin-HRP working solution was added. The plate was covered with a sealing film and incubated at 37°C for 30 minutes.
g) Plate washing: Step 5 was repeated.
h) Color development: 100 µL/well of TMB was added, then the plate was incubated at 37°C in the dark for 5 to 30 minutes, and the reaction was terminated depending on the intensity of the color (dark blue) in the well. Typically, a color development period of 10 to 20 minutes was found to yield optimal results.
i) Reaction termination: 100 µL/well of Stop solution was quickly added to terminate the reaction.
j) Plate reading: Within 10 minutes after termination, the values were read at a measurement wavelength of 450 nm. Dual wavelengths were recommended for reading the plate, specifically measuring at a measurement wavelength of 450 nm and a reference wavelength of 610 to 630 nm simultaneously.

### 4) Data analysis

The expression level of each sample was calculated based on the standard curve, and statistical analysis of the results for each group was performed, including mean and standard error of the mean (SEM). The signal values for each group were normalized against the blank control group. Statistical analysis was then conducted to assess differences between groups based on this data. A T-test was used for analysis, and for comparisons among three or more groups, one-way ANOVA was employed. All data were analyzed using GraphPad Prism 6.02. The inhibition rate of serum IFN-γ following a single administration of the compound is shown in Table 24.

**Table 24: Inhibition rate of serum IFN-γ following single administration of the compound**

| **Compound** | **Dose** | **Serum IFN-γ inhibition rate (%)** |
|---|---|---|
| **WX-011A** | 3 mg/kg | 64% |
| **WX-011A** | 10 mg/kg | 96% |
| **WX-020** | 3 mg/kg | 40% |
| **WX-020** | 10 mg/kg | 86% |

**Conclusion:** The compound of the present disclosure exhibits a significant dose-dependent inhibitory effect on IL-12/IL-18-induced IFNγ release in mice.

### Experimental example 9: Pharmacodynamic experiment in a mouse model of acute colitis induced by CD40 antibody

The purpose of this experiment was to evaluate the alleviating effect of the compound on colitis in a mouse model induced by CD40 antibody.

### 1 Experimental materials

### 1) Experimental animals

CB-17 SCID mice, 8 weeks old, weighing 18 to 20 g, female, supplied by Beijing Vital River Laboratory Animal Technology Co., Ltd.

### 2) Reagents: see Table 25

**Table 25: Information on reagents**

| **Reagent** | **Source** |
|---|---|
| Anti-CD40 antibody | BioXCell |
| IL-12 P40 antibody | BioXCell |
| Occult blood test reagent (Piramidon method) | Baso Diagnostic Inc |
| Saline | Shandong Kelun Phaemaceutical |
| Dulbecco's phosphate buffered saline (DPBS) | Corning |

### 2 Experimental methods

### 1) Experimental design

CB-17 SCID mice were randomly divided into groups. Each group consisted of 6 mice, which received an intraperitoneal injection (IP) of CD40 antibody (80 µg per mouse) on Day 0. The positive control drug IL-12 P40 antibody and the test compound were administered from Day-1 to Day 4, with Day 5 being the endpoint of the experiment. The specific grouping of animals is shown in Table 26. The positive control drug IL-12 P40 antibody was administered via intraperitoneal injection (IP) once every three days; the test compound was administered by gavage (PO) twice a day (BID).

**Table 26: Animal grouping and administration regimen**

| **Group** | **Drug** | **Dose (mg/kg)** | **Administration volume (mL/kg)** | **Route of administration** | **Frequency of administration** | **Number of animals** |
|---|---|---|---|---|---|---|
| 1 | Blank control group | --- | 10 | PO | BID | 6 |
| 2 | IL-12 P40 antibody | 20 | 10 | IP | Once/3 days | 6 |
| 3 | Hydrochloride of WX-011A | 30 | 10 | PO | BID | 6 |
| 4 | Hydrochloride of WX-011A | 50 | 10 | PO | BID | 6 |
| 5 | Sulfate of WX-020 | 50 | 10 | PO | BID | 6 |
| 6 | Sulfate of WX-020 | 100 | 10 | PO | BID | 6 |

### 2) Drug preparation

The compounds were all prepared into a clear solution with 10% DMSO + 10% solutol + 80% (10% HP-β-CD) as a solvent for PO (oral gavage) administration. Administration volume parameters: 10 mL/kg based on the body weight of the mice.

Preparation of IL-12 P40 antibody: An appropriate amount of IL-12 P40 antibody stock solution was transferred to a 15 mL centrifuge tube, and a certain volume of DPBS was added to prepare a working solution with a concentration of 2 mg/mL. The solution was gently shaken to mix and used immediately.

### 3) Model construction

IL-12 P40 antibody and the test compound were administered from Day-1 to Day 4. On Day 0, 30 minutes after administration, 200 µL of CD40 antibody (4 mg/kg) was intraperitoneally injected, while mice in the blank control group were intraperitoneally injected with 200 µL of DPBS. Body weight was recorded and feces were scored during the experiment.

### 4) Description of fecal observation process in animals

Fecal scoring involved placing each mouse individually in a specially designed cage for about 10 minutes for scoring. The fecal scoring criteria were as per Table 27.

### 5) Methodology description for fecal occult blood testing

If blood is observed in the feces or around the anus, occult blood testing is not conducted. For the remaining mice without visible bloody stools, feces are collected for occult blood testing. Occult blood scoring is based on the assumption that the daily test results for the mice in the Normal group are 0. Each day, the fecal samples of four mice from the blank control group are observed for the time taken to develop color on the occult blood test strips. The shortest time required for color development in the feces of these four mice is set as the daily threshold. If the feces develop color before this time point and the color intensifies within 1 to 2 minutes, a score of 2 is assigned. If no visible color or only a weak color is seen within this threshold time, and later a color develops but is significantly less intense than that of the feces scoring 2, a score of 1 is assigned.

### 4 Data acquisition

The body weight and Disease Activity Index (DAI) scores of the animals were recorded daily to assess the disease progression in each group and the effect of the test compound on the disease. The daily Disease Activity Index (DAI) score consists of three parts, with specific criteria referenced in Table 27. The results are shown in Table 28. DAI scores are shown in Table 29.

Note: Mean refers to the average value.

**Table 27: DAI scoring criteria**

| **Score** | **Weight loss %** | **Stool consistency** | **Occult blood or bloody stools** |
|---|---|---|---|
| 0 | 0 | Normal | Occult blood negative |
| 1 | 1 to 5 | Soft stool | Occult blood weak positive |
| 2 | 6 to 10 | Loose stool | Occult blood positive |
| 3 | 11 to 20 | Watery stool | Small amount of blood |
| 4 | >20 | Watery diarrhea | Large amount of blood |

**Table 28: Mouse weight change rate (%)**

| Group | | Weight change rate (%) | | | | | | |
|---|---|---|---|---|---|---|---|---|
| | Day | -1 | 0 | 1 | 2 | 3 | 4 | 5 |
| Blank control group | Mean | 100.00 | 97.58 | 98.63 | 89.07 | 86.11 | 92.24 | 93.88 |
| IL-12 P40 antibody, 20 mg/kg | Mean | 100.00 | 100.28 | 101.07 | 98.73 | 102.65 | 104.08 | 108.98 |
| Hydrochloride of WX-011A 50 mg/kg, BID | Mean | 100.00 | 100.63 | 99.54 | 97.69 | 100.43 | 100.96 | 99.34 |
| Hydrochloride of WX-011A 30 mg/kg, BID | Mean | 100.00 | 100.60 | 100.01 | 95.93 | 98.85 | 99.69 | 97.07 |
| Sulfate of WX-020 100 mg/kg, BID | Mean | 100.00 | 100.78 | 99.88 | 97.14 | 99.65 | 101.16 | 100.90 |
| Sulfate of WX-020 50 mg/kg, BID | Mean | 100.00 | 100.48 | 98.37 | 95.22 | 100.73 | 100.81 | 100.44 |

**Table 29: DAI scores**

| Group | | DAI score | | | | | | |
|---|---|---|---|---|---|---|---|---|
| | Day | -1 | 0 | 1 | 2 | 3 | 4 | 5 |
| Blank control group | Mean | 0.00 | 0.67 | 1.33 | 4.33 | 5.17 | 3.17 | 2.67 |
| IL-12 P40 antibody, 20 mg/kg | Mean | 0.00 | 0.33 | 0.33 | 1.17 | 0.67 | 0.00 | 0.00 |
| Hydrochloride of WX-011A 50 mg/kg, BID | Mean | 0.00 | 0.33 | 1.00 | 1.83 | 0.67 | 0.50 | 1.17 |
| Hydrochloride of WX-011A 30 mg/kg, BID | Mean | 0.00 | 0.17 | 1.00 | 2.17 | 0.83 | 0.50 | 1.00 |
| Sulfate of WX-020 100 mg/kg, BID | Mean | 0.00 | 0.33 | 1.33 | 1.67 | 0.50 | 0.33 | 0.33 |
| Sulfate of WX-020 50 mg/kg, BID | Mean | 0.00 | 0.33 | 1.17 | 2.67 | 0.83 | 0.33 | 0.50 |

Conclusion: In the mouse model of colitis induced by CD40 antibody, the compound of the present disclosure significantly outperformed the blank control group in controlling weight loss and reducing DAI scores at various doses. The compound of the present disclosure exhibits a significant alleviating effect on mouse colitis induced by CD40 antibody.

### Experimental example 10: Pharmacodynamic experiment in a mouse psoriasis-like model induced by IL-23

The purpose of this experiment was to evaluate the prophylactic and therapeutic effect of the test substance on IL-23-induced psoriasis-like skin lesions in mice by intradermal injection of IL-23 into the ear pinnae in a psoriasis-like skin lesion model.

### 1 Experimental materials

### 1) Experimental animals

C57BL/6 mice, SPF grade, weighing 19±2 g, female, sourced from the Laboratory Animal Business Department of Shanghai Institute of Planned Parenthood Research. The acclimatization period is 5 to 7 days.

**2) Reagents: see Table 30**

**Table 30: Information on reagents**

| **Reagent** | **Source** |
|---|---|
| Recombinant human interleukin-23 (IL-23) | Novoprotein |
| Ustekinumab (anti-IL-12/IL-23) | Selleck |
| Sterile water for injection | Guangdong Aixida Pharmaceutical Co., Ltd. |
| Saline | Chimin Health Management Co., Ltd. |

### 3 Experimental methods

### 1) Experimental design

Female C57BL/6 mice of qualified body weight were randomly divided into groups, with 6 mice per group. The grouping and administration information was as follows. The groups were: blank control group, model group, positive control Ustekinumab (5 mg/kg) group, WX-011A hydrochloride (30, 50 mg/kg) group, and WX-020 sulfate (30, 50 mg/kg) group. Except for the blank control group, the animals in the other groups received an intradermal injection of IL-23 (3 µg/10 µL/mouse/day, QD) in the right ear for 8 consecutive days (Day 0 to Day 7). The blank control group received an intradermal injection of an equal volume of saline solution (10 µL/mouse/day, QD) in the right ear for 8 consecutive days (Day 0 to Day 7). Concurrent with model induction, the solvent or test drug was orally administered twice a day (Bid) at 6-hour intervals for 8 consecutive days (Day 0 to Day 7). The positive control was subcutaneously injected on Day 0 and Day 3 (a total of 2 doses). During the administration period, the body weight of the animals was monitored every two days. On Day 0, Day 2, Day 4, Day 6 (all before IL-23 injection), and Day 8, the thickness of the right ear of the mice was measured, and the appearance of the ear pinnae was observed and scored. Animal grouping and administration regimen are shown in Table 31.

**Table 31: Animal grouping and administration regimen**

| Group | Drug | Single dose (mg/kg) | Administration volume (mL/kg) | Route and frequency of administration |
|---|---|---|---|---|
| 1 | Blank control group | Solvent | 10 | POBID (Administered at 6-hour intervals) |
| 2 | Model group | Solvent | 10 | |
| 3 | Ustekinumab (anti-IL-12/IL-23) | 5 | 10 | SC (Administered on Day 0 and Day 3) |
| 4 | Hydrochloride of WX-011A | 30 | 10 | POBID (Administered at 6-hour intervals) |
| 5 | | 50 | 10 | |
| 6 | Sulfate of WX-020 | 30 | 10 | |
| 7 | | 50 | 10 | |

| | | | | |
|---|---|---|---|---|
| *SC: subcutaneous injection; PO: oral administration; BID: twice a day | | | | |

### 2) Instruments: see Table 32

**Table 32: Instrument information**

| **Instruments** | **Model** | **Manufacturer** |
|---|---|---|
| Electronic digital micrometer | 0-10X30 | Zhejiang Deqing Shengtaixin Electronic Technology Co., Ltd. |
| Electric ear edema piercer | YLS-25A | Jinan Yiyan Technology Development Co., Ltd. |

### 3) Drug preparation

The compounds were all prepared into a clear solution with 10% DMSO + 10% solutol + 80% (10% HP-β-CD) as a solvent for PO (oral gavage) administration. Administration volume parameters: 10 mL/kg based on the body weight of the mice.

For the preparation of inducer IL-23, 3 vials were taken. 1.667 mL of sterile water for injection was added to each vial (500 µg), shaken and mixed, then transferred to a 10 mL EP tube, and shaken and mixed to obtain IL-23 solution at a concentration of 0.3 mg/mL. The solution was divided into 8 portions, aliquoted, and stored in a -80°C freezer.

Ustekinumab (anti-IL-12/IL-23) preparation: 0.15 mL ofUstekinumab (anti-IL-12/IL-23) with a concentration of 5 mg/mL was pipetted and diluted with 1.35 mL of PBS (pH 7.2) solution to obtain Ustekinumab (anti-IL-12/IL-23) solution at a concentration of 0.5 mg/mL.

### 3) Model construction and administration

After grouping, IL-23 (3 µg/10 µL/mouse/day, QD) was injected intradermally into the right ears of the mice for 8 consecutive days (Day 0 to Day 7). Measurements of the thickness of the right ear and observations and scoring of the appearance of the ear pinnae were performed on Day 0, Day 2, Day 4, Day 6 (all before IL-23 injection), and Day 8. At the end of the experiment (Day 8), the left ear tissues of the mice from groups G4 to G10 (3 animals per group at each time point) were collected. The right ear, which had been modeled, was excised, an 8 mm ear punch was taken along the edge of the pinna, and its weight was measured. The excised right ear tissues were fixed in formalin for histopathological examination (H&E staining).

Concurrent with model induction, corresponding drug treatment was given for administration. Except for the positive control drug (Ustekinumab), which was subcutaneously injected on Day 0 and Day 3 (once a day), control and test drugs for each dose group were orally administered by gavage twice a day at 6-hour intervals for 8 consecutive days (Day 0 to Day 7).

The pathological scoring of mouse ear tissue sections was conducted according to the criteria in Table 33.

**Table 33: Pathological scoring criteria**

| **Score** | **Degree of keratinization** | **Epidermal thickness** | **Dermal thickness** | **Inflammatory cell infiltration** |
|---|---|---|---|---|
| 0 | Relatively normal | Relatively normal | Relatively normal | Relatively normal |
| 1 | Mild | Mild | Mild | Mild |
| 2 | Moderate | Moderate | Moderate | Moderate |
| 3 | Moderate to significant | Moderate to significant | Moderate to significant | Moderate to significant |
| 4 | Significant | Significant | Significant | Significant |

### 4) Data statistics

The experimental data are represented as Mean±SD; statistical analysis was performed using IBM SPSS Statistics 21, with p<0.05 considered to indicate a statistically significant difference between the two groups. The effect of the test substance on the thickness of mouse ears in the IL-23 induced mouse pinna epidermal hyperplasia model is shown in Table 34. The effect of the test substance on the total score and AUC for IL-23 induced mouse pinna epidermal hyperplasia is shown in Table 35. The effect of the test substance on the pathological scoring of right ear tissue sections stained with H&E is shown in Table 36.

**Table 34: Effect of the test substance on ear thickness in IL-23 induced mouse pinna epidermal hyperplasia model ( x̅)**

| Group | Ear thickness (mm)/Day | | | | |
|---|---|---|---|---|---|
| | Day 0 | Day 2 | Day 4 | Day 6 | Day 8 |
| Blank control group | 0.196 | 0.211 | 0.213 | 0.213 | 0.217 |
| Model group | 0.198 | 0.297 | 0.354 | 0.446 | 0.482 |
| Ustekinumab 5 mg/kg, SC | 0.196 | 0.277* | 0.288** | 0.316** | 0.329* |
| Hydrochloride of WX-011A 30 mg/kg, BID | 0.199 | 0.274* | 0.299* | 0.297** | 0.301** |
| Hydrochloride of WX-011A 50 mg/kg, BID | 0.197 | 0.279^{*p*=0.081} | 0.291** | 0.299** | 0.300** |
| Sulfate of WX-020 30 mg/kg, BID | 0.197 | 0.283 | 0.311 | 0.351** | 0.343^{*p*=0.082} |
| Sulfate of WX-020 50 mg/kg, BID | 0.195 | 0.289 | 0.303* | 0.311** | 0.317** |

| | | | | | |
|---|---|---|---|---|---|
| ^{##}*P*<0.01 vs. blank control group **P*<0.05 vs. model group ***P*<0.01 vs. model group | | | | | |

**Table 35: Effect of the test substance on the total score and AUC for epidermal hyperplasia of mouse pinnae induced by IL-23 ( x̅)**

| Group | Total score for epidermal hyperplasia of ear pinnae/Day | | | | | AUC |
|---|---|---|---|---|---|---|
| | Day 0 | Day 2 | Day 4 | Day 6 | Day 8 | |
| Blank control group | 0.00 | 0.00 | 0.00 | 0.00 | 0.00 | 0.00 |
| Model group | 0.00 | 0.00 | 2.33^{##} | 5.83^{##} | 7.83^{##} | 24.17^{##} |
| Ustekinumab 5 mg/kg, SC | 0.00 | 0.00 | 0.33* | 0.67** | 0.83** | 2.83** |
| Hydrochloride of WX-011A | 0.00 | 0.00 | 0.33* | 0.67** | 1.17** | 3.17** |
| 30 mg/kg, BID | | | | | | |
| Hydrochloride of WX-011A 50 mg/kg, BID | 0.00 | 0.00 | 0.17** | 0.67** | 1.00* | 2.67** |
| Sulfate of WX-020 30 mg/kg, BID | 0.00 | 0.00 | 0.50* | 2.5 | 1.67* | 7.67 |
| Sulfate of WX-020 50 mg/kg, BID | 0.00 | 0.00 | 0.50* | 1.33* | 1.33** | 5.00* |

| | | | | | | |
|---|---|---|---|---|---|---|
| ^{##}*P*<0.01 vs. blank control group **P*<0.05 vs. model group ***P*<0.01 vs. model group | | | | | | |

**Table 36: Effect of the test substance on pathological scoring of right ear tissue sections stained with H&E ( x̅)**

| Group | Pathological score | | | | |
|---|---|---|---|---|---|
| | Degree of keratinization | Epidermal thickness | Dermal thickness | Inflammatory cell infiltration | Comprehensive pathological score |
| Blank control group | 0.00 | 0.17 | 0.00 | 0.33 | 0.50 |
| Model group | 1.67^{##} | 2.50^{##} | 2.17^{##} | 3.00^{##} | 9.33^{##} |
| Ustekinumab 5 mg/kg, SC | 0.50** | 1.17** | 0.67 | 1.17** | 3.50** |
| Hydrochloride of WX-011A 30 mg/kg, BID | 0.17** | 0.67** | 0.17 | 0.17** | 1.17** |
| Hydrochloride of WX-011A 50 mg/kg, BID | 0.33** | 0.50** | 0.50 | 0.50** | 1.83** |
| Sulfate of WX-020 | 0.83** | 0.83** | 1.33 | 1.17** | 4.17** |
| 30 mg/kg, BID | | | | | |
| Sulfate of WX-020 50 mg/kg, BID | 0.33** | 0.67** | 0.50 | 0.67** | 2.17** |

| | | | | | |
|---|---|---|---|---|---|
| ^{##}*P*<0.01 vs. blank control group **P*<0.05 vs. model group ***P*<0.01 vs. model group | | | | | |

**Conclusion:** Compared to the model group, the compound of the present disclosure, at various doses, exhibits a significant alleviating effect on the IL-23 induced mouse psoriasis-like condition. This was evidenced in the increase thickness of the modeled side ear, the total score of the modeled side ear, and the comprehensive pathological score of the ear tissue sections, demonstrating significant pharmacological efficacy.

## Claims

1. A compound of formula (V) or a pharmaceutically acceptable salt thereof, 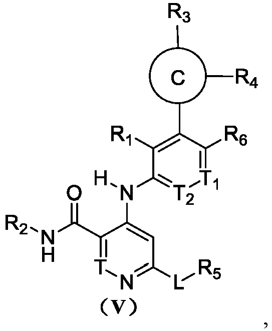 wherein
L is selected from -NH-, -NHC(=O)-, -NHC(=O)O-, and -NHC(=O)NH-;
T, T₁, and T₂ are each independently selected from N and CH, and the CH is optionally substituted by 1 halogen;
R₁ is selected from C₁₋₃ alkoxy, and the C₁₋₃ alkoxy is optionally substituted by 1, 2, or 3 Rₐ;
R₂ is selected from H and C₁₋₃ alkyl, and the C₁₋₃ alkyl is optionally substituted by 1, 2, or 3 R_{b};
ring C is selected from phenyl and 6-membered heteroaryl;
R₃ is selected from -P(=O)(C₁₋₃ alkyl)₂, -P(=O)(C₃₋₅ cycloalkyl)₂, -S(=O)ₙC₁₋₄ alkyl, - S(=O)ₙC₁₋₃ alkylamino, -S(=O)ₙ-4- to 5-membered heterocycloalkyl, -S(=O)ₙNH₂, - S(=O)(=NR)C₁₋₄ alkyl, -S(=O)(=NR)C₁₋₃ alkylamino, -S(=O)(=NR)C₃₋₅ cycloalkyl, 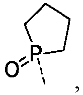 and 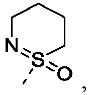 and the -P(=O)(C₁₋₃ alkyl)₂, -P(=O)(C₃₋₅ cycloalkyl)₂, -S(=O)ₙC₁₋₄ alkyl, - S(=O)ₙC₁₋₃ alkylamino, -S(=O)ₙ-4- to 5-membered heterocycloalkyl, -S(=O)ₙNH₂, - S(=O)(=NR)C₁₋₄ alkyl, -S(=O)(=NR)C₁₋₃ alkylamino, -S(=O)(=NR)C₃₋₅ cycloalkyl, 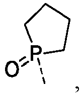 and 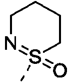 are each independently and optionally substituted by 1, 2, or 3 halogens;
R₅ is selected from C₁₋₃ alkyl, C₃₋₅ cycloalkyl, and 5- to 6-membered heteroaryl, and the C₁₋₃ alkyl, C₃₋₅ cycloalkyl, and 5- to 6-membered heteroaryl are each independently and optionally substituted by 1, 2, or 3 R_{c};
R₄ and R₆ are each independently selected from H, F, Cl, Br, and I;
alternatively, R₃, R₄ together with the carbon atom to which they are attached form 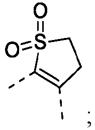
Rₐ and R_{b} are each independently selected from H, D, F, Cl, Br, and I;
R_{c} is selected from F, Cl, Br, I, and C₁₋₃ alkyl;
R is selected from H and C₁₋₃ alkyl;
n is 1 or 2;
provided that
when T is N, R₃ is not -S(=O)ₙC₁₋₄ alkyl;
"hetero" in the 4- to 5-membered heterocycloalkyl and 5- to 6-membered heteroaryl represents 1, 2, or 3 heteroatoms or heteroatom groups independently selected from -O-, -NH-, -S-, and -N-.

2. The compound or the pharmaceutically acceptable salt thereof according to claim 1, wherein R₁ is selected from OCH₃, and the OCH₃ is optionally substituted by 1, 2, or 3 Rₐ.

3. The compound or the pharmaceutically acceptable salt thereof according to claim 2, wherein R₁ is selected from OCH₃ and OCF₃.

4. The compound or the pharmaceutically acceptable salt thereof according to claim 1, wherein R₂ is selected from H and CH₃, and the CH₃ is optionally substituted by 1, 2, or 3 R_{b}.

5. The compound or the pharmaceutically acceptable salt thereof according to claim 4, wherein R₂ is selected from H, CH₃, and CD₃.

6. The compound or the pharmaceutically acceptable salt thereof according to claim 1, wherein R₃ is selected from

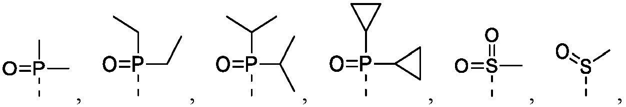

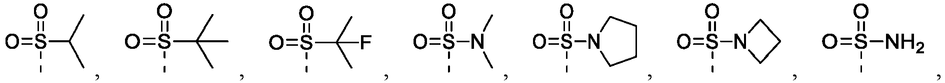

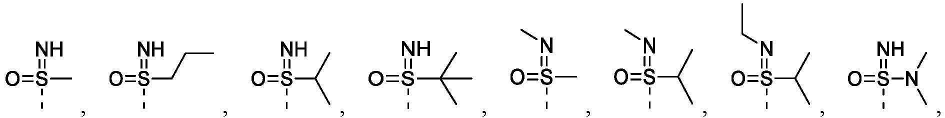

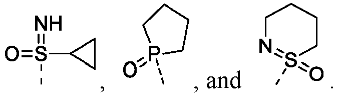

7. The compound or the pharmaceutically acceptable salt thereof according to claim 1, wherein the structural moiety 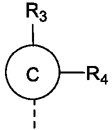 is selected from

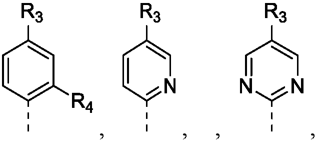

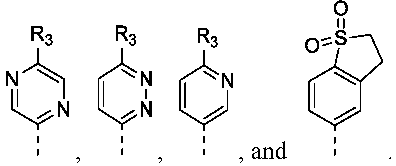

8. The compound or the pharmaceutically acceptable salt thereof according to claim 6 or 7, wherein the structural moiety 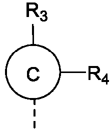 is selected from 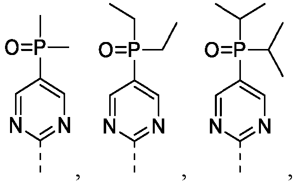 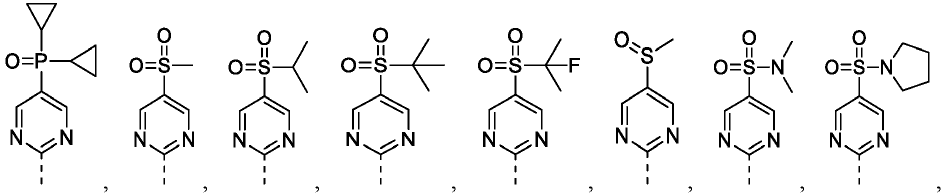 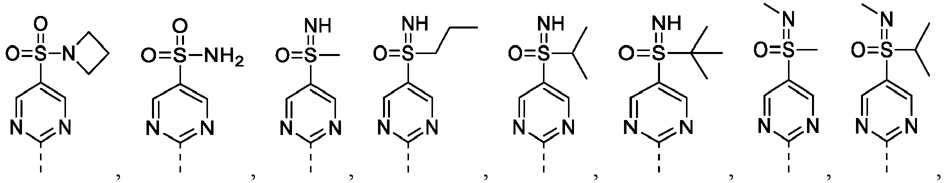 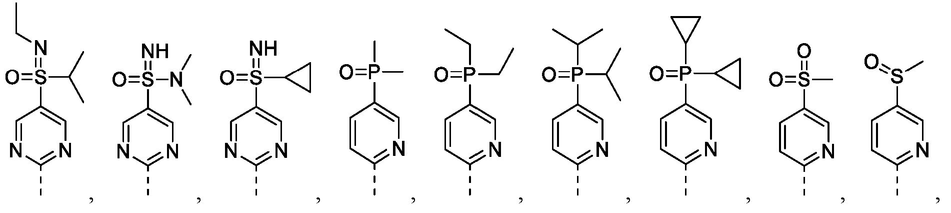 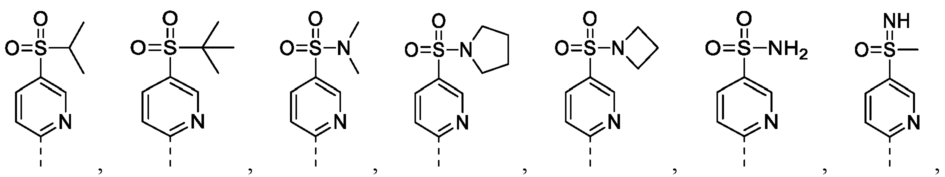 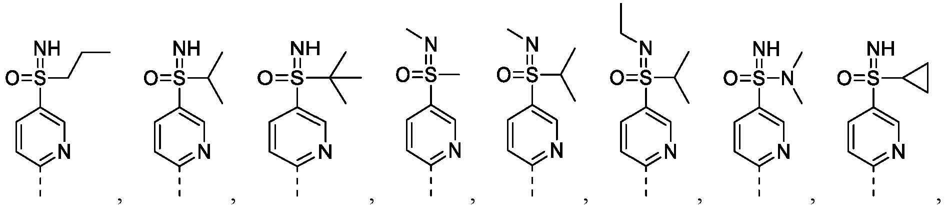 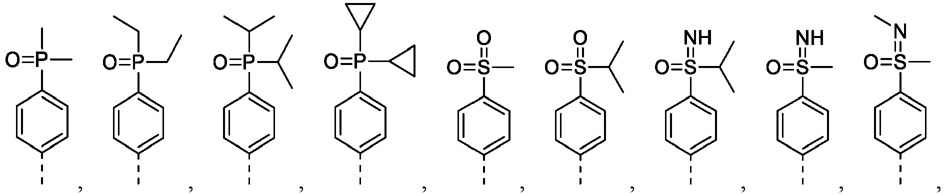 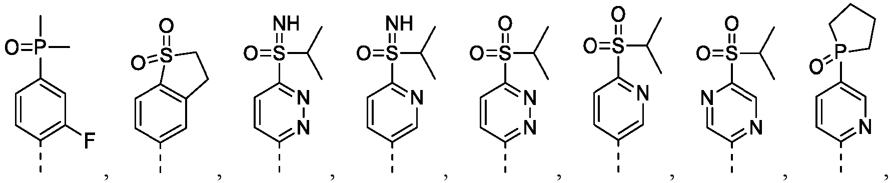 and

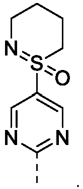

9. The compound or the pharmaceutically acceptable salt thereof according to claim 1, wherein R₅ is selected from CH₃, cyclopropyl, imidazolyl, pyrazolyl, and pyridyl, and the CH₃, cyclopropyl, imidazolyl, pyrazolyl, and pyridyl are each independently and optionally substituted by 1, 2, or 3 R_{c}.

10. The compound or the pharmaceutically acceptable salt thereof according to claim 9, wherein R₅ is selected from CH₃, 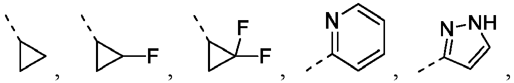 and

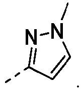

11. The compound or the pharmaceutically acceptable salt thereof according to claim 1, wherein the structural moiety 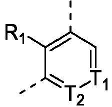 is selected from 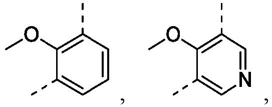 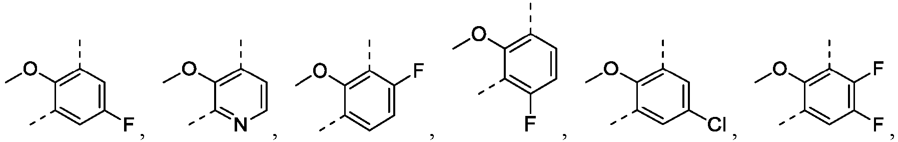 and

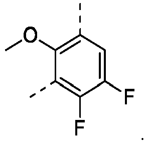

12. The compound or the pharmaceutically acceptable salt thereof according to any one of claims 1 to 11, selected from: 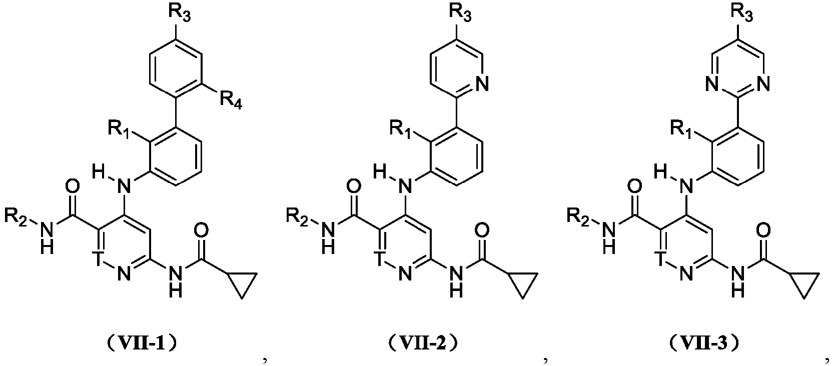 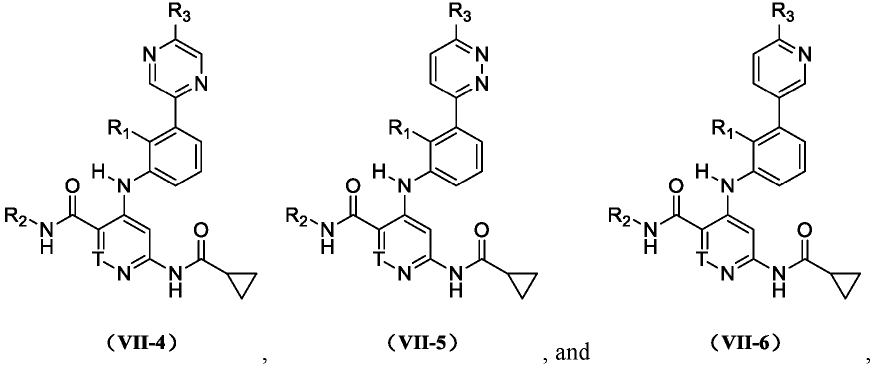 wherein
R₁, R₂, R₃, R₄, and T are as defined in any one of claims 1 to 11.

13. A compound of the following formula or a pharmaceutically acceptable salt thereof,

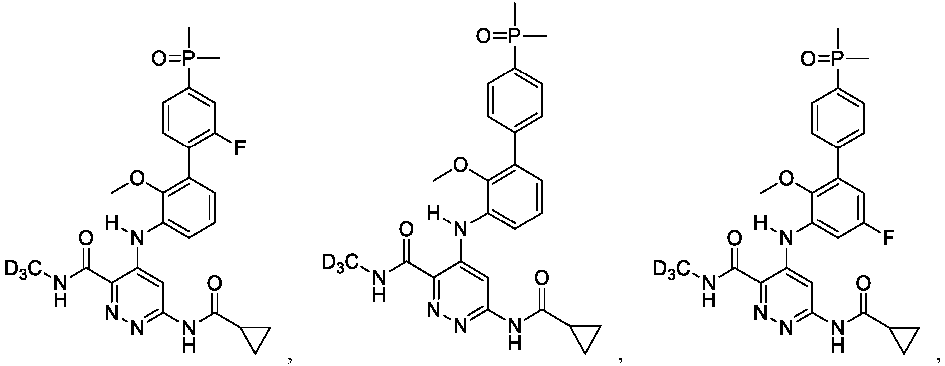

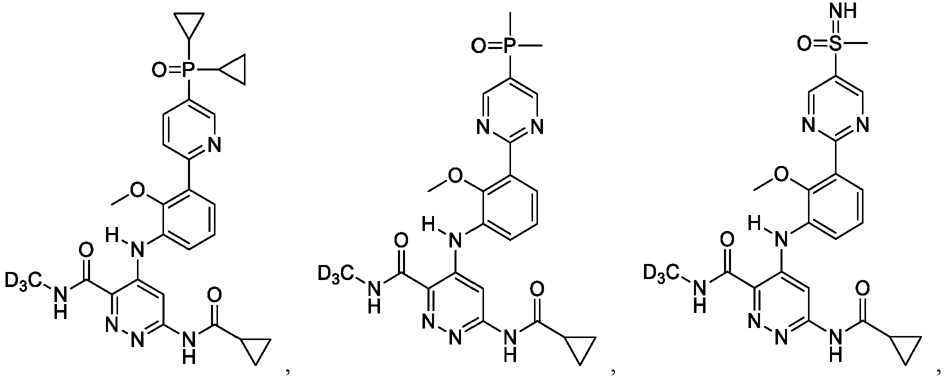

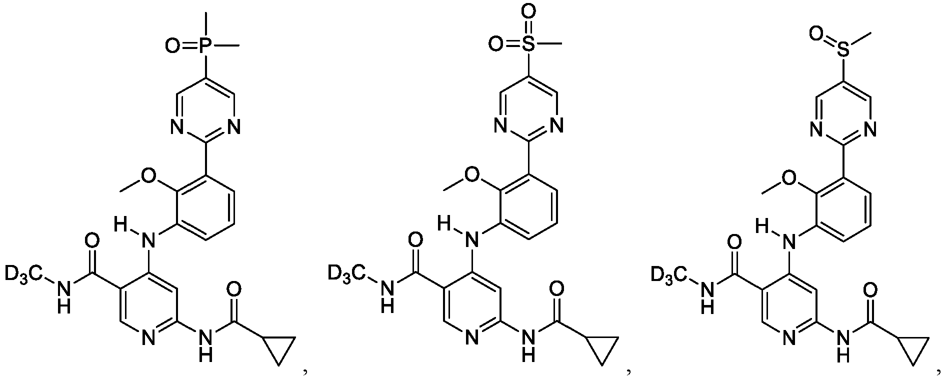

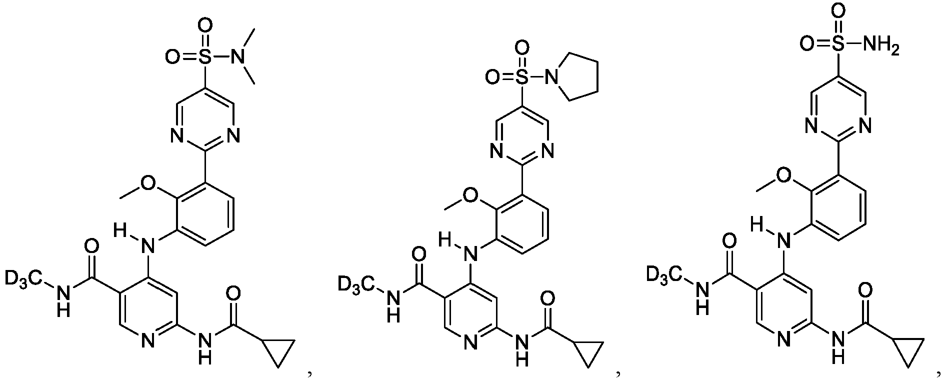

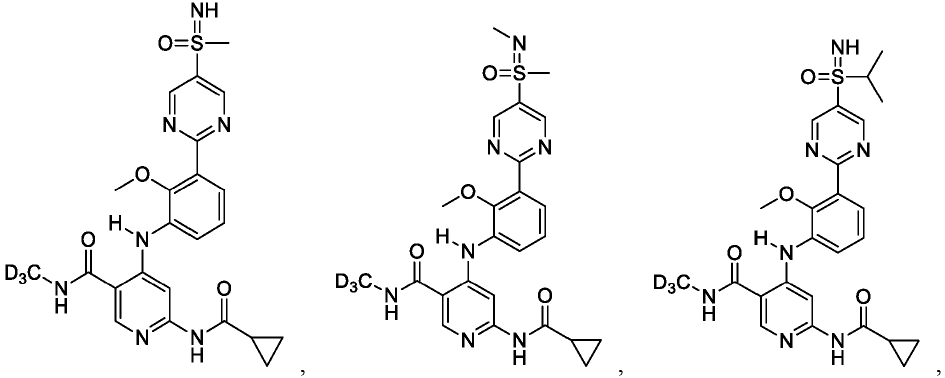

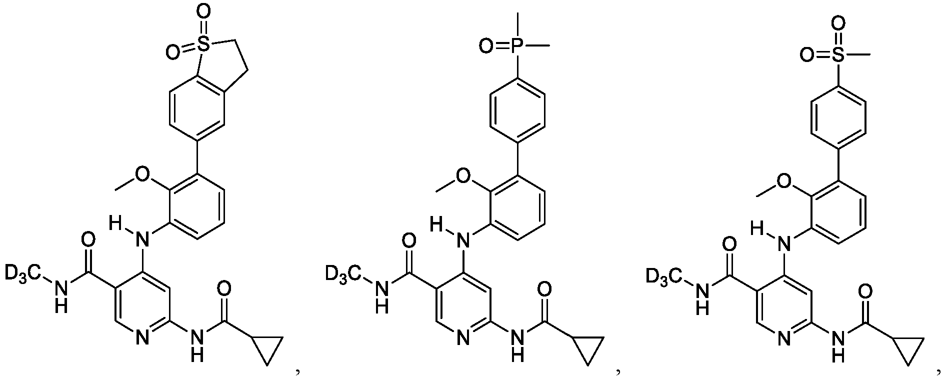

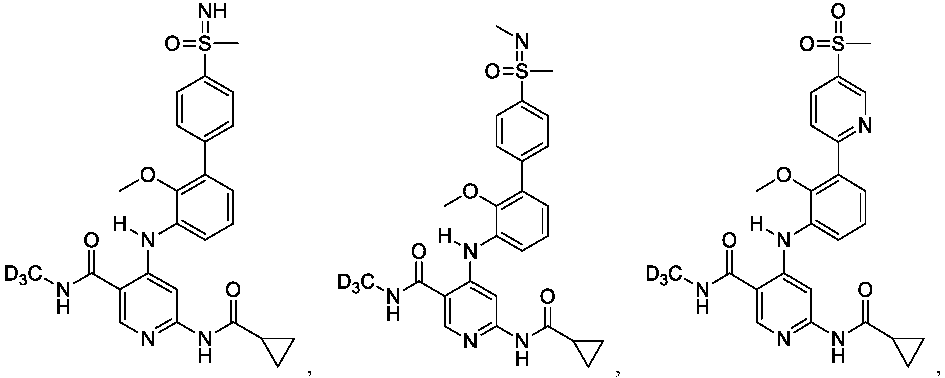

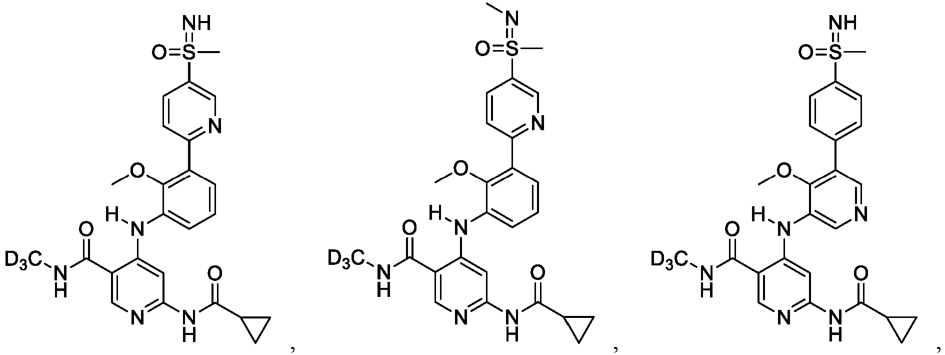

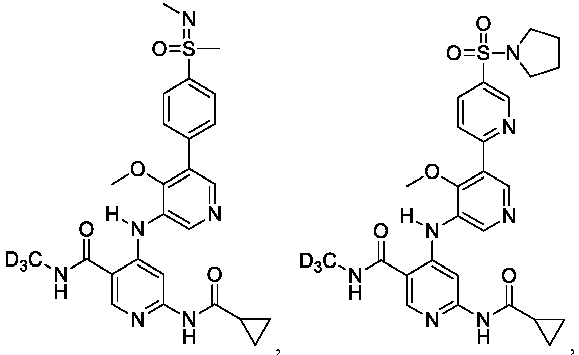

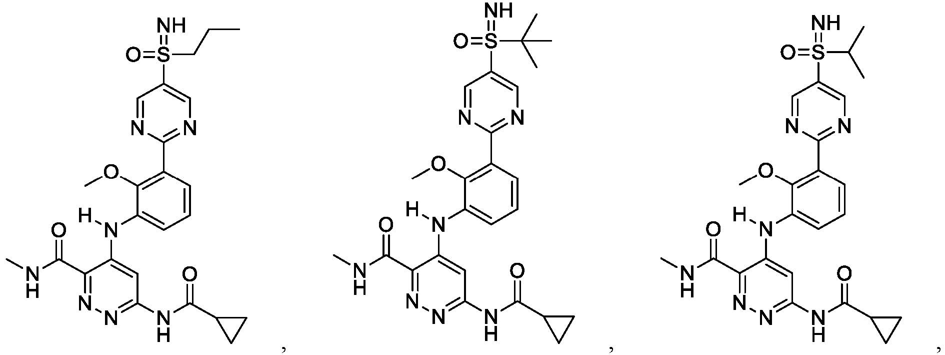

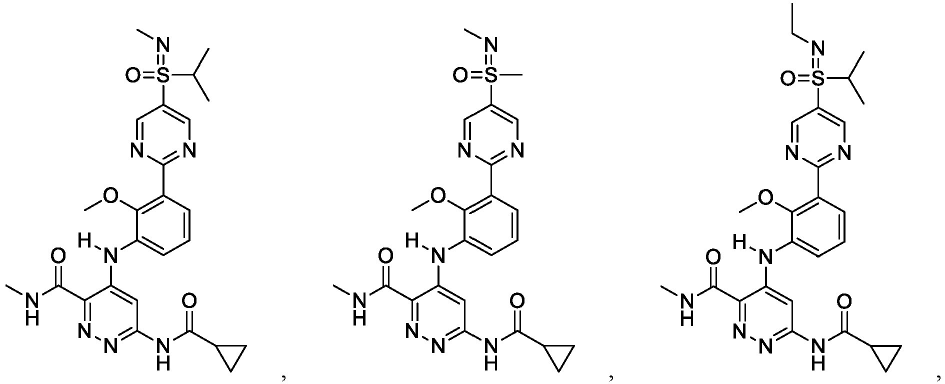

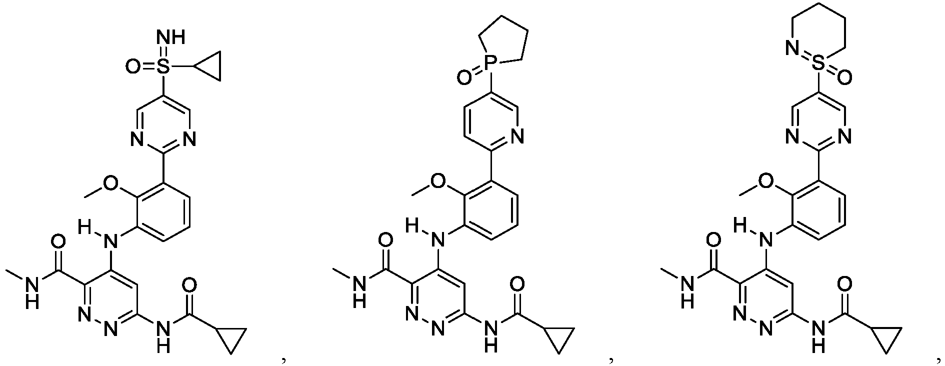

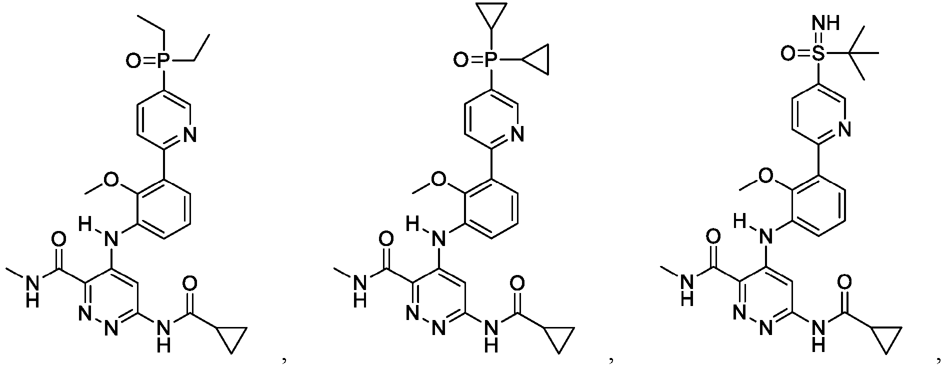

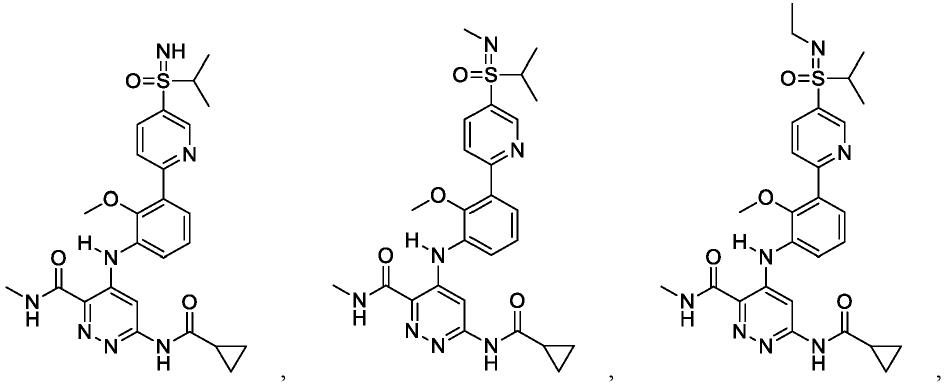

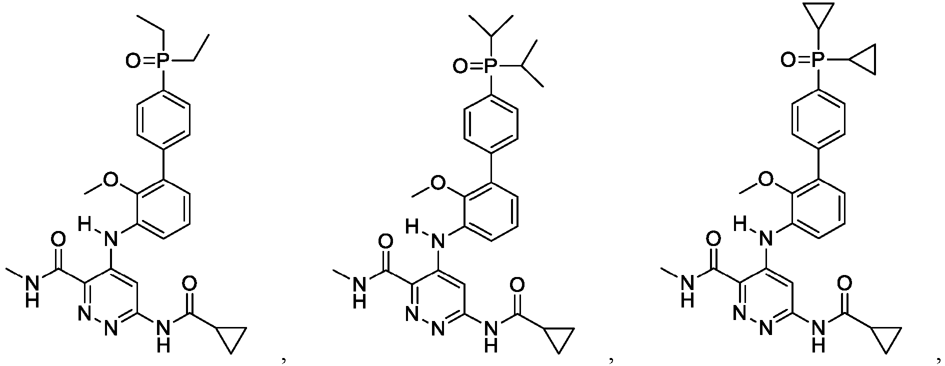

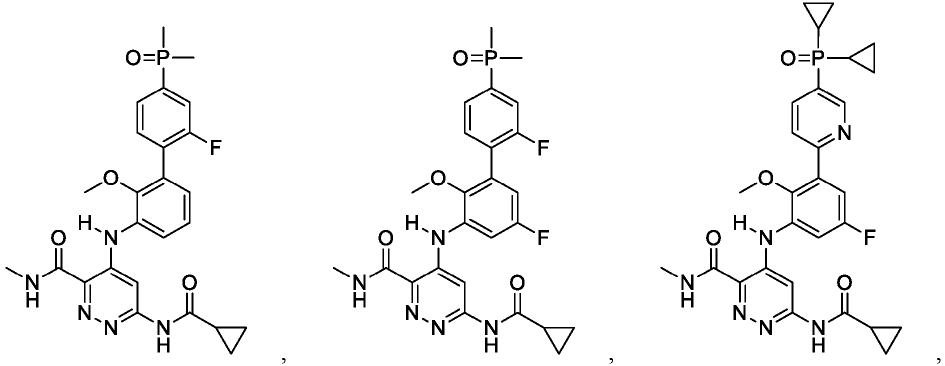

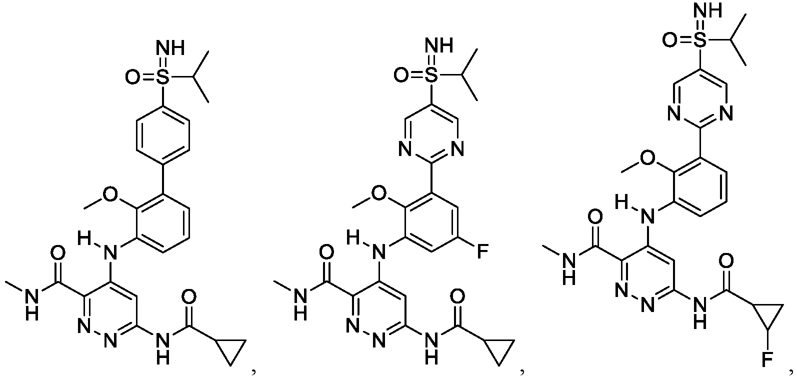

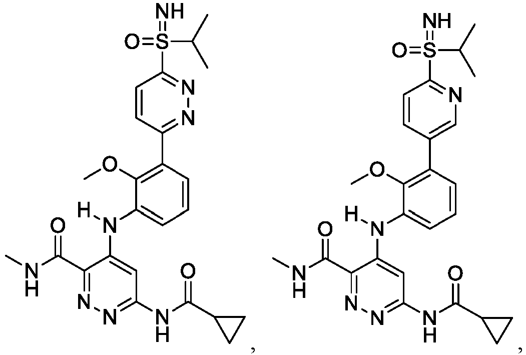

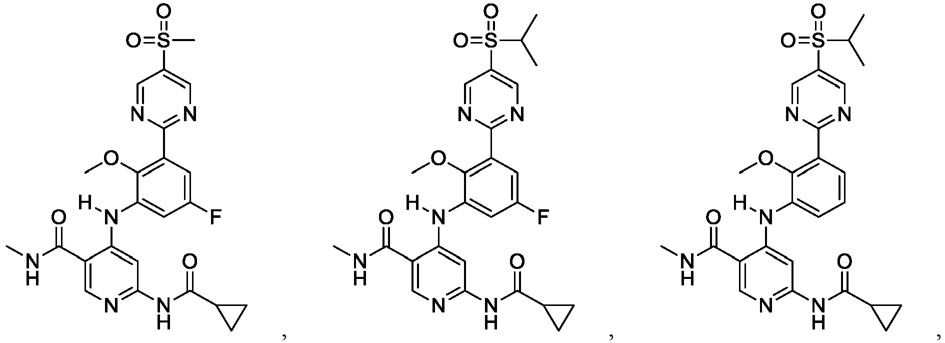

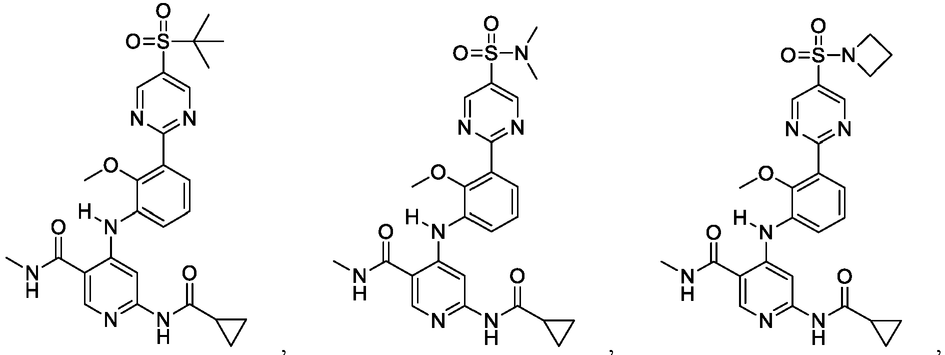

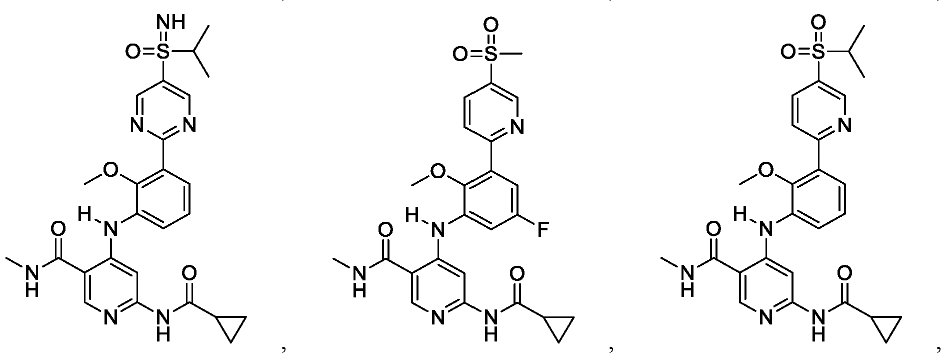

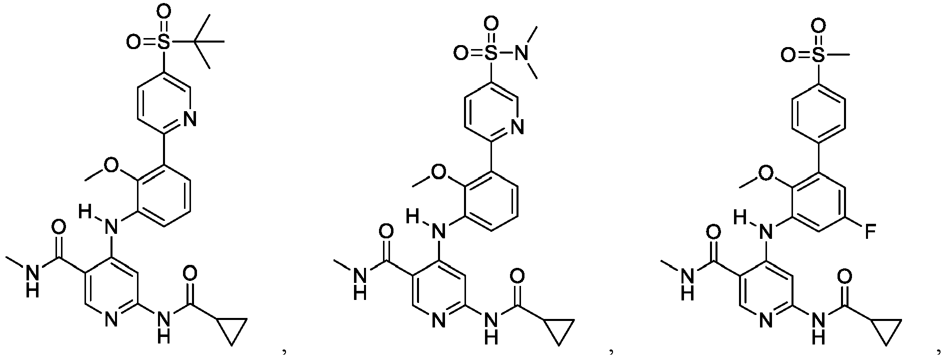

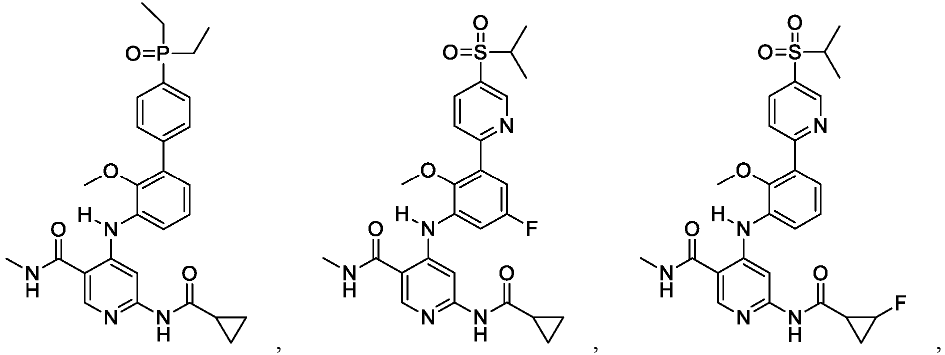

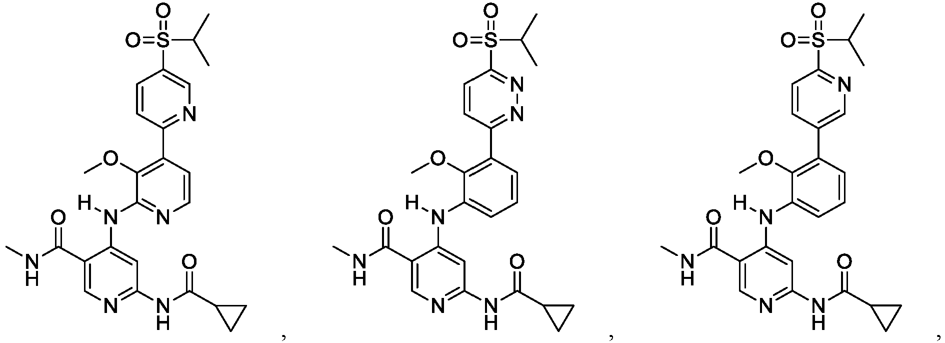

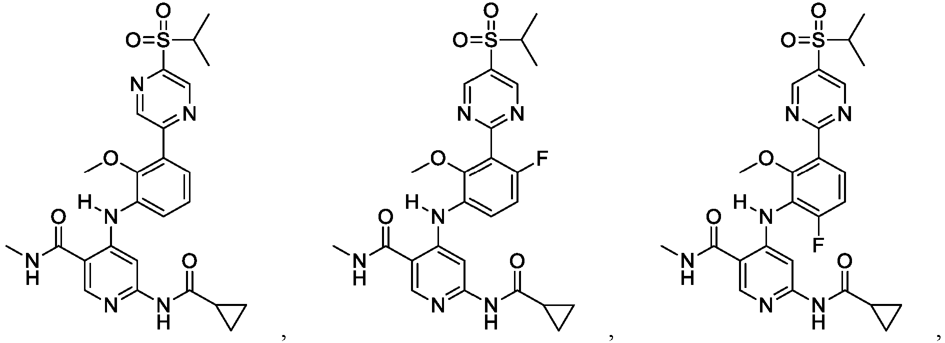

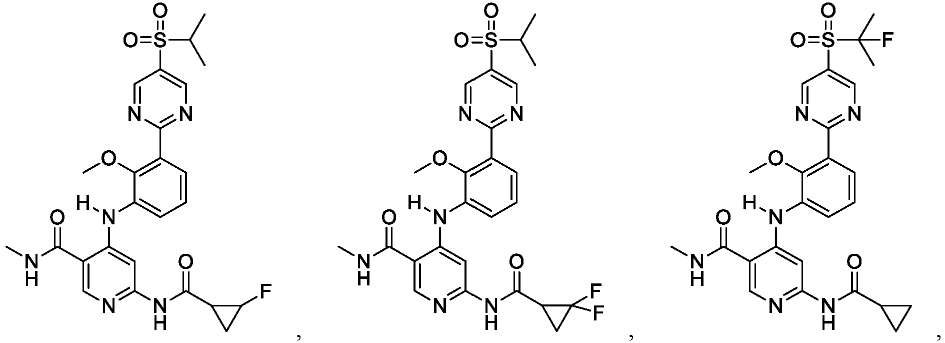

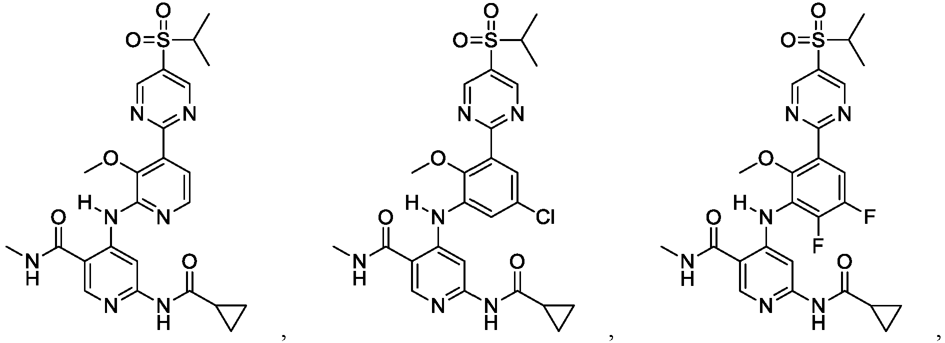

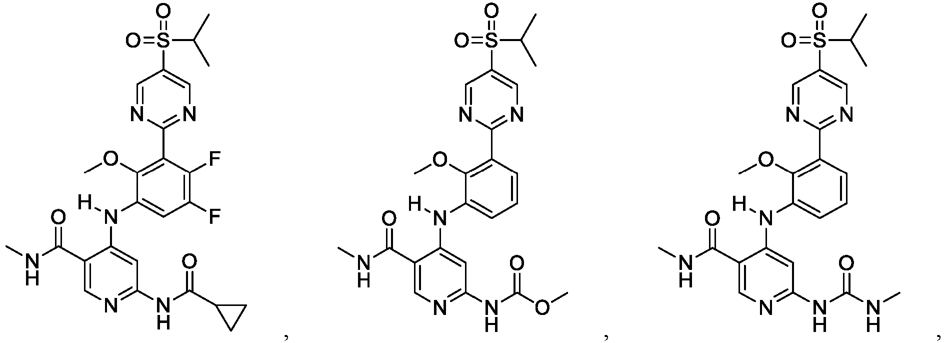

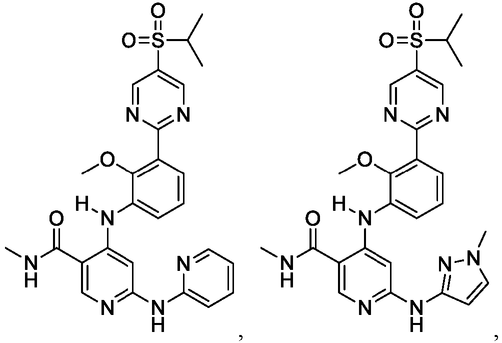

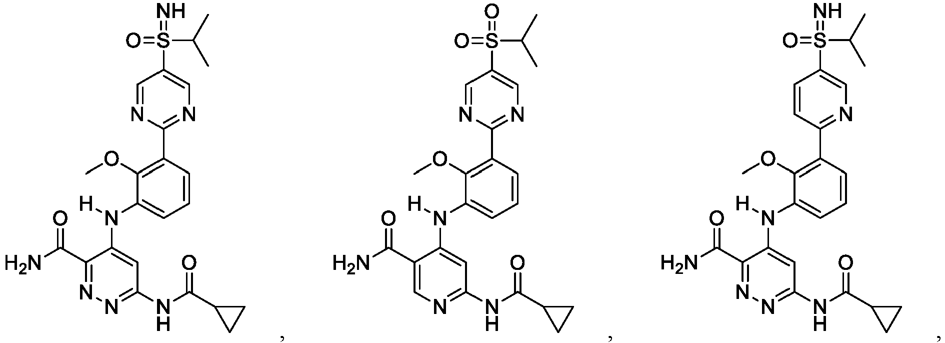

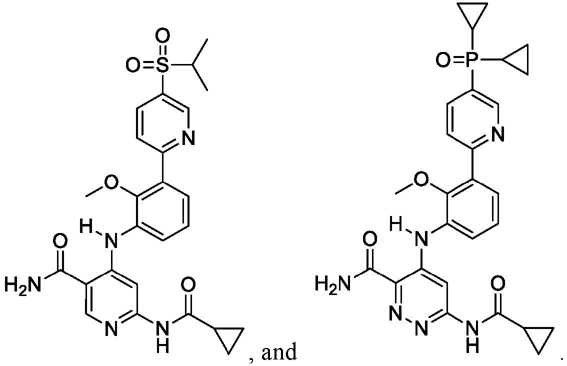

14. The compound or the pharmaceutically acceptable salt thereof according to claim 13, selected from:
